# EUROPEAN PATENT APPLICATION

(11) **EP 3 971 292 A1**
(43) Date of publication of application: **23.03.2022**
(21) Application number: 20382834.8
(22) Date of filing: 22.09.2020
(51) Int. Cl.: C12N 15/11, C12Q 1/6816, C12N 15/67

(54) **RNA MOLECULE, USE THEREOF AND A PROCESS FOR DETECTING A DISEASE BY USING THEREOF**

(71) Applicant: Moirai Biodesign, S.L., 08028 Barcelona (ES)
(72) Inventor: DOTU RODRÍGUEZ, Ivan Javier, 08028 Barcelona (ES); MINUESA DINARÈS, Gerard, 08028 Barcelona (ES); ALSINA VERDÚ, Cristina, 08028 Barcelona (ES)
(74) Representative: Ponti & Partners, S.L.P

(57) **Abstract**

The present invention relates to an RNA molecule. The present invention relates to the use of said RNA molecule in a colorimetric or fluorometric assay upon ligand binding of said RNA molecule. The present invention relates to a process for detecting in vitro a disease using said RNA molecule.

SEQUENCE LISTING

<110>  Moirai Biodesign, S.L.

<120>  RNA molecule, use thereof and a process for detecting a disease
       by using thereof

<130>  P33376EP00

<160>  86

<170>  PatentIn version 3.5

<210>  1
<211>  30
<212>  RNA
<213>  artificial

<220>
<223>  Artificial

<400>  1
guccaacagc gccagagaac aacuaugaug                                        30


<210>  2
<211>  30
<212>  RNA
<213>  artificial

<220>
<223>  Artificial

<400>  2
cgccgcuggc ccggguugga uaauauuuag                                        30


<210>  3
<211>  2799
<212>  DNA
<213>  escherichia coli

<400>  3
atgcacggtt acgatgcgcc catctacacc aacgtgacct atcccattac ggtcaatccg      60

ccgtttgttc ccacggagaa tccgacgggt tgttactcgc tcacatttaa tgttgatgaa     120

agctggctac aggaaggcca gacgcgaatt atttttgatg gcgttaactc ggcgtttcat     180

ctgtggtgca acgggcgctg ggtcggttac ggccaggaca gtcgtttgcc gtctgaattt     240

gacctgagcg catttttacg cgccggagaa aaccgcctcg cggtgatggt gctgcgctgg     300

agtgacggca gttatctgga agatcaggat atgtggcgga tgagcggcat tttccgtgac     360

gtctcgttgc tgcataaacc gactacacaa atcagcgatt tccatgttgc cactcgcttt     420

aatgatgatt tcagccgcgc tgtactggag gctgaagttc agatgtgcgg cgagttgcgt     480

gactacctac gggtaacagt ttctttatgg cagggtgaaa cgcaggtcgc cagcggcacc     540

gcgcctttcg gcggtgaaat tatcgatgag cgtggtggtt atgccgatcg cgtcacacta     600

cgtctgaacg tcgaaaaccc gaaactgtgg agcgccgaaa tcccgaatct ctatcgtgcg 660

gtggttgaac tgcacaccgc cgacggcacg ctgattgaag cagaagcctg cgatgtcggt 720

ttccgcgagg tgcggattga aaatggtctg ctgctgctga acggcaagcc gttgctgatt 780

cgaggcgtta accgtcacga gcatcatcct ctgcatggtc aggtcatgga tgagcagacg 840

atggtgcagg atatcctgct gatgaagcag aacaacttta acgccgtgcg ctgttcgcat 900

tatccgaacc atccgctgtg gtacacgctg tgcgaccgct acggcctgta tgtggtggat 960

gaagccaata ttgaaaccca cggcatggtg ccaatgaatc gtctgaccga tgatccgcgc 1020

tggctaccgg cgatgagcga acgcgtaacg cgaatggtgc agcgcgatcg taatcacccg 1080

agtgtgatca tctggtcgct ggggaatgaa tcaggccacg gcgctaatca cgacgcgctg 1140

tatcgctgga tcaaatctgt cgatccttcc cgcccggtgc agtatgaagg cggcggagcc 1200

gacaccacgg ccaccgatat tatttgcccg atgtacgcgc gcgtggatga agaccagccc 1260

ttcccggctg tgccgaaatg gtccatcaaa aaatggcttt cgctacctgg agagacgcgc 1320

ccgctgatcc tttgcgaata cgcccacgcg atgggtaaca gtcttggcgg tttcgctaaa 1380

tactggcagg cgtttcgtca gtatccccgt ttacagggcg gcttcgtctg ggactgggtg 1440

gatcagtcgc tgattaaata tgatgaaaac ggcaacccgt ggtcggctta cggcggtgat 1500

tttggcgata cgccgaacga tcgccagttc tgtatgaacg gtctggtctt tgccgaccgc 1560

acgccgcatc cagcgctgac ggaagcaaaa caccagcagc agtttttcca gttccgttta 1620

tccgggcaaa ccatcgaagt gaccagcgaa tacctgttcc gtcatagcga taacgagctc 1680

ctgcactgga tggtggcgct ggatggtaag ccgctggcaa gcggtgaagt gcctctggat 1740

gtcgctccac aaggtaaaca gttgattgaa ctgcctgaac taccgcagcc ggagagcgcc 1800

gggcaactct ggctcacagt acgcgtagtg caaccgaacg cgaccgcatg gtcagaagcc 1860

gggcacatca gcgcctggca gcagtggcgt ctggcggaaa acctcagtgt gacgctcccc 1920

gccgcgtccc acgccatccc gcatctgacc accagcgaaa tggatttttg catcgagctg 1980

ggtaataagc gttggcaatt taaccgccag tcaggctttc tttcacagat gtggattggc 2040

gataaaaaac aactgctgac gccgctgcgc gatcagttca cccgtgcacc gctggataac 2100

gacattggcg taagtgaagc gacccgcatt gaccctaacg cctgggtcga acgctggaag 2160

gcggcgggcc attaccaggc cgaagcagcg ttgttgcagt gcacggcaga tacacttgct 2220

gatgcggtgc tgattacgac cgctcacgcg tggcagcatc aggggaaaac cttatttatc 2280

agccggaaaa cctaccggat tgatggtagt ggtcaaatgg cgattaccgt tgatgttgaa 2340

gtggcgagcg atacaccgca tccggcgcgg attggcctga actgccagct ggcgcaggta 2400

gcagagcggg taaactggct cggattaggg ccgcaagaaa actatcccga ccgccttact 2460

gccgcctgtt ttgaccgctg ggatctgcca ttgtcagaca tgtatacccc gtacgtcttc 2520

ccgagcgaaa acggtctgcg ctgcgggacg cgcgaattga attatggccc acaccagtgg 2580

cgcggcgact tccagttcaa catcagccgc tacagtcaac agcaactgat ggaaaccagc 2640

catcgccatc tgctgcacgc ggaagaaggc acatggctga atatcgacgg tttccatatg 2700

gggattggtg gcgacgactc ctggagcccg tcagtatcgg cggaattcca gctgagcgcc 2760

ggtcgctacc attaccagtt ggtctggtgt caaaaataa 2799

<210> 4
<211> 160
<212> RNA
<213> artificial

<220>
<223> Artificial

<400> 4
ggccaucaua guuguucucu ggcgcuguug gacgaaaaca guccagcagc gccgggaagg 60

aggggcgagg cauaaguucc uccuucuugg cguuguugga ccuaagaagg aggaauuuau 120

gucauaucuc acacaacggg aggucguuuu acaacgucgu 160

<210> 5
<211> 160
<212> RNA
<213> artificial

<220>
<223> Artificial

<400> 5
ggccaucaua guuguucucu ggcgcuguug gacgaaaaca guccagcagc gccgggaagg 60

aggggcgaga gauaaguucc uccuucuugg cguuguugga ccuaagaagg aggaauuuau 120

gucauaucuc acacaacggg aggucguuuu acaacgucgu 160

<210> 6
<211> 160
<212> RNA
<213> Artificial Sequence

<220>
<223> Artificial

<400> 6
ggccuaaaua uuauccaacc cgggccagcg gcggauaaaa ugucguuggu ccgggagagg 60

gaggcgugaa aaaauugucu uucuuuuucg gauuaaugau acgaaaaagg aggauaucau 120

guacuacagc uucgaucccg ccgucguuuu acaacgucgu 160

<210> 7
<211> 7964
<212> DNA

<213>  Artificial Sequence

<220>
<223>  Artificial

<400>  7
gaattcgagc tcggtacccg gggatcctct agagtcgacc tgcaggcatg caagcttcga      60

tcccgccgtc gttttacaac gtcgtgactg ggaaaaccct ggcgttaccc aacttaatcg     120

ccttgcagca catccccctt tcgccagctg gcgtaatagc gaagaggccc gcaccgatcg     180

cccttcccaa cagttgcgca gcctgaatgg cgaatggcgc tttgcctggt ttccggcacc     240

agaagcggtg ccggaaagct ggctggagtg cgatcttcct gaggccgata ctgtcgtcgt     300

cccctcaaac tggcagatgc acggttacga tgcgcccatc tacaccaacg taacctatcc     360

cattacggtc aatccgccgt ttgttcccac ggagaatccg acgggttgtt actcgctcac     420

atttaatgtt gatgaaagct ggctacagga aggccagacg cgaattattt ttgatggcgt     480

taactcggcg tttcatctgt ggtgcaacgg gcgctgggtc ggttacggcc aggacagtcg     540

tttgccgtct gaatttgacc tgagcgcatt tttacgcgcc ggagaaaacc gcctcgcggt     600

gatggtgctg cgttggagtg acggcagtta tctggaagat caggatatgt ggcggatgag     660

cggcattttc cgtgacgtct cgttgctgca taaaccgact acacaaatca gcgatttcca     720

tgttgccact cgctttaatg atgatttcag ccgcgctgta ctggaggctg aagttcagat     780

gtgcggcgag ttgcgtgact acctacgggt aacagtttct ttatggcagg gtgaaacgca     840

ggtcgccagc ggcaccgcgc ctttcggcgg tgaaattatc gatgagcgtg gtggttatgc     900

cgatcgcgtc acactacgtc tgaacgtcga aaacccgaaa ctgtggagcg ccgaaatccc     960

gaatctctat cgtgcggtgg ttgaactgca caccgccgac ggcacgctga ttgaagcaga    1020

agcctgcgat gtcggtttcc gcgaggtgcg gattgaaaat ggtctgctgc tgctgaacgg    1080

caagccgttg ctgattcgag gcgttaaccg tcacgagcat catcctctgc atggtcaggt    1140

catggatgag cagacgatgg tgcaggatat cctgctgatg aagcagaaca actttaacgc    1200

cgtgcgctgt tcgcattatc cgaaccatcc gctgtggtac acgctgtgcg accgctacgg    1260

cctgtatgtg gtggatgaag ccaatattga aacccacggc atggtgccaa tgaatcgtct    1320

gaccgatgat ccgcgctggc taccggcgat gagcgaacgc gtaacgcgaa tggtgcagcg    1380

cgatcgtaat cacccgagtg tgatcatctg gtcgctgggg aatgaatcag gccacggcgc    1440

taatcacgac gcgctgtatc gctggatcaa atctgtcgat ccttcccgcc cggtgcagta    1500

tgaaggcggc ggagccgaca ccacggccac cgatattatt tgcccgatgt acgcgcgcgt    1560

ggatgaagac cagcccttcc cggctgtgcc gaaatggtcc atcaaaaaat ggctttcgct    1620

acctggagag acgcgcccgc tgatcctttg cgaatacgcc cacgcgatgg gtaacagtct    1680

tggcggtttc gctaaatact ggcaggcgtt tcgtcagtat ccccgtttac agggcggctt    1740

cgtctgggac tgggtggatc agtcgctgat taaatatgat gaaaacggca acccgtggtc 1800

ggcttacggc ggtgattttg gcgatacgcc gaacgatcgc cagttctgta tgaacggtct 1860

ggtctttgcc gaccgcacgc cgcatccagc gctgacggaa gcaaaacacc agcagcagtt 1920

tttccagttc cgtttatccg ggcaaaccat cgaagtgacc agcgaatacc tgttccgtca 1980

tagcgataac gagctcctgc actggatggt ggcgctggat ggtaagccgc tggcaagcgg 2040

tgaagtgcct ctggatgtcg ctccacaagg taaacagttg attgaactgc ctgaactacc 2100

gcagccggag agcgccgggc aactctggct cacagtacgc gtagtgcaac cgaacgcgac 2160

cgcatggtca gaagccgggc acatcagcgc ctggcagcag tggcgtctgg cggaaaacct 2220

cagtgtgacg ctccccgccg cgtcccacgc catcccgcat ctgaccacca gcgaaatgga 2280

tttttgcatc gagctgggta ataagcgttg gcaatttaac cgccagtcag gctttctttc 2340

acagatgtgg attggcgata aaaaacaact gctgacgccg ctgcgcgatc agttcacccg 2400

tgcaccgctg gataacgaca ttggcgtaag tgaagcgacc cgcattgacc ctaacgcctg 2460

ggtcgaacgc tggaaggcgg cgggccatta ccaggccgaa gcagcgttgt tgcagtgcac 2520

ggcagataca cttgctgatg cggtgctgat tacgaccgct cacgcgtggc agcatcaggg 2580

gaaaacctta tttatcagcc ggaaaaccta ccggattgat ggtagtggtc aaatggcgat 2640

taccgttgat gttgaagtgg cgagcgatac accgcatccg gcgcggattg gcctgaactg 2700

ccagctggcg caggtagcag agcgggtaaa ctggctcgga ttagggccgc aagaaaacta 2760

tcccgaccgc cttactgccg cctgttttga ccgctgggat ctgccattgt cagacatgta 2820

taccccgtac gtcttcccga gcgaaaacgg tctgcgctgc gggacgcgcg aattgaatta 2880

tggcccacac cagtggcgcg gcgacttcca gttcaacatc agccgctaca gtcaacagca 2940

actgatggaa accagccatc gccatctgct gcacgcggaa gaaggcacat ggctgaatat 3000

cgacggtttc catatgggga ttggtggcga cgactcctgg agcccgtcag tatcggcgga 3060

attacagctg agcgccggtc gctaccatta ccagttggtc tggtgtcaaa aataataata 3120

accgggcagg ccatgtctgc ccgtatttcg cgtaaggaaa tccattatgt actatttcgc 3180

ctgatgcggt attttctcct tacgcatctg tgcggtattt cacaccgcat agggtaataa 3240

ctgatataat taaattgaag ctctaatttg tgagtttagt atacatgcat ttacttataa 3300

tacagttttt tagttttgct ggccgcatct tctcaaatat gcttcccagc ctgcttttct 3360

gtaacgttca ccctctacct tagcatccct tccctttgca aatagtcctc ttccaacaat 3420

aataatgtca gatcctgtag agaccacatc atccacggtt ctatactgtt gacccaatgc 3480

gtctcccttg tcatctaaac ccacaccggg tgtcataatc aaccaatcgt aaccttcatc 3540

tcttccaccc atgtctcttt gagcaataaa gccgataaca aaatctttgt cgctcttcgc 3600

aatgtcaaca gtacccttag tatattctcc agtagatagg gagcccttgc atgacaattc 3660

tgctaacatc aaaaggcctc taggttcctt tgttacttct tctgccgcct gcttcaaacc 3720

gctaacaata cctgggccca ccacaccgtg tgcattcgta atgtctgccc attctgctat 3780

tctgtataca cccgcagagt actgcaattt gactgtatta ccaatgtcag caaattttct 3840

gtcttcgaag agtaaaaaat tgtacttggc ggataatgcc tttagcggct taactgtgcc 3900

ctccatggaa aaatcagtca agatatccac atgtgttttt agtaaacaaa ttttgggacc 3960

taatgcttca actaactcca gtaattcctt ggtggtacga acatccaatg aagcacacaa 4020

gtttgtttgc ttttcgtgca tgatattaaa tagcttggca gcaacaggac taggatgagt 4080

agcagcacgt tccttatatg tagctttcga catgatttat cttcgttttt tgttctgtgc 4140

agttgggtta agaatactgg gcaatttcat gtttcttcaa cactacatat gcgtatatat 4200

accaatctaa gtctgtgctc cttccttcgt tcttccttct gttcggagat taccgaatca 4260

aaaaaatttc aaagaaaccg aaatcaaaaa aaagaataaa aaaaaaatga tgaattgaat 4320

tgaaaagcta cttgttaccc atcattgaat tttgaacatc cgaacctggg agttttccct 4380

gaaacagata gtatatttga acctgtataa taatatatag tctagcgctt tacggaagac 4440

aatgtatgta tttcggttcc tggagaaact attgcatcta ttgcataggt aatcttgcac 4500

gtcgcatccc cggttcattt tctgcgtttc catcttgcac ttcaatagca tatctttgtt 4560

aacgaagcat ctgtgcttca ttttgtagaa caaaaatgca acgcgagagc gctaattttt 4620

caaacaaaga atctgagctg catttttaca gaacagaaat gcaacgcgaa agcgctattt 4680

taccaacgaa gaatctgtgc ttcatttttg taaaacaaaa atgcaacgcg agagcgctaa 4740

tttttcaaac aaagaatctg agctgcattt ttacagaaca gaaatgcaac gcgagagcgc 4800

tattttacca acaaagaatc tatacttctt ttttgttcta caaaaatgca tcccgagagc 4860

gctatttttc taacaaagca tcttagatta ctttttttct cctttgtgcg ctctataatg 4920

cagtctcttg ataacttttt gcactgtagg tccgttaagg ttagaagaag gctactttgg 4980

tgtctatttt ctcttccata aaaaaagcct gactccactt cccgcgttta ctgattacta 5040

gcgaagctgc gggtgcattt tttcaagata aaggcatccc cgattatatt ctataccgat 5100

gtggattgcg catactttgt gaacagaaag tgatagcgtt gatgattctt cattggtcag 5160

aaaattatga acggtttctt ctattttgtc tctatatact acgtatagga aatgtttaca 5220

ttttcgtatt gttttcgatt cactctatga atagttctta ctacaatttt tttgtctaaa 5280

gagtaatact agagataaac ataaaaaatg tagaggtcga gtttagatgc aagttcaagg 5340

agcgaaaggt ggatgggtag gttatatagg gatatagcac agagatatat agcaaagaga 5400

tacttttgag caatgtttgt ggaagcggta ttcgcaatat tttagtagct cgttacagtc 5460

cggtgcgttt ttggtttttt gaaagtgcgt cttcagagcg cttttggttt tcaaaagcgc 5520

tctgaagttc ctatactttc tagctagaga ataggaactt cggaatagga acttcaaagc 5580

gtttccgaaa acgagcgctt ccgaaaatgc aacgcgagct gcgcacatac agctcactgt 5640

tcacgtcgca cctatatctg cgtgttgcct gtatatatat atacatgaga agaacggcat 5700

agtgcgtgtt tatgcttaaa tgcgttatgg tgcactctca gtacaatctg ctctgatgcc 5760

gcatagttaa gccagccccg acacccgcca acacccgctg acgcgccctg acgggcttgt 5820

ctgctcccgg catccgctta cagacaagct gtgaccgtct ccgggagctg catgtgtcag 5880

aggttttcac cgtcatcacc gaaacgcgcg agacgaaagg gcctcgtgat acgcctattt 5940

ttataggtta atgtcatgat aataatggtt tcttagacgt caggtggcac ttttcgggga 6000

aatgtgcgcg gaacccctat ttgtttattt ttctaaatac attcaaatat gtatccgctc 6060

atgagacaat aaccctgata aatgcttcaa taatattgaa aaaggaagag tatgagtatt 6120

caacatttcc gtgtcgccct tattcccttt tttgcggcat tttgccttcc tgtttttgct 6180

cacccagaaa cgctggtgaa agtaaaagat gctgaagatc agttgggtgc acgagtgggt 6240

tacatcgaac tggatctcaa cagcggtaag atccttgaga gttttcgccc cgaagaacgt 6300

tttccaatga tgagcacttt taaagttctg ctatgtggcg cggtattatc ccgtattgac 6360

gccgggcaag agcaactcgg tcgccgcata cactattctc agaatgactt ggttgagtac 6420

tcaccagtca cagaaaagca tcttacggat ggcatgacag taagagaatt atgcagtgct 6480

gccataacca tgagtgataa cactgcggcc aacttacttc tgacaacgat cggaggaccg 6540

aaggagctaa ccgctttttt gcacaacatg ggggatcatg taactcgcct tgatcgttgg 6600

gaaccggagc tgaatgaagc cataccaaac gacgagcgtg acaccacgat gcctgtagca 6660

atggcaacaa cgttgcgcaa actattaact ggcgaactac ttactctagc ttcccggcaa 6720

caattaatag actggatgga ggcggataaa gttgcaggac cacttctgcg ctcggccctt 6780

ccggctggct ggtttattgc tgataaatct ggagccggtg agcgtgggtc tcgcggtatc 6840

attgcagcac tggggccaga tggtaagccc tcccgtatcg tagttatcta cacgacgggg 6900

agtcaggcaa ctatggatga acgaaataga cagatcgctg agataggtgc ctcactgatt 6960

aagcattggt aactgtcaga ccaagtttac tcatatatac tttagattga tttaaaactt 7020

catttttaat ttaaaaggat ctaggtgaag atcctttttg ataatctcat gaccaaaatc 7080

ccttaacgtg agttttcgtt ccactgagcg tcagaccccg tagaaaagat caaaggatct 7140

tcttgagatc ctttttttct gcgcgtaatc tgctgcttgc aaacaaaaaa accaccgcta 7200

ccagcggtgg tttgtttgcc ggatcaagag ctaccaactc tttttccgaa ggtaactggc 7260

ttcagcagag cgcagatacc aaatactgtc cttctagtgt agccgtagtt aggccaccac 7320

ttcaagaact ctgtagcacc gcctacatac ctcgctctgc taatcctgtt accagtggct 7380

gctgccagtg gcgataagtc gtgtcttacc gggttggact caagacgata gttaccggat 7440

aaggcgcagc ggtcgggctg aacggggggt tcgtgcacac agcccagctt ggagcgaacg 7500

acctacaccg aactgagata cctacagcgt gagcattgag aaagcgccac gcttcccgaa 7560

gggagaaagg cggacaggta tccggtaagc ggcagggtcg gaacaggaga gcgcacgagg 7620

gagcttccag ggggaaacgc ctggtatctt tatagtcctg tcgggtttcg ccacctctga 7680

cttgagcgtc gatttttgtg atgctcgtca ggggggcgga gcctatggaa aaacgccagc 7740

aacgcggcct ttttacggtt cctggccttt tgctggcctt ttgctcacat gttctttcct 7800

gcgttatccc ctgattctgt ggataaccgt attaccgcct ttgagtgagc tgataccgct 7860

cgccgcagcc gaacgaccga gcgcagcgag tcagtgagcg aggaagcgga agagcgccca 7920

atacgcaaac cgcctctccc cgcgcgttgg ccgattcatt cccg 7964

<210> 8
<211> 56
<212> DNA
<213> artificial

<220>
<223> Artificial

<400> 8
aaacccctcc gtttagagag gggttatgct agttattttt gacaccagac caactg 56

<210> 9
<211> 187
<212> DNA
<213> artificial

<220>
<223> Artificial

<400> 9
tcataagctt taatacgact cactataggc catcatagtt gttctctggc gctgttggac 60

gaaaacagtc cagcagcgcc gggaaggagg ggcgaggcat aagttcctcc ttcttggcgt 120

tgttggacct aagaaggagg aatttatgtc atatctcaca caacggaggg tcgttttaca 180

acgtcgt 187

<210> 10
<211> 187
<212> DNA
<213> artificial

<220>
<223> Artificial

<400> 10
tcataagctt taatacgact cactataggc catcatagtt gttctctggc gctgttggac 60

gaaaacagtc cagcagcgcc gggaaggagg ggcgagagat aagttcctcc ttcttggcgt 120

tgttggacct aagaaggagg aatttatgtc atatctcaca caacgggagg tcgttttaca 180

acgtcgt 187

<210> 11
<211> 187
<212> DNA
<213> artificial

<220>
<223> Artificial

<400> 11
tcataagctt taatacgact cactataggc ctaaatatta tccaacccgg gccagcggcg 60

gataaaatgt cgttggtccg ggagagggag gcgtgaaaaa attgtctttc tttttcggat 120

taatgatacg aaaaaggagg atatcatgta ctacagcttc gatcccgccg tcgttttaca 180

acgtcgt 187

<210> 12
<211> 3249
<212> DNA
<213> artificial

<220>
<223> Artificial

<400> 12
tcataagctt taatacgact cactataggc catcatagtt gttctctggc gctgttggac 60

gaaaacagtc cagcagcgcc gggaaggagg ggcgaggcat aagttcctcc ttcttggcgt 120

tgttggacct aagaaggagg aatttatgtc atatctcaca caacgggagg tcgttttaca 180

acgtcgtgac tgggaaaacc ctggcgttac ccaacttaat cgccttgcag cacatccccc 240

tttcgccagc tggcgtaata gcgaagaggc ccgcaccgat cgcccttccc aacagttgcg 300

cagcctgaat ggcgaatggc gctttgcctg gtttccggca ccagaagcgg tgccggaaag 360

ctggctggag tgcgatcttc ctgaggccga tactgtcgtc gtcccctcaa actggcagat 420

gcacggttac gatgcgccca tctacaccaa cgtgacctat cccattacgg tcaatccgcc 480

gtttgttccc acggagaatc cgacgggttg ttactcgctc acatttaatg ttgatgaaag 540

ctggctacag gaaggccaga cgcgaattat ttttgatggc gttaactcgg cgtttcatct 600

gtggtgcaac gggcgctggg tcggttacgg ccaggacagt cgtttgccgt ctgaatttga 660

cctgagcgca tttttacgcg ccggagaaaa ccgcctcgcg gtgatggtgc tgcgctggag 720

tgacggcagt tatctggaag atcaggatat gtggcggatg agcggcattt tccgtgacgt 780

ctcgttgctg cataaaccga ctacacaaat cagcgatttc catgttgcca ctcgctttaa 840

tgatgatttc agccgcgctg tactggaggc tgaagttcag atgtgcggcg agttgcgtga 900

ctacctacgg gtaacagttt ctttatggca gggtgaaacg caggtcgcca gcggcaccgc 960

gcctttcggc ggtgaaatta tcgatgagcg tggtggttat gccgatcgcg tcacactacg 1020

tctgaacgtc gaaaacccga aactgtggag cgccgaaatc ccgaatctct atcgtgcggt 1080

ggttgaactg cacaccgccg acggcacgct gattgaagca gaagcctgcg atgtcggttt 1140

ccgcgaggtg cggattgaaa atggtctgct gctgctgaac ggcaagccgt tgctgattcg 1200

aggcgttaac cgtcacgagc atcatcctct gcatggtcag gtcatggatg agcagacgat 1260

ggtgcaggat atcctgctga tgaagcagaa caactttaac gccgtgcgct gttcgcatta 1320

tccgaaccat ccgctgtggt acacgctgtg cgaccgctac ggcctgtatg tggtggatga 1380

agccaatatt gaaacccacg gcatggtgcc aatgaatcgt ctgaccgatg atccgcgctg 1440

gctaccggcg atgagcgaac gcgtaacgcg aatggtgcag cgcgatcgta atcacccgag 1500

tgtgatcatc tggtcgctgg ggaatgaatc aggccacggc gctaatcacg acgcgctgta 1560

tcgctggatc aaatctgtcg atccttcccg cccggtgcag tatgaaggcg gcggagccga 1620

caccacggcc accgatatta tttgcccgat gtacgcgcgc gtggatgaag accagccctt 1680

cccggctgtg ccgaaatggt ccatcaaaaa atggctttcg ctacctggag agacgcgccc 1740

gctgatcctt tgcgaatacg cccacgcgat gggtaacagt cttggcggtt tcgctaaata 1800

ctggcaggcg tttcgtcagt atccccgttt acagggcggc ttcgtctggg actgggtgga 1860

tcagtcgctg attaaatatg atgaaaacgg caacccgtgg tcggcttacg gcggtgattt 1920

tggcgatacg ccgaacgatc gccagttctg tatgaacggt ctggtctttg ccgaccgcac 1980

gccgcatcca gcgctgacgg aagcaaaaca ccagcagcag tttttccagt tccgtttatc 2040

cgggcaaacc atcgaagtga ccagcgaata cctgttccgt catagcgata acgagctcct 2100

gcactggatg gtggcgctgg atggtaagcc gctggcaagc ggtgaagtgc ctctggatgt 2160

cgctccacaa ggtaaacagt tgattgaact gcctgaacta ccgcagccgg agagcgccgg 2220

gcaactctgg ctcacagtac gcgtagtgca accgaacgcg accgcatggt cagaagccgg 2280

gcacatcagc gcctggcagc agtggcgtct ggcggaaaac ctcagtgtga cgctccccgc 2340

cgcgtcccac gccatcccgc atctgaccac cagcgaaatg gatttttgca tcgagctggg 2400

taataagcgt tggcaattta accgccagtc aggctttctt tcacagatgt ggattggcga 2460

taaaaaacaa ctgctgacgc cgctgcgcga tcagttcacc cgtgcaccgc tggataacga 2520

cattggcgta agtgaagcga cccgcattga ccctaacgcc tgggtcgaac gctggaaggc 2580

ggcgggccat taccaggccg aagcagcgtt gttgcagtgc acggcagata cacttgctga 2640

tgcggtgctg attacgaccg ctcacgcgtg gcagcatcag gggaaaacct tatttatcag 2700

ccggaaaacc taccggattg atggtagtgg tcaaatggcg attaccgttg atgttgaagt 2760

ggcgagcgat acaccgcatc cggcgcggat tggcctgaac tgccagctgg cgcaggtagc 2820

agagcgggta aactggctcg gattagggcc gcaagaaaac tatcccgacc gccttactgc 2880

cgcctgtttt gaccgctggg atctgccatt gtcagacatg tataccccgt acgtcttccc 2940

gagcgaaaac ggtctgcgct gcgggacgcg cgaattgaat tatggcccac accagtggcg 3000

cggcgacttc cagttcaaca tcagccgcta cagtcaacag caactgatgg aaaccagcca 3060

tcgccatctg ctgcacgcgg aagaaggcac atggctgaat atcgacggtt tccatatggg 3120

gattggtggc gacgactcct ggagcccgtc agtatcggcg gaattccagc tgagcgccgg 3180

tcgctaccat taccagttgg tctggtgtca aaaataacta gcataacccc tctctaaacg 3240

gaggggttt 3249

<210> 13
<211> 3249
<212> DNA
<213> artificial

<220>
<223> Artificial

<400> 13
tcataagctt taatacgact cactataggc catcatagtt gttctctggc gctgttggac 60

gaaaacagtc cagcagcgcc gggaaggagg ggcgagagat aagttcctcc ttcttggcgt 120

tgttggacct aagaaggagg aatttatgtc atatctcaca caacgggagg tcgttttaca 180

acgtcgtgac tgggaaaacc ctggcgttac ccaacttaat cgccttgcag cacatccccc 240

tttcgccagc tggcgtaata gcgaagaggc ccgcaccgat cgcccttccc aacagttgcg 300

cagcctgaat ggcgaatggc gctttgcctg gtttccggca ccagaagcgg tgccggaaag 360

ctggctggag tgcgatcttc ctgaggccga tactgtcgtc gtcccctcaa actggcagat 420

gcacggttac gatgcgccca tctacaccaa cgtgacctat cccattacgg tcaatccgcc 480

gtttgttccc acggagaatc cgacgggttg ttactcgctc acatttaatg ttgatgaaag 540

ctggctacag gaaggccaga cgcgaattat ttttgatggc gttaactcgg cgtttcatct 600

gtggtgcaac gggcgctggg tcggttacgg ccaggacagt cgtttgccgt ctgaatttga 660

cctgagcgca tttttacgcg ccggagaaaa ccgcctcgcg gtgatggtgc tgcgctggag 720

tgacggcagt tatctggaag atcaggatat gtggcggatg agcggcattt tccgtgacgt 780

ctcgttgctg cataaaccga ctacacaaat cagcgatttc catgttgcca ctcgctttaa 840

tgatgatttc agccgcgctg tactggaggc tgaagttcag atgtgcggcg agttgcgtga 900

ctacctacgg gtaacagttt ctttatggca gggtgaaacg caggtcgcca gcggcaccgc 960

gcctttcggc ggtgaaatta tcgatgagcg tggtggttat gccgatcgcg tcacactacg 1020

tctgaacgtc gaaaacccga aactgtggag cgccgaaatc ccgaatctct atcgtgcggt 1080

ggttgaactg cacaccgccg acggcacgct gattgaagca gaagcctgcg atgtcggttt 1140

ccgcgaggtg cggattgaaa atggtctgct gctgctgaac ggcaagccgt tgctgattcg 1200

aggcgttaac cgtcacgagc atcatcctct gcatggtcag gtcatggatg agcagacgat 1260

ggtgcaggat atcctgctga tgaagcagaa caactttaac gccgtgcgct gttcgcatta 1320

tccgaaccat ccgctgtggt acacgctgtg cgaccgctac ggcctgtatg tggtggatga 1380

agccaatatt gaaacccacg gcatggtgcc aatgaatcgt ctgaccgatg atccgcgctg 1440

gctaccggcg atgagcgaac gcgtaacgcg aatggtgcag cgcgatcgta atcacccgag 1500

tgtgatcatc tggtcgctgg ggaatgaatc aggccacggc gctaatcacg acgcgctgta 1560

tcgctggatc aaatctgtcg atccttcccg cccggtgcag tatgaaggcg gcggagccga 1620

caccacggcc accgatatta tttgcccgat gtacgcgcgc gtggatgaag accagccctt 1680

cccggctgtg ccgaaatggt ccatcaaaaa atggctttcg ctacctggag agacgcgccc 1740

gctgatcctt tgcgaatacg cccacgcgat gggtaacagt cttggcggtt tcgctaaata 1800

ctggcaggcg tttcgtcagt atccccgttt acagggcggc ttcgtctggg actgggtgga 1860

tcagtcgctg attaaatatg atgaaaacgg caacccgtgg tcggcttacg gcggtgattt 1920

tggcgatacg ccgaacgatc gccagttctg tatgaacggt ctggtctttg ccgaccgcac 1980

gccgcatcca gcgctgacgg aagcaaaaca ccagcagcag tttttccagt tccgtttatc 2040

cgggcaaacc atcgaagtga ccagcgaata cctgttccgt catagcgata acgagctcct 2100

gcactggatg gtggcgctgg atggtaagcc gctggcaagc ggtgaagtgc ctctggatgt 2160

cgctccacaa ggtaaacagt tgattgaact gcctgaacta ccgcagccgg agagcgccgg 2220

gcaactctgg ctcacagtac gcgtagtgca accgaacgcg accgcatggt cagaagccgg 2280

gcacatcagc gcctggcagc agtggcgtct ggcggaaaac ctcagtgtga cgctccccgc 2340

cgcgtcccac gccatcccgc atctgaccac cagcgaaatg gatttttgca tcgagctggg 2400

taataagcgt tggcaattta accgccagtc aggctttctt tcacagatgt ggattggcga 2460

taaaaaacaa ctgctgacgc cgctgcgcga tcagttcacc cgtgcaccgc tggataacga 2520

cattggcgta agtgaagcga cccgcattga ccctaacgcc tgggtcgaac gctggaaggc 2580

ggcgggccat taccaggccg aagcagcgtt gttgcagtgc acggcagata cacttgctga 2640

tgcggtgctg attacgaccg ctcacgcgtg gcagcatcag gggaaaacct tatttatcag 2700

ccggaaaacc taccggattg atggtagtgg tcaaatggcg attaccgttg atgttgaagt 2760

ggcgagcgat acaccgcatc cggcgcggat tggcctgaac tgccagctgg cgcaggtagc 2820

agagcgggta aactggctcg gattagggcc gcaagaaaac tatcccgacc gccttactgc 2880

cgcctgtttt gaccgctggg atctgccatt gtcagacatg tataccccgt acgtcttccc 2940

gagcgaaaac ggtctgcgct gcgggacgcg cgaattgaat tatggcccac accagtggcg 3000

cggcgacttc cagttcaaca tcagccgcta cagtcaacag caactgatgg aaaccagcca 3060

tcgccatctg ctgcacgcgg aagaaggcac atggctgaat atcgacggtt tccatatggg 3120

gattggtggc gacgactcct ggagcccgtc agtatcggcg gaattccagc tgagcgccgg 3180

tcgctaccat taccagttgg tctggtgtca aaaataacta gcataacccc tctctaaacg 3240

gaggggttt 3249

<210> 14
<211> 3249
<212> DNA
<213> artificial

<220>
<223> Artificial

<400> 14
tcataagctt taatacgact cactataggc ctaaatatta tccaacccgg gccagcggcg 60

gataaaatgt cgttggtccg ggagagggag gcgtgaaaaa attgtctttc tttttcggat 120

taatgatacg aaaaaggagg atatcatgta ctacagcttc gatcccgccg tcgttttaca 180

acgtcgtgac tgggaaaacc ctggcgttac ccaacttaat cgccttgcag cacatccccc 240

tttcgccagc tggcgtaata gcgaagaggc ccgcaccgat cgcccttccc aacagttgcg 300

cagcctgaat ggcgaatggc gctttgcctg gtttccggca ccagaagcgg tgccggaaag 360

ctggctggag tgcgatcttc ctgaggccga tactgtcgtc gtcccctcaa actggcagat 420

gcacggttac gatgcgccca tctacaccaa cgtgacctat cccattacgg tcaatccgcc 480

gtttgttccc acggagaatc cgacgggttg ttactcgctc acatttaatg ttgatgaaag 540

ctggctacag gaaggccaga cgcgaattat ttttgatggc gttaactcgg cgtttcatct 600

gtggtgcaac gggcgctggg tcggttacgg ccaggacagt cgtttgccgt ctgaatttga 660

cctgagcgca tttttacgcg ccggagaaaa ccgcctcgcg gtgatggtgc tgcgctggag 720

tgacggcagt tatctggaag atcaggatat gtggcggatg agcggcattt tccgtgacgt 780

ctcgttgctg cataaaccga ctacacaaat cagcgatttc catgttgcca ctcgctttaa 840

tgatgatttc agccgcgctg tactggaggc tgaagttcag atgtgcggcg agttgcgtga 900

ctacctacgg gtaacagttt ctttatggca gggtgaaacg caggtcgcca gcggcaccgc 960

gcctttcggc ggtgaaatta tcgatgagcg tggtggttat gccgatcgcg tcacactacg 1020

tctgaacgtc gaaaacccga aactgtggag cgccgaaatc ccgaatctct atcgtgcggt 1080

ggttgaactg cacaccgccg acggcacgct gattgaagca gaagcctgcg atgtcggttt 1140

ccgcgaggtg cggattgaaa atggtctgct gctgctgaac ggcaagccgt tgctgattcg 1200

aggcgttaac cgtcacgagc atcatcctct gcatggtcag gtcatggatg agcagacgat 1260

ggtgcaggat atcctgctga tgaagcagaa caactttaac gccgtgcgct gttcgcatta 1320

tccgaaccat ccgctgtggt acacgctgtg cgaccgctac ggcctgtatg tggtggatga 1380

agccaatatt gaaacccacg gcatggtgcc aatgaatcgt ctgaccgatg atccgcgctg 1440

gctaccggcg atgagcgaac gcgtaacgcg aatggtgcag cgcgatcgta atcacccgag 1500

tgtgatcatc tggtcgctgg ggaatgaatc aggccacggc gctaatcacg acgcgctgta 1560

tcgctggatc aaatctgtcg atccttcccg cccggtgcag tatgaaggcg gcggagccga 1620

caccacggcc accgatatta tttgcccgat gtacgcgcgc gtggatgaag accagccctt 1680

cccggctgtg ccgaaatggt ccatcaaaaa atggctttcg ctacctggag agacgcgccc 1740

gctgatcctt tgcgaatacg cccacgcgat gggtaacagt cttggcggtt tcgctaaata 1800

ctggcaggcg tttcgtcagt atccccgttt acagggcggc ttcgtctggg actgggtgga 1860

tcagtcgctg attaaatatg atgaaaacgg caacccgtgg tcggcttacg gcggtgattt 1920

tggcgatacg ccgaacgatc gccagttctg tatgaacggt ctggtctttg ccgaccgcac 1980

gccgcatcca gcgctgacgg aagcaaaaca ccagcagcag tttttccagt tccgtttatc 2040

cgggcaaacc atcgaagtga ccagcgaata cctgttccgt catagcgata acgagctcct 2100

gcactggatg gtggcgctgg atggtaagcc gctggcaagc ggtgaagtgc ctctggatgt 2160

cgctccacaa ggtaaacagt tgattgaact gcctgaacta ccgcagccgg agagcgccgg 2220

gcaactctgg ctcacagtac gcgtagtgca accgaacgcg accgcatggt cagaagccgg 2280

gcacatcagc gcctggcagc agtggcgtct ggcggaaaac ctcagtgtga cgctccccgc 2340

cgcgtcccac gccatcccgc atctgaccac cagcgaaatg gatttttgca tcgagctggg 2400

taataagcgt tggcaattta accgccagtc aggctttctt tcacagatgt ggattggcga 2460

taaaaaacaa ctgctgacgc cgctgcgcga tcagttcacc cgtgcaccgc tggataacga 2520

cattggcgta agtgaagcga cccgcattga ccctaacgcc tgggtcgaac gctggaaggc 2580

ggcgggccat taccaggccg aagcagcgtt gttgcagtgc acggcagata cacttgctga 2640

tgcggtgctg attacgaccg ctcacgcgtg gcagcatcag gggaaaacct tatttatcag 2700

ccggaaaacc taccggattg atggtagtgg tcaaatggcg attaccgttg atgttgaagt 2760

ggcgagcgat acaccgcatc cggcgcggat tggcctgaac tgccagctgg cgcaggtagc 2820

agagcgggta aactggctcg gattagggcc gcaagaaaac tatcccgacc gccttactgc 2880

cgcctgtttt gaccgctggg atctgccatt gtcagacatg tataccccgt acgtcttccc 2940

gagcgaaaac ggtctgcgct gcgggacgcg cgaattgaat tatggcccac accagtggcg 3000

cggcgacttc cagttcaaca tcagccgcta cagtcaacag caactgatgg aaaccagcca 3060

tcgccatctg ctgcacgcgg aagaaggcac atggctgaat atcgacggtt tccatatggg 3120

gattggtggc gacgactcct ggagcccgtc agtatcggcg gaattccagc tgagcgccgg 3180

tcgctaccat taccagttgg tctggtgtca aaaataacta gcataacccc tctctaaacg 3240

gaggggttt 3249

<210> 15
<211> 1450
<212> DNA
<213> escherichia coli

<400> 15
agtttgatca tggctcagat tgaacgctgg cggcaggcct aacacatgca agtcgaacgg 60

taacaggaag cagcttgctg ctttgctgac gagtggcgga cgggtgagta atgtctggga 120

aactgcctga tggaggggga taactactgg aaacggtagc taataccgca taacgtcgca 180

agcacaaaga gggggacctt agggcctctt gccatcggat gtgcccagat gggattagct 240

agtaggtggg gtaacggctc acctaggcga cgatccctag ctggtctgag aggatgacca 300

gcaacactgg aactgagaca cggtccagac tcctacggga ggcagcagtg gggaatattg 360

cacaatgggc gcaagcctga tgcagccatg ccgcgtgtat gaagaaggcc ttcgggttgt 420

aaagtacttt cagcggggag gaagggagta aagttaatac ctttgctcat tgacgttacc 480

cgcagaagaa gcaccggcta actccgtgcc agcagccgcg gtaatacgga gggtgcaagc 540

gttaatcgga attactgggc gtaaagcgca cgcaggcggt ttgttaagtc agatgtgaaa 600

tccccgggct caacctggga actgcatctg atactggcaa gcttgagtct cgtagagggg 660

ggtagaattc caggtgtagc ggtgaaatgc gtagagatct ggaggaatac cggtggcgaa 720

ggcggccccc tggacgaaga ctgacgctca ggtgcgaaag cgtggggagc aaacaggatt 780

agataccctg gtagtccacg ccgtaaacga tgtcgacttg gaggttgtgc ccttgaggcg 840

tggcttccgg agctaacgcg ttaagtcgac cgcctgggga gtacggccgc aaggttaaaa 900

ctcaaatgaa ttgacggggg ccgcacaagc ggtggagcat gtggtttaat tcgatgcaac 960

gcgaagaacc ttacctggtc ttgacatcca cggaagtttt cagagatgag aatgtgcctt 1020

cgggaaccgt gagacaggtg ctgcatggct gtcgtcagct cgtgttgtga aatgttgggt 1080

taagtcccgc aacgagcgca acccttatcc tttgttgcca gcggtccggc cgggaactca 1140

aaggagactg ccagtgataa actggaggaa ggtggggatg acgtcaagtc atcatggccc 1200

ttacgaccag ggctacacac gtgctacaat ggcgcataca aagagaagcg acctcgcgag 1260

agcaagcgga cctcataaag tgcgtcgtag tccggattgg agtctgcaac tcgactccat 1320

gaagtcggaa tcgctagtaa tcgtggatca gaatgccacg gtgaatacgt tcccgggcct 1380

tgtacacacc gcccgtcaca ccatgggagt gggttgcaaa agaagtaggt agcttaactt 1440

cgggagggcg 1450

<210>  16
<211>  159
<212>  RNA
<213>  artificial

<220>
<223>  Artificial

<400>  16
ggcguguagu ugugcugcgu gugugaggag ggcugaaaga gcucuuuucg uauauaaagg      60

aggguaccua uaaucugucc uccuuuauau acgaaaagag uauauaaagg aggacaauau      120

gcacauacaa agcuucgauc ccgccgucgu uuuacaacg                            159


<210>  17
<211>  159
<212>  RNA
<213>  artificial

<220>
<223>  Artificial

<400>  17
ggcguguagu ugugcugcgu gugugaggag ggcugagaga gcucuuuucg uauauaaagg      60

aggguaccua uaaucugucc uccuuuauau acgaaaagag uauauaaagg aggacaauau      120

gcacauacaa agcuucgauc ccgccgucgu uuuacaacg                            159


<210>  18
<211>  78
<212>  RNA
<213>  escherichia coli

<400>  18
ugaaaguacu uuacaacccg aaggccuucu ucauacacgc ggcauggcug caucaggcuu      60

guugcgccca uucugcaa                                                    78


<210>  19
<211>  186
<212>  DNA
<213>  artificial

<220>
<223>  Artificial

<400>  19
tcataagctt taatacgact cactataggc gtgtagttgt gctgcgtgtg tgaggagggc      60

tgaaagagct cttttcgtat ataaaggagg gtacctataa tctgtcctcc tttatatacg      120

aaaagagtat ataaaggagg acaatatgca catacaaagc ttcgatcccg ccgtcgtttt      180

acaacg                                                                 186


<210>  20
<211>  186

<212>  DNA
<213>  artificial

<220>
<223>  Artificial

<400>  20
tcataagctt taatacgact cactataggc gtgtagttgt gctgcgtgtg tgaggagggc      60

tgagagagct cttttcgtat ataaaggagg gtacctataa tctgtcctcc tttatatacg     120

aaaagagtat ataaaggagg acaatatgca catacaaagc ttcgatcccg ccgtcgtttt     180

acaacg                                                                186


<210>  21
<211>  3252
<212>  DNA
<213>  artificial

<220>
<223>  Artificial

<400>  21
tcataagctt taatacgact cactataggc gtgtagttgt gctgcgtgtg tgaggagggc      60

tgaaagagct cttttcgtat ataaaggagg gtacctataa tctgtcctcc tttatatacg     120

aaaagagtat ataaaggagg acaatatgca catacaaagc ttcgatcccg ccgtcgtttt     180

acaacgtcgt gactgggaaa accctggcgt tacccaactt aatcgccttg cagcacatcc     240

ccctttcgcc agctggcgta atagcgaaga ggcccgcacc gatcgccctt cccaacagtt     300

gcgcagcctg aatggcgaat ggcgctttgc ctggtttccg gcaccagaag cggtgccgga     360

aagctggctg gagtgcgatc ttcctgaggc cgatactgtc gtcgtcccct caaactggca     420

gatgcacggt tacgatgcgc ccatctacac caacgtgacc tatcccatta cggtcaatcc     480

gccgtttgtt cccacggaga atccgacggg ttgttactcg ctcacattta atgttgatga     540

aagctggcta caggaaggcc agacgcgaat tatttttgat ggcgttaact cggcgtttca     600

tctgtggtgc aacgggcgct gggtcggtta cggccaggac agtcgtttgc cgtctgaatt     660

tgacctgagc gcatttttac gcgccggaga aaaccgcctc gcggtgatgg tgctgcgctg     720

gagtgacggc agttatctgg aagatcagga tatgtggcgg atgagcggca ttttccgtga     780

cgtctcgttg ctgcataaac cgactacaca aatcagcgat ttccatgttg ccactcgctt     840

taatgatgat ttcagccgcg ctgtactgga ggctgaagtt cagatgtgcg gcgagttgcg     900

tgactaccta cgggtaacag tttctttatg gcaggtgaaa acgcaggtcg ccagcggcac     960

cgcgcctttc ggcggtgaaa ttatcgatga gcgtggtggt tatgccgatc gcgtcacact    1020

acgtctgaac gtcgaaaacc cgaaactgtg gagcgccgaa atcccgaatc tctatcgtgc    1080

ggtggttgaa ctgcacaccg ccgacggcac gctgattgaa gcagaagcct gcgatgtcgg    1140

tttccgcgag gtgcggattg aaaatggtct gctgctgctg aacggcaagc cgttgctgat 1200

tcgaggcgtt aaccgtcacg agcatcatcc tctgcatggt caggtcatgg atgagcagac 1260

gatggtgcag gatatcctgc tgatgaagca gaacaacttt aacgccgtgc gctgttcgca 1320

ttatccgaac catccgctgt ggtacacgct gtgcgaccgc tacggcctgt atgtggtgga 1380

tgaagccaat attgaaaccc acggcatggt gccaatgaat cgtctgaccg atgatccgcg 1440

ctggctaccg gcgatgagcg aacgcgtaac gcgaatggtg cagcgcgatc gtaatcaccc 1500

gagtgtgatc atctggtcgc tggggaatga atcaggccac ggcgctaatc acgacgcgct 1560

gtatcgctgg atcaaatctg tcgatccttc ccgcccggtg cagtatgaag gcggcggagc 1620

cgacaccacg gccaccgata ttatttgccc gatgtacgcg cgcgtggatg aagaccagcc 1680

cttcccggct gtgccgaaat ggtccatcaa aaaatggctt tcgctacctg gagagacgcg 1740

cccgctgatc ctttgcgaat acgcccacgc gatgggtaac agtcttggcg gtttcgctaa 1800

atactggcag gcgtttcgtc agtatccccg tttacagggc ggcttcgtct gggactgggt 1860

ggatcagtcg ctgattaaat atgatgaaaa cggcaacccg tggtcggctt acggcggtga 1920

ttttggcgat acgccgaacg atcgccagtt ctgtatgaac ggtctggtct ttgccgaccg 1980

cacgccgcat ccagcgctga cggaagcaaa acaccagcag cagtttttcc agttccgttt 2040

atccgggcaa accatcgaag tgaccagcga atacctgttc cgtcatagcg ataacgagct 2100

cctgcactgg atggtggcgc tggatggtaa gccgctggca agcggtgaag tgcctctgga 2160

tgtcgctcca caaggtaaac agttgattga actgcctgaa ctaccgcagc cggagagcgc 2220

cgggcaactc tggctcacag tacgcgtagt gcaaccgaac gcgaccgcat ggtcagaagc 2280

cgggcacatc agcgcctggc agcagtggcg tctggcggaa aacctcagtg tgacgctccc 2340

cgccgcgtcc cacgccatcc cgcatctgac caccagcgaa atggattttt gcatcgagct 2400

gggtaataag cgttggcaat ttaaccgcca gtcaggcttt ctttcacaga tgtggattgg 2460

cgataaaaaa caactgctga cgccgctgcg cgatcagttc acccgtgcac cgctggataa 2520

cgacattggc gtaagtgaag cgacccgcat tgaccctaac gcctgggtcg aacgctggaa 2580

ggcggcgggc cattaccagg ccgaagcagc gttgttgcag tgcacggcag atacacttgc 2640

tgatgcggtg ctgattacga ccgctcacgc gtggcagcat caggggaaaa ccttatttat 2700

cagccggaaa acctaccgga ttgatggtag tggtcaaatg gcgattaccg ttgatgttga 2760

agtggcgagc gatacaccgc atccggcgcg gattggcctg aactgccagc tggcgcaggt 2820

agcagagcgg gtaaactggc tcggattagg gccgcaagaa aactatcccg accgccttac 2880

tgccgcctgt tttgaccgct gggatctgcc attgtcagac atgtataccc cgtacgtctt 2940

cccgagcgaa aacggtctgc gctgcgggac gcgcgaattg aattatggcc cacaccagtg 3000

gcgcggcgac ttccagttca acatcagccg ctacagtcaa cagcaactga tggaaaccag 3060

ccatcgccat ctgctgcacg cggaagaagg cacatggctg aatatcgacg gtttccatat 3120

ggggattggt ggcgacgact cctggagccc gtcagtatcg gcggaattcc agctgagcgc 3180

cggtcgctac cattaccagt tggtctggtg tcaaaaataa ctagcataac ccctctctaa 3240

acggaggggt tt 3252

<210> 22
<211> 3252
<212> DNA
<213> artificial

<220>
<223> Artificial

<400> 22
tcataagctt taatacgact cactataggc gtgtagttgt gctgcgtgtg tgaggagggc 60

tgagagagct cttttcgtat ataaaggagg gtacctataa tctgtcctcc tttatatacg 120

aaaagagtat ataaaggagg acaatatgca catacaaagc ttcgatcccg ccgtcgtttt 180

acaacgtcgt gactgggaaa accctggcgt tacccaactt aatcgccttg cagcacatcc 240

ccctttcgcc agctggcgta atagcgaaga ggcccgcacc gatcgccctt cccaacagtt 300

gcgcagcctg aatggcgaat ggcgctttgc ctggtttccg gcaccagaag cggtgccgga 360

aagctggctg gagtgcgatc ttcctgaggc cgatactgtc gtcgtcccct caaactggca 420

gatgcacggt tacgatgcgc ccatctacac caacgtgacc tatcccatta cggtcaatcc 480

gccgtttgtt cccacggaga atccgacggg ttgttactcg ctcacattta atgttgatga 540

aagctggcta caggaaggcc agacgcgaat tatttttgat ggcgttaact cggcgtttca 600

tctgtggtgc aacgggcgct gggtcggtta cggccaggac agtcgtttgc cgtctgaatt 660

tgacctgagc gcatttttac gcgccggaga aaaccgcctc gcggtgatgg tgctgcgctg 720

gagtgacggc agttatctgg aagatcagga tatgtggcgg atgagcggca ttttccgtga 780

cgtctcgttg ctgcataaac cgactacaca aatcagcgat ttccatgttg ccactcgctt 840

taatgatgat ttcagccgcg ctgtactgga ggctgaagtt cagatgtgcg gcgagttgcg 900

tgactaccta cgggtaacag tttctttatg gcagggtgaa acgcaggtcg ccagcggcac 960

cgcgcctttc ggcggtgaaa ttatcgatga gcgtggtggt tatgccgatc gcgtcacact 1020

acgtctgaac gtcgaaaacc cgaaactgtg gagcgccgaa atcccgaatc tctatcgtgc 1080

ggtggttgaa ctgcacaccg ccgacggcac gctgattgaa gcagaagcct gcgatgtcgg 1140

tttccgcgag gtgcggattg aaaatggtct gctgctgctg aacggcaagc cgttgctgat 1200

tcgaggcgtt aaccgtcacg agcatcatcc tctgcatggt caggtcatgg atgagcagac 1260

gatggtgcag gatatcctgc tgatgaagca gaacaacttt aacgccgtgc gctgttcgca 1320

ttatccgaac catccgctgt ggtacacgct gtgcgaccgc tacggcctgt atgtggtgga 1380

tgaagccaat attgaaaccc acggcatggt gccaatgaat cgtctgaccg atgatccgcg 1440

ctggctaccg gcgatgagcg aacgcgtaac gcgaatggtg cagcgcgatc gtaatcaccc 1500

gagtgtgatc atctggtcgc tggggaatga atcaggccac ggcgctaatc acgacgcgct 1560

gtatcgctgg atcaaatctg tcgatccttc ccgcccggtg cagtatgaag gcggcggagc 1620

cgacaccacg gccaccgata ttatttgccc gatgtacgcg cgcgtggatg aagaccagcc 1680

cttcccggct gtgccgaaat ggtccatcaa aaaatggctt tcgctacctg gagagacgcg 1740

cccgctgatc ctttgcgaat acgcccacgc gatgggtaac agtcttggcg gtttcgctaa 1800

atactggcag gcgtttcgtc agtatccccg tttacagggc ggcttcgtct gggactgggt 1860

ggatcagtcg ctgattaaat atgatgaaaa cggcaacccg tggtcggctt acggcggtga 1920

ttttggcgat acgccgaacg atcgccagtt ctgtatgaac ggtctggtct ttgccgaccg 1980

cacgccgcat ccagcgctga cggaagcaaa acaccagcag cagtttttcc agttccgttt 2040

atccgggcaa accatcgaag tgaccagcga atacctgttc cgtcatagcg ataacgagct 2100

cctgcactgg atggtggcgc tggatggtaa gccgctggca agcggtgaag tgcctctgga 2160

tgtcgctcca caaggtaaac agttgattga actgcctgaa ctaccgcagc cggagagcgc 2220

cgggcaactc tggctcacag tacgcgtagt gcaaccgaac gcgaccgcat ggtcagaagc 2280

cgggcacatc agcgcctggc agcagtggcg tctggcggaa aacctcagtg tgacgctccc 2340

cgccgcgtcc cacgccatcc cgcatctgac caccagcgaa atggattttt gcatcgagct 2400

gggtaataag cgttggcaat ttaaccgcca gtcaggcttt ctttcacaga tgtggattgg 2460

cgataaaaaa caactgctga cgccgctgcg cgatcagttc acccgtgcac cgctggataa 2520

cgacattggc gtaagtgaag cgacccgcat tgaccctaac gcctgggtcg aacgctggaa 2580

ggcggcgggc cattaccagg ccgaagcagc gttgttgcag tgcacggcag atacacttgc 2640

tgatgcggtg ctgattacga ccgctcacgc gtggcagcat caggggaaaa ccttatttat 2700

cagccggaaa acctaccgga ttgatggtag tggtcaaatg gcgattaccg ttgatgttga 2760

agtggcgagc gatacaccgc atccggcgcg gattggcctg aactgccagc tggcgcaggt 2820

agcagagcgg gtaaactggc tcggattagg gccgcaagaa aactatcccg accgccttac 2880

tgccgcctgt tttgaccgct gggatctgcc attgtcagac atgtataccc cgtacgtctt 2940

cccgagcgaa aacggtctgc gctgcgggac gcgcgaattg aattatggcc cacaccagtg 3000

gcgcggcgac ttccagttca acatcagccg ctacagtcaa cagcaactga tggaaaccag 3060

ccatcgccat ctgctgcacg cggaagaagg cacatggctg aatatcgacg gtttccatat 3120

ggggattggt ggcgacgact cctggagccc gtcagtatcg gcggaattcc agctgagcgc 3180

cggtcgctac cattaccagt tggtctggtg tcaaaaataa ctagcataac ccctctctaa 3240

acggaggggt tt                                                          3252

<210>  23
<211>  759
<212>  DNA
<213>  artificial

<220>
<223>  Artificial

<400>  23
atgcgtaaag gagaagaact tttcactgga gttgtcccaa ttcttgttga attagatggt      60

gatgttaatg ggcacaaatt ttctgtcagt ggagagggtg aaggtgatgc aacatacgga     120

aaacttaccc ttaaatttat ttgcactact ggaaaactac ctgttccgtg gccaacactt     180

gtcactactt tcggttatgg tgttcaatgc tttgcgagat acccagatca catgaaacag     240

catgactttt tcaagagtgc catgcccgaa ggttacgtac aggaaagaac tatatttttc     300

aaagatgacg ggaactacaa gacacgtgct gaagtcaagt ttgaaggtga tacccttgtt     360

aatagaatcg agttaaaagg tattgatttt aaagaagatg gaaacattct tggacacaaa     420

ttggaataca actataactc acacaatgta tacatcatgg cagacaaaca aaagaatgga     480

atcaaagtta acttcaaaat tagacacaac attgaagatg gaagcgttca actagcagac     540

cattatcaac aaaatactcc gattggcgat ggccctgtcc ttttaccaga caaccattac     600

ctgtccacac aatctgccct ttcgaaagat cccaacgaaa agagagacca catggtcctt     660

cttgagtttg taaccgctgc tgggattaca catggcatgg atgaactata caaaaggcct     720

gcagcaaacg acgaaaacta cgctgcatca gtttaataa                            759

<210>  24
<211>  167
<212>  RNA
<213>  artificial

<220>
<223>  Artificial

<400>  24
ggcguggcga guuuggugcg gucgugcugu gugguaaaua cguauaguac gaucggaggg      60

agggagaaca aucgguuuuc uccuuuugau cguauuauac gaucaaaagg aggagauaau     120

guacauuugc aaccuggcgg cagcgcaaaa gaugcguaaa ggagaag                   167

<210>  25
<211>  167
<212>  RNA
<213>  artificial

<220>
<223>  Artificial

<400>  25
ggcguguagc uauguugcgu guauggggag ggcgaaaauc gcuuuucucg uauauaaagg      60

agggagcacu uaauacuucc uccuuuauau acgagaaggg uauauaaagg aggaagcaau      120

ggaaguuggc aaccuggcgg cagcgcaaaa gaugcguaaa ggagaag                    167


<210>  26
<211>  167
<212>  RNA
<213>  artificial

<220>
<223>  Artificial

<400>  26
ggcgugugga ggagguuuuc ggguuguaaa guaauacauu uauuuugcga uucgauaggg      60

aggaaguaca cauaucuucc uccuuauugg aucgcgagau aucaauaagg aggaagaaau      120

gcaaauugcg aaccuggcgg cagcgcaaaa gaugcguaaa ggagaag                    167


<210>  27
<211>  1105
<212>  DNA
<213>  artificial

<220>
<223>  Artificial

<400>  27
ggcaacagat ctcacagcta acaccacgtc gtccctatct gctgccctag gtctatgagt      60

ggttgctgga taactttacg ggcatgcata aggctcgtat aatatattca agtaatactg      120

gaagcaatgt gagcaatgtc gtgctgttat agttatgaga caagaacaga ggagacataa      180

catgaacagc acgaacgctg ttaacctggc ggcagcgcaa aagatgcgta aaggagaaga      240

acttttcact ggagttgtcc caattcttgt tgaattagat ggtgatgtta atgggcacaa      300

attttctgtc agtggagagg gtgaaggtga tgcaacatac ggaaaactta cccttaaatt      360

tatttgcact actggaaaac tacctgttcc gtggccaaca cttgtcacta ctttcggtta      420

tggtgttcaa tgctttgcga gatacccaga tcacatgaaa cagcatgact ttttcaagag      480

tgccatgccc gaaggttacg tacaggaaag aactatattt ttcaaagatg acgggaacta      540

caagacacgt gctgaagtca agtttgaagg tgataccctt gttaatagaa tcgagttaaa      600

aggtattgat tttaaagaag atggaaacat tcttggacac aaattggaat acaactataa      660

ctcacacaat gtatacatca tggcagacaa acaaaagaat ggaatcaaag ttaacttcaa      720

aattagacac aacattgaag atggaagcgt tcaactagca gaccattatc aacaaaatac      780

tccgattggc gatggccctg tccttttacc agacaaccat tacctgtcca cacaatctgc      840

cctttcgaaa gatcccaacg aaaagagaga ccacatggtc cttcttgagt ttgtaaccgc      900

tgctgggatt acacatggca tggatgaact atacaaaagg cctgcagcaa acgacgaaaa 960

ctacgctgca tcagtttaat aagataaacc agagcggcac ggcaagcaga gtatacgaga 1020

ttcggtagcc accgctgagc aataactagc ataacccctt ggggcctcta aacgggtctt 1080

gaggggtttt ttgcatatgc aacgg 1105

<210> 28
<211> 43
<212> DNA
<213> artificial

<220>
<223> Artificial

<400> 28
ccgttgcata tgcaaaaaac ccctcaagac ccgtttagag gcc 43

<210> 29
<211> 194
<212> DNA
<213> artificial

<220>
<223> Artificial

<400> 29
tcataagctt taatacgact cactataggc gtggcgagtt tggtgcggtc gtgctgtgtg 60

gtaaatacgt atagtacgat cggagggagg gagaacaatc ggttttctcc ttttgatcgt 120

attatacgat caaaaggagg agataatgta catttgcaac ctggcggcag cgcaaaagat 180

gcgtaaagga gaag 194

<210> 30
<211> 194
<212> DNA
<213> artificial

<220>
<223> Artificial

<400> 30
tcataagctt taatacgact cactataggc gtgtagctat gttgcgtgta tggggagggc 60

gaaaatcgct tttctcgtat ataaaggagg gagcacttaa tacttcctcc tttatatacg 120

agaagggtat ataaaggagg aagcaatgga agttggcaac ctggcggcag cgcaaaagat 180

gcgtaaagga gaag 194

<210> 31
<211> 194
<212> DNA
<213> artificial

<220>
<223>  Artificial

<400>  31
tcataagctt taatacgact cactataggc gtgtggagga ggttttcggg ttgtaaagta      60

atacatttat tttgcgattc gatagggagg aagtacacat atcttcctcc ttattggatc     120

gcgagatatc aataaggagg aagaaatgca aattgcgaac ctggcggcag cgcaaaagat     180

gcgtaaagga gaag                                                       194


<210>  32
<211>  1063
<212>  DNA
<213>  artificial

<220>
<223>  Artificial

<400>  32
tcataagctt taatacgact cactataggc gtggcgagtt tggtgcggtc gtgctgtgtg      60

gtaaatacgt atagtacgat cggagggagg gagaacaatc ggttttctcc ttttgatcgt     120

attatacgat caaaaggagg agataatgta catttgcaac ctggcggcag cgcaaaagat     180

gcgtaaagga gaagaacttt tcactggagt tgtcccaatt cttgttgaat tagatggtga     240

tgttaatggg cacaaatttt ctgtcagtgg agagggtgaa ggtgatgcaa catacggaaa     300

acttaccctt aaatttattt gcactactgg aaaactacct gttccgtggc caacacttgt     360

cactactttc ggttatggtg ttcaatgctt tgcgagatac ccagatcaca tgaaacagca     420

tgactttttc aagagtgcca tgcccgaagg ttacgtacag gaaagaacta tatttttcaa     480

agatgacggg aactacaaga cacgtgctga agtcaagttt gaaggtgata cccttgttaa     540

tagaatcgag ttaaaaggta ttgattttaa agaagatgga aacattcttg gacacaaatt     600

ggaatacaac tataactcac acaatgtata catcatggca gacaaacaaa agaatggaat     660

caaagttaac ttcaaaatta gacacaacat tgaagatgga agcgttcaac tagcagacca     720

ttatcaacaa aatactccga ttggcgatgg ccctgtcctt ttaccagaca accattacct     780

gtccacacaa tctgcccttt cgaaagatcc caacgaaaag agagaccaca tggtccttct     840

tgagtttgta accgctgctg ggattacaca tggcatggat gaactataca aaaggcctgc      900

agcaaacgac gaaaactacg ctgcatcagt ttaataataa gataaaccag agcggcacgg     960

caagcagagt atacgagatt cggtagccac cgctgagcaa taactagcat aaccccttgg    1020

ggcctctaaa cgggtcttga ggggtttttt gcatatgcaa cgg                      1063


<210>  33
<211>  1063
<212>  DNA
<213>  artificial

<220>
<223>  Artificial

<400>  33
tcataagctt taatacgact cactataggc gtgtagctat gttgcgtgta tggggagggc      60

gaaaatcgct tttctcgtat ataaaggagg gagcacttaa tacttcctcc tttatatacg     120

agaagggtat ataaaggagg aagcaatgga agttggcaac ctggcggcag cgcaaaagat     180

gcgtaaagga gaagaacttt tcactggagt tgtcccaatt cttgttgaat tagatggtga     240

tgttaatggg cacaaatttt ctgtcagtgg agagggtgaa ggtgatgcaa catacggaaa     300

acttaccctt aaatttattt gcactactgg aaaactacct gttccgtggc caacacttgt     360

cactactttc ggttatggtg ttcaatgctt tgcgagatac ccagatcaca tgaaacagca     420

tgactttttc aagagtgcca tgcccgaagg ttacgtacag gaaagaacta tatttttcaa     480

agatgacggg aactacaaga cacgtgctga agtcaagttt gaaggtgata cccttgttaa     540

tagaatcgag ttaaaaggta ttgattttaa agaagatgga aacattcttg gacacaaatt     600

ggaatacaac tataactcac acaatgtata catcatggca gacaaacaaa agaatggaat     660

caaagttaac ttcaaaatta gacacaacat tgaagatgga agcgttcaac tagcagacca     720

ttatcaacaa aatactccga ttggcgatgg ccctgtcctt ttaccagaca accattacct     780

gtccacacaa tctgcccttt cgaaagatcc caacgaaaag agagaccaca tggtccttct     840

tgagtttgta accgctgctg ggattacaca tggcatggat gaactataca aaaggcctgc     900

agcaaacgac gaaaactacg ctgcatcagt ttaataataa gataaaccag agcggcacgg     960

caagcagagt atacgagatt cggtagccac cgctgagcaa taactagcat aaccccttgg    1020

ggcctctaaa cgggtcttga ggggtttttt gcatatgcaa cgg                      1063

<210>  34
<211>  1063
<212>  DNA
<213>  artificial

<220>
<223>  Artificial

<400>  34
tcataagctt taatacgact cactataggc gtgtggagga ggttttcggg ttgtaaagta      60

atacatttat tttgcgattc gatagggagg aagtacacat atcttcctcc ttattggatc     120

gcgagatatc aataaggagg aagaaatgca aattgcgaac ctggcggcag cgcaaaagat     180

gcgtaaagga gaagaacttt tcactggagt tgtcccaatt cttgttgaat tagatggtga     240

tgttaatggg cacaaatttt ctgtcagtgg agagggtgaa ggtgatgcaa catacggaaa     300

acttaccctt aaatttattt gcactactgg aaaactacct gttccgtggc caacacttgt     360

cactactttc ggttatggtg ttcaatgctt tgcgagatac ccagatcaca tgaaacagca 420

tgactttttc aagagtgcca tgcccgaagg ttacgtacag gaaagaacta tatttttcaa 480

agatgacggg aactacaaga cacgtgctga agtcaagttt gaaggtgata cccttgttaa 540

tagaatcgag ttaaaaggta ttgattttaa agaagatgga aacattcttg gacacaaatt 600

ggaatacaac tataactcac acaatgtata catcatggca gacaaacaaa agaatggaat 660

caaagttaac ttcaaaatta gacacaacat tgaagatgga agcgttcaac tagcagacca 720

ttatcaacaa aatactccga ttggcgatgg ccctgtcctt ttaccagaca accattacct 780

gtccacacaa tctgcccttt cgaaagatcc caacgaaaag agagaccaca tggtccttct 840

tgagtttgta accgctgctg ggattacaca tggcatggat gaactataca aaaggcctgc 900

agcaaacgac gaaaactacg ctgcatcagt ttaataataa gataaaccag agcggcacgg 960

caagcagagt atacgagatt cggtagccac cgctgagcaa taactagcat aaccccttgg 1020

ggcctctaaa cgggtcttga ggggtttttt gcatatgcaa cgg 1063

<210> 35
<211> 21290
<212> DNA
<213> severe acute respiratory syndrome coronavirus 2 isolate wuhan-hu-1
(sars-cov-2)

<400> 35
atggagagcc ttgtccctgg tttcaacgag aaaacacacg tccaactcag tttgcctgtt 60

ttacaggttc gcgacgtgct cgtacgtggc tttggagact ccgtggagga ggtcttatca 120

gaggcacgtc aacatcttaa agatggcact tgtggcttag tagaagttga aaaaggcgtt 180

ttgcctcaac ttgaacagcc ctatgtgttc atcaaacgtt cggatgctcg aactgcacct 240

catggtcatg ttatggttga gctggtagca gaactcgaag gcattcagta cggtcgtagt 300

ggtgagacac ttggtgtcct tgtccctcat gtgggcgaaa taccagtggc ttaccgcaag 360

gttcttcttc gtaagaacgg taataaagga gctggtggcc atagttacgg cgccgatcta 420

aagtcatttg acttaggcga cgagcttggc actgatcctt atgaagattt tcaagaaaac 480

tggaacacta aacatagcag tggtgttacc cgtgaactca tgcgtgagct taacggaggg 540

gcatacactc gctatgtcga taacaacttc tgtggccctg atggctaccc tcttgagtgc 600

attaaagacc ttctagcacg tgctggtaaa gcttcatgca ctttgtccga acaactggac 660

tttattgaca ctaagagggg tgtatactgc tgccgtgaac atgagcatga aattgcttgg 720

tacacggaac gttctgaaaa gagctatgaa ttgcagacac cttttgaaat taaattggca 780

aagaaatttg acaccttcaa tggggaatgt ccaaattttg tatttccctt aaattccata 840

atcaagacta ttcaaccaag ggttgaaaag aaaaagcttg atggctttat gggtagaatt 900

cgatctgtct atccagttgc gtcaccaaat gaatgcaacc aaatgtgcct ttcaactctc 960

atgaagtgtg atcattgtgg tgaaacttca tggcagacgg gcgattttgt taaagccact 1020

tgcgaatttt gtggcactga gaatttgact aaagaaggtg ccactacttg tggttactta 1080

ccccaaaatg ctgttgttaa aatttattgt ccagcatgtc acaattcaga agtaggacct 1140

gagcatagtc ttgccgaata ccataatgaa tctggcttga aaaccattct tcgtaagggt 1200

ggtcgcacta ttgcctttgg aggctgtgtg ttctcttatg ttggttgcca taacaagtgt 1260

gcctattggg ttccacgtgc tagcgctaac ataggttgta accatacagg tgttgttgga 1320

gaaggttccg aaggtcttaa tgacaacctt cttgaaatac tccaaaaaga gaaagtcaac 1380

atcaatattg ttggtgactt taaacttaat gaagagatcg ccattatttt ggcatctttt 1440

tctgcttcca caagtgcttt tgtggaaact gtgaaaggtt tggattataa agcattcaaa 1500

caaattgttg aatcctgtgg taattttaaa gttacaaaag gaaaagctaa aaaaggtgcc 1560

tggaatattg gtgaacagaa atcaatactg agtcctcttt atgcatttgc atcagaggct 1620

gctcgtgttg tacgatcaat tttctcccgc actcttgaaa ctgctcaaaa ttctgtgcgt 1680

gttttacaga aggccgctat aacaatacta gatggaattt cacagtattc actgagactc 1740

attgatgcta tgatgttcac atctgatttg gctactaaca atctagttgt aatggcctac 1800

attacaggtg gtgttgttca gttgacttcg cagtggctaa ctaacatctt tggcactgtt 1860

tatgaaaaac tcaaacccgt ccttgattgg cttgaagaga agtttaagga aggtgtagag 1920

tttcttagag acggttggga aattgttaaa tttatctcaa cctgtgcttg tgaaattgtc 1980

ggtggacaaa ttgtcacctg tgcaaaggaa attaaggaga gtgttcagac attctttaag 2040

cttgtaaata aatttttggc tttgtgtgct gactctatca ttattggtgg agctaaactt 2100

aaagccttga atttaggtga aacatttgtc acgcactcaa agggattgta cagaaagtgt 2160

gttaaatcca gagaagaaac tggcctactc atgcctctaa aagccccaaa agaaattatc 2220

ttcttagagg gagaaacact tcccacagaa gtgttaacag aggaagttgt cttgaaaact 2280

ggtgatttac aaccattaga acaacctact agtgaagctg ttgaagctcc attggttggt 2340

acaccagttt gtattaacgg gcttatgttg ctcgaaatca aagacacaga aaagtactgt 2400

gcccttgcac ctaatatgat ggtaacaaac aataccttca cactcaaagg cggtgcacca 2460

acaaaggtta cttttggtga tgacactgtg atagaagtgc aaggttacaa gagtgtgaat 2520

atcacttttg aacttgatga aaggattgat aaagtactta atgagaagtg ctctgcctat 2580

acagttgaac tcggtacaga agtaaatgag ttcgcctgtg ttgtggcaga tgctgtcata 2640

aaaactttgc aaccagtatc tgaattactt acaccactgg gcattgattt agatgagtgg 2700

agtatggcta catactactt atttgatgag tctggtgagt ttaaattggc ttcacatatg 2760

tattgttctt tctaccctcc agatgaggat gaagaagaag gtgattgtga agaagaagag 2820

tttgagccat caactcaata tgagtatggt actgaagatg attaccaagg taaacctttg 2880

gaatttggtg ccacttctgc tgctcttcaa cctgaagaag agcaagaaga agattggtta 2940

gatgatgata gtcaacaaac tgttggtcaa caagacggca gtgaggacaa tcagacaact 3000

actattcaaa caattgttga ggttcaacct caattagaga tggaacttac accagttgtt 3060

cagactattg aagtgaatag ttttagtggt tatttaaaac ttactgacaa tgtatacatt 3120

aaaaatgcag acattgtgga agaagctaaa aaggtaaaac caacagtggt tgttaatgca 3180

gccaatgttt accttaaaca tggaggaggt gttgcaggag ccttaaataa ggctactaac 3240

aatgccatgc aagttgaatc tgatgattac atagctacta atggaccact taaagtgggt 3300

ggtagttgtg ttttaagcgg acacaatctt gctaaacact gtcttcatgt tgtcggccca 3360

aatgttaaca aaggtgaaga cattcaactt cttaagagtg cttatgaaaa ttttaatcag 3420

cacgaagttc tacttgcacc attattatca gctggtattt ttggtgctga ccctatacat 3480

tctttaagag tttgtgtaga tactgttcgc acaaatgtct acttagctgt ctttgataaa 3540

aatctctatg acaaacttgt ttcaagcttt ttggaaatga agagtgaaaa gcaagttgaa 3600

caaaagatcg ctgagattcc taaagaggaa gttaagccat ttataactga aagtaaacct 3660

tcagttgaac agagaaaaca agatgataag aaaatcaaag cttgtgttga agaagttaca 3720

acaactctgg aagaaactaa gttcctcaca gaaaacttgt tactttatat tgacattaat 3780

ggcaatcttc atccagattc tgccactctt gttagtgaca ttgacatcac tttcttaaag 3840

aaagatgctc catatatagt gggtgatgtt gttcaagagg gtgttttaac tgctgtggtt 3900

atacctacta aaaaggctgg tggcactact gaaatgctag cgaaagcttt gagaaaagtg 3960

ccaacagaca attatataac cacttacccg ggtcagggtt taaatggtta cactgtagag 4020

gaggcaaaga cagtgcttaa aaagtgtaaa agtgcctttt acattctacc atctattatc 4080

tctaatgaga agcaagaaat tcttggaact gtttcttgga atttgcgaga aatgcttgca 4140

catgcagaag aaacacgcaa attaatgcct gtctgtgtgg aaactaaagc catagtttca 4200

actatacagc gtaaatataa gggtattaaa atacaagagg gtgtggttga ttatggtgct 4260

agattttact tttacaccag taaaacaact gtagcgtcac ttatcaacac acttaacgat 4320

ctaaatgaaa ctcttgttac aatgccactt ggctatgtaa cacatggctt aaatttggaa 4380

gaagctgctc ggtatatgag atctctcaaa gtgccagcta cagtttctgt ttcttcacct 4440

gatgctgtta cagcgtataa tggttatctt acttcttctt ctaaaacacc tgaagaacat 4500

tttattgaaa ccatctcact tgctggttcc tataaagatt ggtcctattc tggacaatct 4560

acacaactag gtatagaatt tcttaagaga ggtgataaaa gtgtatatta cactagtaat 4620

cctaccacat tccacctaga tggtgaagtt atcacctttg acaatcttaa gacacttctt 4680

tctttgagag aagtgaggac tattaaggtg tttacaacag tagacaacat taacctccac 4740

acgcaagttg tggacatgtc aatgacatat ggacaacagt ttggtccaac ttatttggat 4800

ggagctgatg ttactaaaat aaaacctcat aattcacatg aaggtaaaac attttatgtt 4860

ttacctaatg atgacactct acgtgttgag gcttttgagt actaccacac aactgatcct 4920

agttttctgg gtaggtacat gtcagcatta aatcacacta aaaagtggaa atacccacaa 4980

gttaatggtt taacttctat taaatgggca gataacaact gttatcttgc cactgcattg 5040

ttaacactcc aacaaataga gttgaagttt aatccacctg ctctacaaga tgcttattac 5100

agagcaaggg ctggtgaagc tgctaacttt tgtgcactta tcttagccta ctgtaataag 5160

acagtaggtg agttaggtga tgttagagaa acaatgagtt acttgtttca acatgccaat 5220

ttagattctt gcaaaagagt cttgaacgtg gtgtgtaaaa cttgtggaca acagcagaca 5280

acccttaagg gtgtagaagc tgttatgtac atgggcacac tttcttatga acaatttaag 5340

aaaggtgttc agataccttg tacgtgtggt aaacaagcta caaaatatct agtacaacag 5400

gagtcacctt ttgttatgat gtcagcacca cctgctcagt atgaacttaa gcatggtaca 5460

tttacttgtg ctagtgagta cactggtaat taccagtgtg gtcactataa acatataact 5520

tctaaagaaa ctttgtattg catagacggt gctttactta caaagtcctc agaatacaaa 5580

ggtcctatta cggatgtttt ctacaaagaa aacagttaca caacaaccat aaaaccagtt 5640

acttataaat tggatggtgt tgtttgtaca gaaattgacc ctaagttgga caattattat 5700

aagaaagaca attcttattt cacagagcaa ccaattgatc ttgtaccaaa ccaaccatat 5760

ccaaacgcaa gcttcgataa ttttaagttt gtatgtgata atatcaaatt tgctgatgat 5820

ttaaaccagt taactggtta taagaaacct gcttcaagag agcttaaagt tacatttttc 5880

cctgacttaa atggtgatgt ggtggctatt gattataaac actacacacc ctcttttaag 5940

aaaggagcta aattgttaca taaacctatt gtttggcatg ttaacaatgc aactaataaa 6000

gccacgtata aaccaaatac ctggtgtata cgttgtcttt ggagcacaaa accagttgaa 6060

acatcaaatt cgtttgatgt actgaagtca gaggacgcgc agggaatgga taatcttgcc 6120

tgcgaagatc taaaaccagt ctctgaagaa gtagtggaaa atcctaccat acagaaagac 6180

gttcttgagt gtaatgtgaa aactaccgaa gttgtaggag acattatact taaaccagca 6240

aataatagtt taaaaattac agaagaggtt ggccacacag atctaatggc tgcttatgta 6300

gacaattcta gtcttactat taagaaacct aatgaattat ctagagtatt aggtttgaaa 6360

acccttgcta ctcatggttt agctgctgtt aatagtgtcc cttgggatac tatagctaat 6420

tatgctaagc cttttcttaa caaagttgtt agtacaacta ctaacatagt tacacggtgt 6480

ttaaaccgtg tttgtactaa ttatatgcct tatttcttta ctttattgct acaattgtgt 6540

acttttacta gaagtacaaa ttctagaatt aaagcatcta tgccgactac tatagcaaag 6600

aatactgtta agagtgtcgg taaattttgt ctagaggctt catttaatta tttgaagtca 6660

cctaattttt ctaaactgat aaatattata atttggtttt tactattaag tgtttgccta 6720

ggttctttaa tctactcaac cgctgcttta ggtgttttaa tgtctaattt aggcatgcct 6780

tcttactgta ctggttacag agaaggctat ttgaactcta ctaatgtcac tattgcaacc 6840

tactgtactg gttctatacc ttgtagtgtt tgtcttagtg gtttagattc tttagacacc 6900

tatccttctt tagaaactat acaaattacc atttcatctt ttaaatggga tttaactgct 6960

tttggcttag ttgcagagtg gtttttggca tatattcttt tcactaggtt tttctatgta 7020

cttggattgg ctgcaatcat gcaattgttt ttcagctatt ttgcagtaca ttttattagt 7080

aattcttggc ttatgtggtt aataattaat cttgtacaaa tggccccgat ttcagctatg 7140

gttagaatgt acatcttctt tgcatcattt tattatgtat ggaaaagtta tgtgcatgtt 7200

gtagacggtt gtaattcatc aacttgtatg atgtgttaca aacgtaatag agcaacaaga 7260

gtcgaatgta caactattgt taatggtgtt agaaggtcct tttatgtcta tgctaatgga 7320

ggtaaaggct tttgcaaact acacaattgg aattgtgtta attgtgatac attctgtgct 7380

ggtagtacat ttattagtga tgaagttgcg agagacttgt cactacagtt taaaagacca 7440

ataaatccta ctgaccagtc ttcttacatc gttgatagtg ttacagtgaa gaatggttcc 7500

atccatcttt actttgataa agctggtcaa aagacttatg aaagacattc tctctctcat 7560

tttgttaact tagacaacct gagagctaat aacactaaag gttcattgcc tattaatgtt 7620

atagtttttg atggtaaatc aaaatgtgaa gaatcatctg caaaatcagc gtctgtttac 7680

tacagtcagc ttatgtgtca acctatactg ttactagatc aggcattagt gtctgatgtt 7740

ggtgatagtg cggaagttgc agttaaaatg tttgatgctt acgttaatac gttttcatca 7800

acttttaacg taccaatgga aaaactcaaa acactagttg caactgcaga agctgaactt 7860

gcaaagaatg tgtccttaga caatgtctta tctactttta tttcagcagc tcggcaaggg 7920

tttgttgatt cagatgtaga aactaaagat gttgttgaat gtcttaaatt gtcacatcaa 7980

tctgacatag aagttactgg cgatagttgt aataactata tgctcaccta taacaaagtt 8040

gaaaacatga caccccgtga ccttggtgct tgtattgact gtagtgcgcg tcatattaat 8100

gcgcaggtag caaaaagtca caacattgct ttgatatgga acgttaaaga tttcatgtca 8160

ttgtctgaac aactacgaaa acaaatacgt agtgctgcta aaaagaataa cttacctttt 8220

aagttgacat gtgcaactac tagacaagtt gttaatgttg taacaacaaa gatagcactt 8280

aagggtggta aaattgttaa taattggttg aagcagttaa ttaaagttac acttgtgttc 8340

ctttttgttg ctgctatttt ctatttaata acacctgttc atgtcatgtc taaacatact 8400

gacttttcaa gtgaaatcat aggatacaag gctattgatg gtggtgtcac tcgtgacata 8460

gcatctacag atacttgttt tgctaacaaa catgctgatt ttgacacatg gtttagccag 8520

cgtggtggta gttatactaa tgacaaagct tgcccattga ttgctgcagt cataacaaga 8580

gaagtgggtt ttgtcgtgcc tggtttgcct ggcacgatat tacgcacaac taatggtgac 8640

tttttgcatt tcttacctag agtttttagt gcagttggta acatctgtta cacaccatca 8700

aaacttatag agtacactga ctttgcaaca tcagcttgtg ttttggctgc tgaatgtaca 8760

atttttaaag atgcttctgg taagccagta ccatattgtt atgataccaa tgtactagaa 8820

ggttctgttg cttatgaaag tttacgccct gacacacgtt atgtgctcat ggatggctct 8880

attattcaat ttcctaacac ctaccttgaa ggttctgtta gagtggtaac aacttttgat 8940

tctgagtact gtaggcacgg cacttgtgaa agatcagaag ctggtgtttg tgtatctact 9000

agtggtagat gggtacttaa caatgattat tacagatctt taccaggagt tttctgtggt 9060

gtagatgctg taaatttact tactaatatg tttacaccac taattcaacc tattggtgct 9120

ttggacatat cagcatctat agtagctggt ggtattgtag ctatcgtagt aacatgcctt 9180

gcctactatt ttatgaggtt tagaagagct tttggtgaat acagtcatgt agttgccttt 9240

aatactttac tattccttat gtcattcact gtactctgtt taacaccagt ttactcattc 9300

ttacctggtg tttattctgt tatttacttg tacttgacat tttatcttac taatgatgtt 9360

tcttttttag cacatattca gtggatggtt atgttcacac ctttagtacc tttctggata 9420

acaattgctt atatcatttg tatttccaca aagcatttct attggttctt tagtaattac 9480

ctaaagagac gtgtagtctt taatggtgtt tcctttagta cttttgaaga agctgcgctg 9540

tgcacctttt tgttaaataa agaaatgtat ctaaagttgc gtagtgatgt gctattacct 9600

cttacgcaat ataatagata cttagctctt tataataagt acaagtattt tagtggagca 9660

atggatacaa ctagctacag agaagctgct tgttgtcatc tcgcaaaggc tctcaatgac 9720

ttcagtaact caggttctga tgttctttac caaccaccac aaacctctat cacctcagct 9780

gttttgcaga gtggttttag aaaaatggca ttcccatctg gtaaagttga gggttgtatg 9840

gtacaagtaa cttgtggtac aactacactt aacggtcttt ggcttgatga cgtagtttac 9900

tgtccaagac atgtgatctg cacctctgaa gacatgctta accctaatta tgaagattta 9960

ctcattcgta agtctaatca taatttcttg gtacaggctg gtaatgttca actcagggtt 10020

attggacatt ctatgcaaaa ttgtgtactt aagcttaagg ttgatacagc caatcctaag 10080

acacctaagt ataagtttgt tcgcattcaa ccaggacaga ctttttcagt gttagcttgt 10140

tacaatggtt caccatctgg tgtttaccaa tgtgctatga ggcccaattt cactattaag 10200

ggttcattcc ttaatggttc atgtggtagt gttggtttta acatagatta tgactgtgtc 10260

tctttttgtt acatgcacca tatggaatta ccaactggag ttcatgctgg cacagactta 10320

gaaggtaact tttatggacc ttttgttgac aggcaaacag cacaagcagc tggtacggac 10380

acaactatta cagttaatgt tttagcttgg ttgtacgctg ctgttataaa tggagacagg 10440

tggtttctca atcgatttac cacaactctt aatgacttta accttgtggc tatgaagtac 10500

aattatgaac ctctaacaca agaccatgtt gacatactag gacctctttc tgctcaaact 10560

ggaattgccg ttttagatat gtgtgcttca ttaaaagaat tactgcaaaa tggtatgaat 10620

ggacgtacca tattgggtag tgctttatta gaagatgaat ttacaccttt tgatgttgtt 10680

agacaatgct caggtgttac tttccaaagt gcagtgaaaa gaacaatcaa gggtacacac 10740

cactggttgt tactcacaat tttgacttca cttttagttt tagtccagag tactcaatgg 10800

tctttgttct tttttttgta tgaaaatgcc tttttacctt ttgctatggg tattattgct 10860

atgtctgctt ttgcaatgat gtttgtcaaa cataagcatg catttctctg tttgtttttg 10920

ttaccttctc ttgccactgt agcttatttt aatatggtct atatgcctgc tagttgggtg 10980

atgcgtatta tgacatggtt ggatatggtt gatactagtt tgtctggttt taagctaaaa 11040

gactgtgtta tgtatgcatc agctgtagtg ttactaatcc ttatgacagc aagaactgtg 11100

tatgatgatg gtgctaggag agtgtggaca cttatgaatg tcttgacact cgtttataaa 11160

gtttattatg gtaatgcttt agatcaagcc atttccatgt gggctcttat aatctctgtt 11220

acttctaact actcaggtgt agttacaact gtcatgtttt tggccagagg tattgttttt 11280

atgtgtgttg agtattgccc tattttcttc ataactggta atacacttca gtgtataatg 11340

ctagtttatt gtttcttagg ctatttttgt acttgttact ttggcctctt ttgtttactc 11400

aaccgctact ttagactgac tcttggtgtt tatgattact tagtttctac acaggagttt 11460

agatatatga attcacaggg actactccca cccaagaata gcatagatgc cttcaaactc 11520

aacattaaat tgttgggtgt tggtggcaaa ccttgtatca aagtagccac tgtacagtct 11580

aaaatgtcag atgtaaagtg cacatcagta gtcttactct cagttttgca acaactcaga 11640

gtagaatcat catctaaatt gtgggctcaa tgtgtccagt tacacaatga cattctctta 11700

gctaaagata ctactgaagc ctttgaaaaa atggtttcac tactttctgt tttgctttcc 11760

atgcagggtg ctgtagacat aaacaagctt tgtgaagaaa tgctggacaa cagggcaacc 11820

ttacaagcta tagcctcaga gtttagttcc cttccatcat atgcagcttt tgctactgct 11880

caagaagctt atgagcaggc tgttgctaat ggtgattctg aagttgttct taaaaagttg 11940

aagaagtctt tgaatgtggc taaatctgaa tttgaccgtg atgcagccat gcaacgtaag 12000

ttggaaaaga tggctgatca agctatgacc caaatgtata aacaggctag atctgaggac 12060

aagagggcaa aagttactag tgctatgcag acaatgcttt tcactatgct tagaaagttg 12120

gataatgatg cactcaacaa cattatcaac aatgcaagag atggttgtgt tcccttgaac 12180

ataatacctc ttacaacagc agccaaacta atggttgtca taccagacta taacacatat 12240

aaaaatacgt gtgatggtac aacatttact tatgcatcag cattgtggga aatccaacag 12300

gttgtagatg cagatagtaa aattgttcaa cttagtgaaa ttagtatgga caattcacct 12360

aatttagcat ggcctcttat tgtaacagct ttaagggcca attctgctgt caaattacag 12420

aataatgagc ttagtcctgt tgcactacga cagatgtctt gtgctgccgg tactacacaa 12480

actgcttgca ctgatgacaa tgcgttagct tactacaaca caacaaaggg aggtaggttt 12540

gtacttgcac tgttatccga tttacaggat ttgaaatggg ctagattccc taagagtgat 12600

ggaactggta ctatctatac agaactggaa ccaccttgta ggtttgttac agacacacct 12660

aaaggtccta aagtgaagta tttatacttt attaaaggat taaacaacct aaatagaggt 12720

atggtacttg gtagtttagc tgccacagta cgtctacaag ctggtaatgc aacagaagtg 12780

cctgccaatt caactgtatt atctttctgt gcttttgctg tagatgctgc taaagcttac 12840

aaagattatc tagctagtgg gggacaacca atcactaatt gtgttaagat gttgtgtaca 12900

cacactggta ctggtcaggc aataacagtt acaccggaag ccaatatgga tcaagaatcc 12960

tttggtggtg catcgtgttg tctgtactgc cgttgccaca tagatcatcc aaatcctaaa 13020

ggattttgtg acttaaaagg taagtatgta caaataccta caacttgtgc taatgaccct 13080

gtgggtttta cacttaaaaa cacagtctgt accgtctgcg gtatgtggaa aggttatggc 13140

tgtagttgtg atcaactccg cgaacccatg cttcagtcag ctgatgcaca atcgttttta 13200

aacgggtttg cggtgtaagt gcagcccgtc ttacaccgtg cggcacaggc actagtactg 13260

atgtcgtata cagggctttt gacatctaca atgataaagt agctggtttt gctaaattcc 13320

taaaaactaa ttgttgtcgc ttccaagaaa aggacgaaga tgacaattta attgattctt 13380

actttgtagt taagagacac actttctcta actaccaaca tgaagaaaca atttataatt 13440

tacttaagga ttgtccagct gttgctaaac atgacttctt taagtttaga atagacggtg 13500

acatggtacc acatatatca cgtcaacgtc ttactaaata cacaatggca gacctcgtct 13560

atgctttaag gcattttgat gaaggtaatt gtgacacatt aaaagaaata cttgtcacat 13620

acaattgttg tgatgatgat tatttcaata aaaaggactg gtatgatttt gtagaaaacc 13680

cagatatatt acgcgtatac gccaacttag gtgaacgtgt acgccaagct ttgttaaaaa 13740

cagtacaatt ctgtgatgcc atgcgaaatg ctggtattgt tggtgtactg acattagata 13800

atcaagatct caatggtaac tggtatgatt tcggtgattt catacaaacc acgccaggta 13860

gtggagttcc tgttgtagat tcttattatt cattgttaat gcctatatta accttgacca 13920

gggctttaac tgcagagtca catgttgaca ctgacttaac aaagccttac attaagtggg 13980

atttgttaaa atatgacttc acggaagaga ggttaaaact ctttgaccgt tattttaaat 14040

attgggatca gacataccac ccaaattgtg ttaactgttt ggatgacaga tgcattctgc 14100

attgtgcaaa ctttaatgtt ttattctcta cagtgttccc acctacaagt tttggaccac 14160

tagtgagaaa aatatttgtt gatggtgttc catttgtagt ttcaactgga taccacttca 14220

gagagctagg tgttgtacat aatcaggatg taaacttaca tagctctaga cttagtttta 14280

aggaattact tgtgtatgct gctgaccctg ctatgcacgc tgcttctggt aatctattac 14340

tagataaacg cactacgtgc ttttcagtag ctgcacttac taacaatgtt gcttttcaaa 14400

ctgtcaaacc cggtaatttt aacaaagact tctatgactt tgctgtgtct aagggtttct 14460

ttaaggaagg aagttctgtt gaattaaaac acttcttctt tgctcaggat ggtaatgctg 14520

ctatcagcga ttatgactac tatcgttata atctaccaac aatgtgtgat atcagacaac 14580

tactatttgt agttgaagtt gttgataagt actttgattg ttacgatggt ggctgtatta 14640

atgctaacca agtcatcgtc aacaacctag acaaatcagc tggttttcca tttaataaat 14700

ggggtaaggc tagactttat tatgattcaa tgagttatga ggatcaagat gcacttttcg 14760

catatacaaa acgtaatgtc atccctacta taactcaaat gaatcttaag tatgccatta 14820

gtgcaaagaa tagagctcgc accgtagctg gtgtctctat ctgtagtact atgaccaata 14880

gacagtttca tcaaaaatta ttgaaatcaa tagccgccac tagaggagct actgtagtaa 14940

ttggaacaag caaattctat ggtggttggc acaacatgtt aaaaactgtt tatagtgatg 15000

tagaaaaccc tcaccttatg ggttgggatt atcctaaatg tgatagagcc atgcctaaca 15060

tgcttagaat tatggcctca cttgttcttg ctcgcaaaca tacaacgtgt tgtagcttgt 15120

cacaccgttt ctatagatta gctaatgagt gtgctcaagt attgagtgaa atggtcatgt 15180

gtggcggttc actatatgtt aaaccaggtg gaacctcatc aggagatgcc acaactgctt 15240

atgctaatag tgtttttaac atttgtcaag ctgtcacggc caatgttaat gcacttttat 15300

ctactgatgg taacaaaatt gccgataagt atgtccgcaa tttacaacac agactttatg 15360

agtgtctcta tagaaataga gatgttgaca cagactttgt gaatgagttt tacgcatatt 15420

tgcgtaaaca tttctcaatg atgatactct ctgacgatgc tgttgtgtgt ttcaatagca 15480

cttatgcatc tcaaggtcta gtggctagca taaagaactt taagtcagtt ctttattatc 15540

aaaacaatgt ttttatgtct gaagcaaaat gttggactga gactgacctt actaaaggac 15600

ctcatgaatt ttgctctcaa catacaatgc tagttaaaca gggtgatgat tatgtgtacc 15660

ttccttaccc agatccatca agaatcctag gggccggctg ttttgtagat gatatcgtaa 15720

aaacagatgg tacacttatg attgaacggt tcgtgtcttt agctatagat gcttacccac 15780

ttactaaaca tcctaatcag gagtatgctg atgtctttca tttgtactta caatacataa 15840

gaaagctaca tgatgagtta acaggacaca tgttagacat gtattctgtt atgcttacta 15900

atgataacac ttcaaggtat tgggaacctg agttttatga ggctatgtac acaccgcata 15960

cagtcttaca ggctgttggg gcttgtgttc tttgcaattc acagacttca ttaagatgtg 16020

gtgcttgcat acgtagacca ttcttatgtt gtaaatgctg ttacgaccat gtcatatcaa 16080

catcacataa attagtcttg tctgttaatc cgtatgtttg caatgctcca ggttgtgatg 16140

tcacagatgt gactcaactt tacttaggag gtatgagcta ttattgtaaa tcacataaac 16200

cacccattag ttttccattg tgtgctaatg gacaagtttt tggtttatat aaaaatacat 16260

gtgttggtag cgataatgtt actgacttta atgcaattgc aacatgtgac tggacaaatg 16320

ctggtgatta cattttagct aacacctgta ctgaaagact caagcttttt gcagcagaaa 16380

cgctcaaagc tactgaggag acatttaaac tgtcttatgg tattgctact gtacgtgaag 16440

tgctgtctga cagagaatta catctttcat gggaagttgg taaacctaga ccaccactta 16500

accgaaatta tgtctttact ggttatcgtg taactaaaaa cagtaaagta caaataggag 16560

agtacacctt tgaaaaaggt gactatggtg atgctgttgt ttaccgaggt acaacaactt 16620

acaaattaaa tgttggtgat tattttgtgc tgacatcaca tacagtaatg ccattaagtg 16680

cacctacact agtgccacaa gagcactatg ttagaattac tggcttatac ccaacactca 16740

atatctcaga tgagttttct agcaatgttg caaattatca aaaggttggt atgcaaaagt 16800

attctacact ccagggacca cctggtactg gtaagagtca ttttgctatt ggcctagctc 16860

tctactaccc ttctgctcgc atagtgtata cagcttgctc tcatgccgct gttgatgcac 16920

tatgtgagaa ggcattaaaa tatttgccta tagataaatg tagtagaatt atacctgcac 16980

gtgctcgtgt agagtgtttt gataaattca aagtgaattc aacattagaa cagtatgtct 17040

tttgtactgt aaatgcattg cctgagacga cagcagatat agttgtcttt gatgaaattt 17100

caatggccac aaattatgat ttgagtgttg tcaatgccag attacgtgct aagcactatg 17160

tgtacattgg cgaccctgct caattacctg caccacgcac attgctaact aagggcacac 17220

tagaaccaga atatttcaat tcagtgtgta gacttatgaa aactataggt ccagacatgt 17280

tcctcggaac ttgtcggcgt tgtcctgctg aaattgttga cactgtgagt gctttggttt 17340

atgataataa gcttaaagca cataaagaca aatcagctca atgctttaaa atgttttata 17400

agggtgttat cacgcatgat gtttcatctg caattaacag gccacaaata ggcgtggtaa 17460

gagaattcct tacacgtaac cctgcttgga gaaaagctgt ctttatttca ccttataatt 17520

cacagaatgc tgtagcctca aagattttgg gactaccaac tcaaactgtt gattcatcac 17580

agggctcaga atatgactat gtcatattca ctcaaaccac tgaaacagct cactcttgta 17640

atgtaaacag atttaatgtt gctattacca gagcaaaagt aggcatactt tgcataatgt 17700

ctgatagaga cctttatgac aagttgcaat ttacaagtct tgaaattcca cgtaggaatg 17760

tggcaacttt acaagctgaa aatgtaacag gactctttaa agattgtagt aaggtaatca 17820

ctgggttaca tcctacacag gcacctacac acctcagtgt tgacactaaa ttcaaaactg 17880

aaggtttatg tgttgacata cctggcatac ctaaggacat gacctataga agactcatct 17940

ctatgatggg ttttaaaatg aattatcaag ttaatggtta ccctaacatg tttatcaccc 18000

gcgaagaagc tataagacat gtacgtgcat ggattggctt cgatgtcgag gggtgtcatg 18060

ctactagaga agctgttggt accaatttac ctttacagct aggtttttct acaggtgtta 18120

acctagttgc tgtacctaca ggttatgttg atacacctaa taatacagat ttttccagag 18180

ttagtgctaa accaccgcct ggagatcaat ttaaacacct cataccactt atgtacaaag 18240

gacttccttg gaatgtagtg cgtataaaga ttgtacaaat gttaagtgac acacttaaaa 18300

atctctctga cagagtcgta tttgtcttat gggcacatgg ctttgagttg acatctatga 18360

agtattttgt gaaaatagga cctgagcgca cctgttgtct atgtgataga cgtgccacat 18420

gcttttccac tgcttcagac acttatgcct gttggcatca ttctattgga tttgattacg 18480

tctataatcc gtttatgatt gatgttcaac aatggggttt tacaggtaac ctacaaagca 18540

accatgatct gtattgtcaa gtccatggta atgcacatgt agctagttgt gatgcaatca 18600

tgactaggtg tctagctgtc cacgagtgct ttgttaagcg tgttgactgg actattgaat 18660

atcctataat tggtgatgaa ctgaagatta atgcggcttg tagaaaggtt caacacatgg 18720

ttgttaaagc tgcattatta gcagacaaat tcccagttct tcacgacatt ggtaacccta 18780

aagctattaa gtgtgtacct caagctgatg tagaatggaa gttctatgat gcacagcctt 18840

gtagtgacaa agcttataaa atagaagaat tattctattc ttatgccaca cattctgaca 18900

aattcacaga tggtgtatgc ctattttgga attgcaatgt cgatagatat cctgctaatt 18960

ccattgtttg tagatttgac actagagtgc tatctaacct taacttgcct ggttgtgatg 19020

gtggcagttt gtatgtaaat aaacatgcat tccacacacc agcttttgat aaaagtgctt 19080

ttgttaattt aaaacaatta ccatttttct attactctga cagtccatgt gagtctcatg 19140

gaaaacaagt agtgtcagat atagattatg taccactaaa gtctgctacg tgtataacac 19200

gttgcaattt aggtggtgct gtctgtagac atcatgctaa tgagtacaga ttgtatctcg 19260

atgcttataa catgatgatc tcagctggct ttagcttgtg ggtttacaaa caatttgata 19320

cttataacct ctggaacact tttacaagac ttcagagttt agaaaatgtg gcttttaatg 19380

ttgtaaataa gggacacttt gatggacaac agggtgaagt accagtttct atcattaata 19440

acactgttta cacaaaagtt gatggtgttg atgtagaatt gtttgaaaat aaaacaacat 19500

tacctgttaa tgtagcattt gagctttggg ctaagcgcaa cattaaacca gtaccagagg 19560

tgaaaatact caataatttg ggtgtggaca ttgctgctaa tactgtgatc tgggactaca 19620

aaagagatgc tccagcacat atatctacta ttggtgtttg ttctatgact gacatagcca 19680

agaaaccaac tgaaacgatt tgtgcaccac tcactgtctt ttttgatggt agagttgatg 19740

gtcaagtaga cttatttaga aatgcccgta atggtgttct tattacagaa ggtagtgtta 19800

aaggtttaca accatctgta ggtcccaaac aagctagtct taatggagtc acattaattg 19860

gagaagccgt aaaaacacag ttcaattatt ataagaaagt tgatggtgtt gtccaacaat 19920

tacctgaaac ttactttact cagagtagaa atttacaaga atttaaaccc aggagtcaaa 19980

tggaaattga tttcttagaa ttagctatgg atgaattcat tgaacggtat aaattagaag 20040

gctatgcctt cgaacatatc gtttatggag attttagtca tagtcagtta ggtggtttac 20100

atctactgat tggactagct aaacgtttta aggaatcacc ttttgaatta gaagatttta 20160

ttcctatgga cagtacagtt aaaaactatt tcataacaga tgcgcaaaca ggttcatcta 20220

agtgtgtgtg ttctgttatt gatttattac ttgatgattt tgttgaaata ataaaatccc 20280

aagatttatc tgtagtttct aaggttgtca aagtgactat tgactataca gaaatttcat 20340

ttatgctttg gtgtaaagat ggccatgtag aaacatttta cccaaaatta caatctagtc 20400

aagcgtggca accgggtgtt gctatgccta atctttacaa aatgcaaaga atgctattag 20460

aaaagtgtga ccttcaaaat tatggtgata gtgcaacatt acctaaaggc ataatgatga 20520

atgtcgcaaa atatactcaa ctgtgtcaat atttaaacac attaacatta gctgtaccct 20580

ataatatgag agttatacat tttggtgctg gttctgataa aggagttgca ccaggtacag 20640

ctgttttaag acagtggttg cctacgggta cgctgcttgt cgattcagat cttaatgact 20700

ttgtctctga tgcagattca actttgattg gtgattgtgc aactgtacat acagctaata 20760

aatgggatct cattattagt gatatgtacg accctaagac taaaaatgtt acaaaagaaa 20820

atgactctaa agagggtttt ttcacttaca tttgtgggtt tatacaacaa aagctagctc 20880

ttggaggttc cgtggctata aagataacag aacattcttg gaatgctgat ctttataagc 20940

tcatgggaca cttcgcatgg tggacagcct ttgttactaa tgtgaatgcg tcatcatctg 21000

aagcattttt aattggatgt aattatcttg gcaaaccacg cgaacaaata gatggttatg 21060

tcatgcatgc aaattacata ttttggagga atacaaatcc aattcagttg tcttcctatt 21120

ctttatttga catgagtaaa tttcccctta aattaagggg tactgctgtt atgtctttaa 21180

aagaaggtca aatcaatgat atgattttat ctcttcttag taaaggtaga cttataatta 21240

gagaaaacaa cagagttgtt atttctagtg atgttcttgt taacaactaa 21290

<210> 36
<211> 154
<212> RNA
<213> artificial

<220>
<223> Artificial

<400> 36
ggcgccgucu uguugaccaa caguuuguug acuaaauaac gguuaacaag cuguuucaga 60

gaggacgcaa auaaaguccu cuuuggagua guuuguugau uuacuuuaag gagggcaaaa 120

ugggaagcuu cgaucccgcc gucguuuuac aacg 154

<210> 37
<211> 154
<212> RNA
<213> artificial

<220>
<223> Artificial

<400> 37
ggcgccgucu uguugaccaa caguuuguug acuaaauaac gguuaacaag cuguuucaga 60

gaggacgcaa auaaaguccu cuuuggagua guuuguugau uuacuuuaag gagggcaaaa 120

ugggaagcuu cgaucccgcc gucguuuuac aacg 154

<210> 38
<211> 30
<212> RNA
<213> severe acute respiratory syndrome coronavirus 2 isolate wuhan-hu-1 (sars-cov-2)

<400> 38
agucaacaaa cuguugguca acaagacggc 30

<210> 39
<211> 181
<212> DNA
<213> artificial

<220>
<223> Artificial

<400> 39
tcataagctt taatacgact cactataggc gccgtcttgt tgaccaacag tttgttgact 60

aaataacggt taacaagctg tttcagagag gacgcaaata aagtcctctt tggagtagtt 120

tgttgattta ctttaaggag ggcaaaatgg gaagcttcga tcccgccgtc gttttacaac 180

g 181

<210> 40
<211> 181
<212> DNA
<213> artificial

<220>
<223> Artificial

<400> 40
tcataagctt taatacgact cactataggc gccgtcttgt tgaccaacag tttgttgact 60

aaataacggt taacaagctg tttcaaagag gacgcaaata aagtcctctt tggagtagtt 120

tgttgattta ctttaaggag ggcaaaatgg gaagcttcga tcccgccgtc gttttacaac 180

g 181

<210> 41
<211> 3247
<212> DNA
<213> artificial

<220>
<223> Artificial

<400> 41
tcataagctt taatacgact cactataggc gccgtcttgt tgaccaacag tttgttgact 60

aaataacggt taacaagctg tttcagagag gacgcaaata aagtcctctt tggagtagtt 120

tgttgattta ctttaaggag ggcaaaatgg gaagcttcga tcccgccgtc gttttacaac 180

gtcgtgactg ggaaaaccct ggcgttaccc aacttaatcg ccttgcagca catccccctt 240

tcgccagctg gcgtaatagc gaagaggccc gcaccgatcg cccttcccaa cagttgcgca 300

gcctgaatgg cgaatggcgc tttgcctggt ttccggcacc agaagcggtg ccggaaagct 360

ggctggagtg cgatcttcct gaggccgata ctgtcgtcgt cccctcaaac tggcagatgc 420

acggttacga tgcgcccatc tacaccaacg tgacctatcc cattacggtc aatccgccgt 480

ttgttcccac ggagaatccg acgggttgtt actcgctcac atttaatgtt gatgaaagct 540

ggctacagga aggccagacg cgaattattt ttgatggcgt taactcggcg tttcatctgt 600

ggtgcaacgg gcgctgggtc ggttacggcc aggacagtcg tttgccgtct gaatttgacc 660

tgagcgcatt tttacgcgcc ggagaaaacc gcctcgcggt gatggtgctg cgctggagtg 720

acggcagtta tctggaagat caggatatgt ggcggatgag cggcattttc cgtgacgtct 780

cgttgctgca taaaccgact acacaaatca gcgatttcca tgttgccact cgctttaatg 840

atgatttcag ccgcgctgta ctggaggctg aagttcagat gtgcggcgag ttgcgtgact 900

acctacgggt aacagtttct ttatggcagg gtgaaacgca ggtcgccagc ggcaccgcgc 960

ctttcggcgg tgaaattatc gatgagcgtg gtggttatgc cgatcgcgtc acactacgtc 1020

tgaacgtcga aaacccgaaa ctgtggagcg ccgaaatccc gaatctctat cgtgcggtgg 1080

ttgaactgca caccgccgac ggcacgctga ttgaagcaga agcctgcgat gtcggtttcc 1140

gcgaggtgcg gattgaaaat ggtctgctgc tgctgaacgg caagccgttg ctgattcgag 1200

gcgttaaccg tcacgagcat catcctctgc atggtcaggt catggatgag cagacgatgg 1260

tgcaggatat cctgctgatg aagcagaaca actttaacgc cgtgcgctgt tcgcattatc 1320

cgaaccatcc gctgtggtac acgctgtgcg accgctacgg cctgtatgtg gtggatgaag 1380

ccaatattga aacccacggc atggtgccaa tgaatcgtct gaccgatgat ccgcgctggc 1440

taccggcgat gagcgaacgc gtaacgcgaa tggtgcagcg cgatcgtaat cacccgagtg 1500

tgatcatctg gtcgctgggg aatgaatcag gccacggcgc taatcacgac gcgctgtatc 1560

gctggatcaa atctgtcgat ccttcccgcc cggtgcagta tgaaggcggc ggagccgaca 1620

ccacggccac cgatattatt tgcccgatgt acgcgcgcgt ggatgaagac cagcccttcc 1680

cggctgtgcc gaaatggtcc atcaaaaaat ggctttcgct acctggagag acgcgcccgc 1740

tgatcctttg cgaatacgcc cacgcgatgg gtaacagtct tggcggtttc gctaaatact 1800

ggcaggcgtt tcgtcagtat ccccgtttac agggcggctt cgtctgggac tgggtggatc 1860

agtcgctgat taaatatgat gaaaacggca acccgtggtc ggcttacggc ggtgattttg 1920

gcgatacgcc gaacgatcgc cagttctgta tgaacggtct ggtctttgcc gaccgcacgc 1980

cgcatccagc gctgacggaa gcaaaacacc agcagcagtt tttccagttc cgtttatccg 2040

ggcaaaccat cgaagtgacc agcgaatacc tgttccgtca tagcgataac gagctcctgc 2100

actggatggt ggcgctggat ggtaagccgc tggcaagcgg tgaagtgcct ctggatgtcg 2160

ctccacaagg taaacagttg attgaactgc ctgaactacc gcagccggag agcgccgggc 2220

aactctggct cacagtacgc gtagtgcaac cgaacgcgac cgcatggtca gaagccgggc 2280

acatcagcgc ctggcagcag tggcgtctgg cggaaaacct cagtgtgacg ctccccgccg 2340

cgtcccacgc catcccgcat ctgaccacca gcgaaatgga tttttgcatc gagctgggta 2400

ataagcgttg gcaatttaac cgccagtcag gctttctttc acagatgtgg attggcgata 2460

aaaaacaact gctgacgccg ctgcgcgatc agttcacccg tgcaccgctg gataacgaca 2520

ttggcgtaag tgaagcgacc cgcattgacc ctaacgcctg ggtcgaacgc tggaaggcgg 2580

cgggccatta ccaggccgaa gcagcgttgt tgcagtgcac ggcagataca cttgctgatg 2640

cggtgctgat tacgaccgct cacgcgtggc agcatcaggg gaaaacctta tttatcagcc 2700

ggaaaaccta ccggattgat ggtagtggtc aaatggcgat taccgttgat gttgaagtgg 2760

cgagcgatac accgcatccg gcgcggattg gcctgaactg ccagctggcg caggtagcag 2820

agcgggtaaa ctggctcgga ttagggccgc aagaaaacta tcccgaccgc cttactgccg 2880

cctgttttga ccgctgggat ctgccattgt cagacatgta taccccgtac gtcttcccga 2940

gcgaaaacgg tctgcgctgc gggacgcgcg aattgaatta tggcccacac cagtggcgcg 3000

gcgacttcca gttcaacatc agccgctaca gtcaacagca actgatggaa accagccatc 3060

gccatctgct gcacgcggaa gaaggcacat ggctgaatat cgacggtttc catatgggga 3120

ttggtggcga cgactcctgg agcccgtcag tatcggcgga attccagctg agcgccggtc 3180

gctaccatta ccagttggtc tggtgtcaaa aataactagc ataacccctc tctaaacgga 3240

ggggttt 3247

<210> 42
<211> 3247
<212> DNA
<213> artificial

<220>
<223>  Artificial

<400>  42
tcataagctt taatacgact cactataggc gccgtcttgt tgaccaacag tttgttgact      60

aaataacggt taacaagctg tttcaaagag gacgcaaata aagtcctctt tggagtagtt     120

tgttgattta ctttaaggag ggcaaaatgg gaagcttcga tcccgccgtc gttttacaac     180

gtcgtgactg ggaaaaccct ggcgttaccc aacttaatcg ccttgcagca catccccctt     240

tcgccagctg gcgtaatagc gaagaggccc gcaccgatcg cccttcccaa cagttgcgca     300

gcctgaatgg cgaatggcgc tttgcctggt ttccggcacc agaagcggtg ccggaaagct     360

ggctggagtg cgatcttcct gaggccgata ctgtcgtcgt cccctcaaac tggcagatgc     420

acggttacga tgcgcccatc tacaccaacg tgacctatcc cattacggtc aatccgccgt     480

ttgttcccac ggagaatccg acgggttgtt actcgctcac atttaatgtt gatgaaagct     540

ggctacagga aggccagacg cgaattattt ttgatggcgt taactcggcg tttcatctgt     600

ggtgcaacgg gcgctgggtc ggttacggcc aggacagtcg tttgccgtct gaatttgacc     660

tgagcgcatt tttacgcgcc ggagaaaacc gcctcgcggt gatggtgctg cgctggagtg     720

acggcagtta tctggaagat caggatatgt ggcggatgag cggcattttc cgtgacgtct     780

cgttgctgca taaaccgact acacaaatca gcgatttcca tgttgccact cgctttaatg     840

atgatttcag ccgcgctgta ctggaggctg aagttcagat gtgcggcgag ttgcgtgact     900

acctacgggt aacagtttct ttatggcagg gtgaaacgca ggtcgccagc ggcaccgcgc     960

ctttcggcgg tgaaattatc gatgagcgtg gtggttatgc cgatcgcgtc acactacgtc    1020

tgaacgtcga aaacccgaaa ctgtggagcg ccgaaatccc gaatctctat cgtgcggtgg    1080

ttgaactgca caccgccgac ggcacgctga ttgaagcaga agcctgcgat gtcggtttcc    1140

gcgaggtgcg gattgaaaat ggtctgctgc tgctgaacgg caagccgttg ctgattcgag    1200

gcgttaaccg tcacgagcat catcctctgc atggtcaggt catggatgag cagacgatgg    1260

tgcaggatat cctgctgatg aagcagaaca actttaacgc cgtgcgctgt tcgcattatc    1320

cgaaccatcc gctgtggtac acgctgtgcg accgctacgg cctgtatgtg gtggatgaag    1380

ccaatattga aacccacggc atggtgccaa tgaatcgtct gaccgatgat ccgcgctggc    1440

taccggcgat gagcgaacgc gtaacgcgaa tggtgcagcg cgatcgtaat cacccgagtg    1500

tgatcatctg gtcgctgggg aatgaatcag gccacggcgc taatcacgac gcgctgtatc    1560

gctggatcaa atctgtcgat ccttcccgcc cggtgcagta tgaaggcggc ggagccgaca    1620

ccacggccac cgatattatt tgcccgatgt acgcgcgcgt ggatgaagac cagcccttcc    1680

cggctgtgcc gaaatggtcc atcaaaaaat ggctttcgct acctggagag acgcgcccgc    1740

tgatcctttg cgaatacgcc cacgcgatgg gtaacagtct tggcggtttc gctaaatact 1800

ggcaggcgtt tcgtcagtat ccccgtttac agggcggctt cgtctgggac tgggtggatc 1860

agtcgctgat taaatatgat gaaaacggca acccgtggtc ggcttacggc ggtgattttg 1920

gcgatacgcc gaacgatcgc cagttctgta tgaacggtct ggtctttgcc gaccgcacgc 1980

cgcatccagc gctgacggaa gcaaaacacc agcagcagtt tttccagttc cgtttatccg 2040

ggcaaaccat cgaagtgacc agcgaatacc tgttccgtca tagcgataac gagctcctgc 2100

actggatggt ggcgctggat ggtaagccgc tggcaagcgg tgaagtgcct ctggatgtcg 2160

ctccacaagg taaacagttg attgaactgc ctgaactacc gcagccggag agcgccgggc 2220

aactctggct cacagtacgc gtagtgcaac cgaacgcgac cgcatggtca gaagccgggc 2280

acatcagcgc ctggcagcag tggcgtctgg cggaaaacct cagtgtgacg ctccccgccg 2340

cgtcccacgc catcccgcat ctgaccacca gcgaaatgga tttttgcatc gagctgggta 2400

ataagcgttg gcaatttaac cgccagtcag gctttctttc acagatgtgg attggcgata 2460

aaaaacaact gctgacgccg ctgcgcgatc agttcacccg tgcaccgctg gataacgaca 2520

ttggcgtaag tgaagcgacc cgcattgacc ctaacgcctg ggtcgaacgc tggaaggcgg 2580

cgggccatta ccaggccgaa gcagcgttgt tgcagtgcac ggcagataca cttgctgatg 2640

cggtgctgat tacgaccgct cacgcgtggc agcatcaggg gaaaacctta tttatcagcc 2700

ggaaaaccta ccggattgat ggtagtggtc aaatggcgat taccgttgat gttgaagtgg 2760

cgagcgatac accgcatccg gcgcggattg gcctgaactg ccagctggcg caggtagcag 2820

agcgggtaaa ctggctcgga ttagggccgc aagaaaacta tcccgaccgc cttactgccg 2880

cctgttttga ccgctgggat ctgccattgt cagacatgta taccccgtac gtcttcccga 2940

gcgaaaacgg tctgcgctgc gggacgcgcg aattgaatta tggcccacac cagtggcgcg 3000

gcgacttcca gttcaacatc agccgctaca gtcaacagca actgatggaa accagccatc 3060

gccatctgct gcacgcggaa gaaggcacat ggctgaatat cgacggtttc catatgggga 3120

ttggtggcga cgactcctgg agcccgtcag tatcggcgga attccagctg agcgccggtc 3180

gctaccatta ccagttggtc tggtgtcaaa aataactagc ataacccctc tctaaacgga 3240

ggggttt 3247

<210> 43
<211> 366
<212> DNA
<213> severe acute respiratory syndrome coronavirus 2 isolate wuhan-hu-1
(sars-cov-2)

<400> 43
atgaaatttc ttgttttctt aggaatcatc acaactgtag ctgcatttca ccaagaatgt 60

agtttacagt catgtactca acatcaacca tatgtagttg atgacccgtg tcctattcac 120

ttctattcta aatggtatat tagagtagga gctagaaaat cagcaccttt aattgaattg 180

tgcgtggatg aggctggttc taaatcaccc attcagtaca tcgatatcgg taattataca 240

gtttcctgtt taccttttac aattaattgc caggaaccta aattgggtag tcttgtagtg 300

cgttgttcgt tctatgaaga ctttttagag tatcatgacg ttcgtgttgt tttagatttc 360

atctaa 366

<210> 44
<211> 154
<212> RNA
<213> artificial

<220>
<223> Artificial

<400> 44
ggcgguuaau ggguuguugg uuagcgaggc ggcauaaauc gccgucuugc uggccuaggg 60

aggcggccag guacaucgcc uccuuagguu gguaagacgg uaacuuaagg aggcgaaaau 120

gcagaaccug gcggcagcgc aaaagaugcg uaaa 154

<210> 45
<211> 153
<212> RNA
<213> artificial

<220>
<223> Artificial

<400> 45
ggcccuauuc acuuuauucu aaaugguaua uuuaagacug guauaccguu uagauaagga 60

gggcaccggg guauuguccu ccuuguuugg acgguauauc cgaauaagga ggacaagaug 120

caaaaccugg cggcagcgca aaagaugcgu aaa 153

<210> 46
<211> 30
<212> RNA
<213> severe acute respiratory syndrome coronavirus 2 isolate wuhan-hu-1
(sars-cov-2)

<400> 46
gccgucuugu ugaccaacag uuuguugacu 30

<210> 47
<211> 30
<212> RNA
<213> severe acute respiratory syndrome coronavirus 2 isolate wuhan-hu-1
(sars-cov-2)

<400> 47
aauauaccau uuagaauaga agugaauagg 30

<210>  48
<211>  181
<212>  DNA
<213>  artificial

<220>
<223>  Artificial

<400>  48
tcataagctt taatacgact cactataggc ggttaatggg ttgttggtta gcgaggcggc      60

ataaatcgcc gtcttgctgg cctagggagg cggccaggta catcgcctcc ttaggttggt      120

aagacggtaa cttaaggagg cgaaaatgca gaacctggcg gcagcgcaaa agatgcgtaa      180

a                                                                      181


<210>  49
<211>  181
<212>  DNA
<213>  artificial

<220>
<223>  Artificial

<400>  49
tcataagctt taatacgact cactataggc cctattcact tctattctaa atggtatatt      60

taagactggt ataccgttta gataaggagg gcaccggggt attgtcctcc ttgtttggac      120

ggtatatccg aataaggagg acaagatgca aaacctggcg gcagcgcaaa agatgcgtaa      180

a                                                                      181


<210>  50
<211>  1057
<212>  DNA
<213>  artificial

<220>
<223>  Artificial

<400>  50
   tcataagctt taatacgact cactataggc ggttaatggg ttgttggtta gcgaggcggc      60
   ataaatcgcc gtcttgctgg cctagggagg cggccaggta catcgcctcc ttaggttggt      120
   aagacggtaa cttaaggagg cgaaaatgca gaacctggcg gcagcgcaaa agatgcgtaa      180
   aggagaagaa cttttcactg gagttgtccc aattcttgtt gaattagatg gtgatgttaa      240
   tgggcacaaa ttttctgtca gtggagaggg tgaaggtgat gcaacatacg gaaaacttac      300
   ccttaaattt atttgcacta ctggaaaact acctgttccg tggccaacac ttgtcactac      360
   tttcggttat ggtgttcaat gctttgcgag atacccagat cacatgaaac agcatgactt      420
   tttcaagagt gccatgcccg aaggttacgt acaggaaaga actatatttt tcaaagatga     480
   cgggaactac aagacacgtg ctgaagtcaa gtttgaaggt gatacccttg ttaatagaat     540
   cgagttaaaa ggtattgatt ttaaagaaga tggaaacatt cttggacaca aattggaata 600
   caactataac tcacacaatg tatacatcat ggcagacaaa caaaagaatg gaatcaaagt 660
   taacttcaaa attagacaca acattgaaga tggaagcgtt caactagcag accattatca 720
   acaaaatact ccgattggcg atggccctgt ccttttacca gacaaccatt acctgtccac 780
   acaatctgcc ctttcgaaag atcccaacga aaagagagac cacatggtcc ttcttgagtt 840
   tgtaaccgct gctgggatta cacatggcat ggatgaacta tacaaaaggc ctgcagcaaa 900
   cgacgaaaac tacgctgcat cagtttaata ataagataaa ccagagcggc acggcaagca 960
   gagtatacga gattcggtag ccaccgctga gcaataacta gcataacccc ttggggcctc 1020
   taaacgggtc ttgaggggtt ttttgcatat gcaacgg 1057

<210> 51
<211> 1057
<212> DNA
<213> artificial

<220>
<223> Artificial

<400> 51
   tcataagctt taatacgact cactataggc agttaagtta tttatggctg agagtggact 60
   gtaaataagt ccgctctcag tcgaaggagg cgtcttttgc aaacgcctcc tttggttgag 120
   agcggattga ctaaaggagg cgtatatgtc gaacctggcg gcagcgcaaa agatgcgtaa 180
   aggagaagaa cttttcactg gagttgtccc aattcttgtt gaattagatg gtgatgttaa 240
   tgggcacaaa ttttctgtca gtggagaggg tgaaggtgat gcaacatacg gaaaacttac 300
   ccttaaattt atttgcacta ctggaaaact acctgttccg tggccaacac ttgtcactac 360
   tttcggttat ggtgttcaat gctttgcgag atacccagat cacatgaaac agcatgactt 420
   tttcaagagt gccatgcccg aaggttacgt acaggaaaga actatatttt tcaaagatga 480
   cgggaactac aagacacgtg ctgaagtcaa gtttgaaggt gatacccttg ttaatagaat 540
   cgagttaaaa ggtattgatt ttaaagaaga tggaaacatt cttggacaca aattggaata 600
   caactataac tcacacaatg tatacatcat ggcagacaaa caaaagaatg gaatcaaagt 660
   taacttcaaa attagacaca acattgaaga tggaagcgtt caactagcag accattatca 720
   acaaaatact ccgattggcg atggccctgt ccttttacca gacaaccatt acctgtccac 780
   acaatctgcc ctttcgaaag atcccaacga aaagagagac cacatggtcc ttcttgagtt 840
   tgtaaccgct gctgggatta cacatggcat ggatgaacta tacaaaaggc ctgcagcaaa 900
   cgacgaaaac tacgctgcat cagtttaata ataagataaa ccagagcggc acggcaagca 960
   gagtatacga gattcggtag ccaccgctga gcaataacta gcataacccc ttggggcctc 1020
   taaacgggtc ttgaggggtt ttttgcatat gcaacgg 1057

<210> 52
<211> 444
<212> DNA
<213> Artificial Sequence

<220>
<223> Artificial

<400> 52
atggcaaacc tggacaagat gctgaatacc acagtaacag aggtgcggca gttcctgcag 60

gtggacagag tgtgcgtgtt ccagtttgag gaggattata gcggagtggt ggtggtggag 120

gccgtggacg ataggtggat ctccatcctg aagacccagg tgcgggatag atacttcatg 180

gagacaaggg gcgaggagta ttctcacggc cgctaccagg ccatcgccga catctacacc 240

gcaaacctga cagagtgcta cagggatctg ctgacacagt ttcaggtgag agcaatcctg 300

gccgtgccca tcctgcaggg caagaagctg tggggcctgt tggtggcaca ccagctggcg 360

gcccctagac agtggcagac ctgggagatc gactttctga agcagcaggc cgtggtggtg 420

ggcatcgcca tccagcagag ctaa 444

<210> 53
<211> 595
<212> RNA
<213> Artificial Sequence

<220>
<223> Artificial

<400> 53
ggcgguugau ggguuguugg ucaacagggc ggcgauaauc gccguucugu uggcuagcaa 60

ggaggaaaau auggcaaacc uggacaagau gcugaauacc acaguaacag aggugcggca 120

guuccugcag guggacagag ugugcguguu ccaguuugag gaggauuaua gcggaguggu 180

ggugguggag gccguggacg auagguggau cuccauccug aagacccagg ugcgggauag 240

auacuucaug gagacaaggg gcgaggagua uucucacggc cgcuaccagg ccaucgccga 300

caucuacacc gcaaaccuga cagagugcua cagggaucug cugacacagu uucaggugag 360

agcaauccug gccgugccca uccugcaggg caagaagcug uggggccugu ugguggcaca 420

ccagcuggcg gccccuagac aguggcagac cugggagauc gacuuucuga agcagcaggc 480

cgugguggug ggcaucgcca uccagcagag cuaauuuccu ccuuguuagc caauagaaug 540

gucuaacaag gaggaaacaa uguauacaac aaaccuggcg gcagcgcaaa agaug 595

<210> 54
<211> 595
<212> RNA
<213> Artificial Sequence

<220>
<223>  Artificial

<400>  54
ggcgguugau ggguuguugg ucaacagggc ggcgauaauu gccguucugu uggcuagcaa      60

ggaggaaaau auggcaaacc uggacaagau gcugaauacc acaguaacag aggugcggca     120

guuccugcag guggacagag ugugcguguu ccaguuugag gaggauuaua gcggaguggu     180

ggugguggag gccguggacg auagguggau cuccauccug aagacccagg ugcgggauag     240

auacuucaug gagacaaggg gcgaggagua uucucacggc cgcuaccagg ccaucgccga     300

caucuacacc gcaaaccuga cagagugcua cagggaucug cugacacagu uucaggugag     360

agcaauccug gccgugccca uccugcaggg caagaagcug uggggccugu ugguggcaca     420

ccagcuggcg gccccuagac aguggcagac cugggagauc gacuuucuga agcagcaggc     480

cgugguggug ggcaucgcca uccagcagag cuaauuuccu ccuuguuagc caauagaaug     540

gucuaacaag gaggaaacaa uguauacaac aaaccuggcg gcagcgcaaa agaug          595


<210>  55
<211>  115
<212>  DNA
<213>  Artificial Sequence

<220>
<223>  Artificial

<400>  55
tcataagctt taatacgact cactataggc ggttgatggg ttgttggtca acagggcggc      60

gataatcgcc gttctgttgg ctagcaagga ggaaaatatg gcaaacctgg acaag          115


<210>  56
<211>  115
<212>  DNA
<213>  Artificial Sequence

<220>
<223>  Artificial

<400>  56
tcataagctt taatacgact cactataggc ggttgatggg ttgttggtca acagggcggc      60

gataattgcc gttctgttgg ctagcaagga ggaaaatatg gcaaacctgg acaag          115


<210>  57
<211>  52
<212>  DNA
<213>  Artificial Sequence

<220>
<223>  Artificial

<400>  57

cagctggtgt gccaccaaca ggccccacag cttcttgccc tgcaggatgg gc          52

<210>  58
<211>  454
<212>  DNA
<213>  Artificial Sequence

<220>
<223>  Artificial

<400>  58
tcataagctt taatacgact cactataggc ggttgatggg ttgttggtca acagggcggc    60

gataatcgcc gttctgttgg ctagcaagga ggaaaatatg gcaaacctgg acaagatgct   120

gaataccaca gtaacagagg tgcggcagtt cctgcaggtg gacagagtgt gcgtgttcca   180

gtttgaggag gattatagcg gagtggtggt ggtggaggcc gtggacgata ggtggatctc   240

catcctgaag acccaggtgc gggatagata cttcatggag acaaggggcg aggagtattc    300

tcacggccgc taccaggcca tcgccgacat ctacaccgca aacctgacag agtgctacag   360

ggatctgctg acacagtttc aggtgagagc aatcctggcc gtgcccatcc tgcagggcaa   420

gaagctgtgg ggcctgttgg tggcacacca gctg                               454

<210>  59
<211>  454
<212>  DNA
<213>  Artificial Sequence

<220>
<223>  Artificial

<400>  59
tcataagctt taatacgact cactataggc ggttgatggg ttgttggtca acagggcggc    60

gataattgcc gttctgttgg ctagcaagga ggaaaatatg gcaaacctgg acaagatgct   120

gaataccaca gtaacagagg tgcggcagtt cctgcaggtg gacagagtgt gcgtgttcca   180

gtttgaggag gattatagcg gagtggtggt ggtggaggcc gtggacgata ggtggatctc   240

catcctgaag acccaggtgc gggatagata cttcatggag acaaggggcg aggagtattc    300

tcacggccgc taccaggcca tcgccgacat ctacaccgca aacctgacag agtgctacag   360

ggatctgctg acacagtttc aggtgagagc aatcctggcc gtgcccatcc tgcagggcaa   420

gaagctgtgg ggcctgttgg tggcacacca gctg                               454

<210>  60
<211>  200
<212>  DNA
<213>  Artificial Sequence

<220>
<223>  Artificial

<400>  60
agctgtgggg cctgttggtg gcacaccagc tggcggcccc tagacagtgg cagacctggg      60

agatcgactt tctgaagcag caggccgtgg tggtgggcat cgccatccag cagagctaat     120

ttcctccttg ttagccaata gaatggtcta acaaggagga aacaatgtat acaacaaacc     180

tggcggcagc gcaaaagatg                                                 200


<210>  61
<211>  1082
<212>  DNA
<213>  Artificial Sequence

<220>
<223>  Artificial

<400>  61
agctgtgggg cctgttggtg gcacaccagc tggcggcccc tagacagtgg cagacctggg      60

agatcgactt tctgaagcag caggccgtgg tggtgggcat cgccatccag cagagctaat     120

ttcctccttg ttagccaata gaatggtcta acaaggagga aacaatgtat acaacaaacc     180

tggcggcagc gcaaaagatg cgtaaaggag aagaactttt cactggagtt gtcccaattc     240

ttgttgaatt agatggtgat gttaatgggc acaaattttc tgtcagtgga gagggtgaag     300

gtgatgcaac atacggaaaa cttaccctta aatttatttg cactactgga aaactacctg     360

ttccgtggcc aacacttgtc actactttcg gttatggtgt tcaatgcttt gcgagatacc     420

cagatcacat gaaacagcat gactttttca agagtgccat gcccgaaggt tacgtacagg     480

aaagaactat atttttcaaa gatgacggga actacaagac acgtgctgaa gtcaagtttg     540

aaggtgatac ccttgttaat agaatcgagt taaaaggtat tgattttaaa gaagatggaa     600

acattcttgg acacaaattg gaatacaact ataactcaca caatgtatac atcatggcag     660

acaaacaaaa gaatggaatc aaagttaact tcaaaattag acacaacatt gaagatggaa     720

gcgttcaact agcagaccat tatcaacaaa atactccgat tggcgatggc cctgtccttt     780

taccagacaa ccattacctg tccacacaat ctgccctttc gaaagatccc aacgaaaaga     840

gagaccacat ggtccttctt gagtttgtaa ccgctgctgg gattacacat ggcatggatg     900

aactatacaa aaggcctgca gcaaacgacg aaaactacgc tgcatcagtt taataataag     960

ataaaccaga gcggcacggc aagcagagta tacgagattc ggtagccacc gctgagcaat    1020

aactagcata accccttggg gcctctaaac gggtcttgag gggttttttg catatgcaac    1080

gg                                                                   1082


<210>  62
<211>  1082
<212>  DNA
<213>  Artificial Sequence

<220>
<223>  Artificial

<400>  62
agctgtgggg cctgttggtg gcacaccagc tggcggcccc tagacagtgg cagacctggg      60

agatcgactt tctgaagcag caggccgtgg tggtgggcat cgccatccag cagagctaat     120

ttcctccttg ttagccaata gaatggtcta acaaggagga aacaatgtat acaacaaacc     180

tggcggcagc gcaaaagatg cgtaaaggag aagaactttt cactggagtt gtcccaattc     240

ttgttgaatt agatggtgat gttaatgggc acaaattttc tgtcagtgga gagggtgaag     300

gtgatgcaac atacggaaaa cttaccctta aatttatttg cactactgga aaactacctg     360

ttccgtggcc aacacttgtc actactttcg gttatggtgt tcaatgcttt gcgagatacc     420

cagatcacat gaaacagcat gactttttca agagtgccat gcccgaaggt tacgtacagg     480

aaagaactat atttttcaaa gatgacggga actacaagac acgtgctgaa gtcaagtttg     540

aaggtgatac ccttgttaat agaatcgagt taaaaggtat tgattttaaa gaagatggaa     600

acattcttgg acacaaattg gaatacaact ataactcaca caatgtatac atcatggcag     660

acaaacaaaa gaatggaatc aaagttaact tcaaaattag acacaacatt gaagatggaa     720

gcgttcaact agcagaccat tatcaacaaa atactccgat tggcgatggc cctgtccttt     780

taccagacaa ccattacctg tccacacaat ctgccctttc gaaagatccc aacgaaaaga     840

gagaccacat ggtccttctt gagtttgtaa ccgctgctgg gattacacat ggcatggatg     900

aactatacaa aaggcctgca gcaaacgacg aaaactacgc tgcatcagtt taataataag     960

ataaaccaga gcggcacggc aagcagagta tacgagattc ggtagccacc gctgagcaat    1020

aactagcata accccttggg gcctctaaac gggtcttgag gggttttttg catatgcaac    1080

gg                                                                   1082


<210>  63
<211>  21
<212>  DNA
<213>  Artificial Sequence

<220>
<223>  Artificial

<400>  63
tcataagctt taatacgact c                                                21


<210>  64
<211>  21
<212>  DNA
<213>  Artificial Sequence

<220>
<223>  Artificial

<400>  64
ccgttgcata tgcaaaaaac c                                                21


<210>  65
<211>  1504
<212>  DNA
<213>  Artificial Sequence

<220>
<223>  Artificial

<400>  65
tcataagctt taatacgact cactataggc ggttgatggg ttgttggtca acagggcggc        60

gataatcgcc gttctgttgg ctagcaagga ggaaaatatg gcaaacctgg acaagatgct       120

gaataccaca gtaacagagg tgcggcagtt cctgcaggtg gacagagtgt gcgtgttcca       180

gtttgaggag gattatagcg gagtggtggt ggtggaggcc gtggacgata ggtggatctc       240

catcctgaag acccaggtgc gggatagata cttcatggag acaaggggcg aggagtattc        300

tcacggccgc taccaggcca tcgccgacat ctacaccgca aacctgacag agtgctacag       360

ggatctgctg acacagtttc aggtgagagc aatcctggcc gtgcccatcc tgcagggcaa       420

gaagctgtgg ggcctgttgg tggcacacca gctggcggcc cctagacagt ggcagacctg       480

ggagatcgac tttctgaagc agcaggccgt ggtggtgggc atcgccatcc agcagagcta       540

atttcctcct tgttagccaa tagaatggtc taacaaggag gaaacaatgt atacaacaaa       600

cctggcggca gcgcaaaaga tgcgtaaagg agaagaactt ttcactggag ttgtcccaat       660

tcttgttgaa ttagatggtg atgttaatgg gcacaaattt tctgtcagtg gagagggtga       720

aggtgatgca acatacggaa aacttaccct taaatttatt tgcactactg gaaaactacc       780

tgttccgtgg ccaacacttg tcactacttt cggttatggt gttcaatgct ttgcgagata       840

cccagatcac atgaaacagc atgacttttt caagagtgcc atgcccgaag gttacgtaca       900

ggaaagaact atatttttca aagatgacgg gaactacaag acacgtgctg aagtcaagtt       960

tgaaggtgat acccttgtta atagaatcga gttaaaaggt attgatttta aagaagatgg      1020

aaacattctt ggacacaaat tggaatacaa ctataactca cacaatgtat acatcatggc      1080

agacaaacaa aagaatggaa tcaaagttaa cttcaaaatt agacacaaca ttgaagatgg      1140

aagcgttcaa ctagcagacc attatcaaca aaatactccg attggcgatg gccctgtcct      1200

tttaccagac aaccattacc tgtccacaca atctgccctt tcgaaagatc ccaacgaaaa      1260

gagagaccac atggtccttc ttgagtttgt aaccgctgct gggattacac atggcatgga      1320

tgaactatac aaaaggcctg cagcaaacga cgaaaactac gctgcatcag tttaataata      1380

agataaacca gagcggcacg gcaagcagag tatacgagat tcggtagcca ccgctgagca      1440

ataactagca taaccccttg gggcctctaa acgggtcttg aggggttttt tgcatatgca      1500

acgg                                                                1504

<210>  66
<211>  1504
<212>  DNA
<213>  Artificial Sequence

<220>
<223>  Artificial

<400>  66
tcataagctt taatacgact cactataggc ggttgatggg ttgttggtca acagggcggc      60

gataattgcc gttctgttgg ctagcaagga ggaaaatatg gcaaacctgg acaagatgct     120

gaataccaca gtaacagagg tgcggcagtt cctgcaggtg gacagagtgt gcgtgttcca     180

gtttgaggag gattatagcg gagtggtggt ggtggaggcc gtggacgata ggtggatctc     240

catcctgaag acccaggtgc gggatagata cttcatggag acaaggggcg aggagtattc     300

tcacggccgc taccaggcca tcgccgacat ctacaccgca aacctgacag agtgctacag     360

ggatctgctg acacagtttc aggtgagagc aatcctggcc gtgcccatcc tgcagggcaa     420

gaagctgtgg ggcctgttgg tggcacacca gctggcggcc cctagacagt ggcagacctg     480

ggagatcgac tttctgaagc agcaggccgt ggtggtgggc atcgccatcc agcagagcta     540

atttcctcct tgttagccaa tagaatggtc taacaaggag gaaacaatgt atacaacaaa     600

cctggcggca gcgcaaaaga tgcgtaaagg agaagaactt ttcactggag ttgtcccaat     660

tcttgttgaa ttagatggtg atgttaatgg gcacaaattt tctgtcagtg gagagggtga     720

aggtgatgca acatacggaa aacttaccct taaatttatt tgcactactg gaaaactacc     780

tgttccgtgg ccaacacttg tcactacttt cggttatggt gttcaatgct ttgcgagata     840

cccagatcac atgaaacagc atgacttttt caagagtgcc atgcccgaag gttacgtaca     900

ggaaagaact atatttttca aagatgacgg gaactacaag acacgtgctg aagtcaagtt     960

tgaaggtgat acccttgtta atagaatcga gttaaaaggt attgatttta aagaagatgg    1020

aaacattctt ggacacaaat tggaatacaa ctataactca cacaatgtat acatcatggc    1080

agacaaacaa aagaatggaa tcaaagttaa cttcaaaatt agacacaaca ttgaagatgg    1140

aagcgttcaa ctagcagacc attatcaaca aaatactccg attggcgatg gccctgtcct    1200

tttaccagac aaccattacc tgtccacaca atctgccctt tcgaaagatc ccaacgaaaa    1260

gagagaccac atggtccttc ttgagtttgt aaccgctgct gggattacac atggcatgga    1320

tgaactatac aaaaggcctg cagcaaacga cgaaaactac gctgcatcag tttaataata    1380

agataaacca gagcggcacg gcaagcagag tatacgagat tcggtagcca ccgctgagca    1440

ataactagca taaccccttg gggcctctaa acgggtcttg aggggttttt tgcatatgca    1500

acgg                                                                1504

<210>  67
<211>  595
<212>  RNA
<213>  Artificial Sequence

<400>  67
ggcaaguuug auguggcugu gcugcgugua uggggauaac uuguaugugu ggcgcagcaa      60

ggaggaaaau auggcaaacc uggacaagau gcugaauacc acaguaacag aggugcggca     120

guuccugcag guggacagag ugugcguguu ccaguuugag gaggauuaua gcggaguggu     180

ggugguggag gccguggacg auagguggau cuccauccug aagacccagg ugcgggauag     240

auacuucaug gagacaaggg gcgaggagua uucucacggc cgcuaccagg ccaucgccga     300

caucuacacc gcaaaccuga cagagugcua cagggaucug cugacacagu uucaggugag     360

agcaauccug gccgugccca uccugcaggg caagaagcug uggggccugu ugguggcaca     420

ccagcuggcg gccccuagac aguggcagac cugggagauc gacuuucuga agcagcaggc     480

cgugguggug ggcaucgcca uccagcagag cuaauuuccu ccuuguugcg cuguauguac     540

gggcaauaag gaggaaauaa ugucaaccac caaccuggcg gcagcgcaaa agaug          595

<210>  68
<211>  595
<212>  RNA
<213>  Artificial Sequence

<400>  68
ggcuaguugu guugugugua ugaagaaggc cuugaauaac ggggucuucu uugugguuaa      60

ggaggaaaau auggcaaacc uggacaagau gcugaauacc acaguaacag aggugcggca     120

guuccugcag guggacagag ugugcguguu ccaguuugag gaggauuaua gcggaguggu     180

ggugguggag gccguggacg auagguggau cuccauccug aagacccagg ugcgggauag     240

auacuucaug gagacaaggg gcgaggagua uucucacggc cgcuaccagg ccaucgccga     300

caucuacacc gcaaaccuga cagagugcua cagggaucug cugacacagu uucaggugag     360

agcaauccug gccgugccca uccugcaggg caagaagcug uggggccugu ugguggcaca     420

ccagcuggcg gccccuagac aguggcagac cugggagauc gacuuucuga agcagcaggc     480

cgugguggug ggcaucgcca uccagcagag cuaauuuccu ccuuagucac gaagaagacu     540

ucugacuaag gagggaauaa ugucgaagac gaaccuggcg gcagcgcaaa agaug          595

<210>  69
<211>  595
<212>  RNA
<213>  Artificial Sequence

<400>  69
ggcuaguugu guugugugua ugaagaaggc cuugaauaac ggggucuucu ucgugguuaa      60

ggaggaaaau auggcaaacc uggacaagau gcugaauacc acaguaacag aggugcggca 120

guuccugcag guggacagag ugugcguguu ccaguuugag gaggauuaua gcggaguggu 180

ggugguggag gccguggacg auagguggau cuccauccug aagacccagg ugcgggauag 240

auacuucaug gagacaaggg gcgaggagua uucucacggc cgcuaccagg ccaucgccga 300

caucuacacc gcaaaccuga cagagugcua cagggaucug cugacacagu uucaggugag 360

agcaauccug gccgugccca uccugcaggg caagaagcug uggggccugu ugguggcaca 420

ccagcuggcg gccccuagac aguggcagac cugggagauc gacuuucuga agcagcaggc 480

cgugguggug ggcaucgcca uccagcagag cuaauuuccu ccuuagucac gaagaagacu 540

ucugacuaag gagggaauaa ugucgaagac gaaccuggcg gcagcgcaaa agaug 595

<210> 70
<211> 595
<212> RNA
<213> Artificial Sequence

<400> 70
ggcuaguugu guugugugua ugaagagggc cuugaauaac ggggucuucu ucgugguuaa 60

ggaggaaaau auggcaaacc uggacaagau gcugaauacc acaguaacag aggugcggca 120

guuccugcag guggacagag ugugcguguu ccaguuugag gaggauuaua gcggaguggu 180

ggugguggag gccguggacg auagguggau cuccauccug aagacccagg ugcgggauag 240

auacuucaug gagacaaggg gcgaggagua uucucacggc cgcuaccagg ccaucgccga 300

caucuacacc gcaaaccuga cagagugcua cagggaucug cugacacagu uucaggugag 360

agcaauccug gccgugccca uccugcaggg caagaagcug uggggccugu ugguggcaca 420

ccagcuggcg gccccuagac aguggcagac cugggagauc gacuuucuga agcagcaggc 480

cgugguggug ggcaucgcca uccagcagag cuaauuuccu ccuuagucac gaagaagacu 540

ucugacuaag gagggaauaa ugucgaagac gaaccuggcg gcagcgcaaa agaug 595

<210> 71
<211> 115
<212> DNA
<213> Artificial Sequence

<400> 71
tcataagctt taatacgact cactataggc aagtttgatg tggctgtgct gcgtgtatgg 60

ggataacttg tatgtgtggc gcagcaagga ggaaaatatg gcaaacctgg acaag 115

<210> 72
<211> 115
<212> DNA
<213> Artificial Sequence

<400>  72
tcataagctt taatacgact cactataggc tagttgtgtt gtgtgtatga agaaggcctt      60

gaataacggg gtcttctttg tggttaagga ggaaaatatg gcaaacctgg acaag          115


<210>  73
<211>  115
<212>  DNA
<213>  Artificial Sequence

<400>  73
tcataagctt taatacgact cactataggc tagttgtgtt gtgtgtatga agaaggcctt      60

gaataacggg gtcttcttcg tggttaagga ggaaaatatg gcaaacctgg acaag          115


<210>  74
<211>  115
<212>  DNA
<213>  Artificial Sequence

<400>  74
tcataagctt taatacgact cactataggc tagttgtgtt gtgtgtatga agagggcctt      60

gaataacggg gtcttcttcg tggttaagga ggaaaatatg gcaaacctgg acaag          115


<210>  75
<211>  454
<212>  DNA
<213>  Artificial Sequence

<400>  75
tcataagctt taatacgact cactataggc aagtttgatg tggctgtgct gcgtgtatgg      60

ggataacttg tatgtgtggc gcagcaagga ggaaaatatg gcaaacctgg acaagatgct     120

gaataccaca gtaacagagg tgcggcagtt cctgcaggtg gacagagtgt gcgtgttcca     180

gtttgaggag gattatagcg gagtggtggt ggtggaggcc gtggacgata ggtggatctc     240

catcctgaag acccaggtgc gggatagata cttcatggag acaaggggcg aggagtattc     300

tcacggccgc taccaggcca tcgccgacat ctacaccgca aacctgacag agtgctacag     360

ggatctgctg acacagtttc aggtgagagc aatcctggcc gtgcccatcc tgcagggcaa     420

gaagctgtgg ggcctgttgg tggcacacca gctg                                 454


<210>  76
<211>  454
<212>  DNA
<213>  Artificial Sequence

<400>  76
tcataagctt taatacgact cactataggc tagttgtgtt gtgtgtatga agaaggcctt      60

gaataacggg gtcttctttg tggttaagga ggaaaatatg gcaaacctgg acaagatgct     120

gaataccaca gtaacagagg tgcggcagtt cctgcaggtg gacagagtgt gcgtgttcca     180

gtttgaggag gattatagcg gagtggtggt ggtggaggcc gtggacgata ggtggatctc 240

catcctgaag acccaggtgc gggatagata cttcatggag acaaggggcg aggagtattc 300

tcacggccgc taccaggcca tcgccgacat ctacaccgca aacctgacag agtgctacag 360

ggatctgctg acacagtttc aggtgagagc aatcctggcc gtgcccatcc tgcagggcaa 420

gaagctgtgg ggcctgttgg tggcacacca gctg 454

<210> 77
<211> 454
<212> DNA
<213> Artificial Sequence

<400> 77
tcataagctt taatacgact cactataggc tagttgtgtt gtgtgtatga agaaggcctt 60

gaataacggg gtcttcttcg tggttaagga ggaaaatatg gcaaacctgg acaagatgct 120

gaataccaca gtaacagagg tgcggcagtt cctgcaggtg gacagagtgt gcgtgttcca 180

gtttgaggag gattatagcg gagtggtggt ggtggaggcc gtggacgata ggtggatctc 240

catcctgaag acccaggtgc gggatagata cttcatggag acaaggggcg aggagtattc 300

tcacggccgc taccaggcca tcgccgacat ctacaccgca aacctgacag agtgctacag 360

ggatctgctg acacagtttc aggtgagagc aatcctggcc gtgcccatcc tgcagggcaa 420

gaagctgtgg ggcctgttgg tggcacacca gctg 454

<210> 78
<211> 454
<212> DNA
<213> Artificial Sequence

<400> 78
tcataagctt taatacgact cactataggc tagttgtgtt gtgtgtatga agagggcctt 60

gaataacggg gtcttcttcg tggttaagga ggaaaatatg gcaaacctgg acaagatgct 120

gaataccaca gtaacagagg tgcggcagtt cctgcaggtg gacagagtgt gcgtgttcca 180

gtttgaggag gattatagcg gagtggtggt ggtggaggcc gtggacgata ggtggatctc 240

catcctgaag acccaggtgc gggatagata cttcatggag acaaggggcg aggagtattc 300

tcacggccgc taccaggcca tcgccgacat ctacaccgca aacctgacag agtgctacag 360

ggatctgctg acacagtttc aggtgagagc aatcctggcc gtgcccatcc tgcagggcaa 420

gaagctgtgg ggcctgttgg tggcacacca gctg 454

<210> 79
<211> 200
<212> DNA
<213> Artificial Sequence

<400> 79

agctgtgggg cctgttggtg gcacaccagc tggcggcccc tagacagtgg cagacctggg 60

agatcgactt tctgaagcag caggccgtgg tggtgggcat cgccatccag cagagctaat 120

ttcctccttg ttgcgctgta tgtacgggca ataaggagga aataatgtca accaccaacc 180

tggcggcagc gcaaaagatg 200

<210> 80
<211> 200
<212> DNA
<213> Artificial Sequence

<400> 80
agctgtgggg cctgttggtg gcacaccagc tggcggcccc tagacagtgg cagacctggg 60

agatcgactt tctgaagcag caggccgtgg tggtgggcat cgccatccag cagagctaat 120

ttcctcctta gtcacgaaga agacttctga ctaaggaggg aataatgtcg aagacgaacc 180

tggcggcagc gcaaaagatg 200

<210> 81
<211> 1082
<212> DNA
<213> Artificial Sequence

<400> 81
agctgtgggg cctgttggtg gcacaccagc tggcggcccc tagacagtgg cagacctggg 60

agatcgactt tctgaagcag caggccgtgg tggtgggcat cgccatccag cagagctaat 120

ttcctccttg ttgcgctgta tgtacgggca ataaggagga aataatgtca accaccaacc 180

tggcggcagc gcaaaagatg cgtaaaggag aagaactttt cactggagtt gtcccaattc 240

ttgttgaatt agatggtgat gttaatgggc acaaattttc tgtcagtgga gagggtgaag 300

gtgatgcaac atacggaaaa cttaccctta aatttatttg cactactgga aaactacctg 360

ttccgtggcc aacacttgtc actactttcg gttatggtgt tcaatgcttt gcgagatacc 420

cagatcacat gaaacagcat gactttttca agagtgccat gcccgaaggt tacgtacagg 480

aaagaactat atttttcaaa gatgacggga actacaagac acgtgctgaa gtcaagtttg 540

aaggtgatac ccttgttaat agaatcgagt taaaaggtat tgattttaaa gaagatggaa 600

acattcttgg acacaaattg gaatacaact ataactcaca caatgtatac atcatggcag 660

acaaacaaaa gaatggaatc aaagttaact tcaaaattag acacaacatt gaagatggaa 720

gcgttcaact agcagaccat tatcaacaaa atactccgat tggcgatggc cctgtccttt 780

taccagacaa ccattacctg tccacacaat ctgccctttc gaaagatccc aacgaaaaga 840

gagaccacat ggtccttctt gagtttgtaa ccgctgctgg gattacacat ggcatggatg 900

aactatacaa aaggcctgca gcaaacgacg aaaactacgc tgcatcagtt taataataag 960

ataaaccaga gcggcacggc aagcagagta tacgagattc ggtagccacc gctgagcaat 1020

aactagcata accccttggg gcctctaaac gggtcttgag gggttttttg catatgcaac 1080

gg 1082

<210> 82
<211> 1082
<212> DNA
<213> Artificial Sequence

<400> 82
agctgtgggg cctgttggtg gcacaccagc tggcggcccc tagacagtgg cagacctggg 60

agatcgactt tctgaagcag caggccgtgg tggtgggcat cgccatccag cagagctaat 120

ttcctcctta gtcacgaaga agacttctga ctaaggaggg aataatgtcg aagacgaacc 180

tggcggcagc gcaaaagatg cgtaaaggag aagaactttt cactggagtt gtcccaattc 240

ttgttgaatt agatggtgat gttaatgggc acaaattttc tgtcagtgga gagggtgaag 300

gtgatgcaac atacggaaaa cttaccctta aatttatttg cactactgga aaactacctg 360

ttccgtggcc aacacttgtc actactttcg gttatggtgt tcaatgcttt gcgagatacc 420

cagatcacat gaaacagcat gactttttca agagtgccat gcccgaaggt tacgtacagg 480

aaagaactat atttttcaaa gatgacggga actacaagac acgtgctgaa gtcaagtttg 540

aaggtgatac ccttgttaat agaatcgagt taaaaggtat tgattttaaa gaagatggaa 600

acattcttgg acacaaattg gaatacaact ataactcaca caatgtatac atcatggcag 660

acaaacaaaa gaatggaatc aaagttaact tcaaaattag acacaacatt gaagatggaa 720

gcgttcaact agcagaccat tatcaacaaa atactccgat tggcgatggc cctgtccttt 780

taccagacaa ccattacctg tccacacaat ctgccctttc gaaagatccc aacgaaaaga 840

gagaccacat ggtccttctt gagtttgtaa ccgctgctgg gattacacat ggcatggatg 900

aactatacaa aaggcctgca gcaaacgacg aaaactacgc tgcatcagtt taataataag 960

ataaaccaga gcggcacggc aagcagagta tacgagattc ggtagccacc gctgagcaat 1020

aactagcata accccttggg gcctctaaac gggtcttgag gggttttttg catatgcaac 1080

gg 1082

<210> 83
<211> 1504
<212> DNA
<213> Artificial Sequence

<400> 83
tcataagctt taatacgact cactataggc aagtttgatg tggctgtgct gcgtgtatgg 60

ggataacttg tatgtgtggc gcagcaagga ggaaaatatg gcaaacctgg acaagatgct 120

gaataccaca gtaacagagg tgcggcagtt cctgcaggtg gacagagtgt gcgtgttcca 180

gtttgaggag gattatagcg gagtggtggt ggtggaggcc gtggacgata ggtggatctc 240

catcctgaag acccaggtgc gggatagata cttcatggag acaaggggcg aggagtattc 300

tcacggccgc taccaggcca tcgccgacat ctacaccgca aacctgacag agtgctacag 360

ggatctgctg acacagtttc aggtgagagc aatcctggcc gtgcccatcc tgcagggcaa 420

gaagctgtgg ggcctgttgg tggcacacca gctggcggcc cctagacagt ggcagacctg 480

ggagatcgac tttctgaagc agcaggccgt ggtggtgggc atcgccatcc agcagagcta 540

atttcctcct tgttgcgctg tatgtacggg caataaggag gaaataatgt caaccaccaa 600

cctggcggca gcgcaaaaga tgcgtaaagg agaagaactt ttcactggag ttgtcccaat 660

tcttgttgaa ttagatggtg atgttaatgg gcacaaattt tctgtcagtg gagagggtga 720

aggtgatgca acatacggaa aacttaccct taaatttatt tgcactactg gaaaactacc 780

tgttccgtgg ccaacacttg tcactacttt cggttatggt gttcaatgct ttgcgagata 840

cccagatcac atgaaacagc atgacttttt caagagtgcc atgcccgaag gttacgtaca 900

ggaaagaact atatttttca aagatgacgg gaactacaag acacgtgctg aagtcaagtt 960

tgaaggtgat acccttgtta atagaatcga gttaaaaggt attgatttta aagaagatgg 1020

aaacattctt ggacacaaat tggaatacaa ctataactca cacaatgtat acatcatggc 1080

agacaaacaa aagaatggaa tcaaagttaa cttcaaaatt agacacaaca ttgaagatgg 1140

aagcgttcaa ctagcagacc attatcaaca aaatactccg attggcgatg gccctgtcct 1200

tttaccagac aaccattacc tgtccacaca atctgccctt tcgaaagatc ccaacgaaaa 1260

gagagaccac atggtccttc ttgagtttgt aaccgctgct gggattacac atggcatgga 1320

tgaactatac aaaaggcctg cagcaaacga cgaaaactac gctgcatcag tttaataata 1380

agataaacca gagcggcacg gcaagcagag tatacgagat tcggtagcca ccgctgagca 1440

ataactagca taaccccttg gggcctctaa acgggtcttg aggggttttt tgcatatgca 1500

acgg 1504

<210> 84
<211> 1504
<212> DNA
<213> Artificial Sequence

<400> 84
tcataagctt taatacgact cactataggc tagttgtgtt gtgtgtatga agaaggcctt 60

gaataacggg gtcttctttg tggttaagga ggaaaatatg gcaaacctgg acaagatgct 120

gaataccaca gtaacagagg tgcggcagtt cctgcaggtg gacagagtgt gcgtgttcca 180

gtttgaggag gattatagcg gagtggtggt ggtggaggcc gtggacgata ggtggatctc 240

catcctgaag acccaggtgc gggatagata cttcatggag acaaggggcg aggagtattc 300

tcacggccgc taccaggcca tcgccgacat ctacaccgca aacctgacag agtgctacag 360

ggatctgctg acacagtttc aggtgagagc aatcctggcc gtgcccatcc tgcagggcaa 420

gaagctgtgg ggcctgttgg tggcacacca gctggcggcc cctagacagt ggcagacctg 480

ggagatcgac tttctgaagc agcaggccgt ggtggtgggc atcgccatcc agcagagcta 540

atttcctcct tagtcacgaa gaagacttct gactaaggag ggaataatgt cgaagacgaa 600

cctggcggca gcgcaaaaga tgcgtaaagg agaagaactt ttcactggag ttgtcccaat 660

tcttgttgaa ttagatggtg atgttaatgg gcacaaattt tctgtcagtg gagagggtga 720

aggtgatgca acatacggaa aacttaccct taaatttatt tgcactactg gaaaactacc 780

tgttccgtgg ccaacacttg tcactacttt cggttatggt gttcaatgct ttgcgagata 840

cccagatcac atgaaacagc atgacttttt caagagtgcc atgcccgaag gttacgtaca 900

ggaaagaact atatttttca aagatgacgg gaactacaag acacgtgctg aagtcaagtt 960

tgaaggtgat acccttgtta atagaatcga gttaaaaggt attgatttta aagaagatgg 1020

aaacattctt ggacacaaat tggaatacaa ctataactca cacaatgtat acatcatggc 1080

agacaaacaa aagaatggaa tcaaagttaa cttcaaaatt agacacaaca ttgaagatgg 1140

aagcgttcaa ctagcagacc attatcaaca aaatactccg attggcgatg gccctgtcct 1200

tttaccagac aaccattacc tgtccacaca atctgccctt tcgaaagatc ccaacgaaaa 1260

gagagaccac atggtccttc ttgagtttgt aaccgctgct gggattacac atggcatgga 1320

tgaactatac aaaaggcctg cagcaaacga cgaaaactac gctgcatcag tttaataata 1380

agataaacca gagcggcacg gcaagcagag tatacgagat tcggtagcca ccgctgagca 1440

ataactagca taaccccttg gggcctctaa acgggtcttg aggggttttt tgcatatgca 1500

acgg 1504

<210> 85
<211> 1504
<212> DNA
<213> Artificial Sequence

<400> 85
tcataagctt taatacgact cactataggc tagttgtgtt gtgtgtatga agaaggcctt 60

gaataacggg gtcttcttcg tggttaagga ggaaaatatg gcaaacctgg acaagatgct 120

gaataccaca gtaacagagg tgcggcagtt cctgcaggtg gacagagtgt gcgtgttcca 180

gtttgaggag gattatagcg gagtggtggt ggtggaggcc gtggacgata ggtggatctc 240

catcctgaag acccaggtgc gggatagata cttcatggag acaaggggcg aggagtattc 300

tcacggccgc taccaggcca tcgccgacat ctacaccgca aacctgacag agtgctacag 360

ggatctgctg acacagtttc aggtgagagc aatcctggcc gtgcccatcc tgcagggcaa 420

gaagctgtgg ggcctgttgg tggcacacca gctggcggcc cctagacagt ggcagacctg 480

ggagatcgac tttctgaagc agcaggccgt ggtggtgggc atcgccatcc agcagagcta 540

atttcctcct tagtcacgaa gaagacttct gactaaggag ggaataatgt cgaagacgaa 600

cctggcggca gcgcaaaaga tgcgtaaagg agaagaactt ttcactggag ttgtcccaat 660

tcttgttgaa ttagatggtg atgttaatgg gcacaaattt tctgtcagtg gagagggtga 720

aggtgatgca acatacggaa aacttaccct taaatttatt tgcactactg gaaaactacc 780

tgttccgtgg ccaacacttg tcactacttt cggttatggt gttcaatgct ttgcgagata 840

cccagatcac atgaaacagc atgacttttt caagagtgcc atgcccgaag gttacgtaca 900

ggaaagaact atatttttca aagatgacgg gaactacaag acacgtgctg aagtcaagtt 960

tgaaggtgat acccttgtta atagaatcga gttaaaaggt attgatttta aagaagatgg 1020

aaacattctt ggacacaaat tggaatacaa ctataactca cacaatgtat acatcatggc 1080

agacaaacaa aagaatggaa tcaaagttaa cttcaaaatt agacacaaca ttgaagatgg 1140

aagcgttcaa ctagcagacc attatcaaca aaatactccg attggcgatg gccctgtcct 1200

tttaccagac aaccattacc tgtccacaca atctgccctt tcgaaagatc ccaacgaaaa 1260

gagagaccac atggtccttc ttgagtttgt aaccgctgct gggattacac atggcatgga 1320

tgaactatac aaaaggcctg cagcaaacga cgaaaactac gctgcatcag tttaataata 1380

agataaacca gagcggcacg gcaagcagag tatacgagat tcggtagcca ccgctgagca 1440

ataactagca taaccccttg gggcctctaa acgggtcttg aggggttttt tgcatatgca 1500

acgg 1504

<210> 86
<211> 1504
<212> DNA
<213> Artificial Sequence

<400> 86
tcataagctt taatacgact cactataggc tagttgtgtt gtgtgtatga agagggcctt 60

gaataacggg gtcttcttcg tggttaagga ggaaaatatg gcaaacctgg acaagatgct 120

gaataccaca gtaacagagg tgcggcagtt cctgcaggtg gacagagtgt gcgtgttcca 180

gtttgaggag gattatagcg gagtggtggt ggtggaggcc gtggacgata ggtggatctc 240

catcctgaag acccaggtgc gggatagata cttcatggag acaaggggcg aggagtattc 300

tcacggccgc taccaggcca tcgccgacat ctacaccgca aacctgacag agtgctacag 360

ggatctgctg acacagtttc aggtgagagc aatcctggcc gtgcccatcc tgcagggcaa 420

gaagctgtgg ggcctgttgg tggcacacca gctggcggcc cctagacagt ggcagacctg 480

ggagatcgac tttctgaagc agcaggccgt ggtggtgggc atcgccatcc agcagagcta 540

atttcctcct tagtcacgaa gaagacttct gactaaggag ggaataatgt cgaagacgaa 600

cctggcggca gcgcaaaaga tgcgtaaagg agaagaactt ttcactggag ttgtcccaat 660

tcttgttgaa ttagatggtg atgttaatgg gcacaaattt tctgtcagtg gagagggtga 720

aggtgatgca acatacggaa aacttaccct taaatttatt tgcactactg gaaaactacc 780

tgttccgtgg ccaacacttg tcactacttt cggttatggt gttcaatgct ttgcgagata 840

cccagatcac atgaaacagc atgacttttt caagagtgcc atgcccgaag gttacgtaca 900

ggaaagaact atatttttca aagatgacgg gaactacaag acacgtgctg aagtcaagtt 960

tgaaggtgat acccttgtta atagaatcga gttaaaaggt attgatttta aagaagatgg 1020

aaacattctt ggacacaaat tggaatacaa ctataactca cacaatgtat acatcatggc 1080

agacaaacaa aagaatggaa tcaaagttaa cttcaaaatt agacacaaca ttgaagatgg 1140

aagcgttcaa ctagcagacc attatcaaca aaatactccg attggcgatg gccctgtcct 1200

tttaccagac aaccattacc tgtccacaca atctgccctt tcgaaagatc ccaacgaaaa 1260

gagagaccac atggtccttc ttgagtttgt aaccgctgct gggattacac atggcatgga 1320

tgaactatac aaaaggcctg cagcaaacga cgaaaactac gctgcatcag tttaataata 1380

agataaacca gagcggcacg gcaagcagag tatacgagat tcggtagcca ccgctgagca 1440

ataactagca taaccccttg gggcctctaa acgggtcttg aggggttttt tgcatatgca 1500

acgg 1504

## Description

### Field of the invention

The present invention relates to novel RNA molecules comprising a sensor part and a trigger part. Due to these two parts, these molecules are also called herein RNA Plug&Play molecules. The present invention further relates to the use of novel RNA molecules and to a process for detecting a disease in vitro using said novel RNA molecules.

### Background of the invention

Riboregulators are sequences of RNA that effect changes in cells in response to a nucleic acid sequence. These RNA-based devices, which typically regulate protein translation or trigger mRNA degradation, have been used for a number of applications in synthetic biology, including sensitive control over gene expression, shunting of metabolic flux through different metabolic pathways, and synthetic control over cell death. (see WO2014074648)

In riboregulators that control gene expression, repression of protein translation has relied on sequestration of the normally single- stranded context of the ribosome binding site (RBS) within the loop region of a duplex RNA region (hairpin loop) that is upstream of a gene of interest (GOI). An mRNA in which the RBS is sequestered within a hairpin upstream of the GOI is thus a cis-repressed RNA (crRNA). A riboregulator based on an engineered crRNA can be constructed in which a trans-activating RNA (taRNA) binds to the crRNA and unwinds the repressing RNA duplex thereby exposing a now single-stranded RBS and activating translation of the downstream gene. In riboregulators that decrease expression of the GOI, the RBS and initiation codon of the GOI are both exposed in the absence of the trigger RNA. However, a trans-repressing RNA (trRNA), which bears anti-sense to the RBS and start codon, can bind to the riboregulator and strongly suppress translation of the downstream gene.

Over the last decade, researchers have developed a number of different riboregulator systems to control gene expression in prokaryotic cells. These previous systems have utilized a number of recurring design motifs. The vast majority of riboregulators have employed loop- linear interactions to drive the crRN A/trans -RNA hybridization reaction forward. In these interactions, a linear, single-stranded region in one of the strands binds to a loop established at the end of a duplex in the other strand. Essential in this interaction is the formation of a kissing loop structure in which the tertiary structure of the RNA sequence causes bases within the loop to flip outwards, facilitating binding with the linear RNA strand. Importantly, this kissing loop structure is only established with specific sequences inside the loop region, which can severely limit the number of possible crRNA designs.

All previous riboregulator systems have relied on sequestration of the RBS to impede translation of the GOI. This design choice has two crucial implications. First, much of the work in optimization of genetic circuits in synthetic biology relies on varying the strength of the RBS to finely tune protein levels inside the cell. Since the RBS sequence is a functional part of these riboregulators, one cannot simply replace the riboregulator RBS with variants and expect the output characteristics of the device to vary in a predictable manner following the strength of the new RBS. Furthermore, changes to the RBS will require corresponding modifications in the sequence of the trans-RNA. Second, for riboregulators that activate gene expression, riboregulators that sequester the RBS must be activated by taRNA sequences that are at least partially complementary to the crRNA RBS sequence. Consequently, unbound taRNAs can compete with de-repressed crRNA species for ribosome binding. Alternatively, RBS sequences within the taRNAs can also be sequestered within stem regions. This additional secondary structure can decrease the kinetics of binding with the crRNA and the dynamic range of the riboregulator.

WO2014074648A2 - A riboregulator or toehold switch [P1] is a riboregulator that activates gene expression by switching from a secondary structure that prevents translation to a configuration that promotes translation upon binding of a cognate trans-acting RNA. This trans-acting RNA is called the *trigger* and in the diagnostics setting this trigger is endogenous to the cell or organism of interest.

The idea is that the start codon AUG is sequestered within a strong double stranded region, while the Ribosome Binding Site (RBS) is accessible at a loop region of the stem. This configuration allows ribosome binding but it prevents scanning of the gene and therefore translation is compromised. Upon binding the trigger RNA, where regions a* and b* are complementary to regions a and b in the toehold, hybridization of these regions occurs which simultaneously unfolds the double stranded region around the AUG thus allowing translation of the downstream gene.

In the diagnostics setting the trigger RNA is a region of the transcriptome that can only be found in cells with a disease state. In the literature, this trigger RNA is a region of a virus (ebola, zika, etc...) that has infected the host. The gene downstream of the toehold is typically a certain fluorescence protein such as GFP or Luciferase. Therefore, when adding the toehold switch to the infected cell, the fluorescence protein will be translated and this signal will allow for the diagnostic of the disease.

The implementation of this diagnostics protocol has been carried out by using cell lysates of bacteria, which contain all the necessary translation and transcription machinery, where the toehold switch is added along with blood of the patient. If the fluorescence protein can be seen then the patient is infected.

No known approaches have so far arisen to implement this protocol in human cells or to detect other diseases than viral infections.

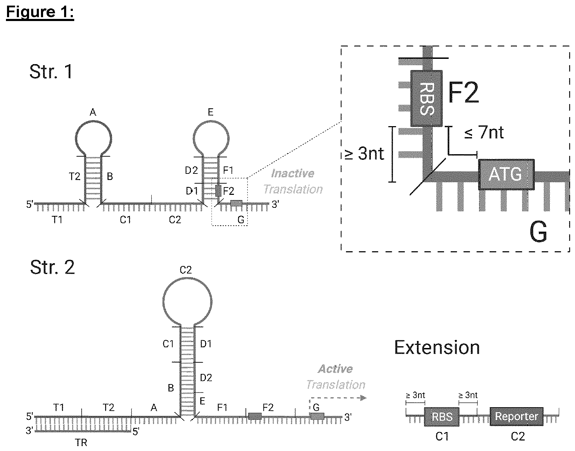

The present invention provides a new approach by developing new RNA molecules named as RNA Plug&Play (RNA P&P), useful for detecting in vitro a disease. The differences between a toehold switch and an RNA Plug&Play are twofold: first, the expression control is achieved via complete RNA duplex obfuscation of the RBS and second, the potential addition of a reporter gene to both report positive and negative biomarker detections. The present inventors have been able to develop such a structure of an RNA P&P that it can surprisingly potentially detect any RNA sequence without sequence constraints. Figure 1 depicts an RNA P&P with all of the regions and specifications.

### Summary of the invention

In a first aspect, the present invention relates to an RNA molecule as defined in the claims.

In a second aspect, the present invention relates to the use of the RNA molecule according to the first aspect of the invention in a colorimetric or fluorometric assay upon ligand binding of said RNA molecule according to the first aspect of the invention.

In a third aspect, the present invention relates to a process for detecting in vitro a disease using the RNA molecule according to the first aspect of the invention.

In a fourth aspect, the present invention relates to a kit for detecting in vitro a disease according to the third aspect of the invention.

### Brief description of the drawings

**Figure 1****. Schematics of an RNA Plug&Play molecule**. An RNA P&P along with all its regions as defined in claim 1, without (upper left region) and with (lower left region) the biomarker (RNA target). Relevant constraints are shown in the upper right region and the extension according to claim 2 is shown in the bottom right region.
**Figure 2****. RNA P&P detection of synthetic artificial RNA with a colorimetric readout.** Depicted is the reaction of 100 ng RNA P&P artificial *lacZ*-1 and artificial *lacZ-2* in the absence (control) or presence of 2.35 µM synthetic artificial RNA (sRNA, **SEQ ID NO: 1** for RNA P&P artificial *lacZ*-1 and artificial *lacZ*-2) after 45 minutes (panels **A, B**); and 100 ng RNA P&P artificial *lacZ*-3 in the absence (control) or presence of 0.8, 1.6, 2.35 µM sRNA **(SEQ ID NO: 2)** after 1, 2 and 3 hours (panels **C, D, E respectively**)**.** The colorimetric reaction is based on β-galactosidase enzymatic activity (yellow -negative- CPRG is transformed to purple -positive- colored chlorophenol red) only occurring when RNA P&P interacts with *E.coli* 16S rRNA fragment. RNA P&P *lacZ*-1 and *lacZ-2* show a certain level of leakiness so that the negative control shows a more orange color, while *lacZ-3* is very robust and the negative control stays yellow for at least 3 hours.

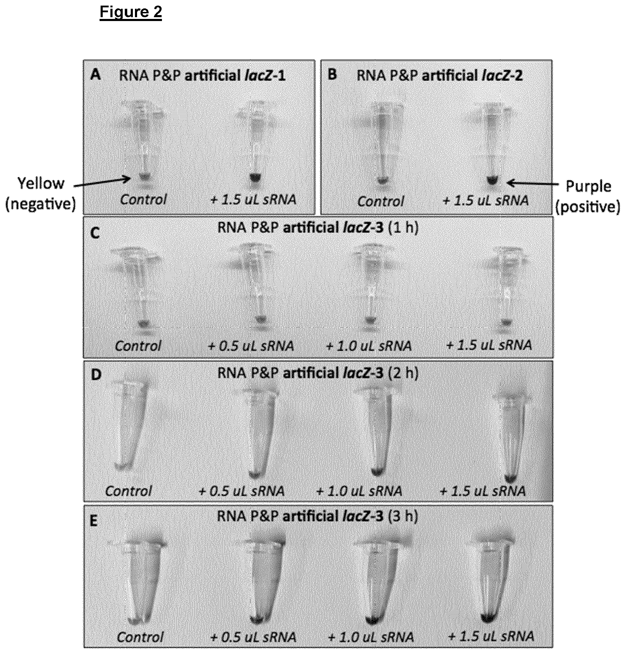

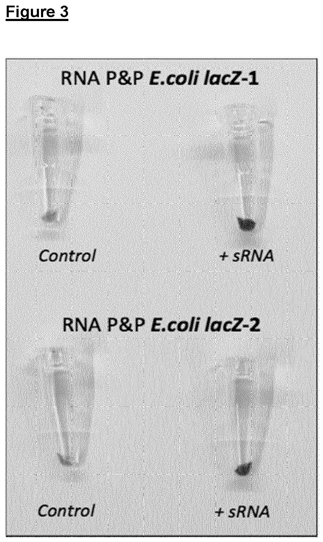

**Figure 3****. RNA P&P detection of *E.coli* 16 rRNA with a colorimetric readout.** Depicted is the reaction of 100 ng RNA P&P *E.coli* lacZ-1 and RNA P&P *E.coli* lacZ-2 in the absence or presence of synthetic RNA of *E.coli* 16S rRNA (sRNA, 1 µL of **SEQ ID NO: 18,** equivalent to 1.6 µM) after 1 hour. The colorimetric reaction is based on β-galactosidase enzymatic activity (yellow -negative- CPRG is transformed to purple -positive- colored chlorophenol red) only occurring when RNA P&P interacts with sRNA.

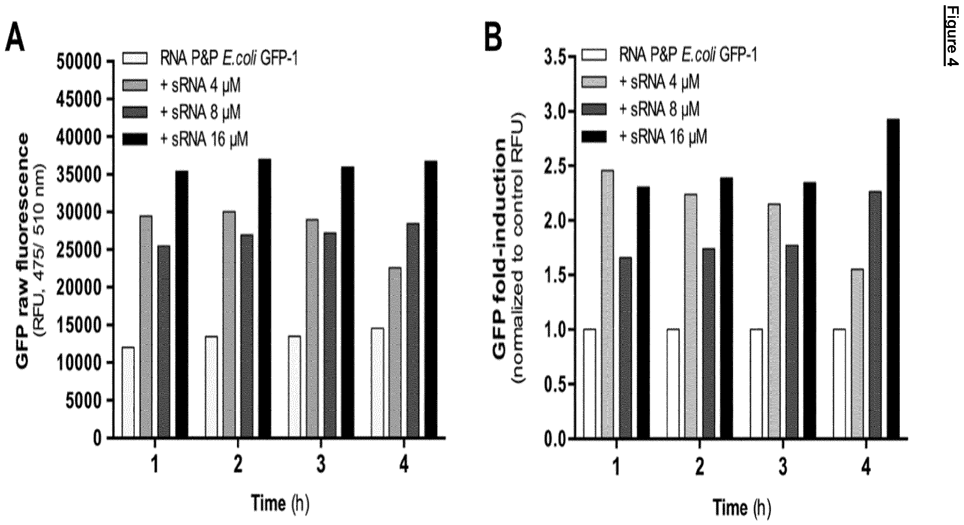

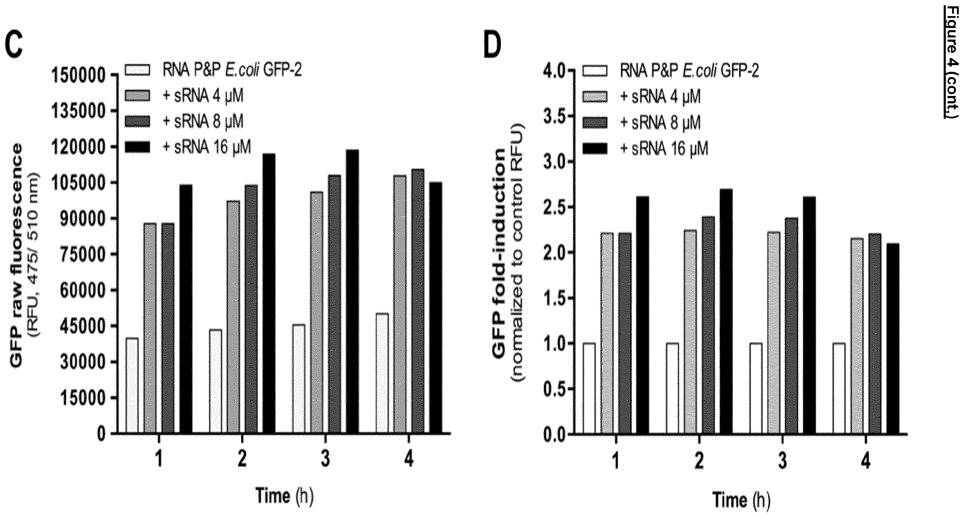

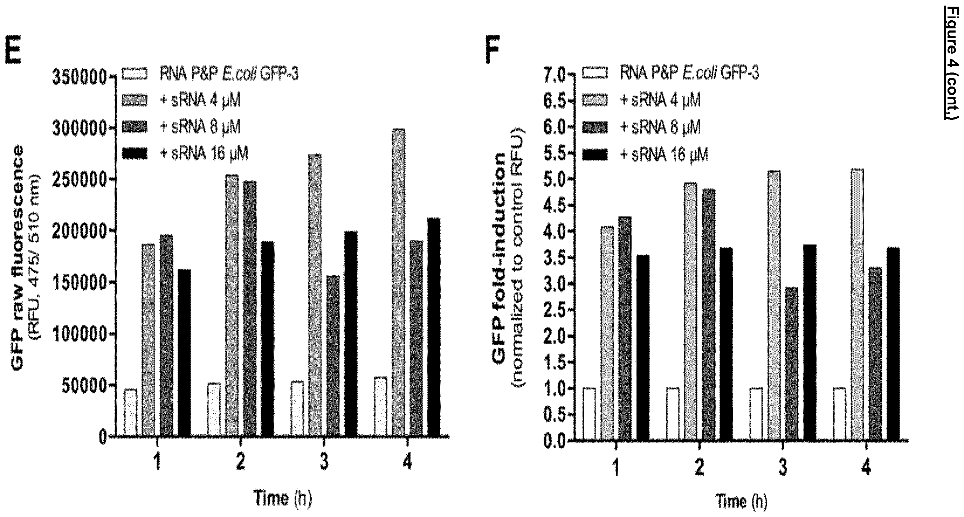

**Figure 4****. RNA P&P detection of *E.coli* 16S rRNA with a fluorescent (GFP) readout.** These graphs show the raw fluorescence GFP signal (RFU = raw fluorescence units) of 100 ng RNA P&P *E.coli* GFP-1 and 50 ng of RNA P&P *E.coli* GFP-2 and RNA P&P *E.coli* GFP-3 **(A, C, E)** and the fold-induction of GFP expression (normalized to RNA P&P *E.coli* GFP 1 and *E.coli* GFP 2 controls) **(B, D, F)** in the absence or presence of *E.coli* 16S rRNA sRNA **(SEQ ID NO: 15)** at 4, 8 and 16 µM after 1 to 4 hours. Fluorescence signal was read at 475 and 510 nm (excitation and emission wavelengths, respectively) in black bottom low-volume 384-well plates (Corning) on a Synergy H1 Hybrid Multi-Mode Plate Reader.

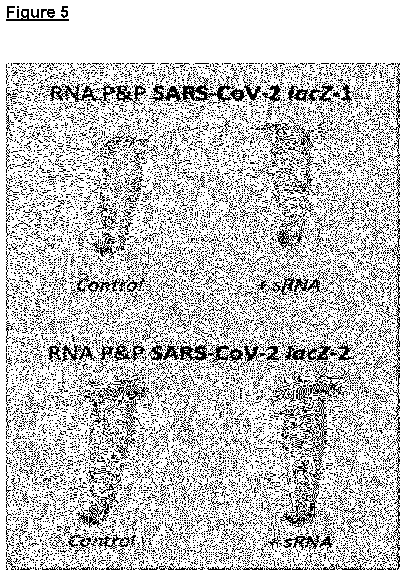

**Figure 5****. RNA P&P detection of SARS-CoV-2 *orf1ab* gene with a colorimetric readout.** Depicted is the reaction of 100 ng SARS-CoV-2 *lacZ-1* and SARS-CoV-2 *lacZ-2* in the absence or presence of sRNA (**SEQ ID NO: 38,** *orf1ab* gene region) (1.5 µL equivalent to 2.35 µM sRNA, respectively) after 2 hours. Final colorimetric reaction is based on β-galactosidase enzymatic activity (yellow -negative- CPRG is transformed to purple -positive-colored chlorophenol red) occurring when RNA P&P interacts with sRNA.

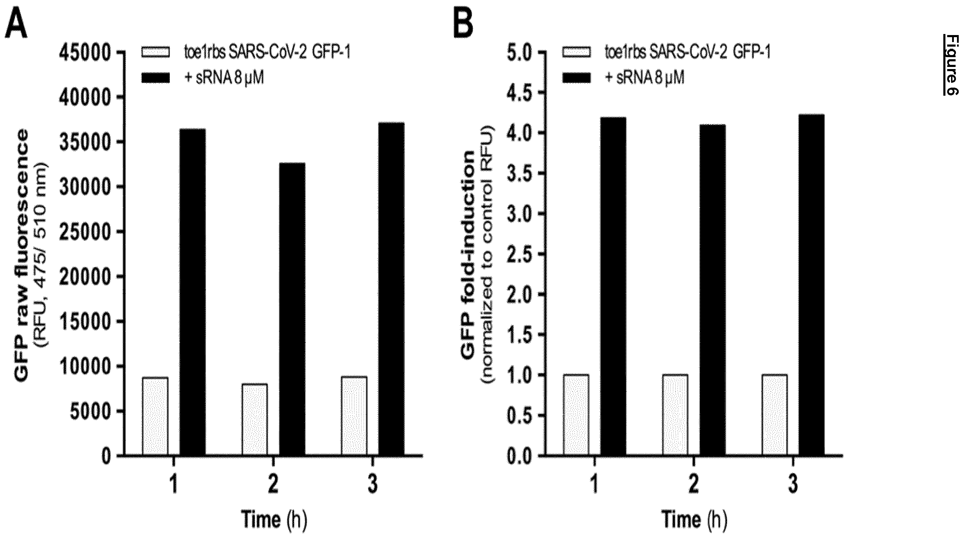

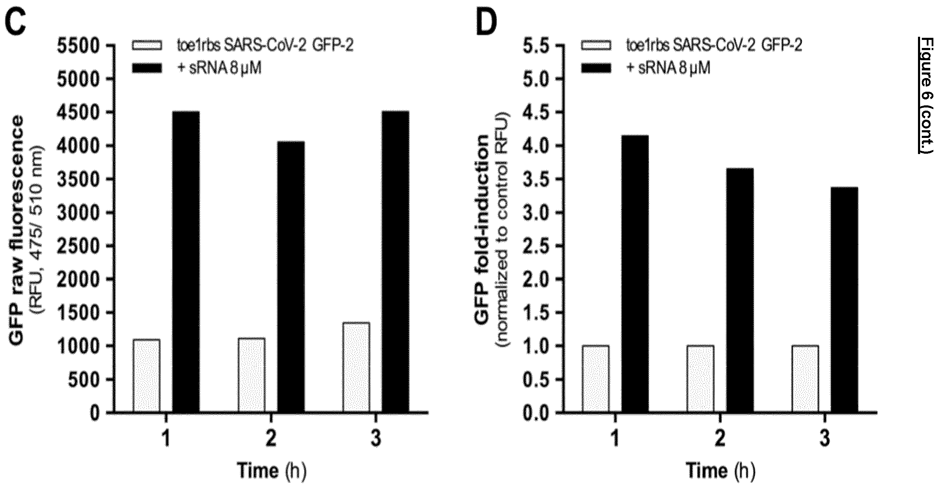

**Figure 6****. RNA P&P detection of SARS-CoV-2 with a fluorescent (GFP) readout.** These graphs show the raw fluorescence GFP signal (RFU = raw fluorescence units) of 100 ng RNA P&P SARS-CoV-2 GFP-1 and RNA P&P SARS-CoV-2 GFP-2 **(A, C)** and the fold-induction of GFP expression (normalized to RNA P&P SARS-CoV-2 GFP-1 and RNA P&P SARS-CoV-2 GFP-2 controls) **(B, D)** in the absence or presence of sRNA (8 µM, **SEQ ID NO: 46** for RNA P&P SARS-CoV-2 GFP-1 and **SEQ ID NO: 47** for RNA P&P SARS-CoV-2 GFP-2) after 1 to 3 hours. Fluorescence signal was read at 475 and 510 nm (excitation and emission wavelengths, respectively) in black bottom low-volume 384-well plates (Corning) on a Synergy H1 Hybrid Multi-Mode Plate Reader.

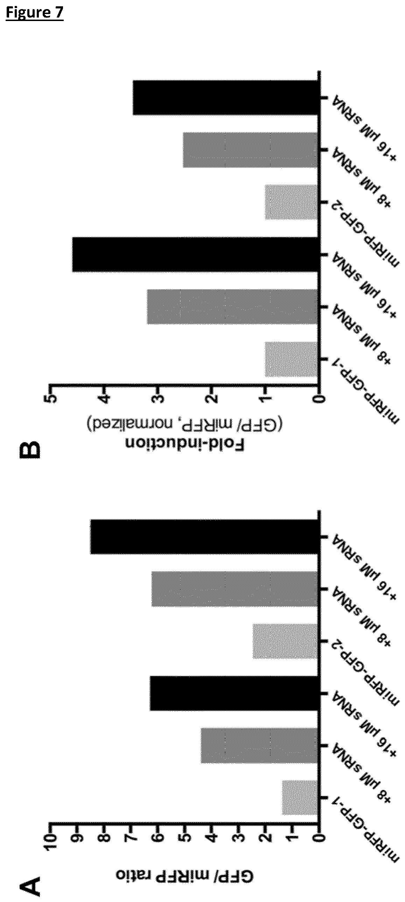

**Figure 7****. RNA P&P detection of SARS-CoV-2 with dual fluorescent (miRFP and GFP) readout.** These graphs show the GFP/ miRFP signal ratio (based on RFU = raw fluorescence units) **(A)** of 150 ng RNA P&P SARS-CoV-2 miRFP/GFP-1 **(SEQ ID NO: 65,** miRFP/GFP-1 in the graph) and RNA P&P SARS-CoV-2 miRFP/GFP-2 **(SEQ ID NO: 66,** miRFP/GFP-2 in the graph) in the absense or the presence of sRNA (8 or 16 µM, **SEQ ID NO: 46)** and **(B)** the fold-induction of GFP of RNA P&P in the presence sRNA versus control (normalizing data on miRFP RFU levels) after 1 hour. Fluorescence signal of miRFP670nano was read at 645 and 670 nm (excitation and emission wavelengths, respectively) and after 30 seconds delay, GFP was read at 475 and 510 nm (excitation and emission wavelengths, respectively) in black bottom low-volume 384-well plates (Corning) on a Synergy H1 Hybrid Multi-Mode Plate Reader.

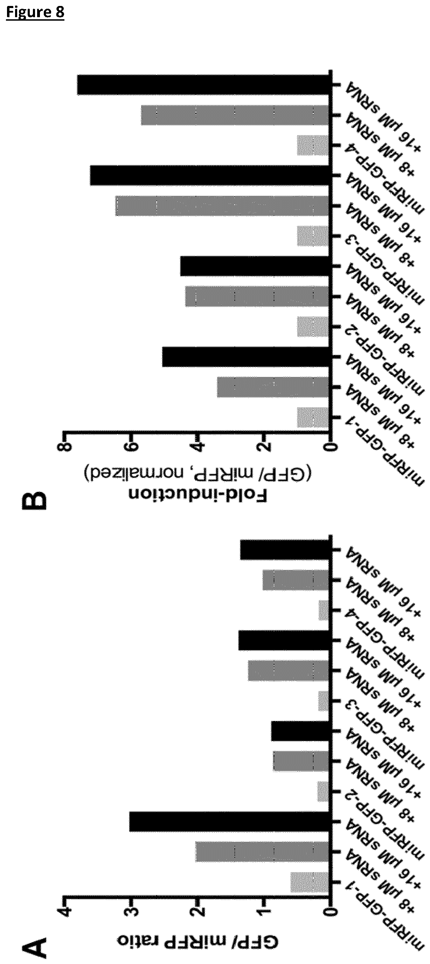

**Figure 8****. RNA P&P detection of *E.coli* with dual fluorescent (miRFP and GFP) readout.** These graphs show the GFP/ miRFP signal ratio (based on RFU = raw fluorescence units) **(A)** of 150 ng RNA P&P *E.coli* miRFP/GFP-1 **(SEQ ID NO: 83,** miRFP/GFP-1 in the graph), RNA P&P *E.coli* miRFP/GFP-2 **(SEQ ID NO: 84,** miRFP/GFP-2 in the graph), RNA P&P *E.coli* miRFP/GFP-3 **(SEQ ID NO: 85,** miRFP/GFP-3 in the graph) and RNA P&P *E.coli* miRFP/GFP-4 **(SEQ ID NO: 86,** miRFP/GFP-4 in the graph) in the absense or the presence of sRNA (8 or 16 µM, **SEQ ID NO: 18)** and **(B)** the fold-induction of GFP of RNA P&P in the presence sRNA versus control (normalizing data on miRFP RFU levels) after 1 hour. Fluorescence signal of miRFP670nano was read at 645 and 670 nm (excitation and emission wavelengths, respectively) and after 30 seconds delay, GFP was read at 475 and 510 nm (excitation and emission wavelengths, respectively) in black bottom low-volume 384-well plates (Corning) on a Synergy H1 Hybrid Multi-Mode Plate Reader.

### Detailed description

In a fist aspect and main embodiment, the present invention relates to an RNA molecule comprising or consisting of a sensor part and a trigger part, wherein the trigger part is an mRNA coding for a protein and the sensor part comprises or consists of 2 stem loops divided in the following regions in order from 5' to 3': T (T1,T2), A, B, C (C1,C2), D (D1,D2), E, F (F1,F2) and G, wherein
- region T1 is unpaired;
- T2 forms a stem with B where A is the loop region;
- C can be paired or unpaired;
- D forms a stem with F, where E is the loop region;
- F2 must contain an RBS;
- stem loop D1 basepairs with F2 and D2 with F1;
- G contains an AUG (corresponding to the start codon) and a linker and is immediately followed by the trigger part;
- further with the following structural limitations:
   a) T has a length of >= 30 nucleotides;
   b) T has the same number of nucleotides as B;
   c) D1 has the same number of nucleotides as F2 and D2 has the same number of nucleotides as F1:
   d) RBS in F1 is at least 3 nucleotides away from its start;
   e) (B + C1) has the same number of nucleotides as (D + E);
   f) D2 + E has the same number of nucleotides as T;
   g) C1 has the same number of nucleotides as D1;
   h) RBS in F2 must be at least 3 nucleotides away from F2 3' end;
further wherein in the absence of a target RNA, the RBS in region F2 is sequestered in the DEF stem loop and thus, translation of the trigger is not possible;
further wherein in the presence of a target RNA, the sensor part hybridizes the target RNA and a conformational change is produced, in which region T is sequestered by target RNA, B and C1 form a stem loop with D and E, where C2 is the loop region, F and G are partially or totally unpaired and thus the RBS in F2 is not sequestered and translation of the trigger is possible; and wherein RBS do not form stems with regions that also hybridize to the target RNA or form a stem with regions that hybridize with the regions which hybridize the target RNA.

In a preferred embodiment, the trigger part is an mRNA coding for a bioluminescence protein, a fluorescent protein or a colorimetric reporter protein.

In a further embodiment and as a particular case, the present invention relates to an RNA molecule, wherein in the sensor part:
- C contains an RBS in C1 and a sensor reporter gene different from the trigger part,
- RBS in C1 must be at least 3 nucleotides away from C1 each end;
- ATG of the reporter gene can be either on C1 or C2 as long as the distance from the RBS is from 3 to 10, preferably 5-6 nucleotides;
- D+E must be proportional to the length of C2; and
wherein in the absence of RNA target, RBS in C1 is partially or totally unpaired, and wherein in the presence of RNA target, C1-D1 are part of the stem BC1-DE.

Preferably, in this further embodiment, both the sensor and the trigger reporter genes are mRNAs coding for a bioluminescence protein, a fluorescent protein or a colorimetric reporter protein, as long as both reporter genes are different.

In the context of the present invention, "RBS" is understood as ribosome binding site, which is a sequence of nucleotides upstream of the start codon of an mRNA transcript that is responsible for the recruitment of a ribosome during the initiation of protein translation.

In the context of the present invention, "ATG" is understood as the codon for methionine or the translation initiation codon. Usually, protein translation can only start at a Methionine codon (although this codon may be found elsewhere within the protein sequence as well). In eukaryotic DNA, the sequence is ATG; in RNA it is AUG. Usually, the first AUG in the mRNA is the point at which translation starts, and an open reading frame follows - i.e. the nucleotides taken three at a time will code for the amino acids of the protein, and a stop codon will be found only when the protein coding region is complete.

In the context of the present invention, "stem" and "loop" regions are used as it is well known in the art. Stem-loop intramolecular base pairing is a pattern that can occur in single-stranded DNA or, more commonly, in RNA. The structure is also known as a hairpin or hairpin loop. It occurs when two regions of the same strand, usually complementary in nucleotide sequence when read in opposite directions, base-pair to form a double helix that ends in an unpaired loop. The resulting structure is a key building block of many RNA secondary structures. As an important secondary structure of RNA, it can direct RNA folding, protect structural stability for messenger RNA (mRNA), provide recognition sites for RNA binding proteins, and serve as a substrate for enzymatic reactions.

In the context of the present invention, a "linker" is an artificial RNA sequence that contains no stop codons in frame with the upstream start codon. It serves as a separation between the structurally relevant regions of the RNA P&P and the trigger. It will be translated as part of the trigger so it needs to maintain the frame of the trigger defined by the AUG upstream and it should be short (between 3 and 30 nucleotides preferably) so that it is not expected to jeopardize the activity of the trigger. Any artificial RNA sequence complying with this definition could be used as a linker in the present invention.

In the context of the present invention, "proportional" means that the longer is one region, the longer needs to be the second region, for example, longer C2 regions should imply longer D+E regions, but there is no need for a linear increase in length.

In the context of the present invention, "paired" is understood as for the bases (A, U, G, C for RNA) which stand for the chemicals adenine (A), uracil (U), guanine (G), and cytosine (C), respectively, adenine always pairs with uracil, and guanine always pairs with either cytosine or uracil. When referring to a region, pair or basepair means that all bases in the region are paired. In contrast "unpaired" means that at least one base is not paired.

In this disclosure and in the claims, terms such as "comprises," "comprising," "containing" and "having" are open-ended terms and can mean "includes," "including," and the like; while terms like "consisting of" or "consists of" refer to the mentioned elements after these terms and others which are not mentioned are excluded.

Unless otherwise explained, all technical and scientific terms used herein have the same meaning as commonly understood by one of ordinary skill in the art to which this disclosure belongs. The singular terms "a", "an", and "the" include plural referents unless context clearly indicates otherwise. Similarly, the word "or" is intended to include "and" unless the context clearly indicate otherwise.

In a preferred embodiment for any of the previous embodiments of the RNA molecule of the invention, said bioluminescence protein is selected from luciferase and aequorin, said fluorescent protein is selected from GFP (green fluorescent protein), YFP (yellow fluorescent protein) and RFP (red fluorescent protein) and said colorimetric reporter protein is LacZ (β-galactosidase).

In a preferred embodiment for the RNA molecule according to the main embodiment, there is no sensor reporter gene and the trigger is a LacZ mRNA and the sensor part corresponds to the following artificial RNA sequences selected from: SEQ ID NO: 4, SEQ ID NO: 5 and SEQ ID NO: 6 , where their respective artificial RNA targets are the following: SEQ ID NO: 1 (for SEQ ID NO: 4,5) and SEQ ID NO: 2 (for SEQ ID NO: 6)

In a preferred embodiment for any of the previous embodiments of RNA molecule of the invention, said artificial RNA target is a biomarker characterizing a disease selected from a viral infection, a bacterial infection, cancer or a genetic disease.

Preferably, according to the main embodiment, said biomarker is a region of the e.coli rRNA16s mRNA reverse complemented, the trigger is a LacZ mRNA and wherein the sensor part corresponds to the following artificial RNA sequence: SEQ ID NO: 16 and SEQ ID NO: 17 or alternatively, where the trigger is a GFP mRNA and wherein the sensor part corresponds to the following artificial RNA sequences: SEQ ID NO: 24, SEQ ID NO: 25, SEQ ID NO: 26 .or alternatively, according to the further embodiment and particular case, wherein the sensor reporter gene is RFP and the trigger is a GFP mRNA.

Also preferably, according to the main embodiment said biomarker is a region of the SARS-CoV-2 viral genome (SEQ ID NO: 38), the trigger is a LacZ mRNA and the sensor part corresponds to the following artificial RNA sequences: SEQ ID NO: 36 and SEQ ID NO: 37, or alternatively, said biomarker are regions of the SARS-CoV-2 viral genome reverse complemented (SEQ ID NO: 46, SEQ ID NO: 47), the trigger is a GFP mRNA and wherein the sensor part corresponds to the following artificial RNA sequences: SEQ ID NO: 44 and SEQ ID NO: 45, or alternatively, according to the further embodiment and particular case, said biomarker are regions of the SARS-CoV-2 viral genome reverse complemented, the sensor reporter gene is RFP and the trigger is a GFP mRNA.

In a second aspect, the present invention relates to the use of the RNA molecule according to any of the previous embodiments, in a colorimetric or fluorometric assay (where a transcription/translation assay is performed with the molecule and patient fluid and the result is read with the appropriate instrumentation) upon RNA target binding of said RNA molecule, wherein said RNA molecule is contained with a cell extract in the recipient to be used in an in-vitro diagnostic of a certain disease, such as bacterial infections, viral infections, cancer, genetic disorders, etc... In particular said disease is a bloodstream infection caused by pathogenic bacteria or COVID-19.

In a third aspect, the present invention relates to a process for detecting in vitro a disease, in particular as defined previously, comprising the steps of:
a) obtaining an isolated sample of blood or saliva or swabs of body fluids from an individual;
c) extracting and purifying the RNA;
d) amplifying the RNA obtained in step c) through isothermal amplification;
e) detecting the RNA from cells and/or microorganisms causing the disease through an RNA molecule as defined in the main embodiment of the present invention; and
f) identifying a positive result of the disease by confirming the expression of the trigger mRNA.

In the context of the present invention, "body fluids" is understood as blood, saliva, urine, etc...

In the context of the present invention, "isothermal amplification" is understood as and RNA to RNA amplification that requires no cyclic temperature changes, such as NASBA or TMA. For further information about NASBA or TMA, see https://www.neb.com/applications/dna-amplification-pcr-and-qpcr/isothermal-amplification/nucleic-acid-sequenced-based-amplification-and-transcription-mediated-amplification

In another process embodiment, the process for detecting in vitro a disease, in particular as defined previously, comprises the steps of:
a) obtaining an isolated sample of blood or saliva or swabs of body fluids from an individual;
c) extracting and purifying the RNA;
d) amplifying the RNA obtained in step c) through isothermal amplification;
e) detecting the RNA from cells and/or microorganisms causing the disease through an RNA molecule as defined in the further embodiment and particular case of the present invention; and
f) identifying a positive result of the disease by confirming the expression of the trigger mRNA.
g) identifying a negative result of the disease by confirming the expression of the sensor mRNA.
h) Null if no expression detected.

In a more preferred embodiment:
1) the isothermal amplification is selected from NASBA, TMA and SMART technologies, and/or
2) the RNA molecule as defined in the present invention is lyophilized, along with a cell extract, in a microfluidics device, and/or
3) the identification of a positive result in step f) is confirmed through a colorimetric or fluorometric assay wherein the trigger is a LacZ mRNA or GFP.
4) the identification of a negative result in step f) is confirmed through a fluorimetric assay wherein the sensor reporter is RFP.

In a further preferred embodiment, the process further comprises between steps a) and c): b) treating said isolated sample or swab with at least a lysate buffer and/or a heating step.

In a fourth aspect, the present invention relates to a kit for detecting in vitro a disease comprising:
a) at least one receiving means for receiving said sample or swab isolated from the individual;
b) a lysate buffer for treating the sample or swab;
c) heating capacity (a thermoblock for example)
d) at least the RNA molecule as defined in the present invention for the detection of a disease in the individual;
e) instructions for using the kit.

In a more preferred embodiment for the process, the kit, or the use, as previously characterized, said disease is a bloodstream infection caused by pathogenic bacteria or COVID-19.

A number of examples will be provided below that are intended to illustrate the invention and in no way limit the scope of the invention, which is established by the attached claims.

### EXAMPLES

### Example 1: Artificial RNA Plug&Plays (lacZ reporter)

We designed three RNA P&Ps for the detection of two different synthetic artificial RNAs **(SEQ ID NO: 1** and **SEQ ID NO: 2).** These RNA P&Ps are composed of a sensor region and a trigger. The trigger region corresponds to the *lacZ* gene **(SEQ ID NO: 3),** while the sensor regions correspond to RNA P&P artificial *lacZ*-1 **(SEQ ID NO: 4),** RNA P&P artificial *lacZ-2* **(SEQ ID NO: 5)** and RNA P&P artificial *lacZ*-3 **(SEQ ID NO: 6).** All sensor regions are designed to detect a synthetic artificial RNA **(SEQ ID NO: 1** for both RNA P&P artificial *lacZ-1* and *lacZ-2,* and **SEQ ID NO: 2** for RNA P&P artificial *lacZ*-3). In order to generate the complete RNA P&P sequences we performed PCR using YEp358 vector **(SEQ ID NO: 7)** with a 3' *lacZ* terminator primer **(SEQ ID NO: 8)** and 5' primers for P&P artificial *lacZ*-1 **(SEQ ID NO: 9),** for P&P artificial *lacZ*-2 **(SEQ ID NO: 10)** and for artificial *lacZ*-3 **(SEQ ID NO: 11).** The PCR reaction results in the three complete dsDNA products containing a T7 promoter for *in vitro* transcription, the P&P with a ribosome binding site (RBS) and the trigger region (*lacZ* gene) **(SEQ ID NO: 12, 13** and **14,** respectively).

In order to test the efficiency of our RNA P&P, we used **100 ng** of purified PCR product (dsDNA) in a cell-free system that permits mRNA transcription and protein synthesis using recombinant elements (PURExpress, New England Biolabs) in the absence or presence of the corresponding artificial synthetic RNA (sRNA) **(SEQ ID NO: 1** and **SEQ ID NO: 2).** In the absence of the latter, the P&P structure of RNA (blocking access to the RBS) should prevent translation of β-galactosidase (protein product of *lacZ* gene). On the contrary, in the presence of the sRNA **(SEQ ID NO: 1** for both RNA P&P artificial *lacZ*-1 and *lacZ-2,* and **SEQ ID NO: 2** for RNA P&P artificial *lacZ-3),* RNA P&P structure changes conformation upon hybridization and translation of β-galactosidase occurs. **Figure 1** depicts the result of all P&Ps with and without the addition of 2.35 µM of sRNA (1.5 uL of **SEQ ID NO: 1)** after 45 minutes (for RNA P&P artificial *lacZ*-1 and *lacZ-2)* and with and without the addition of 0.8, 1.6 and 2.35 µM of sRNA (0.5, 1.0 and 1.5 uL of **SEQ ID NO: 2,** respectively) for RNA P&P artificial *lacZ*-3 after 1, 2 and 3 hours (for RNA P&P artificial *lacZ-3)* from the start of the reaction.

In this case, using a colorimetric reaction readout, the enzyme expressed cleaves the *yellow* (negative, control) chloro phenol red-β-D-galactopyranoside (CPRG) substrate present in the mix to produce the *purple* (positive, with sRNA) chlorophenol red product.

### Example 2: RNA Plug&Play for E.coli 16S rRNA detection (lacZ reporter)

We designed two RNA P&Ps for the detection of *E.coli* 16S rRNA **(SEQ ID NO: 15).** These RNA P&Ps are composed of a sensor region and a trigger. The trigger region corresponds to the *lacZ* gene **(SEQ ID NO: 3),** while the sensor regions correspond to RNA P&P *E.coli lacZ*-1 **(SEQ ID NO: 16)** and *E.coli lacZ-2* **(SEQ ID NO: 17).** Both sensor regions are designed to detect the reverse complement of a region of the 16S rRNA of *E.coli* **(SEQ ID NO: 18).** The region is reverse complemented to simulate the result of a prior step of NASBA amplification (Kao et al. Detection of Escherichia coli Using Nucleic Acid Sequence-Based Amplification and Oligonucleotide Probes for 16S Ribosomal RNA. Analytical Letters, 43, 1756-1769 (2010). In order to generate the complete RNA P&P sequences we performed PCR using YEp358 vector **(SEQ ID NO: 7)** with a 3' *lacZ* terminator primer **(SEQ ID NO: 8)** and 5' primers for RNA P&P *E.coli lacZ*-1 **(SEQ ID NO: 19)** and for RNA P&P *E.coli lacZ-*2 **(SEQ ID NO: 20).** The PCR results in the two complete dsDNA products containing a T7 promoter for transcription, the P&P with a ribosome binding site (RBS) and the trigger region (*lacZ* gene) **(SEQ ID NO: 21** and **SEQ ID NO: 22,** respectively).

In order to test the efficiency of our RNA P&P, we used 100 ng of purified PCR product (dsDNA) in a cell-free system that permits mRNA transcription and protein synthesis using recombinant elements (PURExpress, New England Biolabs) in the absence or presence of the synthetic RNA target (sRNA) of *E.coli* 16S RNA **(SEQ ID NO: 18).** In the absence of the latter, the P&P structure of RNA (blocking access of RBS) should prevent translation of β-galactosidase (protein product of *lacZ*). On the contrary, in the presence of the sRNA **(SEQ ID NO: 18),** RNA P&P structure changes conformation upon hybridization and translation of β-galactosidase occurs. **Figure 2** depicts the result of both P&Ps with and without the addition of 1.6 µM of sRNA after 1 hour from the start of the reaction.

In this case, using colorimetric reaction readout, the enzyme expressed cleaves the *yellow* (negative, control) chloro phenol red-β-D-galactopyranoside (CPRG) substrate present in the mix to produce the *purple* (positive, with sRNA) chlorophenol red product.

### Example 3: RNA Plug&Play for E.coli 16S rRNA detection (GFP reporter)

We designed three RNA P&Ps for the detection of *E.coli* 16S rRNA **(SEQ ID NO: 15).** These RNA P&Ps are composed of a sensor region and a trigger. The trigger region corresponds to the *green fluorescent protein (gfp) mut3b-ASV* (*gfpmut3b-ASV*) gene **(SEQ ID NO: 23),** while the sensor regions correspond to RNA P&P *E.coli* GFP-1 **(SEQ ID NO: 24),** RNA P&P *E.coli* GFP-2 **(SEQ ID NO: 25)** and RNA P&P *E.coli* GFP-3 **(SEQ ID NO: 26).** Both sensor regions are designed to detect the reverse complement of a region of the 16S rRNA of *E.coli* **(SEQ ID NO: 18).** The region is reverse complemented to simulate the result of a prior step of NASBA amplification (Kao et al. Detection of Escherichia coli Using Nucleic Acid Sequence-Based Amplification and Oligonucleotide Probes for 16S Ribosomal RNA. Analytical Letters, 43, 1756-1769 (2010)). In order to generate the complete RNA P&P sequences we performed PCR using a *gfpmut3b-ASV* containing synthetic construct **(SEQ ID NO: 27)** with a 3' GFP terminator primer **(SEQ ID NO: 28)** and 5' primers for RNA P&P *E.coli* GFP-1 **(SEQ ID NO: 29),** for RNA P&P *E.coli* GFP-2 **(SEQ ID NO: 30)** and for RNA P&P *E.coli* GFP-3 **(SEQ ID NO: 31).** The PCR results in the complete dsDNA products containing a T7 promoter for transcription, the P&P with a ribosome binding site (RBS) and the trigger region (*gfpmut3b-ASV* gene) **(SEQ ID NO: 32, 33** and **34** for P&P *E.coli* GFP-1, -2 and -3, respectively).

In order to test the efficiency of our RNA P&P, we used 100 ng (RNA P&P *E.coli* GFP-1) and 50 ng (RNA P&P *E.coli* GFP-2 and RNA P&P *E.coli* GFP-3) of purified PCR product (dsDNA) in a cell-free system that permits mRNA transcription and protein synthesis using recombinant elements (PURExpress, New England Biolabs) in the absence or presence of the sRNA of *E.coli* 16S rRNA **(SEQ ID NO: 15).** In the absence of the latter, the P&P structure of RNA (blocking access to the RBS) should prevent translation of GFPmut3b-ASV protein. On the contrary, in the presence of the sRNA **(SEQ ID NO: 15),** RNA P&P structure changes conformation upon hybridization and translation of β-galactosidase occurs. **Figure 3** shows the result (in terms of both raw fluorescence and fold change) of both RNA P&Ps with and without the addition of 4, 8 and 16 µM of sRNA **(SEQ ID NO: 15)** after 1 to 4 hours from the start of the reaction.

In this case, the fluorescent readout of the translated GFPmut3b-ASV was used considering a ratio of 2.0 (P&P+sRNA over control) as the threshold for an effective detection. When expressed, GFPmut3b-ASV fluoresces with an excitation and emission wavelengths of 475 and 510 nm, respectively. It is an unstable GFP form with a half life of 110 min, what reduces the background (autofluorescence) to minimum levels, particularly when working in the cellular environment (Chapel et al. Computational design of small transcription activating RNAs for versatile and dynamic gene regulation. Nature Communications, 8, 1051 (2017).

### Example 4: RNA Plug&Play for Severe Acute Respiratory Syndrome Coronavirus-2 (SARS-CoV-2) detection (lacZ reporter)

We designed two RNA P&Ps for the detection of SARS-CoV-2 *orf1ab* gene **(SEQ ID NO: 35).** These RNA P&Ps are composed of a sensor region and a trigger. The trigger region corresponds to the *lacZ* gene **(SEQ ID NO: 3),** while the sensor regions correspond to RNA P&P SARS-CoV-2 *lacZ*-1 **(SEQ ID NO: 36)** and RNA P&P SARS-CoV-2 *lacZ-2* **(SEQ ID NO: 37).** Both sensor regions are designed to detect a specific region of the *orf1ab* gene of SARS-CoV-2 **(SEQ ID NO: 38).** In order to generate the complete RNA P&P sequences we performed PCR using YEp358 vector **(SEQ ID NO: 7)** with a 3' *lacZ* terminator primer **(SEQ ID NO: 8)** and 5' primers for SARS-CoV-2 *lacZ*-1 **(SEQ ID NO: 39)** and for SARS-CoV-2 *lacZ-2* **(SEQ ID NO: 40).** The PCR results in the complete dsDNA products containing a T7 promoter for transcription, the P&P with a ribosome binding site (RBS) and the trigger region (*lacZ* gene) **(SEQ ID NO: 41** and **42** for SARS-CoV-2 *lacZ-1* and *lacZ-2,* respectively)

In order to test the efficiency of our RNA P&P, we used 100 ng of purified PCR product (dsDNA) in a recombinant protein transcription/ translation system that permits mRNA transcription and protein synthesis using recombinant elements (PURExpress, New England Biolabs) in the absence or presence of the SARS-CoV-2 sRNA of the *orf1ab* gene region of interest **(SEQ ID NO: 38).** In the absence of the latter, the P&P structure of RNA (blocking access to the RBS) should prevent translation of β-galactosidase (product of *lacZ*). On the contrary, in the presence of the sRNA **(SEQ ID NO: 38),** RNA P&P structure changes conformation upon binding and translation of β-galactosidase occurs. **Figure 4** depicts the result of both P&Ps with and without the addition of 2.35 µM of RNA target after 2 hours from the start of the reaction.

In this case, using colorimetric reaction readout, the enzyme expressed cleaves the *yellow* (negative, control) chloro phenol red-β-D-galactopyranoside (CPRG) substrate present in the mix to produce the *purple* (positive, with sRNA) chlorophenol red product.

### Example 5: RNA Plug&Play for SARS-CoV-2 detection (GFP reporter)

We designed two RNA P&Ps for the detection of SARS-CoV-2 *orf1ab* gene **(SEQ ID NO: 35)** and *orf8* gene **(SEQ ID NO: 43).** These RNA P&Ps are composed of a sensor region and a trigger. The trigger region corresponds to the *green fluorescent protein (gfp) mut3b-ASV* (*gfpmut3b-ASV*) gene **(SEQ ID NO: 23),** while the sensor regions correspond to RNA P&P SARS-CoV-2 GFP-1 **(SEQ ID NO: 44)** and RNA P&P SARS-CoV-2 GFP-2 **(SEQ ID NO: 45).** Both sensor regions are designed to detect the reverse complement of a region of the SARS-CoV-2 *orf1ab* gene **(SEQ ID NO: 46** for RNA P&P SARS-CoV-2 GFP-1) and *orf8* gene **(SEQ ID NO: 47** for RNA P&P SARS-CoV-2 GFP-2). The region is reverse complemented to simulate the result of a prior step of NASBA amplification. In order to generate the complete RNA P&P sequences we performed PCR using a *gfpmut3b-ASV* containing synthetic construct **(SEQ ID NO: 27)** with a 3' GFP terminator primer **(SEQ ID NO: 28)** and 5' primers for RNA P&P SARS-CoV-2 GFP-1 **(SEQ ID NO: 48)** and for RNA P&P SARS-CoV-2 GFP-2 **(SEQ ID NO: 49).** The PCR results in the complete dsDNA products containing a T7 promoter for transcription, the P&P with a ribosome binding site (RBS) and the trigger region (*gfpmut3b-ASV* gene) **(SEQ ID NO: 50** and **51** for P&P SARS-CoV-2 GFP-1 and GFP-2, respectively).

In order to test the efficiency of our RNA P&P, we used 100 ng of purified PCR product (dsDNA) in a cell-free system that permits mRNA transcription and protein synthesis using recombinant elements (PURExpress, New England Biolabs) in the absence or presence of the SARS-CoV-2 sRNA **(SEQ ID NO: 46** for RNA P&P SARS-CoV-2 GFP-1 and **SEQ ID NO: 47** for RNA P&P SARS-CoV-2 GFP-2). In the absence of the sRNA, the P&P structure of RNA (blocking access to the RBS) should prevent translation of GFPmut3b-ASV protein. On the contrary, in the presence of the sRNA **(SEQ ID NO: 46** and **SEQ ID NO: 47),** RNA P&P structure changes conformation upon hybridization and translation of GFPmut3b-ASV occurs. **Figure 5** shows the result of both RNA P&Ps (in terms of both raw fluorescence and fold change) with and without the addition of 8 µM of sRNA after **1 to 3 hours** from the start of the reaction.

In this case, the fluorescent readout of the translated GFPmut3b-ASV was used considering a ratio of 2.0 (P&P+sRNA over control) as positive. When expressed, GFPmut3b-ASV fluoresces with an excitation and emission wavelengths of 475 and 510 nm, respectively. It is an unstable GFP form with a half life of ∼110 min, what reduces the background (autofluorescence) to minimum levels, specially when working in the cellular environment (Chapell et al. Computational design of small transcription activating RNAs for versatile and dynamic gene regulation. Nature Communications, 8, 1051 (2017)).

### Example 6: RNA Plug&Play for SARS-CoV-2 detection (miRFP/GFP reporter)

We designed two RNA P&Ps RFP/GFP for the detection of SARS-CoV-2 *orf1ab* gene **(SEQ ID NO: 35).** These RNA P&Ps miRFP/GFP are composed of a sensor region, a sensor reporter gene and a trigger region (claim 2). The sensor reporter corresponds to the *miRFP670nano* gene **(SEQ ID NO: 52),** while the trigger corresponds to *gfpmut3b-ASV* gene **(SEQ ID NO: 23).** The sensor regions correspond to RNA P&P SARS-CoV-2 RFP/GFP-1 and **-2 (SEQ ID NO: 53** and **SEQ ID NO: 54),** which include the sensor reporter gene. These sensor regions are designed to detect the reverse complement of a region of the *orf1ab* gene **(SEQ ID NO: 46).**

In order to generate the complete RNA P&P sequences we performed two initial PCR reactions (PCR#1 and PCR#2) and an Overlap Extension PCR (OE-PCR). The first PCR (PCR#1) used the dsDNA *miRFP670nano* synthetic gene **(SEQ ID NO: 52)** as template, a 5' primer #1 containing a T7 for RNA P&P SARS-CoV-2 miRFP/GFP-1 and -2 **(SEQ ID NO: 55** and **SEQ ID NO: 56)** and a common 3' primer (with 32 overlapping nucleotides with PCR#2 5' region) **(SEQ ID NO: 57)** resulting in the PCR product #1 for RNA P&P SARS-CoV-2 miRFP/GFP-1 and -2 **(SEQ ID NO: 58** and **SEQ ID NO: 59,** respectively). The second PCR (PCR#2) reaction used the the *gfpmut3b-ASV* synthetic gene **(SEQ ID NO: 27)** as template, a common 5' primer #2 for RNA P&P SARS-CoV-2 RFP/GFP-1 and -2 **(SEQ ID NO: 60)** (containing the end of *miRFP670nano* gene, RBS and upstream region of GFP start codon) and a common 3' GFP terminator primer **(SEQ ID NO: 28)** resulting in the PCR product #2 for RNA P&P SARS-CoV-2 miRFP/GFP-1 and -2 **(SEQ ID NO: 61** and **SEQ ID NO: 62,** respectively). With the two PCR products for each P&P miRFP/GFP, we performed an OE-PCR by running 15 cycles (without primers addition) to anneal the 32-nt overlapping region (marked in grey in **SEQ ID Nos: 57 to 62)** and then adding primers 5' T7 **(SEQ ID NO: 63)** and 3' GFP term **(SEQ ID NO: 64)** to perform 30 cycles to finish the reaction. The products of this OE-PCR were purified and constituted the final constructs to be assayed **(SEQ ID NO: 65** and **66).**

In order to test the efficiency of our RNA P&P, we used 150 ng of the final PCR products **(SEQ ID NO: 65** and **66)** in a cell-free system that permits mRNA transcription and protein synthesis using recombinant elements (PURExpress, New England Biolabs) in the absence or presence of the SARS-CoV-2 *orf1ab* gene sRNA **(SEQ ID NO: 46).** In the absence of the sRNA, the RNA P&P structure of the sensor allows the translation of miRFP670nano protein while, at the same time, prevents the translation of GFPmut3b-ASV protein. On the contrary, in the presence of the sRNA and upon hybridization, RNA P&P structure of the sensor changes conformation and translation of GFPmut3b-ASV occurs whereas miRFP670nano translation is blocked. **Figure 7** shows the result of both RNA P&Ps with and without the addition of 8 and 16 µM of the SARS-CoV-2 sRNA **(SEQ ID NO: 46)** after 1 hour from the start of the reaction.

In this case, the fluorescent readout of the translated GFPmut3b-ASV is read at an excitation and emission wavelengths of 475 and 510 nm, respectively. miRFP670nano fluorescence in the presence of biliverdin is read at an excitation and emission wavelengths of 645 and 675 nm, respectively. Ratios of GFP/miRFP > 2.5 (or fold-induction over 2.0) indicate a positive outcome (presence of sRNA) and ratios of GFP/miRFP ≤ 2.5 (or fold-induction below 2.0) indicate a negative outcome (sRNA is not present or null/poor hybridization).

### Example 7: RNA Plug&Play for E.coli 16S rRNA detection (miRFP/GFP reporter)

We designed two RNA P&Ps miRFP/GFP for the detection of *E.coli* 16S rRNA **(SEQ ID NO: 15).** These RNA P&Ps miRFP/GFP are composed of a sensor region, a sensor reporter gene and a trigger region (claim 2). The sensor reporter corresponds to the *miRFP670nano* gene **(SEQ ID NO: 52),** while the trigger corresponds to *gfpmut3b-ASV* gene **(SEQ ID NO: 23).** The sensor regions correspond to RNA P&P *E.coli* miRFP/GFP-1, -2, -3 and -4 **(SEQ ID NOs: 67 to 70),** which include the sensor reporter gene. These sensor regions are designed to detect the reverse complement of a region of the 16S rRNA of *E.coli* **(SEQ ID NO: 18).** The region is reverse complemented to simulate the result of a prior step of NASBA amplification (Kao et al. Anal Letters, 2010).

In order to generate the complete RNA P&P sequences we performed two initial PCR reactions (PCR#1 and PCR#2) and an Overlap Extension PCR (OE-PCR). The first PCR (PCR#1) used the dsDNA *miRFP670nano* synthetic gene **(SEQ ID NO: 52)** as template, a 5' primer #1 containing a T7 for RNA P&P *E.coli* miRFP/GFP-1, -2, -3 and -4 **(SEQ ID Nos: 71 to 74)** and a common 3' primer (with 32 overlapping nucleotides with PCR#2 5' region) **(SEQ ID NO: 57)** resulting in the PCR product #1 for RNA P&P *E.coli* miRFP/GFP-1, -2, -3 and -4 **(SEQ ID Nos: 75 to 78).** The second PCR (PCR#2) reaction used the the *gfpmut3b-ASV* synthetic gene **(SEQ ID NO: 27)** as template, a 5' primer #2 **(SEQ ID NO: 79** for RNA P&P *E.coli* miRFP/GFP-1 and **SEQ ID NO: 80** for *E.coli* miRFP/GFP-2, -3 and -4) containing the end of *miRFP670nano* gene, RBS and upstream region of GFP start codon and a common 3' GFP terminator primer **(SEQ ID NO: 28)** resulting in the PCR products #2 for RNA P&P *E.coli* miRFP/GFP-1 **(SEQ ID NO: 81)** and RNA P&P *E.coli* miRFP/GFP-2, -3 and -4 **(SEQ ID NO: 82).** With the two PCR products for each P&P miRFP/GFP, we performed an OE-PCR by running 15 cycles (without primers addition) to anneal the 32-nt overlapping region (marked in grey in **SEQ ID NOs: 57, 75 to 82)** and then adding primers 5' T7 **(SEQ ID NO: 63)** and 3' GFP term **(SEQ ID NO: 64)** to perform 30 cycles to finish the reaction. The products of this OE-PCR were purified and constituted the final constructs to be assayed **(SEQ ID NOs: 83 to 86).**

In order to test the efficiency of our RNA P&P, we used 150 ng of the final PCR products **(SEQ ID NOs: 83 to 86)** in a cell-free system that permits mRNA transcription and protein synthesis using recombinant elements (PURExpress, New England Biolabs) in the absence or presence of the 16S rRNA *E.coli* sRNA **(SEQ ID NO: 18).** In the absence of the sRNA, the RNA P&P structure of the sensor allows the translation of miRFP670nano protein while, at the same time, prevents the translation of GFPmut3b-ASV protein. On the contrary, in the presence of the sRNA and upon hybridization, RNA P&P structure of the sensor changes conformation and translation of GFPmut3b-ASV occurs whereas miRFP670nano translation is blocked. **Figure 8** shows the result of both RNA P&Ps with and without the addition of 8 and 16 µM of the *E.coli 16S* sRNA **(SEQ ID NO: 18)** after 1 hour from the start of the reaction.

In this case, the fluorescent readout of the translated GFPmut3b-ASV is read at an excitation and emission wavelengths of 475 and 510 nm, respectively. miRFP670nano fluorescence in the presence of 10 µM biliverdin (chromophore) is read at an excitation and emission wavelengths of 645 and 675 nm, respectively. Ratios of GFP/miRFP > 1.0 indicate a positive outcome (presence of sRNA) and ratios of GFP/miRFP ≤ 1.0, indicate a negative outcome (sRNA is not present or null/poor hybridization).

The list of sequences mentioned herein is as follows:
Sequence #1:
   > **Synthetic RNA target artificial-1/2**
   GUCCAACAGCGCCAGAGAACAACUAUGAUG
Sequence #2:
   > **Synthetic RNA target artificial-3**
   CGCCGCUGGCCCGGGUUGGAUAAUAUUUAG
Sequence #3
   *>* ***lacZ* gene** from NC 000913.3:c366305-363231 *Escherichia coli* str. *K-12* substr. *MG1655,* complete genome 
ATGCACGGTTACGATGCGCCCATCTACACCAACGTGACCTATCCCATTACGGTCAAT
CCGCCGTTTGTTCCCACGGAGAATCCGACGGGTTGTTACTCGCTCACATTTAATGTT
GATGAAAGCTGGCTACAGGAAGGCCAGACGCGAATTATTTTTGATGGCGTTAACTCG
GCGTTTCATCTGTGGTGCAACGGGCGCTGGGTCGGTTACGGCCAGGACAGTCGTTT
GCCGTCTGAATTTGACCTGAGCGCATTTTTACGCGCCGGAGAAAACCGCCTCGCGG
TGATGGTGCTGCGCTGGAGTGACGGCAGTTATCTGGAAGATCAGGATATGTGGCGG
ATGAGCGGCATTTTCCGTGACGTCTCGTTGCTGCATAAACCGACTACACAAATCAGC
GATTTCCATGTTGCCACTCGCTTTAATGATGATTTCAGCCGCGCTGTACTGGAGGCT
GAAGTTCAGATGTGCGGCGAGTTGCGTGACTACCTACGGGTAACAGTTTCTTTATGG
CAGGGTGAAACGCAGGTCGCCAGCGGCACCGCGCCTTTCGGCGGTGAAATTATCGA
TGAGCGTGGTGGTTATGCCGATCGCGTCACACTACGTCTGAACGTCGAAAACCCGA
AACTGTGGAGCGCCGAAATCCCGAATCTCTATCGTGCGGTGGTTGAACTGCACACC
GCCGACGGCACGCTGATTGAAGCAGAAGCCTGCGATGTCGGTTTCCGCGAGGTGC
 
GGATTGAAAATGGTCTGCTGCTGCTGAACGGCAAGCCGTTGCTGATTCGAGGCGTTA
ACCGTCACGAGCATCATCCTCTGCATGGTCAGGTCATGGATGAGCAGACGATGGTG
CAGGATATCCTGCTGATGAAGCAGAACAACTTTAACGCCGTGCGCTGTTCGCATTAT
CCGAACCATCCGCTGTGGTACACGCTGTGCGACCGCTACGGCCTGTATGTGGTGGA
TGAAGCCAATATTGAAACCCACGGCATGGTGCCAATGAATCGTCTGACCGATGATCC
GCGCTGGCTACCGGCGATGAGCGAACGCGTAACGCGAATGGTGCAGCGCGATCGT
AATCACCCGAGTGTGATCATCTGGTCGCTGGGGAATGAATCAGGCCACGGCGCTAA
TCACGACGCGCTGTATCGCTGGATCAAATCTGTCGATCCTTCCCGCCCGGTGCAGTA
TGAAGGCGGCGGAGCCGACACCACGGCCACCGATATTATTTGCCCGATGTACGCGC
GCGTGGATGAAGACCAGCCCTTCCCGGCTGTGCCGAAATGGTCCATCAAAAAATGG
CTTTCGCTACCTGGAGAGACGCGCCCGCTGATCCTTTGCGAATACGCCCACGCGAT
GGGTAACAGTCTTGGCGGTTTCGCTAAATACTGGCAGGCGTTTCGTCAGTATCCCCG
TTTACAGGGCGGCTTCGTCTGGGACTGGGTGGATCAGTCGCTGATTAAATATGATGA
AAACGGCAACCCGTGGTCGGCTTACGGCGGTGATTTTGGCGATACGCCGAACGATC
GCCAGTTCTGTATGAACGGTCTGGTCTTTGCCGACCGCACGCCGCATCCAGCGCTG
ACGGAAGCAAAACACCAGCAGCAGTTTTTCCAGTTCCGTTTATCCGGGCAAACCATC
GAAGTGACCAGCGAATACCTGTTCCGTCATAGCGATAACGAGCTCCTGCACTGGATG
GTGGCGCTGGATGGTAAGCCGCTGGCAAGCGGTGAAGTGCCTCTGGATGTCGCTCC
ACAAGGTAAACAGTTGATTGAACTGCCTGAACTACCGCAGCCGGAGAGCGCCGGGC
AACTCTGGCTCACAGTACGCGTAGTGCAACCGAACGCGACCGCATGGTCAGAAGCC
GGGCACATCAGCGCCTGGCAGCAGTGGCGTCTGGCGGAAAACCTCAGTGTGACGC
TCCCCGCCGCGTCCCACGCCATCCCGCATCTGACCACCAGCGAAATGGATTTTTGC
ATCGAGCTGGGTAATAAGCGTTGGCAATTTAACCGCCAGTCAGGCTTTCTTTCACAG
ATGTGGATTGGCGATAAAAAACAACTGCTGACGCCGCTGCGCGATCAGTTCACCCGT
GCACCGCTGGATAACGACATTGGCGTAAGTGAAGCGACCCGCATTGACCCTAACGC
CTGGGTCGAACGCTGGAAGGCGGCGGGCCATTACCAGGCCGAAGCAGCGTTGTTG
CAGTGCACGGCAGATACACTTGCTGATGCGGTGCTGATTACGACCGCTCACGCGTG
GCAGCATCAGGGGAAAACCTTATTTATCAGCCGGAAAACCTACCGGATTGATGGTAG
TGGTCAAATGGCGATTACCGTTGATGTTGAAGTGGCGAGCGATACACCGCATCCGG
CGCGGATTGGCCTGAACTGCCAGCTGGCGCAGGTAGCAGAGCGGGTAAACTGGCT
CGGATTAGGGCCGCAAGAAAACTATCCCGACCGCCTTACTGCCGCCTGTTTTGACC
GCTGGGATCTGCCATTGTCAGACATGTATACCCCGTACGTCTTCCCGAGCGAAAACG
GTCTGCGCTGCGGGACGCGCGAATTGAATTATGGCCCACACCAGTGGCGCGGCGA
CTTCCAGTTCAACATCAGCCGCTACAGTCAACAGCAACTGATGGAAACCAGCCATCG
CCATCTGCTGCACGCGGAAGAAGGCACATGGCTGAATATCGACGGTTTCCATATGG
GGATTGGTGGCGACGACTCCTGGAGCCCGTCAGTATCGGCGGAATTCCAGCTGAGC
GCCGGTCGCTACCATTACCAGTTGGTCTGGTGTCAAAAATAA
Sequence #4
   **> RNA P&P artificial *lacZ*-1** 
GGCCAUCAUAGUUGUUCUCUGGCGCUGUUGGACGAAAACAGUCCAGCAGCGCCG
GGAAGGAGGGGCGAGGCAUAAGUUCCUCCUUCUUGGCGUUGUUGGACCUAAGAA
GGAGGAAUUUAUGUCAUAUCUCACACAACGGGAGGUCGUUUUACAACGUCGU
Sequence #5
   **> RNA P&P artificial *lacZ*-2** 
GGCCAUCAUAGUUGUUCUCUGGCGCUGUUGGACGAAAACAGUCCAGCAGCGCCG
GGAAGGAGGGGCGAGAGAUAAGUUCCUCCUUCUUGGCGUUGUUGGACCUAAGAA
GGAGGAAUUUAUGUCAUAUCUCACACAACGGGAGGUCGUUUUACAACGUCGU
Sequence #6
   > **RNA P&P artificial *lacZ*-3** 
GGCCUAAAUAUUAUCCAACCCGGGCCAGCGGCGGAUAAAAUGUCGUUGGUCCGG
GAGAGGGAGGCGUGAAAAAAUUGUCUUUCUUUUUCGGAUUAAUGAUACGAAAAAG
GAGGAUAUCAUGUACUACAGCUUCGAUCCCGCCGUCGUUUUACAACGUCGU
Sequence #7
   > **YEp358 vector**
   *http:*//*genome-www.stanford.edu*/*vectordb*/*vector descrip*/*COMPLETE*/*YEP358. SEQ.html* 
GAATTCGAGCTCGGTACCCGGGGATCCTCTAGAGTCGACCTGCAGGCATGCAAGCT
TCGATCCCGCCGTCGTTTTACAACGTCGTGACTGGGAAAACCCTGGCGTTACCCAAC
TTAATCGCCTTGCAGCACATCCCCCTTTCGCCAGCTGGCGTAATAGCGAAGAGGCCC
GCACCGATCGCCCTTCCCAACAGTTGCGCAGCCTGAATGGCGAATGGCGCTTTGCC
TGGTTTCCGGCACCAGAAGCGGTGCCGGAAAGCTGGCTGGAGTGCGATCTTCCTGA
GGCCGATACTGTCGTCGTCCCCTCAAACTGGCAGATGCACGGTTACGATGCGCCCA
TCTACACCAACGTAACCTATCCCATTACGGTCAATCCGCCGTTTGTTCCCACGGAGA
ATCCGACGGGTTGTTACTCGCTCACATTTAATGTTGATGAAAGCTGGCTACAGGAAG
GCCAGACGCGAATTATTTTTGATGGCGTTAACTCGGCGTTTCATCTGTGGTGCAACG
GGCGCTGGGTCGGTTACGGCCAGGACAGTCGTTTGCCGTCTGAATTTGACCTGAGC
GCATTTTTACGCGCCGGAGAAAACCGCCTCGCGGTGATGGTGCTGCGTTGGAGTGA
CGGCAGTTATCTGGAAGATCAGGATATGTGGCGGATGAGCGGCATTTTCCGTGACG
 
TCTCGTTGCTGCATAAACCGACTACACAAATCAGCGATTTCCATGTTGCCACTCGCTT
TAATGATGATTTCAGCCGCGCTGTACTGGAGGCTGAAGTTCAGATGTGCGGCGAGTT
GCGTGACTACCTACGGGTAACAGTTTCTTTATGGCAGGGTGAAACGCAGGTCGCCA
GCGGCACCGCGCCTTTCGGCGGTGAAATTATCGATGAGCGTGGTGGTTATGCCGAT
CGCGTCACACTACGTCTGAACGTCGAAAACCCGAAACTGTGGAGCGCCGAAATCCC
GAATCTCTATCGTGCGGTGGTTGAACTGCACACCGCCGACGGCACGCTGATTGAAG
CAGAAGCCTGCGATGTCGGTTTCCGCGAGGTGCGGATTGAAAATGGTCTGCTGCTG
CTGAACGGCAAGCCGTTGCTGATTCGAGGCGTTAACCGTCACGAGCATCATCCTCTG
CATGGTCAGGTCATGGATGAGCAGACGATGGTGCAGGATATCCTGCTGATGAAGCA
GAACAACTTTAACGCCGTGCGCTGTTCGCATTATCCGAACCATCCGCTGTGGTACAC
GCTGTGCGACCGCTACGGCCTGTATGTGGTGGATGAAGCCAATATTGAAACCCACG
GCATGGTGCCAATGAATCGTCTGACCGATGATCCGCGCTGGCTACCGGCGATGAGC
GAACGCGTAACGCGAATGGTGCAGCGCGATCGTAATCACCCGAGTGTGATCATCTG
GTCGCTGGGGAATGAATCAGGCCACGGCGCTAATCACGACGCGCTGTATCGCTGGA
TCAAATCTGTCGATCCTTCCCGCCCGGTGCAGTATGAAGGCGGCGGAGCCGACACC
ACGGCCACCGATATTATTTGCCCGATGTACGCGCGCGTGGATGAAGACCAGCCCTT
CCCGGCTGTGCCGAAATGGTCCATCAAAAAATGGCTTTCGCTACCTGGAGAGACGC
GCCCGCTGATCCTTTGCGAATACGCCCACGCGATGGGTAACAGTCTTGGCGGTTTC
GCTAAATACTGGCAGGCGTTTCGTCAGTATCCCCGTTTACAGGGCGGCTTCGTCTGG
GACTGGGTGGATCAGTCGCTGATTAAATATGATGAAAACGGCAACCCGTGGTCGGCT
TACGGCGGTGATTTTGGCGATACGCCGAACGATCGCCAGTTCTGTATGAACGGTCTG
GTCTTTGCCGACCGCACGCCGCATCCAGCGCTGACGGAAGCAAAACACCAGCAGCA
GTTTTTCCAGTTCCGTTTATCCGGGCAAACCATCGAAGTGACCAGCGAATACCTGTT
CCGTCATAGCGATAACGAGCTCCTGCACTGGATGGTGGCGCTGGATGGTAAGCCGC
TGGCAAGCGGTGAAGTGCCTCTGGATGTCGCTCCACAAGGTAAACAGTTGATTGAAC
TGCCTGAACTACCGCAGCCGGAGAGCGCCGGGCAACTCTGGCTCACAGTACGCGTA
GTGCAACCGAACGCGACCGCATGGTCAGAAGCCGGGCACATCAGCGCCTGGCAGC
AGTGGCGTCTGGCGGAAAACCTCAGTGTGACGCTCCCCGCCGCGTCCCACGCCATC
CCGCATCTGACCACCAGCGAAATGGATTTTTGCATCGAGCTGGGTAATAAGCGTTGG
CAATTTAACCGCCAGTCAGGCTTTCTTTCACAGATGTGGATTGGCGATAAAAAACAAC
TGCTGACGCCGCTGCGCGATCAGTTCACCCGTGCACCGCTGGATAACGACATTGGC
GTAAGTGAAGCGACCCGCATTGACCCTAACGCCTGGGTCGAACGCTGGAAGGCGGC
GGGCCATTACCAGGCCGAAGCAGCGTTGTTGCAGTGCACGGCAGATACACTTGCTG
ATGCGGTGCTGATTACGACCGCTCACGCGTGGCAGCATCAGGGGAAAACCTTATTTA
TCAGCCGGAAAACCTACCGGATTGATGGTAGTGGTCAAATGGCGATTACCGTTGATG
TTGAAGTGGCGAGCGATACACCGCATCCGGCGCGGATTGGCCTGAACTGCCAGCTG
GCGCAGGTAGCAGAGCGGGTAAACTGGCTCGGATTAGGGCCGCAAGAAAACTATCC
 
CGACCGCCTTACTGCCGCCTGTTTTGACCGCTGGGATCTGCCATTGTCAGACATGTA
TACCCCGTACGTCTTCCCGAGCGAAAACGGTCTGCGCTGCGGGACGCGCGAATTGA
ATTATGGCCCACACCAGTGGCGCGGCGACTTCCAGTTCAACATCAGCCGCTACAGT
CAACAGCAACTGATGGAAACCAGCCATCGCCATCTGCTGCACGCGGAAGAAGGCAC
ATGGCTGAATATCGACGGTTTCCATATGGGGATTGGTGGCGACGACTCCTGGAGCC
CGTCAGTATCGGCGGAATTACAGCTGAGCGCCGGTCGCTACCATTACCAGTTGGTCT
GGTGTCAAAAATAATAATAACCGGGCAGGCCATGTCTGCCCGTATTTCGCGTAAGGA
AATCCATTATGTACTATTTCGCCTGATGCGGTATTTTCTCCTTACGCATCTGTGCGGT
ATTTCACACCGCATAGGGTAATAACTGATATAATTAAATTGAAGCTCTAATTTGTGAGT
TTAGTATACATGCATTTACTTATAATACAGTTTTTTAGTTTTGCTGGCCGCATCTTCTC
AAATATGCTTCCCAGCCTGCTTTTCTGTAACGTTCACCCTCTACCTTAGCATCCCTTC
CCTTTGCAAATAGTCCTCTTCCAACAATAATAATGTCAGATCCTGTAGAGACCACATC
ATCCACGGTTCTATACTGTTGACCCAATGCGTCTCCCTTGTCATCTAAACCCACACCG
GGTGTCATAATCAACCAATCGTAACCTTCATCTCTTCCACCCATGTCTCTTTGAGCAA
TAAAGCCGATAACAAAATCTTTGTCGCTCTTCGCAATGTCAACAGTACCCTTAGTATA
TTCTCCAGTAGATAGGGAGCCCTTGCATGACAATTCTGCTAACATCAAAAGGCCTCTA
GGTTCCTTTGTTACTTCTTCTGCCGCCTGCTTCAAACCGCTAACAATACCTGGGCCCA
CCACACCGTGTGCATTCGTAATGTCTGCCCATTCTGCTATTCTGTATACACCCGCAGA
GTACTGCAATTTGACTGTATTACCAATGTCAGCAAATTTTCTGTCTTCGAAGAGTAAAA
AATTGTACTTGGCGGATAATGCCTTTAGCGGCTTAACTGTGCCCTCCATGGAAAAATC
AGTCAAGATATCCACATGTGTTTTTAGTAAACAAATTTTGGGACCTAATGCTTCAACTA
ACTCCAGTAATTCCTTGGTGGTACGAACATCCAATGAAGCACACAAGTTTGTTTGCTT
TTCGTGCATGATATTAAATAGCTTGGCAGCAACAGGACTAGGATGAGTAGCAGCACG
TTCCTTATATGTAGCTTTCGACATGATTTATCTTCGTTTTTTGTTCTGTGCAGTTGGGT
TAAGAATACTGGGCAATTTCATGTTTCTTCAACACTACATATGCGTATATATACCAATC
TAAGTCTGTGCTCCTTCCTTCGTTCTTCCTTCTGTTCGGAGATTACCGAATCAAAAAA
ATTTCAAAGAAACCGAAATCAAAAAAAAGAATAAAAAAAAAATGATGAATTGAATTGAA
AAGCTACTTGTTACCCATCATTGAATTTTGAACATCCGAACCTGGGAGTTTTCCCTGA
AACAGATAGTATATTTGAACCTGTATAATAATATATAGTCTAGCGCTTTACGGAAGACA
ATGTATGTATTTCGGTTCCTGGAGAAACTATTGCATCTATTGCATAGGTAATCTTGCA
CGTCGCATCCCCGGTTCATTTTCTGCGTTTCCATCTTGCACTTCAATAGCATATCTTT
GTTAACGAAGCATCTGTGCTTCATTTTGTAGAACAAAAATGCAACGCGAGAGCGCTA
ATTTTTCAAACAAAGAATCTGAGCTGCATTTTTACAGAACAGAAATGCAACGCGAAAG
CGCTATTTTACCAACGAAGAATCTGTGCTTCATTTTTGTAAAACAAAAATGCAACGCG
AGAGCGCTAATTTTTCAAACAAAGAATCTGAGCTGCATTTTTACAGAACAGAAATGCA
ACGCGAGAGCGCTATTTTACCAACAAAGAATCTATACTTCTTTTTTGTTCTACAAAAAT
GCATCCCGAGAGCGCTATTTTTCTAACAAAGCATCTTAGATTACTTTTTTTCTCCTTTG
 
TGCGCTCTATAATGCAGTCTCTTGATAACTTTTTGCACTGTAGGTCCGTTAAGGTTAG
AAGAAGGCTACTTTGGTGTCTATTTTCTCTTCCATAAAAAAAGCCTGACTCCACTTCC
CGCGTTTACTGATTACTAGCGAAGCTGCGGGTGCATTTTTTCAAGATAAAGGCATCC
CCGATTATATTCTATACCGATGTGGATTGCGCATACTTTGTGAACAGAAAGTGATAGC
GTTGATGATTCTTCATTGGTCAGAAAATTATGAACGGTTTCTTCTATTTTGTCTCTATA
TACTACGTATAGGAAATGTTTACATTTTCGTATTGTTTTCGATTCACTCTATGAATAGT
TCTTACTACAATTTTTTTGTCTAAAGAGTAATACTAGAGATAAACATAAAAAATGTAGA
GGTCGAGTTTAGATGCAAGTTCAAGGAGCGAAAGGTGGATGGGTAGGTTATATAGG
GATATAGCACAGAGATATATAGCAAAGAGATACTTTTGAGCAATGTTTGTGGAAGCG
GTATTCGCAATATTTTAGTAGCTCGTTACAGTCCGGTGCGTTTTTGGTTTTTTGAAAGT
GCGTCTTCAGAGCGCTTTTGGTTTTCAAAAGCGCTCTGAAGTTCCTATACTTTCTAGC
TAGAGAATAGGAACTTCGGAATAGGAACTTCAAAGCGTTTCCGAAAACGAGCGCTTC
CGAAAATGCAACGCGAGCTGCGCACATACAGCTCACTGTTCACGTCGCACCTATATC
TGCGTGTTGCCTGTATATATATATACATGAGAAGAACGGCATAGTGCGTGTTTATGCT
TAAATGCGTTATGGTGCACTCTCAGTACAATCTGCTCTGATGCCGCATAGTTAAGCCA
GCCCCGACACCCGCCAACACCCGCTGACGCGCCCTGACGGGCTTGTCTGCTCCCG
GCATCCGCTTACAGACAAGCTGTGACCGTCTCCGGGAGCTGCATGTGTCAGAGGTT
TTCACCGTCATCACCGAAACGCGCGAGACGAAAGGGCCTCGTGATACGCCTATTTTT
ATAGGTTAATGTCATGATAATAATGGTTTCTTAGACGTCAGGTGGCACTTTTCGGGGA
AATGTGCGCGGAACCCCTATTTGTTTATTTTTCTAAATACATTCAAATATGTATCCGCT
CATGAGACAATAACCCTGATAAATGCTTCAATAATATTGAAAAAGGAAGAGTATGAGT
ATTCAACATTTCCGTGTCGCCCTTATTCCCTTTTTTGCGGCATTTTGCCTTCCTGTTTT
TGCTCACCCAGAAACGCTGGTGAAAGTAAAAGATGCTGAAGATCAGTTGGGTGCACG
AGTGGGTTACATCGAACTGGATCTCAACAGCGGTAAGATCCTTGAGAGTTTTCGCCC
CGAAGAACGTTTTCCAATGATGAGCACTTTTAAAGTTCTGCTATGTGGCGCGGTATTA
TCCCGTATTGACGCCGGGCAAGAGCAACTCGGTCGCCGCATACACTATTCTCAGAAT
GACTTGGTTGAGTACTCACCAGTCACAGAAAAGCATCTTACGGATGGCATGACAGTA
AGAGAATTATGCAGTGCTGCCATAACCATGAGTGATAACACTGCGGCCAACTTACTT
CTGACAACGATCGGAGGACCGAAGGAGCTAACCGCTTTTTTGCACAACATGGGGGA
TCATGTAACTCGCCTTGATCGTTGGGAACCGGAGCTGAATGAAGCCATACCAAACGA
CGAGCGTGACACCACGATGCCTGTAGCAATGGCAACAACGTTGCGCAAACTATTAAC
TGGCGAACTACTTACTCTAGCTTCCCGGCAACAATTAATAGACTGGATGGAGGCGGA
TAAAGTTGCAGGACCACTTCTGCGCTCGGCCCTTCCGGCTGGCTGGTTTATTGCTGA
TAAATCTGGAGCCGGTGAGCGTGGGTCTCGCGGTATCATTGCAGCACTGGGGCCAG
ATGGTAAGCCCTCCCGTATCGTAGTTATCTACACGACGGGGAGTCAGGCAACTATGG
ATGAACGAAATAGACAGATCGCTGAGATAGGTGCCTCACTGATTAAGCATTGGTAAC
TGTCAGACCAAGTTTACTCATATATACTTTAGATTGATTTAAAACTTCATTTTTAATTTA
 
AAAGGATCTAGGTGAAGATCCTTTTTGATAATCTCATGACCAAAATCCCTTAACGTGA
GTTTTCGTTCCACTGAGCGTCAGACCCCGTAGAAAAGATCAAAGGATCTTCTTGAGA
TCCTTTTTTTCTGCGCGTAATCTGCTGCTTGCAAACAAAAAAACCACCGCTACCAGCG
GTGGTTTGTTTGCCGGATCAAGAGCTACCAACTCTTTTTCCGAAGGTAACTGGCTTCA
GCAGAGCGCAGATACCAAATACTGTCCTTCTAGTGTAGCCGTAGTTAGGCCACCACT
TCAAGAACTCTGTAGCACCGCCTACATACCTCGCTCTGCTAATCCTGTTACCAGTGG
CTGCTGCCAGTGGCGATAAGTCGTGTCTTACCGGGTTGGACTCAAGACGATAGTTAC
CGGATAAGGCGCAGCGGTCGGGCTGAACGGGGGGTTCGTGCACACAGCCCAGCTT
GGAGCGAACGACCTACACCGAACTGAGATACCTACAGCGTGAGCATTGAGAAAGCG
CCACGCTTCCCGAAGGGAGAAAGGCGGACAGGTATCCGGTAAGCGGCAGGGTCGG
AACAGGAGAGCGCACGAGGGAGCTTCCAGGGGGAAACGCCTGGTATCTTTATAGTC
CTGTCGGGTTTCGCCACCTCTGACTTGAGCGTCGATTTTTGTGATGCTCGTCAGGGG
GGCGGAGCCTATGGAAAAACGCCAGCAACGCGGCCTTTTTACGGTTCCTGGCCTTTT
GCTGGCCTTTTGCTCACATGTTCTTTCCTGCGTTATCCCCTGATTCTGTGGATAACCG
TATTACCGCCTTTGAGTGAGCTGATACCGCTCGCCGCAGCCGAACGACCGAGCGCA
GCGAGTCAGTGAGCGAGGAAGCGGAAGAGCGCCCAATACGCAAACCGCCTCTCCC
CGCGCGTTGGCCGATTCATTCCCG
Sequence #8
   > **3' *lacZ* terminator** (reverse DNA primer)
   AAACCCCTCCGTTTAGAGAGGGGTTATGCTAGTTATTTTTGACACCAGACCAACTG
Sequence #9
   > **5' primer of RNA P&P artificial *lacZ*-1**
   T7 promoter (in *italics)* 
TCATAAGCTTTAATACGACTCACTATAGGCCATCATAGTTGTTCTCTGGCGCTGTTGG
ACGAAAACAGTCCAGCAGCGCCGGGAAGGAGGGGCGAGGCATAAGTTCCTCCTTCT
TGGCGTTGTTGGACCTAAGAAGGAGGAATTTATGTCATATCTCACACAACGGGAGGT
CGTTTTACAACGTCGT
Sequence #10
   > **5' primer of RNA P&P artificial *lacZ*-2**
   T7 promoter (in *italics)* 
TCATAAGCTTTAATACGACTCACTATAGGCCATCATAGTTGTTCTCTGGCGCTGTTGG
ACGAAAACAGTCCAGCAGCGCCGGGAAGGAGGGGCGAGAGATAAGTTCCTCCTTCT
TGGCGTTGTTGGACCTAAGAAGGAGGAATTTATGTCATATCTCACACAACGGGAGGT
CGTTTTACAACGTCGT
Sequence #11
   > **5' primer of RNA P&P artificial *lacZ*-3**
   T7 promoter (in *italics)* 
TCATAAGCTTTAATACGACTCACTATAGGCCTAAATATTATCCAACCCGGGCCAGCGG
CGGATAAAATGTCGTTGGTCCGGGAGAGGGAGGCGTGAAAAATTGTCTTTCTTTTT
CGGATTAATGATACGAAAAAGGAGGATATCATGTACTACAGCTTCGATCCCGCCGTC
GTTTTACAACGTCGT
Sequence #12
   > **PCR product of RNA P&P artificial *lacZ*-1**
   T7 promoter (in *italics),* YEp358 vector underlined, *lacZ* CDS in grey 
TCATAAGCTTTAATACGACTCACTATAGGCCATCATAGTTGTTCTCTGGCGCTGTTGG
ACGAAAACAGTCCAGCAGCGCCGGAAGGAGGGCGAGGCATAAGTTCCTCCTTCT
TGGCGTTGTTGGACCTAAGAAGGAGGAATTTATGTCATATCTCACACAACGGAGGT
CGTTTTACAACGTCGTGACTGGGAAAACCCTGGCGTTACCCAACTTAATCGCCTTGC
AGCACATCCCCCTTTCGCCAGCTGGCGTAATAGCGAAGAGGCCCGCACCGATCGCC
CTTCCCAACAGTTGCGCAGCCTGAATGGCGAATGGCGCTTTGCCTGGTTTCCGGCA
CCAGAAGCGGTGCCGGAAAGCTGGCTGGAGTGCGATCTTCCTGAGGCCGATACTGT
CGTCGTCCCCTCAAACTGGCAGATGCACGGTTACGATGCGCCCATCTACACCAACG
TGACCTATCCCATTACGGTCAATCCGCCGTTTGTTCCCACGGAGAATCCGACGGGTT
GTTACTCGCTCACATTTAATGTTGATGAAAGCTGGCTACAGGAAGGCCAGACGCGAA
TTATTTTTGATGGCGTTAACTCGGCGTTTCATCTGTGGTGCAACGGGCGCTGGGTCG
GTTACGGCCAGGACAGTCGTTTGCCGTCTGAATTTGACCTGAGCGCATTTTTACGCG
CCGGAGAAAACCGCCTCGCGGTGATGGTGCTGCGCTGGAGTGACGGCAGTTATCTG
GAAGATCAGGATATGTGGCGGATGAGCGGCATTTTCCGTGACGTCTCGTTGCTGCAT
AAACCGACTACACAAATCAGCGATTTCCATGTTGCCACTCGCTTTAATGATGATTTCA
GCCGCGCTGTACTGGAGGCTGAAGTTCAGATGTGCGGCGAGTTGCGTGACTACCTA
CGGGTAACAGTTTCTTTATGGCAGGGTGAAACGCAGGTCGCCAGCGGCACCGCGCC
TTTCGGCGGTGAAATTATCGATGAGCGTGGTGGTTATGCCGATCGCGTCACACTACG
TCTGAACGTCGAAAACCCGAAACTGTGGAGCGCCGAAATCCCGAATCTCTATCGTGC
GGTGGTTGAACTGCACACCGCCGACGGCACGCTGATTGAAGCAGAAGCCTGCGATG
TCGGTTTCCGCGAGGTGCGGATTGAAAATGGTCTGCTGCTGCTGAACGGCAAGCCG
TTGCTGATTCGAGGCGTTAACCGTCACGAGCATCATCCTCTGCATGGTCAGGTCATG
GATGAGCAGACGATGGTGCAGGATATCCTGCTGATGAAGCAGAACAACTTTAACGCC
GTGCGCTGTTCGCATTATCCGAACCATCCGCTGTGGTACACGCTGTGCGACCGCTA
CGGCCTGTATGTGGTGGATGAAGCCAATATTGAAACCCACGGCATGGTGCCAATGAA
 
TCGTCTGACCGATGATCCGCGCTGGCTACCGGCGATGAGCGAACGCGTAACGCGAA
TGGTGCAGCGCGATCGTAATCACCCGAGTGTGATCATCTGGTCGCTGGGGAATGAA
TCAGGCCACGGCGCTAATCACGACGCGCTGTATCGCTGGATCAAATCTGTCGATCCT
TCCCGCCCGGTGCAGTATGAAGGCGGCGGAGCCGACACCACGGCCACCGATATTAT
TTGCCCGATGTACGCGCGCGTGGATGAAGACCAGCCCTTCCCGGCTGTGCCGAAAT
GGTCCATCAAAAAATGGCTTTCGCTACCTGGAGAGACGCGCCCGCTGATCCTTTGCG
AATACGCCCACGCGATGGGTAACAGTCTTGGCGGTTTCGCTAAATACTGGCAGGCG
TTTCGTCAGTATCCCCGTTTACAGGGCGGCTTCGTCTGGGACTGGGTGGATCAGTC
GCTGATTAAATATGATGAAAACGGCAACCCGTGGTCGGCTTACGGCGGTGATTTTGG
CGATACGCCGAACGATCGCCAGTTCTGTATGAACGGTCTGGTCTTTGCCGACCGCA
CGCCGCATCCAGCGCTGACGGAAGCAAAACACCAGCAGCAGTTTTTCCAGTTCCGTT
TATCCGGGCAAACCATCGAAGTGACCAGCGAATACCTGTTCCGTCATAGCGATAACG
AGCTCCTGCACTGGATGGTGGCGCTGGATGGTAAGCCGCTGGCAAGCGGTGAAGT
GCCTCTGGATGTCGCTCCACAAGGTAAACAGTTGATTGAACTGCCTGAACTACCGCA
GCCGGAGAGCGCCGGGCAACTCTGGCTCACAGTACGCGTAGTGCAACCGAACGCG
ACCGCATGGTCAGAAGCCGGGCACATCAGCGCCTGGCAGCAGTGGCGTCTGGCGG
AAAACCTCAGTGTGACGCTCCCCGCCGCGTCCCACGCCATCCCGCATCTGACCACC
AGCGAAATGGATTTTTGCATCGAGCTGGGTAATAAGCGTTGGCAATTTAACCGCCAG
TCAGGCTTTCTTTCACAGATGTGGATTGGCGATAAAAAACAACTGCTGACGCCGCTG
CGCGATCAGTTCACCCGTGCACCGCTGGATAACGACATTGGCGTAAGTGAAGCGAC
CCGCATTGACCCTAACGCCTGGGTCGAACGCTGGAAGGCGGCGGGCCATTACCAG
GCCGAAGCAGCGTTGTTGCAGTGCACGGCAGATACACTTGCTGATGCGGTGCTGAT
TACGACCGCTCACGCGTGGCAGCATCAGGGGAAAACCTTATTTATCAGCCGGAAAAC
CTACCGGATTGATGGTAGTGGTCAAATGGCGATTACCGTTGATGTTGAAGTGGCGAG
CGATACACCGCATCCGGCGCGGATTGGCCTGAACTGCCAGCTGGCGCAGGTAGCA
GAGCGGGTAAACTGGCTCGGATTAGGGCCGCAAGAAAACTATCCCGACCGCCTTAC
TGCCGCCTGTTTTGACCGCTGGGATCTGCCATTGTCAGACATGTATACCCCGTACGT
CTTCCCGAGCGAAAACGGTCTGCGCTGCGGGACGCGCGAATTGAATTATGGCCCAC
ACCAGTGGCGCGGCGACTTCCAGTTCAACATCAGCCGCTACAGTCAACAGCAACTG
ATGGAAACCAGCCATCGCCATCTGCTGCACGCGGAAGAAGGCACATGGCTGAATAT
CGACGGTTTCCATATGGGGATTGGTGGCGACGACTCCTGGAGCCCGTCAGTATCGG
CGGAATTCCAGCTGAGCGCCGGTCGCTACCATTACCAGTTGGTCTGGTGTCAAAAAT
AACTAGCATAACCCCTCTCTAAACGGAGGGGTTT
Sequence #13
   > **PCR product of RNA P&P artificial *lacZ*-2**
   T7 promoter (in *italics),* YEp358 vector underlined, *lacZ* CDS in grey 
TCATAAGCTTTAATACGACTCACTATAGGCCATCATAGTTGTTCTCTGGCGCTGTTGG
ACGAAAACAGTCCAGCAGCGCCGGGAAGGAGGGCGAGAGATAAGTTCCTCCTTCT
TGGCGTTGTTGGACCTAAGAAGGAGGAATTTATGTCATATCTCACACAACGGGAGGT
CGTTTTACAACGTCGTGACTGGGAAAACCCTGGCGTTACCCAACTTAATCGCCTTGC
AGCACATCCCCCTTTCGCCAGCTGGCGTAATAGCGAAGAGGCCCGCACCGATCGCC
CTTCCCAACAGTTGCGCAGCCTGAATGGCGAATGGCGCTTTGCCTGGTTTCCGGCA
CCAGAAGCGGTGCCGGAAAGCTGGCTGGAGTGCGATCTTCCTGAGGCCGATACTGT
CGTCGTCCCCTCAAACTGGCAGATGCACGGTTACGATGCGCCCATCTACACCAACG
TGACCTATCCCATTACGGTCAATCCGCCGTTTGTTCCCACGGAGAATCCGACGGGTT
GTTACTCGCTCACATTTAATGTTGATGAAAGCTGGCTACAGGAAGGCCAGACGCGAA
TTATTTTTGATGGCGTTAACTCGGCGTTTCATCTGTGGTGCAACGGGCGCTGGGTCG
GTTACGGCCAGGACAGTCGTTTGCCGTCTGAATTTGACCTGAGCGCATTTTTACGCG
CCGGAGAAAACCGCCTCGCGGTGATGGTGCTGCGCTGGAGTGACGGCAGTTATCTG
GAAGATCAGGATATGTGGCGGATGAGCGGCATTTTCCGTGACGTCTCGTTGCTGCAT
AAACCGACTACACAAATCAGCGATTTCCATGTTGCCACTCGCTTTAATGATGATTTCA
GCCGCGCTGTACTGGAGGCTGAAGTTCAGATGTGCGGCGAGTTGCGTGACTACCTA
CGGGTAACAGTTTCTTTATGGCAGGGTGAAACGCAGGTCGCCAGCGGCACCGCGCC
TTTCGGCGGTGAAATTATCGATGAGCGTGGTGGTTATGCCGATCGCGTCACACTACG
TCTGAACGTCGAAAACCCGAAACTGTGGAGCGCCGAAATCCCGAATCTCTATCGTGC
GGTGGTTGAACTGCACACCGCCGACGGCACGCTGATTGAAGCAGAAGCCTGCGATG
TCGGTTTCCGCGAGGTGCGGATTGAAAATGGTCTGCTGCTGCTGAACGGCAAGCCG
TTGCTGATTCGAGGCGTTAACCGTCACGAGCATCATCCTCTGCATGGTCAGGTCATG
GATGAGCAGACGATGGTGCAGGATATCCTGCTGATGAAGCAGAACAACTTTAACGCC
GTGCGCTGTTCGCATTATCCGAACCATCCGCTGTGGTACACGCTGTGCGACCGCTA
CGGCCTGTATGTGGTGGATGAAGCCAATATTGAAACCCACGGCATGGTGCCAATGAA
TCGTCTGACCGATGATCCGCGCTGGCTACCGGCGATGAGCGAACGCGTAACGCGAA
TGGTGCAGCGCGATCGTAATCACCCGAGTGTGATCATCTGGTCGCTGGGGAATGAA
TCAGGCCACGGCGCTAATCACGACGCGCTGTATCGCTGGATCAAATCTGTCGATCCT
TCCCGCCCGGTGCAGTATGAAGGCGGCGGAGCCGACACCACGGCCACCGATATTAT
TTGCCCGATGTACGCGCGCGTGGATGAAGACCAGCCCTTCCCGGCTGTGCCGAAAT
GGTCCATCAAAAAATGGCTTTCGCTACCTGGAGAGACGCGCCCGCTGATCCTTTGCG
AATACGCCCACGCGATGGGTAACAGTCTTGGCGGTTTCGCTAAATACTGGCAGGCG
TTTCGTCAGTATCCCCGTTTACAGGGCGGCTTCGTCTGGGACTGGGTGGATCAGTC
GCTGATTAAATATGATGAAAACGGCAACCCGTGGTCGGCTTACGGCGGTGATTTTGG
CGATACGCCGAACGATCGCCAGTTCTGTATGAACGGTCTGGTCTTTGCCGACCGCA
CGCCGCATCCAGCGCTGACGGAAGCAAAACACCAGCAGCAGTTTTTCCAGTTCCGTT
TATCCGGGCAAACCATCGAAGTGACCAGCGAATACCTGTTCCGTCATAGCGATAACG
 
AGCTCCTGCACTGGATGGTGGCGCTGGATGGTAAGCCGCTGGCAAGCGGTGAAGT
GCCTCTGGATGTCGCTCCACAAGGTAAACAGTTGATTGAACTGCCTGAACTACCGCA
GCCGGAGAGCGCCGGGCAACTCTGGCTCACAGTACGCGTAGTGCAACCGAACGCG
ACCGCATGGTCAGAAGCCGGGCACATCAGCGCCTGGCAGCAGTGGCGTCTGGCG
AAAACCTCAGTGTGACGCTCCCCGCCGCGTCCCACGCCATCCCGCATCTGACCACC
AGCGAAATGGATTTTTGCATCGAGCTGGGTAATAAGCGTTGGCAATTTAACCGCCAG
TCAGGCTTTCTTTCACAGATGTGGATTGGCGATAAAAAACAACTGCTGACGCCGCTG
CGCGATCAGTTCACCCGTGCACCGCTGGATAACGACATTGGCGTAAGTGAAGCGAC
CCGCATTGACCCTAACGCCTGGGTCGAACGCTGGAAGGCGGCGGGCCATTACCAG
GCCGAAGCAGCGTTGTTGCAGTGCACGGCAGATACACTTGCTGATGCGGTGCTGAT
TACGACCGCTCACGCGTGGCAGCATCAGGGGAAAACCTTATTTATCAGCCGGAAAAC
CTACCGGATTGATGGTAGTGGTCAAATGGCGATTACCGTTGATGTTGAAGTGGCGAG
CGATACACCGCATCCGGCGCGGATTGGCCTGAACTGCCAGCTGGCGCAGGTAGCA
GAGCGGGTAAACTGGCTCGGATTAGGGCCGCAAGAAAACTATCCCGACCGCCTTAC
TGCCGCCTGTTTTGACCGCTGGGATCTGCCATTGTCAGACATGTATACCCCGTACGT
CTTCCCGAGCGAAAACGGTCTGCGCTGCGGGACGCGCGAATTGAATTATGGCCCAC
ACCAGTGGCGCGGCGACTTCCAGTTCAACATCAGCCGCTACAGTCAACAGCAACTG
ATGGAAACCAGCCATCGCCATCTGCTGCACGCGGAAGAAGGCACATGGCTGAATAT
CGACGGTTTCCATATGGGGATTGGTGGCGACGACTCCTGGAGCCCGTCAGTATCGG
CGGAATTCCAGCTGAGCGCCGGTCGCTACCATTACCAGTTGGTCTGGTGTCAAAAAT
AACTAGCATAACCCCTCTCTAAACGGAGGGGTTT
Sequence #14
   > **PCR product of RNA P&P artificial *lacZ*-3**
   T7 promoter (in *italics),* YEp358 vector underlined, *lacZ* CDS in grey 
TCATAAGCTTTAATACGACTCACTATAGGCCTAAATATTATCCAACCCGGGCCAGCGG
CGGATAAAATGTCGTTGGTCCGGGAGAGGAGGCGTGAAAAATTGTCTTTCTTTTT
CGGATTAATGATACGAAAAAGGAGGATATCATGTACTACAGCTTCGATCCCGCCGTC
GTTTTACAACGTCGTGACTGGGAAAACCCTGGCGTTACCCAACTTAATCGCCTTGCA
GCACATCCCCCTTTCGCCAGCTGGCGTAATAGCGAAGAGGCCCGCACCGATCGCCC
TTCCCAACAGTTGCGCAGCCTGAATGGCGAATGGCGCTTTGCCTGGTTTCCGGCAC
CAGAAGCGGTGCCGGAAAGCTGGCTGGAGTGCGATCTTCCTGAGGCCGATACTGTC
GTCGTCCCCTCAAACTGGCAGATGCACGGTTACGATGCGCCCATCTACACCAACGT
GACCTATCCCATTACGGTCAATCCGCCGTTTGTTCCCACGGAGAATCCGACGGGTTG
TTACTCGCTCACATTTAATGTTGATGAAAGCTGGCTACAGGAAGGCCAGACGCGAAT
TATTTTTGATGGCGTTAACTCGGCGTTTCATCTGTGGTGCAACGGGCGCTGGGTCGG
TTACGGCCAGGACAGTCGTTTGCCGTCTGAATTTGACCTGAGCGCATTTTTACGCGC
 
CGGAGAAAACCGCCTCGCGGTGATGGTGCTGCGCTGGAGTGACGGCAGTTATCTGG
AAGATCAGGATATGTGGCGGATGAGCGGCATTTTCCGTGACGTCTCGTTGCTGCATA
AACCGACTACACAAATCAGCGATTTCCATGTTGCCACTCGCTTTAATGATGATTTCAG
CCGCGCTGTACTGGAGGCTGAAGTTCAGATGTGCGGCGAGTTGCGTGACTACCTAC
GGGTAACAGTTTCTTTATGGCAGGGTGAAACGCAGGTCGCCAGCGGCACCGCGCCT
TTCGGCGGTGAAATTATCGATGAGCGTGGTGGTTATGCCGATCGCGTCACACTACGT
CTGAACGTCGAAAACCCGAAACTGTGGAGCGCCGAAATCCCGAATCTCTATCGTGC
GGTGGTTGAACTGCACACCGCCGACGGCACGCTGATTGAAGCAGAAGCCTGCGATG
TCGGTTTCCGCGAGGTGCGGATTGAAAATGGTCTGCTGCTGCTGAACGGCAAGCCG
TTGCTGATTCGAGGCGTTAACCGTCACGAGCATCATCCTCTGCATGGTCAGGTCATG
GATGAGCAGACGATGGTGCAGGATATCCTGCTGATGAAGCAGAACAACTTTAACGCC
GTGCGCTGTTCGCATTATCCGAACCATCCGCTGTGGTACACGCTGTGCGACCGCTA
CGGCCTGTATGTGGTGGATGAAGCCAATATTGAAACCCACGGCATGGTGCCAATGAA
TCGTCTGACCGATGATCCGCGCTGGCTACCGGCGATGAGCGAACGCGTAACGCGAA
TGGTGCAGCGCGATCGTAATCACCCGAGTGTGATCATCTGGTCGCTGGGGAATGAA
TCAGGCCACGGCGCTAATCACGACGCGCTGTATCGCTGGATCAAATCTGTCGATCCT
TCCCGCCCGGTGCAGTATGAAGGCGGCGGAGCCGACACCACGGCCACCGATATTAT
TTGCCCGATGTACGCGCGCGTGGATGAAGACCAGCCCTTCCCGGCTGTGCCGAAAT
GGTCCATCAAAAAATGGCTTTCGCTACCTGGAGAGACGCGCCCGCTGATCCTTTGCG
AATACGCCCACGCGATGGGTAACAGTCTTGGCGGTTTCGCTAAATACTGGCAGGCG
TTTCGTCAGTATCCCCGTTTACAGGGCGGCTTCGTCTGGGACTGGGTGGATCAGTC
GCTGATTAAATATGATGAAAACGGCAACCCGTGGTCGGCTTACGGCGGTGATTTTGG
CGATACGCCGAACGATCGCCAGTTCTGTATGAACGGTCTGGTCTTTGCCGACCGCA
CGCCGCATCCAGCGCTGACGGAAGCAAAACACCAGCAGCAGTTTTTCCAGTTCCGTT
TATCCGGGCAAACCATCGAAGTGACCAGCGAATACCTGTTCCGTCATAGCGATAACG
AGCTCCTGCACTGGATGGTGGCGCTGGATGGTAAGCCGCTGGCAAGCGGTGAAGT
GCCTCTGGATGTCGCTCCACAAGGTAAACAGTTGATTGAACTGCCTGAACTACCGCA
GCCGGAGAGCGCCGGGCAACTCTGGCTCACAGTACGCGTAGTGCAACCGAACGCG
ACCGCATGGTCAGAAGCCGGGCACATCAGCGCCTGGCAGCAGTGGCGTCTGGCGG
AAAACCTCAGTGTGACGCTCCCCGCCGCGTCCCACGCCATCCCGCATCTGACCACC
AGCGAAATGGATTTTTGCATCGAGCTGGGTAATAAGCGTTGGCAATTTAACCGCCAG
TCAGGCTTTCTTTCACAGATGTGGATTGGCGATAAAAAACAACTGCTGACGCCGCTG
CGCGATCAGTTCACCCGTGCACCGCTGGATAACGACATTGGCGTAAGTGAAGCGAC
CCGCATTGACCCTAACGCCTGGGTCGAACGCTGGAAGGCGGCGGGCCATTACCAG
GCCGAAGCAGCGTTGTTGCAGTGCACGGCAGATACACTTGCTGATGCGGTGCTGAT
TACGACCGCTCACGCGTGGCAGCATCAGGGGAAAACCTTATTTATCAGCCGGAAAAC
CTACCGGATTGATGGTAGTGGTCAAATGGCGATTACCGTTGATGTTGAAGTGGCGAG
 
CGATACACCGCATCCGGCGGATTGGCCTGAACTGCCAGCTGGCGCAGGTAGCA
GAGCGGGTAAACTGGCTCGGATTAGGGCCGCAAGAAAACTATCCCGACCGCCTTAC
TGCCGCCTGTTTTGACCGCTGGGATCTGCCATTGTCAGACATGTATACCCCGTACGT
CTTCCCGAGCGAAAACGGTCTGCGCTGCGGGACGCGCGAATTGAATTATGGCCCAC
ACCAGTGGCGCGGCGACTTCCAGTTCAACATCAGCCGCTACAGTCAACAGCAACTG
ATGGAAACCAGCCATCGCCATCTGCTGCACGCGGAAGAAGGCACATGGCTGAATAT
CGACGGTTTCCATATGGGGATTGGTGGCGACGACTCCTGGAGCCCGTCAGTATCGG
CGGAATTCCAGCTGAGCGCCGGTCGCTACCATTACCAGTTGGTCTGGTGTCAAAAAT
AACTAGCATAACCCCTCTCTAAACGGAGGGGTTT
Sequence #15
   > **NR 024570.1 *Escherichia coli* strain U 5/41 16S ribosomal RNA,** partial sequence 
AGTTTGATCATGGCTCAGATTGAACGCTGGCGGCAGGCCTAACACATGCAAGTCGAA
CGGTAACAGGAAGCAGCTTGCTGCTTTGCTGACGAGTGGCGGACGGGTGAGTAATG
TCTGGGAAACTGCCTGATGGAGGGGGATAACTACTGGAAACGGTAGCTAATACCGC
ATAACGTCGCAAGCACAAAGAGGGGGACCTTAGGGCCTCTTGCCATCGGATGTGCC
CAGATGGGATTAGCTAGTAGGTGGGGTAACGGCTCACCTAGGCGACGATCCCTAGC
TGGTCTGAGAGGATGACCAGCAACACTGGAACTGAGACACGGTCCAGACTCCTACG
GGAGGCAGCAGTGGGGAATATTGCACAATGGGCGCAAGCCTGATGCAGCCATGCC
GCGTGTATGAAGAAGGCCTTCGGGTTGTAAAGTACTTTCAGCGGGGAGGAAGGGAG
TAAAGTTAATACCTTTGCTCATTGACGTTACCCGCAGAAGAAGCACCGGCTAACTCC
GTGCCAGCAGCCGCGGTAATACGGAGGGTGCAAGCGTTAATCGGAATTACTGGGCG
TAAAGCGCACGCAGGCGGTTTGTTAAGTCAGATGTGAAATCCCCGGGCTCAACCTG
GGAACTGCATCTGATACTGGCAAGCTTGAGTCTCGTAGAGGGGGGTAGAATTCCAG
GTGTAGCGGTGAAATGCGTAGAGATCTGGAGGAATACCGGTGGCGAAGGCGGCCC
CCTGGACGAAGACTGACGCTCAGGTGCGAAAGCGTGGGGAGCAAACAGGATTAGAT
ACCCTGGTAGTCCACGCCGTAAACGATGTCGACTTGGAGGTTGTGCCCTTGAGGCG
TGGCTTCCGGAGCTAACGCGTTAAGTCGACCGCCTGGGGAGTACGGCCGCAAGGTT
AAAACTCAAATGAATTGACGGGGGCCGCACAAGCGGTGGAGCATGTGGTTTAATTCG
ATGCAACGCGAAGAACCTTACCTGGTCTTGACATCCACGGAAGTTTTCAGAGATGAG
AATGTGCCTTCGGGAACCGTGAGACAGGTGCTGCATGGCT
GTCGTCAGCTCGTGTTGTGAAATGTTGGGTTAAGTCCCGCAACGAGCGCAACCCTTA
TCCTTTGTTGCCAGCGGTCCGGCCGGGAACTCAAAGGAGACTGCCAGTGATAAACT
GGAGGAAGGTGGGGATGACGTCAAGTCATCATGGCCCTTACGACCAGGGCTACACA
CGTGCTACAATGGCGCATACAAAGAGAAGCGACCTCGCGAGAGCAAGCGGACCTCA
TAAAGTGCGTCGTAGTCCGGATTGGAGTCTGCAACTCGACTCCATGAAGTCGGAATC
GCTAGTAATCGTGGATCAGAATGCCACGGTGAATACGTTCCCGGGCCTTGTACACAC
 
CGCCCGTCACACCATGGGAGTGGGTTGCAAAAGAAGTAGGTAGCTTAACTTCGGGA
GGGCG
Sequence #16:
   > **RNA P&P *E.coli lacZ*-1** 
GGCGUGUAGUUGUGCUGCGUGUGUGAGGAGGGCUGAAAGAGCUCUUUUCGUAUA
UAAAGGAGGGUACCUAUAAUCUGUCCUCCUUUAUAUACGAAAAGAGUAUAUAAAGG
AGGACAAUAUGCACAUACAAAGCUUCGAUCCCGCCGUCGUUUUACAACG
Sequence #17:
   > **RNA P&P *E.coli lacZ*-2** 
GGCGUGUAGUUGUGCUGCGUGUGUGAGGAGGGCUGAGAGAGCUCUUUUCGUAUA
UAAAGGAGGGUACCUAUAAUCUGUCCUCCUUUAUAUACGAAAAGAGUAUAUAAAGG
AGGACAAUAUGCACAUACAAAGCUUCGAUCCCGCCGUCGUUUUACAACG
Sequence #18:
   > **Synthetic RNA target of 16S ribosomal RNA (nt** 357-432, reverse complement) 
UGAAAGUACUUUACAACCCGAAGGCCUUCUUCAUACACGCGGCAUGGCUGCAUCA
GGCUUGUUGCGCCCAUUCUGCAA
Sequence #19:
   > **5' primer of P&P *E.coli lacZ*-1**
   T7 promoter (in *italics)* 
TCATAAGCTTTAATACGACTCACTATAGGCGTGTAGTTGTGCTGCGTGTGTGAGGAG
GGCTGAAAGAGCTCTTTTCGTATATAAAGGAGGGTACCTATAATCTGTCCTCCTTTAT
ATACGAAAAGAGTATATAAAGGAGGACAATATGCACATACAAAGCTTCGATCCCGCC
GTCGTTTTACAACG
Sequence #20:
   > **5' primer of RNA P&P *E.coli lacZ*-2**
   T7 promoter (in *italics)* 
TCATAAGCTTTAATACGACTCACTATAGGCGTGTAGTTGTGCTGCGTGTGTGAGGAG
GGCTGAGAGAGCTCTTTTCGTATATAAAGGAGGGTACCTATAATCTGTCCTCCTTTAT
ATACGAAAAGAGTATATAAAGGAGGACAATATGCACATACAAAGCTTCGATCCCGCC
GTCGTTTTACAACG
Sequence #21:
   > **PCR product of RNA P&P *E.coli lacZ*-1**
   T7 promoter (in *italics),* YEp358 vector underlined, *lacZ* CDS in grey 
TCATAAGCTTTAATACGACTCACTATAGGCGTGTAGTTGTGCTGCGTGTGTGAGGAG
GGCTGAAAGAGCTCTTTTCGTATATAAAGGAGGGTACCTATAATCTGTCCTCCTTTAT
ATACGAAAAGAGTATATAAAGGAGGACAATATGCACATACAAAGCTTCGATCCCGCC
GTCGTTTTACAACGTCGTGACTGGGAAAACCCTGGCGTTACCCAACTTAATCGCCTT
GCAGCACATCCCCCTTTCGCCAGCTGGCGTAATAGCGAAGAGGCCCGCACCGATCG
CCCTTCCCAACAGTTGCGCAGCCTGAATGGCGAATGGCGCTTTGCCTGGTTTCCGG
CACCAGAAGCGGTGCCGGAAAGCTGGCTGGAGTGCGATCTTCCTGAGGCCGATACT
GTCGTCGTCCCCTCAAACTGGCAGATGCACGGTTACGATGCGCCCATCTACACCAA
CGTGACCTATCCCATTACGGTCAATCCGCCGTTTGTTCCCACGGAGAATCCGACGGG
TTGTTACTCGCTCACATTTAATGTTGATGAAAGCTGGCTACAGGAAGGCCAGACGCG
AATTATTTTTGATGGCGTTAACTCGGCGTTTCATCTGTGGTGCAACGGGCGCTGGGT
CGGTTACGGCCAGGACAGTCGTTTGCCGTCTGAATTTGACCTGAGCGCATTTTTACG
CGCCGGAGAAAACCGCCTCGCGGTGATGGTGCTGCGCTGGAGTGACGGCAGTTAT
CTGGAAGATCAGGATATGTGGCGGATGAGCGGCATTTTCCGTGACGTCTCGTTGCT
GCATAAACCGACTACACAAATCAGCGATTTCCATGTTGCCACTCGCTTTAATGATGAT
TTCAGCCGCGCTGTACTGGAGGCTGAAGTTCAGATGTGCGGCGAGTTGCGTGACTA
CCTACGGGTAACAGTTTCTTTATGGCAGGGTGAAACGCAGGTCGCCAGCGGCACCG
CGCCTTTCGGCGGTGAAATTATCGATGAGCGTGGTGGTTATGCCGATCGCGTCACA
CTACGTCTGAACGTCGAAAACCCGAAACTGTGGAGCGCCGAAATCCCGAATCTCTAT
CGTGCGGTGGTTGAACTGCACACCGCCGACGGCACGCTGATTGAAGCAGAAGCCTG
CGATGTCGGTTTCCGCGAGGTGCGGATTGAAAATGGTCTGCTGCTGCTGAACGGCA
AGCCGTTGCTGATTCGAGGCGTTAACCGTCACGAGCATCATCCTCTGCATGGTCAGG
TCATGGATGAGCAGACGATGGTGCAGGATATCCTGCTGATGAAGCAGAACAACTTTA
ACGCCGTGCGCTGTTCGCATTATCCGAACCATCCGCTGTGGTACACGCTGTGCGAC
CGCTACGGCCTGTATGTGGTGGATGAAGCCAATATTGAAACCCACGGCATGGTGCC
AATGAATCGTCTGACCGATGATCCGCGCTGGCTACCGGCGATGAGCGAACGCGTAA
CGCGAATGGTGCAGCGCGATCGTAATCACCCGAGTGTGATCATCTGGTCGCTGGGG
AATGAATCAGGCCACGGCGCTAATCACGACGCGCTGTATCGCTGGATCAAATCTGTC
GATCCTTCCCGCCCGGTGCAGTATGAAGGCGGCGGAGCCGACACCACGGCCACCG
ATATTATTTGCCCGATGTACGCGCGCGTGGATGAAGACCAGCCCTTCCCGGCTGTG
CCGAAATGGTCCATCAAAAAATGGCTTTCGCTACCTGGAGAGACGCGCCCGCTGATC
CTTTGCGAATACGCCCACGCGATGGGTAACAGTCTTGGCGGTTTCGCTAAATACTGG
CAGGCGTTTCGTCAGTATCCCCGTTTACAGGGCGGCTTCGTCTGGGACTGGGTGGA
TCAGTCGCTGATTAAATATGATGAAAACGGCAACCCGTGGTCGGCTTACGGCGGTGA
TTTTGGCGATACGCCGAACGATCGCCAGTTCTGTATGAACGGTCTGGTCTTTGCCGA
 
CCGCACGCCGCATCCAGCGCTGACGGAAGCAAAACACCAGCAGCAGTTTTCCAGT
TCCGTTTATCCGGGCAAACCATCGAAGTGACCAGCGAATACCTGTTCCGTCATAGCG
ATAACGAGCTCCTGCACTGGATGGTGGCGCTGGATGGTAAGCCGCTGGCAAGCGGT
GAAGTGCCTCTGGATGTCGCTCCACAAGGTAAACAGTTGATTGAACTGCCTGAACTA
CCGCAGCCGGAGAGCGCCGGGCAACTCTGGCTCACAGTACGCGTAGTGCAACCGA
ACGCGACCGCATGGTCAGAAGCCGGGCACATCAGCGCCTGGCAGCAGTGGCGTCT
GGCGGAAAACCTCAGTGTGACGCTCCCCGCCGCGTCCCACGCCATCCCGCATCTGA
CCACCAGCGAAATGGATTTTTGCATCGAGCTGGGTAATAAGCGTTGGCAATTTAACC
GCCAGTCAGGCTTTCTTTCACAGATGTGGATTGGCGATAAAAAACAACTGCTGACGC
CGCTGCGCGATCAGTTCACCCGTGCACCGCTGGATAACGACATTGGCGTAAGTGAA
GCGACCCGCATTGACCCTAACGCCTGGGTCGAACGCTGGAAGGCGGCGGGCCATT
ACCAGGCCGAAGCAGCGTTGTTGCAGTGCACGGCAGATACACTTGCTGATGCGGTG
CTGATTACGACCGCTCACGCGTGGCAGCATCAGGGGAAAACCTTATTTATCAGCCGG
AAAACCTACCGGATTGATGGTAGTGGTCAAATGGCGATTACCGTTGATGTTGAAGTG
GCGAGCGATACACCGCATCCGGCGCGGATTGGCCTGAACTGCCAGCTGGCGCAGG
TAGCAGAGCGGGTAAACTGGCTCGGATTAGGGCCGCAAGAAAACTATCCCGACCGC
CTTACTGCCGCCTGTTTTGACCGCTGGGATCTGCCATTGTCAGACATGTATACCCCG
TACGTCTTCCCGAGCGAAAACGGTCTGCGCTGCGGGACGCGCGAATTGAATTATGG
CCCACACCAGTGGCGCGGCGACTTCCAGTTCAACATCAGCCGCTACAGTCAACAGC
AACTGATGGAAACCAGCCATCGCCATCTGCTGCACGCGGAAGAAGGCACATGGCTG
AATATCGACGGTTTCCATATGGGGATTGGTGGCGACGACTCCTGGAGCCCGTCAGT
ATCGGCGGAATTCCAGCTGAGCGCCGGTCGCTACCATTACCAGTTGGTCTGGTGTC
AAAAATAACTAGCATAACCCCTCTCTAAACGGAGGGGTTT
Sequence #22:
   > **PCR product of RNA P&P *E.coli lacZ*-2**
   T7 promoter (in *italics),* YEp358 vector underlined, *lacZ* CDS in grey 
TCATAAGCTTTAATACGACTCACTATAGGCGTGTAGTTGTGCTGCGTGTGTGAGGAG
GGCTGAGAGAGCTCTTTTCGTATATAAAGGAGGGTACCTATAATCTGTCCTCCTTTAT
ATACGAAAAGAGTATATAAAGGAGGACAATATGCACATACAAAGCTTCGATCCCGCC
GTCGTTTTACAACGTCGTGACTGGGAAAACCCTGGCGTTACCCAACTTAATCGCCTT
GCAGCACATCCCCCTTTCGCCAGCTGGCGTAATAGCGAAGAGGCCCGCACCGATCG
CCCTTCCCAACAGTTGCGCAGCCTGAATGGCGAATGGCGCTTTGCCTGGTTTCCGG
CACCAGAAGCGGTGCCGGAAAGCTGGCTGGAGTGCGATCTTCCTGAGGCCGATACT
GTCGTCGTCCCCTCAAACTGGCAGATGCACGGTTACGATGCGCCCATCTACACCAA
CGTGACCTATCCCATTACGGTCAATCCGCCGTTTGTTCCCACGGAGAATCCGACGGG
TTGTTACTCGCTCACATTTAATGTTGATGAAAGCTGGCTACAGGAAGGCCAGACGCG
 
AATTATTTTTGATGGCGTTAACTCGGCGTTTCATCTGTGGTGCAACGGGCGCTGGGT
CGGTTACGGCCAGGACAGTCGTTTGCCGTCTGAATTTGACCTGAGCGCATTTTTACG
CGCCGGAGAAAACCGCCTCGCGGTGATGGTGCTGCGCTGGAGTGACGGCAGTTAT
CTGGAAGATCAGGATATGTGGCGGATGAGCGGCATTTTCCGTGACGTCTCGTTGCT
GCATAAACCGACTACACAAATCAGCGATTTCCATGTTGCCACTCGCTTTAATGATGAT
TTCAGCCGCGCTGTACTGGAGGCTGAAGTTCAGATGTGCGGCGAGTTGCGTGACTA
CCTACGGGTAACAGTTTCTTTATGGCAGGGTGAAACGCAGGTCGCCAGCGGCACCG
CGCCTTTCGGCGGTGAAATTATCGATGAGCGTGGTGGTTATGCCGATCGCGTCACA
CTACGTCTGAACGTCGAAAACCCGAAACTGTGGAGCGCCGAAATCCCGAATCTCTAT
CGTGCGGTGGTTGAACTGCACACCGCCGACGGCACGCTGATTGAAGCAGAAGCCTG
CGATGTCGGTTTCCGCGAGGTGCGGATTGAAAATGGTCTGCTGCTGCTGAACGGCA
AGCCGTTGCTGATTCGAGGCGTTAACCGTCACGAGCATCATCCTCTGCATGGTCAGG
TCATGGATGAGCAGACGATGGTGCAGGATATCCTGCTGATGAAGCAGAACAACTTTA
ACGCCGTGCGCTGTTCGCATTATCCGAACCATCCGCTGTGGTACACGCTGTGCGAC
CGCTACGGCCTGTATGTGGTGGATGAAGCCAATATTGAAACCCACGGCATGGTGCC
AATGAATCGTCTGACCGATGATCCGCGCTGGCTACCGGCGATGAGCGAACGCGTAA
CGCGAATGGTGCAGCGCGATCGTAATCACCCGAGTGTGATCATCTGGTCGCTGGGG
AATGAATCAGGCCACGGCGCTAATCACGACGCGCTGTATCGCTGGATCAAATCTGTC
GATCCTTCCCGCCCGGTGCAGTATGAAGGCGGCGGAGCCGACACCACGGCCACCG
ATATTATTTGCCCGATGTACGCGCGCGTGGATGAAGACCAGCCCTTCCCGGCTGTG
CCGAAATGGTCCATCAAAAAATGGCTTTCGCTACCTGGAGAGACGCGCCCGCTGATC
CTTTGCGAATACGCCCACGCGATGGGTAACAGTCTTGGCGGTTTCGCTAAATACTGG
CAGGCGTTTCGTCAGTATCCCCGTTTACAGGGCGGCTTCGTCTGGGACTGGGTGGA
TCAGTCGCTGATTAAATATGATGAAAACGGCAACCCGTGGTCGGCTTACGGCGGTGA
TTTTGGCGATACGCCGAACGATCGCCAGTTCTGTATGAACGGTCTGGTCTTTGCCGA
CCGCACGCCGCATCCAGCGCTGACGGAAGCAAAACACCAGCAGCAGTTTTTCCAGT
TCCGTTTATCCGGGCAAACCATCGAAGTGACCAGCGAATACCTGTTCCGTCATAGCG
ATAACGAGCTCCTGCACTGGATGGTGGCGCTGGATGGTAAGCCGCTGGCAAGCGGT
GAAGTGCCTCTGGATGTCGCTCCACAAGGTAAACAGTTGATTGAACTGCCTGAACTA
CCGCAGCCGGAGAGCGCCGGGCAACTCTGGCTCACAGTACGCGTAGTGCAACCGA
ACGCGACCGCATGGTCAGAAGCCGGGCACATCAGCGCCTGGCAGCAGTGGCGTCT
GGCGGAAAACCTCAGTGTGACGCTCCCCGCCGCGTCCCACGCCATCCCGCATCTGA
CCACCAGCGAAATGGATTTTTGCATCGAGCTGGGTAATAAGCGTTGGCAATTTAACC
GCCAGTCAGGCTTTCTTTCACAGATGTGGATTGGCGATAAAAAACAACTGCTGACGC
CGCTGCGCGATCAGTTCACCCGTGCACCGCTGGATAACGACATTGGCGTAAGTGAA
GCGACCCGCATTGACCCTAACGCCTGGGTCGAACGCTGGAAGGCGGCGGGCCATT
ACCAGGCCGAAGCAGCGTTGTTGCAGTGCACGGCAGATACACTTGCTGATGCGGTG
 
CTGATTACGACCGCTCACGCGTGGCAGCATCAGGGGAAAACCTTATTTATCAGCCGG
AAAACCTACCGGATTGATGGTAGTGGTCAAATGGCGATTACCGTTGATGTTGAAGTG
GCGAGCGATACACCGCATCCGGCGCGGATTGGCCTGAACTGCCAGCTGGCGCAGG
TAGCAGAGCGGGTAAACTGGCTCGGATTAGGGCCGCAAGAAAACTATCCCGACCGC
CTTACTGCCGCCTGTTTTGACCGCTGGGATCTGCCATTGTCAGACATGTATACCCCG
TACGTCTTCCCGAGCGAAAACGGTCTGCGCTGCGGGACGCGCGAATTGAATTATGG
CCCACACCAGTGGCGCGGCGACTTCCAGTTCAACATCAGCCGCTACAGTCAACAGC
AACTGATGGAAACCAGCCATCGCCATCTGCTGCACGCGGAAGAAGGCACATGGCTG
AATATCGACGGTTTCCATATGGGGATTGGTGGCGACGACTCCTGGAGCCCGTCAGT
ATCGGCGGAATTCCAGCTGAGCGCCGGTCGCTACCATTACCAGTTGGTCTGGTGTC
AAAAATAACTAGCATAACCCCTCTCTAAACGGAGGGGTTT
Sequence #23:
   *>* ***gfpmut3B-ASV* gene** (CDS) 
ATGCGTAAAGGAGAAGAACTTTTCACTGGAGTTGTCCCAATTCTTGTTGAATTAGATG
GTGATGTTAATGGGCACAAATTTTCTGTCAGTGGAGAGGGTGAAGGTGATGCAACAT
ACGGAAAACTTACCCTTAAATTTATTTGCACTACTGGAAAACTACCTGTTCCGTGGCC
AACACTTGTCACTACTTTCGGTTATGGTGTTCAATGCTTTGCGAGATACCCAGATCAC
ATGAAACAGCATGACTTTTTCAAGAGTGCCATGCCCGAAGGTTACGTACAGGAAAGA
ACTATATTTTTCAAAGATGACGGGAACTACAAGACACGTGCTGAAGTCAAGTTTGAAG
GTGATACCCTTGTTAATAGAATCGAGTTAAAAGGTATTGATTTTAAAGAAGATGGAAA
CATTCTTGGACACAAATTGGAATACAACTATAACTCACACAATGTATACATCATGGCA
GACAAACAAAAGAATGGAATCAAAGTTAACTTCAAAATTAGACACAACATTGAAGATG
GAAGCGTTCAACTAGCAGACCATTATCAACAAAATACTCCGATTGGCGATGGCCCTG
TCCTTTTACCAGACAACCATTACCTGTCCACACAATCTGCCCTTTCGAAAGATCCCAA
CGAAAAGAGAGACCACATGGTCCTTCTTGAGTTTGTAACCGCTGCTGGGATTACACA
TGGCATGGATGAACTATACAAAGGCCTGCAGCAAACGACGAAACTACGCTGCATC
AGTTTAATAA
Sequence #24:
   > **RNA P&P *E.coli* GFP-1** 
GGCGUGGCGAGUUUGGUGCGGUCGUGCUGUGUGGUAAAUACGUAUAGUACGAUC
GGAGGGAGGGAGAACAAUCGGUUUCUCCUUUUGAUCGUAUUAUACGAUCAAAAG
GAGGAGAUAAUGUACAUUUGCAACCUGGCGGCAGCGCAAAAGAUGCGUAAAGGAG
AAG
Sequence #25:
   > **RNA P&P *E.coli* GFP-2** 
GGCGUGUAGCUAUGUUGCGUGUAUGGGAGGGCGAAAAUCGCUUUUCUCGUAUA
UAAAGGAGGGAGCACUUAAUACUUCCUCCUUUAUAUACGAGAAGGGUAUAUAAAG
GAGGAAGCAAUGGAAGUUGGCAACCUGGCGGCAGCGCAAAAGAUGCGUAAAGGAG
AAG
Sequence #26:
   > **RNA P&P *E.coli* GFP-3** 
GGCGUGUGGAGGAGGUUUUCGGGUUGUAAAGUAAUACAUUUAUUUUGCGAUUCG
AUAGGGAGGAAGUACACAUAUCUUCCUCCUUAUUGGAUCGCGAGAUAUCAAUAAG
GAGGAAGAAAUGCAAAUUGCGAACCUGGCGGCAGCGCAAAAGAUGCGUAAAGGAG
AAG
Sequence #27:
   > **Synthetic construct containing *gfpmut3b-ASV* gene**
   proD promoter (in *italics*), *gfpmut3b-ASV* CDS in grey 
GGCAACAGATCTCACAGCTAACACCACGTCGTCCCTATCTGCTGCCCTAGGTCTATG
AGTGGTTGCTGGATAACTTTACGGGCATGCATAAGGCTCGTATAATATATTCAAGTAA
TACTGGAAGCAATGTGAGCAATGTCGTGCTGTTATAGTTATGAGACAAGAACAGAGG
AGACATAACATGAACAGCACGAACGCTGTTAACCTGGCGGCAGCGCAAAAGATGCG
TAAAGGAGAAGAACTTTTCACTGGAGTTGTCCCAATTCTTGTTGAATTAGATGGTGAT
GTTAATGGGCACAAATTTTCTGTCAGTGGAGAGGGTGAAGGTGATGCAACATACGGA
AAACTTACCCTTAAATTTATTTGCACTACTGGAAAACTACCTGTTCCGTGGCCAACAC
TTGTCACTACTTTCGGTTATGGTGTTCAATGCTTTGCGAGATACCCAGATCACATGAA
ACAGCATGACTTTTTCAAGAGTGCCATGCCCGAAGGTTACGTACAGGAAAGAACTAT
ATTTTTCAAAGATGACGGGAACTACAAGACACGTGCTGAAGTCAAGTTTGAAGGTGA
TACCCTTGTTAATAGAATCGAGTTAAAAGGTATTGATTTTAAAGAAGATGGAAACATTC
TTGGACACAAATTGGAATACAACTATAACTCACACAATGTATACATCATGGCAGACAA
ACAAAAGAATGGAATCAAAGTTAACTTCAAAATTAGACACAACATTGAAGATGGAAGC
GTTCAACTAGCAGACCATTATCAACAAAATACTCCGATTGGCGATGGCCCTGTCCTTT
TACCAGACAACCATTACCTGTCCACACAATCTGCCCTTTCGAAAGATCCCAACGAAAA
GAGAGACCACATGGTCCTTCTTGAGTTTGTAACCGCTGCTGGGATTACACATGGCAT
GGATGAACTATACAAAGGCCTGCAGCAAACGACGAAAACTACGCTGCATCAGTTTA
ATAAGATAAACCAGAGCGGCACGGCAAGCAGAGTATACGAGATTCGGTAGCCACCG
CTGAGCAATAACTAGCATAACCCCTTGGGGCCTCTAAACGGGTCTTGAGGGGTTTTT
TGCATATGCAACGG
Sequence #28:
   > **3' *GFPmut3b-ASVterminator*** (reverse DNA primer)
   CCGTTGCATATGCAAAAAACCCCTCAAGACCCGTTTAGAGGCC
Sequence #29:
   > **5' primer of RNA P&P *E.coli* GFP-1**
   T7 promoter (in *italics)* 
TCATAAGCTTTAATACGACTCACTATAGGCGTGGCGAGTTTGGTGCGGTCGTGCTGT
GTGGTAAATACGTATAGTACGATCGGAGGGAGGGAGAACAATCGGTTTTCTCCTTTT
GATCGTATTATACGATCAAAAGGAGGAGATAATGTACATTTGCAACCTGGCGGCAGC
GCAAAAGATGCGTAAAGGAGAAG
Sequence #30:
   > **5' primer of RNA P&P *E.coli* GFP-2**
   T7 promoter (in *italics*) 
TCATAAGCTTTAATACGACTCACTATAGGCGTGTAGCTATGTTGCGTGTATGGGGAG
GGCGAAAATCGCTTTTCTCGTATATAAAGGAGGGAGCACTTAATACTTCCTCCTTTAT
ATACGAGAAGGGTATATAAAGGAGGAAGCAATGGAAGTTGGCAACCTGGCGGCAGC
GCAAAAGATGCGTAAAGGAGAAG
Sequence #31:
   > **5' primer of RNA P&P *E.coli* GFP-3**
   T7 promoter (in *italics*) 
TCATAAGCTTTAATACGACTCACTATAGGCGTGTGGAGGAGGTTTTCGGGTTGTAAA
GTAATACATTTATTTTGCGATTCGATAGGGAGGAAGTACACATATCTTCCTCCTTATTG
GATCGCGAGATATCAATAAGGAGGAAGAAATGCAAATTGCGAACCTGGCGGCAGCG
CAAAAGATGCGTAAAGGAGAAG
Sequence #32:
   > **PCR product of RNA P&P *E.coli* GFP-1**
   T7 promoter (in *italics*), *gfpmut3b-ASV* CDS in grey 
TCATAAGCTTTAATACGACTCACTATAGGCGTGGCGAGTTTGGTGCGGTCGTGCTGT
GTGGTAAATACGTATAGTACGATCGGAGGGAGGGAGAACAATCGGTTTTCTCCTTTT
GATCGTATTATACGATCAAAAGGAGGAGATAATGTACATTTGCAACCTGGCGGCAGC
GCAAAAGATGCGTAAAGGAGAAGAACTTTTCACTGGAGTTGTCCCAATTCTTGTTGAA
TTAGATGGTGATGTTAATGGGCACAAATTTTCTGTCAGTGGAGAGGGTGAAGGTGAT
 
GCAACATACGGAAAACTTACCCTTAAATTTATTTGCACTACTGGAAAACTACCTGTTC
CGTGGCCAACACTTGTCACTACTTTCGGTTATGGTGTTCAATGCTTTGCGAGATACCC
AGATCACATGAAACAGCATGACTTTTTCAAGAGTGCCATGCCCGAAGGTTACGTACA
GGAAAGAACTATATTTTTCAAAGATGACGGGAACTACAAGACACGTGCTGAAGTCAA
GTTTGAAGGTGATACCCTTGTTAATAGAATCGAGTTAAAAGGTATTGATTTTAAAGAA
GATGGAAACATTCTTGGACACAAATTGGAATACAACTATAACTCACACAATGTATACA
TCATGGCAGACAAACAAAAGAATGGAATCAAAGTTAACTTCAAAATTAGACACAACAT
TGAAGATGGAAGCGTTCAACTAGCAGACCATTATCAACAAAATACTCCGATTGGCGA
TGGCCCTGTCCTTTTACCAGACAACCATTACCTGTCCACACAATCTGCCCTTTCGAAA
GATCCCAACGAAAAGAGAGACCACATGGTCCTTCTTGAGTTTGTAACCGCTGCTGGG
ATTACACATGGCATGGATGAACTATACAAAGGCCTGCAGCAAACGACGAAAACTAC
GCTGCATCAGTTTAATAATAAGATAAACCAGAGCGGCACGGCAAGCAGAGTATACGA
GATTCGGTAGCCACCGCTGAGCAATAACTAGCATAACCCCTTGGGGCCTCTAAACGG
GTCTTGAGGGGTTTTTTGCATATGCAACGG
Sequence #33:
   > **PCR product of RNA P&P *E.coli* GFP-2**
   T7 promoter (in *italics*), *gfpmut3b-ASV* CDS in grey 
TCATAAGCTTTAATACGACTCACTATAGGCGTGTAGCTATGTTGCGTGTATGGGGAG
GGCGAAAATCGCTTTTCTCGTATATAAAGGAGGGAGCACTTAATACTTCCTCCTTTAT
ATACGAGAAGGGTATATAAAGGAGGAAGCAATGGAAGTTGGCAACCTGGCGGCAGC
GCAAAAGATGCGTAAAGGAGAAGAACTTTTCACTGGAGTTGTCCCAATTCTTGTTGAA
TTAGATGGTGATGTTAATGGGCACAAATTTTCTGTCAGTGGAGAGGGTGAAGGTGAT
GCAACATACGGAAAACTTACCCTTAAATTTATTTGCACTACTGGAAAACTACCTGTTC
CGTGGCCAACACTTGTCACTACTTTCGGTTATGGTGTTCAATGCTTTGCGAGATACCC
AGATCACATGAAACAGCATGACTTTTTCAAGAGTGCCATGCCCGAAGGTTACGTACA
GGAAAGAACTATATTTTTCAAAGATGACGGGAACTACAAGACACGTGCTGAAGTCAA
GTTTGAAGGTGATACCCTTGTTAATAGAATCGAGTTAAAAGGTATTGATTTTAAAGAA
GATGGAAACATTCTTGGACACAAATTGGAATACAACTATAACTCACACAATGTATACA
TCATGGCAGACAAACAAAAGAATGGAATCAAAGTTAACTTCAAAATTAGACACAACAT
TGAAGATGGAAGCGTTCAACTAGCAGACCATTATCAACAAAATACTCCGATTGGCGA
TGGCCCTGTCCTTTTACCAGACAACCATTACCTGTCCACACAATCTGCCCTTTCGAAA
GATCCCAACGAAAAGAGAGACCACATGGTCCTTCTTGAGTTTGTAACCGCTGCTGGG
ATTACACATGGCATGGATGAACTATACAAAGGCCTGCAGCAAACGACGAAAACTAC
GCTGCATCAGTTTAATAATAAGATAAACCAGAGCGGCACGGCAAGCAGAGTATACGA
GATTCGGTAGCCACCGCTGAGCAATAACTAGCATAACCCCTTGGGGCCTCTAAACGG
GTCTTGAGGGGTTTTTTGCATATGCAACGG
Sequence #34:
   > **PCR product of RNA P&P *E.coli* GFP-3**
   T7 promoter (in *italics*), *gfpmut3b-ASV* CDS in grey 
TCATAAGCTTTAATACGACTCACTATAGGCGTGGAGGAGGTTTTCGGGTTGTAAA
GTAATACATTTATTTTGCGATTCGATAGGGAGGAAGTACACATATCTTCCTCCTTATTG
GATCGCGAGATATCAATAAGGAGGAAGAAATGCAAATTGCGAACCTGGCGGCAGCG
CAAAAGATGCGTAAAGGAGAAGAACTTTTCACTGGAGTTGTCCCAATTCTTGTTGAAT
TAGATGGTGATGTTAATGGGCACAAATTTTCTGTCAGTGGAGAGGGTGAAGGTGATG
CAACATACGGAAAACTTACCCTTAAATTTATTTGCACTACTGGAAAACTACCTGTTCC
GTGGCCAACACTTGTCACTACTTTCGGTTATGGTGTTCAATGCTTTGCGAGATACCCA
GATCACATGAAACAGCATGACTTTTTCAAGAGTGCCATGCCCGAAGGTTACGTACAG
GAAAGAACTATATTTTTCAAAGATGACGGGAACTACAAGACACGTGCTGAAGTCAAGT
TTGAAGGTGATACCCTTGTTAATAGAATCGAGTTAAAAGGTATTGATTTTAAAGAAGAT
GGAAACATTCTTGGACACAAATTGGAATACAACTATAACTCACACAATGTATACATCA
TGGCAGACAAACAAAAGAATGGAATCAAAGTTAACTTCAAAATTAGACACAACATTGA
AGATGGAAGCGTTCAACTAGCAGACCATTATCAACAAAATACTCCGATTGGCGATGG
CCCTGTCCTTTTACCAGACAACCATTACCTGTCCACACAATCTGCCCTTTCGAAAGAT
CCCAACGAAAAGAGAGACCACATGGTCCTTCTTGAGTTTGTAACCGCTGCTGGGATT
ACACATGGCATGGATGAACTATACAAAGGCCTGCAGCAAACGACGAAACTACGCT
GCATCAGTTTAATAATAAGATAAACCAGAGCGGCACGGCAAGCAGAGTATACGAGAT
TCGGTAGCCACCGCTGAGCAATAACTAGCATAACCCCTTGGGGCCTCTAAACGGGT
CTTGAGGGGTTTTTTGCATATGCAACGG
Sequence #35:
   *>* ***orf1ab* gene** (CDS); MN908947.3:266-21555 Severe acute respiratory syndrome coronavirus 2 isolate Wuhan-Hu-1 (SARS-CoV-2), complete genome 
ATGGAGAGCCTTGTCCCTGGTTTCAACGAGAAAACACACGTCCAACTCAGTTTGCCT
GTTTTACAGGTTCGCGACGTGCTCGTACGTGGCTTTGGAGACTCCGTGGAGGAGGT
CTTATCAGAGGCACGTCAACATCTTAAAGATGGCACTTGTGGCTTAGTAGAAGTTGAA
AAAGGCGTTTTGCCTCAACTTGAACAGCCCTATGTGTTCATCAAACGTTCGGATGCTC
GAACTGCACCTCATGGTCATGTTATGGTTGAGCTGGTAGCAGAACTCGAAGGCATTC
AGTACGGTCGTAGTGGTGAGACACTTGGTGTCCTTGTCCCTCATGTGGGCGAAATAC
CAGTGGCTTACCGCAAGGTTCTTCTTCGTAAGAACGGTAATAAAGGAGCTGGTGGCC
ATAGTTACGGCGCCGATCTAAAGTCATTTGACTTAGGCGACGAGCTTGGCACTGATC
CTTATGAAGATTTTCAAGAAAACTGGAACACTAAACATAGCAGTGGTGTTACCCGTGA
ACTCATGCGTGAGCTTAACGGAGGGGCATACACTCGCTATGTCGATAACAACTTCTG
 
TGGCCCTGATGGCTACCCTCTTGAGTGCATTAAAGACCTTCTAGCACGTGCTGGTAA
AGCTTCATGCACTTTGTCCGAACAACTGGACTTTATTGACACTAAGAGGGGTGTATAC
TGCTGCCGTGAACATGAGCATGAAATTGCTTGGTACACGGAACGTTCTGAAAAGAGC
TATGAATTGCAGACACCTTTTGAAATTAAATTGGCAAAGAAATTTGACACCTTCAATG
GGGAATGTCCAAATTTTGTATTTCCCTTAAATTCCATAATCAAGACTATTCAACCAAGG
GTTGAAAAGAAAAAGCTTGATGGCTTTATGGGTAGAATTCGATCTGTCTATCCAGTTG
CGTCACCAAATGAATGCAACCAAATGTGCCTTTCAACTCTCATGAAGTGTGATCATTG
TGGTGAAACTTCATGGCAGACGGGCGATTTTGTTAAAGCCACTTGCGAATTTTGTGG
CACTGAGAATTTGACTAAAGAAGGTGCCACTACTTGTGGTTACTTACCCCAAAATGCT
GTTGTTAAAATTTATTGTCCAGCATGTCACAATTCAGAAGTAGGACCTGAGCATAGTC
TTGCCGAATACCATAATGAATCTGGCTTGAAAACCATTCTTCGTAAGGGTGGTCGCA
CTATTGCCTTTGGAGGCTGTGTGTTCTCTTATGTTGGTTGCCATAACAAGTGTGCCTA
TTGGGTTCCACGTGCTAGCGCTAACATAGGTTGTAACCATACAGGTGTTGTTGGAGA
AGGTTCCGAAGGTCTTAATGACAACCTTCTTGAAATACTCCAAAAAGAGAAAGTCAAC
ATCAATATTGTTGGTGACTTTAAACTTAATGAAGAGATCGCCATTATTTTGGCATCTTT
TTCTGCTTCCACAAGTGCTTTTGTGGAAACTGTGAAAGGTTTGGATTATAAAGCATTC
AAACAAATTGTTGAATCCTGTGGTAATTTTAAAGTTACAAAAGGAAAAGCTAAAAAAG
GTGCCTGGAATATTGGTGAACAGAAATCAATACTGAGTCCTCTTTATGCATTTGCATC
AGAGGCTGCTCGTGTTGTACGATCAATTTTCTCCCGCACTCTTGAAACTGCTCAAAAT
TCTGTGCGTGTTTTACAGAAGGCCGCTATAACAATACTAGATGGAATTTCACAGTATT
CACTGAGACTCATTGATGCTATGATGTTCACATCTGATTTGGCTACTAACAATCTAGT
TGTAATGGCCTACATTACAGGTGGTGTTGTTCAGTTGACTTCGCAGTGGCTAACTAAC
ATCTTTGGCACTGTTTATGAAAAACTCAAACCCGTCCTTGATTGGCTTGAAGAGAAGT
TTAAGGAAGGTGTAGAGTTTCTTAGAGACGGTTGGGAAATTGTTAAATTTATCTCAAC
CTGTGCTTGTGAAATTGTCGGTGGACAAATTGTCACCTGTGCAAAGGAAATTAAGGA
GAGTGTTCAGACATTCTTTAAGCTTGTAAATAAATTTTTGGCTTTGTGTGCTGACTCTA
TCATTATTGGTGGAGCTAAACTTAAAGCCTTGAATTTAGGTGAAACATTTGTCACGCA
CTCAAAGGGATTGTACAGAAAGTGTGTTAAATCCAGAGAAGAAACTGGCCTACTCAT
GCCTCTAAAAGCCCCAAAAGAAATTATCTTCTTAGAGGGAGAAACACTTCCCACAGAA
GTGTTAACAGAGGAAGTTGTCTTGAAAACTGGTGATTTACAACCATTAGAACAACCTA
CTAGTGAAGCTGTTGAAGCTCCATTGGTTGGTACACCAGTTTGTATTAACGGGCTTAT
GTTGCTCGAAATCAAAGACACAGAAAAGTACTGTGCCCTTGCACCTAATATGATGGTA
ACAAACAATACCTTCACACTCAAAGGCGGTGCACCAACAAAGGTTACTTTTGGTGAT
GACACTGTGATAGAAGTGCAAGGTTACAAGAGTGTGAATATCACTTTTGAACTTGATG
AAAGGATTGATAAAGTACTTAATGAGAAGTGCTCTGCCTATACAGTTGAACTCGGTAC
AGAAGTAAATGAGTTCGCCTGTGTTGTGGCAGATGCTGTCATAAAAACTTTGCAACCA
GTATCTGAATTACTTACACCACTGGGCATTGATTTAGATGAGTGGAGTATGGCTACAT
 
ACTACTTATTTGATGAGTCTGGTGAGTTTAAATTGGCTTCACATATGTATTGTTCTTTC
TACCCTCCAGATGAGGATGAAGAAGAAGGTGATTGTGAAGAAGAAGAGTTTGAGCCA
TCAACTCAATATGAGTATGGTACTGAAGATGATTACCAAGGTAAACCTTTGGAATTTG
GTGCCACTTCTGCTGCTCTTCAACCTGAAGAAGAGCAAGAAGAAGATTGGTTAGATG
ATGATAGTCAACAAACTGTTGGTCAACAAGACGGCAGTGAGGACAATCAGACAACTA
CTATTCAAACAATTGTTGAGGTTCAACCTCAATTAGAGATGGAACTTACACCAGTTGT
TCAGACTATTGAAGTGAATAGTTTTAGTGGTTATTTAAAACTTACTGACAATGTATACA
TTAAAAATGCAGACATTGTGGAAGAAGCTAAAAAGGTAAAACCAACAGTGGTTGTTAA
TGCAGCCAATGTTTACCTTAAACATGGAGGAGGTGTTGCAGGAGCCTTAAATAAGGC
TACTAACAATGCCATGCAAGTTGAATCTGATGATTACATAGCTACTAATGGACCACTT
AAAGTGGGTGGTAGTTGTGTTTTAAGCGGACACAATCTTGCTAAACACTGTCTTCATG
TTGTCGGCCCAAATGTTAACAAAGGTGAAGACATTCAACTTCTTAAGAGTGCTTATGA
AAATTTTAATCAGCACGAAGTTCTACTTGCACCATTATTATCAGCTGGTATTTTTGGTG
CTGACCCTATACATTCTTTAAGAGTTTGTGTAGATACTGTTCGCACAAATGTCTACTTA
GCTGTCTTTGATAAAAATCTCTATGACAAACTTGTTTCAAGCTTTTTGGAAATGAAGAG
TGAAAAGCAAGTTGAACAAAAGATCGCTGAGATTCCTAAAGAGGAAGTTAAGCCATTT
ATAACTGAAAGTAAACCTTCAGTTGAACAGAGAAAACAAGATGATAAGAAAATCAAAG
CTTGTGTTGAAGAAGTTACAACAACTCTGGAAGAAACTAAGTTCCTCACAGAAAACTT
GTTACTTTATATTGACATTAATGGCAATCTTCATCCAGATTCTGCCACTCTTGTTAGTG
ACATTGACATCACTTTCTTAAAGAAAGATGCTCCATATATAGTGGGTGATGTTGTTCA
AGAGGGTGTTTTAACTGCTGTGGTTATACCTACTAAAAAGGCTGGTGGCACTACTGA
AATGCTAGCGAAAGCTTTGAGAAAAGTGCCAACAGACAATTATATAACCACTTACCCG
GGTCAGGGTTTAAATGGTTACACTGTAGAGGAGGCAAAGACAGTGCTTAAAAAGTGT
AAAAGTGCCTTTTACATTCTACCATCTATTATCTCTAATGAGAAGCAAGAAATTCTTGG
AACTGTTTCTTGGAATTTGCGAGAAATGCTTGCACATGCAGAAGAAACACGCAAATTA
ATGCCTGTCTGTGTGGAAACTAAAGCCATAGTTTCAACTATACAGCGTAAATATAAGG
GTATTAAAATACAAGAGGGTGTGGTTGATTATGGTGCTAGATTTTACTTTTACACCAG
TAAAACAACTGTAGCGTCACTTATCAACACACTTAACGATCTAAATGAAACTCTTGTTA
CAATGCCACTTGGCTATGTAACACATGGCTTAAATTTGGAAGAAGCTGCTCGGTATAT
GAGATCTCTCAAAGTGCCAGCTACAGTTTCTGTTTCTTCACCTGATGCTGTTACAGCG
TATAATGGTTATCTTACTTCTTCTTCTAAAACACCTGAAGAACATTTTATTGAAACCAT
CTCACTTGCTGGTTCCTATAAAGATTGGTCCTATTCTGGACAATCTACACAACTAGGT
ATAGAATTTCTTAAGAGAGGTGATAAAAGTGTATATTACACTAGTAATCCTACCACATT
CCACCTAGATGGTGAAGTTATCACCTTTGACAATCTTAAGACACTTCTTTCTTTGAGA
GAAGTGAGGACTATTAAGGTGTTTACAACAGTAGACAACATTAACCTCCACACGCAA
GTTGTGGACATGTCAATGACATATGGACAACAGTTTGGTCCAACTTATTTGGATGGAG
CTGATGTTACTAAAATAAAACCTCATAATTCACATGAAGGTAAAACATTTTATGTTTTA
 
CCTAATGATGACACTCTACGTGTTGAGGCTTTTGAGTACTACCACACAACTGATCCTA
GTTTTCTGGGTAGGTACATGTCAGCATTAAATCACACTAAAAAGTGGAAATACCCACA
AGTTAATGGTTTAACTTCTATTAAATGGGCAGATAACAACTGTTATCTTGCCACTGCAT
TGTTAACACTCCAACAAATAGAGTTGAAGTTTAATCCACCTGCTCTACAAGATGCTTA
TTACAGAGCAAGGGCTGGTGAAGCTGCTAACTTTTGTGCACTTATCTTAGCCTACTGT
AATAAGACAGTAGGTGAGTTAGGTGATGTTAGAGAAACAATGAGTTACTTGTTTCAAC
ATGCCAATTTAGATTCTTGCAAAAGAGTCTTGAACGTGGTGTGTAAAACTTGTGGACA
ACAGCAGACAACCCTTAAGGGTGTAGAAGCTGTTATGTACATGGGCACACTTTCTTAT
GAACAATTTAAGAAAGGTGTTCAGATACCTTGTACGTGTGGTAAACAAGCTACAAAAT
ATCTAGTACAACAGGAGTCACCTTTTGTTATGATGTCAGCACCACCTGCTCAGTATGA
ACTTAAGCATGGTACATTTACTTGTGCTAGTGAGTACACTGGTAATTACCAGTGTGGT
CACTATAAACATATAACTTCTAAAGAAACTTTGTATTGCATAGACGGTGCTTTACTTAC
AAAGTCCTCAGAATACAAAGGTCCTATTACGGATGTTTTCTACAAAGAAAACAGTTAC
ACAACAACCATAAAACCAGTTACTTATAAATTGGATGGTGTTGTTTGTACAGAAATTGA
CCCTAAGTTGGACAATTATTATAAGAAAGACAATTCTTATTTCACAGAGCAACCAATTG
ATCTTGTACCAAACCAACCATATCCAAACGCAAGCTTCGATAATTTTAAGTTTGTATGT
GATAATATCAAATTTGCTGATGATTTAAACCAGTTAACTGGTTATAAGAAACCTGCTTC
AAGAGAGCTTAAAGTTACATTTTTCCCTGACTTAAATGGTGATGTGGTGGCTATTGAT
TATAAACACTACACACCCTCTTTTAAGAAAGGAGCTAAATTGTTACATAAACCTATTGT
TTGGCATGTTAACAATGCAACTAATAAAGCCACGTATAAACCAAATACCTGGTGTATA
CGTTGTCTTTGGAGCACAAAACCAGTTGAAACATCAAATTCGTTTGATGTACTGAAGT
CAGAGGACGCGCAGGGAATGGATAATCTTGCCTGCGAAGATCTAAAACCAGTCTCTG
AAGAAGTAGTGGAAAATCCTACCATACAGAAAGACGTTCTTGAGTGTAATGTGAAAAC
TACCGAAGTTGTAGGAGACATTATACTTAAACCAGCAAATAATAGTTTAAAAATTACAG
AAGAGGTTGGCCACACAGATCTAATGGCTGCTTATGTAGACAATTCTAGTCTTACTAT
TAAGAAACCTAATGAATTATCTAGAGTATTAGGTTTGAAAACCCTTGCTACTCATGGTT
TAGCTGCTGTTAATAGTGTCCCTTGGGATACTATAGCTAATTATGCTAAGCCTTTTCTT
AACAAAGTTGTTAGTACAACTACTAACATAGTTACACGGTGTTTAAACCGTGTTTGTA
CTAATTATATGCCTTATTTCTTTACTTTATTGCTACAATTGTGTACTTTTACTAGAAGTA
CAAATTCTAGAATTAAAGCATCTATGCCGACTACTATAGCAAAGAATACTGTTAAGAG
TGTCGGTAAATTTTGTCTAGAGGCTTCATTTAATTATTTGAAGTCACCTAATTTTTCTA
AACTGATAAATATTATAATTTGGTTTTTACTATTAAGTGTTTGCCTAGGTTCTTTAATCT
ACTCAACCGCTGCTTTAGGTGTTTTAATGTCTAATTTAGGCATGCCTTCTTACTGTACT
GGTTACAGAGAAGGCTATTTGAACTCTACTAATGTCACTATTGCAACCTACTGTACTG
GTTCTATACCTTGTAGTGTTTGTCTTAGTGGTTTAGATTCTTTAGACACCTATCCTTCT
TTAGAAACTATACAAATTACCATTTCATCTTTTAAATGGGATTTAACTGCTTTTGGCTTA
GTTGCAGAGTGGTTTTTGGCATATATTCTTTTCACTAGGTTTTTCTATGTACTTGGATT
 
GGCTGCAATCATGCAATTGTTTTCAGCTATTTTGCAGTACATTTTATTAGTAATTCTT
GGCTTATGTGGTTAATAATTAATCTTGTACAAATGGCCCCGATTTCAGCTATGGTTAG
AATGTACATCTTCTTTGCATCATTTTATTATGTATGGAAAAGTTATGTGCATGTTGTAG
ACGGTTGTAATTCATCAACTTGTATGATGTGTTACAAACGTAATAGAGCAACAAGAGT
CGAATGTACAACTATTGTTAATGGTGTTAGAAGGTCCTTTTATGTCTATGCTAATGGA
GGTAAAGGCTTTTGCAAACTACACAATTGGAATTGTGTTAATTGTGATACATTCTGTG
CTGGTAGTACATTTATTAGTGATGAAGTTGCGAGAGACTTGTCACTACAGTTTAAAAG
ACCAATAAATCCTACTGACCAGTCTTCTTACATCGTTGATAGTGTTACAGTGAAGAAT
GGTTCCATCCATCTTTACTTTGATAAAGCTGGTCAAAAGACTTATGAAAGACATTCTCT
CTCTCATTTTGTTAACTTAGACAACCTGAGAGCTAATAACACTAAAGGTTCATTGCCTA
TTAATGTTATAGTTTTTGATGGTAAATCAAAATGTGAAGAATCATCTGCAAAATCAGCG
TCTGTTTACTACAGTCAGCTTATGTGTCAACCTATACTGTTACTAGATCAGGCATTAGT
GTCTGATGTTGGTGATAGTGCGGAAGTTGCAGTTAAAATGTTTGATGCTTACGTTAAT
ACGTTTTCATCAACTTTTAACGTACCAATGGAAAAACTCAAAACACTAGTTGCAACTG
CAGAAGCTGAACTTGCAAAGAATGTGTCCTTAGACAATGTCTTATCTACTTTTATTTCA
GCAGCTCGGCAAGGGTTTGTTGATTCAGATGTAGAAACTAAAGATGTTGTTGAATGT
CTTAAATTGTCACATCAATCTGACATAGAAGTTACTGGCGATAGTTGTAATAACTATAT
GCTCACCTATAACAAAGTTGAAAACATGACACCCCGTGACCTTGGTGCTTGTATTGAC
TGTAGTGCGCGTCATATTAATGCGCAGGTAGCAAAAAGTCACAACATTGCTTTGATAT
GGAACGTTAAAGATTTCATGTCATTGTCTGAACAACTACGAAAACAAATACGTAGTGC
TGCTAAAAAGAATAACTTACCTTTTAAGTTGACATGTGCAACTACTAGACAAGTTGTTA
ATGTTGTAACAACAAAGATAGCACTTAAGGGTGGTAAAATTGTTAATAATTGGTTGAA
GCAGTTAATTAAAGTTACACTTGTGTTCCTTTTTGTTGCTGCTATTTTCTATTTAATAAC
ACCTGTTCATGTCATGTCTAAACATACTGACTTTTCAAGTGAAATCATAGGATACAAG
GCTATTGATGGTGGTGTCACTCGTGACATAGCATCTACAGATACTTGTTTTGCTAACA
AACATGCTGATTTTGACACATGGTTTAGCCAGCGTGGTGGTAGTTATACTAATGACAA
AGCTTGCCCATTGATTGCTGCAGTCATAACAAGAGAAGTGGGTTTTGTCGTGCCTGG
TTTGCCTGGCACGATATTACGCACAACTAATGGTGACTTTTTGCATTTCTTACCTAGA
GTTTTTAGTGCAGTTGGTAACATCTGTTACACACCATCAAAACTTATAGAGTACACTG
ACTTTGCAACATCAGCTTGTGTTTTGGCTGCTGAATGTACAATTTTTAAAGATGCTTCT
GGTAAGCCAGTACCATATTGTTATGATACCAATGTACTAGAAGGTTCTGTTGCTTATG
AAAGTTTACGCCCTGACACACGTTATGTGCTCATGGATGGCTCTATTATTCAATTTCC
TAACACCTACCTTGAAGGTTCTGTTAGAGTGGTAACAACTTTTGATTCTGAGTACTGT
AGGCACGGCACTTGTGAAAGATCAGAAGCTGGTGTTTGTGTATCTACTAGTGGTAGA
TGGGTACTTAACAATGATTATTACAGATCTTTACCAGGAGTTTTCTGTGGTGTAGATG
CTGTAAATTTACTTACTAATATGTTTACACCACTAATTCAACCTATTGGTGCTTTGGAC
ATATCAGCATCTATAGTAGCTGGTGGTATTGTAGCTATCGTAGTAACATGCCTTGCCT
 
ACTATTTTATGAGGTTTAGAAGAGCTTTTGGTGAATACAGTCATGTAGTTGCCTTTAAT
ACTTTACTATTCCTTATGTCATTCACTGTACTCTGTTTAACACCAGTTTACTCATTCTTA
CCTGGTGTTTATTCTGTTATTTACTTGTACTTGACATTTTATCTTACTAATGATGTTTCT
TTTTTAGCACATATTCAGTGGATGGTTATGTTCACACCTTTAGTACCTTTCTGGATAAC
AATTGCTTATATCATTTGTATTTCCACAAAGCATTTCTATTGGTTCTTTAGTAATTACCT
AAAGAGACGTGTAGTCTTTAATGGTGTTTCCTTTAGTACTTTTGAAGAAGCTGCGCTG
TGCACCTTTTTGTTAAATAAAGAAATGTATCTAAAGTTGCGTAGTGATGTGCTATTACC
TCTTACGCAATATAATAGATACTTAGCTCTTTATAATAAGTACAAGTATTTTAGTGGAG
CAATGGATACAACTAGCTACAGAGAAGCTGCTTGTTGTCATCTCGCAAAGGCTCTCA
ATGACTTCAGTAACTCAGGTTCTGATGTTCTTTACCAACCACCACAAACCTCTATCAC
CTCAGCTGTTTTGCAGAGTGGTTTTAGAAAAATGGCATTCCCATCTGGTAAAGTTGAG
GGTTGTATGGTACAAGTAACTTGTGGTACAACTACACTTAACGGTCTTTGGCTTGATG
ACGTAGTTTACTGTCCAAGACATGTGATCTGCACCTCTGAAGACATGCTTAACCCTAA
TTATGAAGATTTACTCATTCGTAAGTCTAATCATAATTTCTTGGTACAGGCTGGTAATG
TTCAACTCAGGGTTATTGGACATTCTATGCAAAATTGTGTACTTAAGCTTAAGGTTGAT
ACAGCCAATCCTAAGACACCTAAGTATAAGTTTGTTCGCATTCAACCAGGACAGACTT
TTTCAGTGTTAGCTTGTTACAATGGTTCACCATCTGGTGTTTACCAATGTGCTATGAG
GCCCAATTTCACTATTAAGGGTTCATTCCTTAATGGTTCATGTGGTAGTGTTGGTTTTA
ACATAGATTATGACTGTGTCTCTTTTTGTTACATGCACCATATGGAATTACCAACTGGA
GTTCATGCTGGCACAGACTTAGAAGGTAACTTTTATGGACCTTTTGTTGACAGGCAAA
CAGCACAAGCAGCTGGTACGGACACAACTATTACAGTTAATGTTTTAGCTTGGTTGTA
CGCTGCTGTTATAAATGGAGACAGGTGGTTTCTCAATCGATTTACCACAACTCTTAAT
GACTTTAACCTTGTGGCTATGAAGTACAATTATGAACCTCTAACACAAGACCATGTTG
ACATACTAGGACCTCTTTCTGCTCAAACTGGAATTGCCGTTTTAGATATGTGTGCTTC
ATTAAAAGAATTACTGCAAAATGGTATGAATGGACGTACCATATTGGGTAGTGCTTTA
TTAGAAGATGAATTTACACCTTTTGATGTTGTTAGACAATGCTCAGGTGTTACTTTCCA
AAGTGCAGTGAAAAGAACAATCAAGGGTACACACCACTGGTTGTTACTCACAATTTTG
ACTTCACTTTTAGTTTTAGTCCAGAGTACTCAATGGTCTTTGTTCTTTTTTTTGTATGAA
AATGCCTTTTTACCTTTTGCTATGGGTATTATTGCTATGTCTGCTTTTGCAATGATGTT
TGTCAAACATAAGCATGCATTTCTCTGTTTGTTTTTGTTACCTTCTCTTGCCACTGTAG
CTTATTTTAATATGGTCTATATGCCTGCTAGTTGGGTGATGCGTATTATGACATGGTT
GGATATGGTTGATACTAGTTTGTCTGGTTTTAAGCTAAAAGACTGTGTTATGTATGCA
TCAGCTGTAGTGTTACTAATCCTTATGACAGCAAGAACTGTGTATGATGATGGTGCTA
GGAGAGTGTGGACACTTATGAATGTCTTGACACTCGTTTATAAAGTTTATTATGGTAA
TGCTTTAGATCAAGCCATTTCCATGTGGGCTCTTATAATCTCTGTTACTTCTAACTACT
CAGGTGTAGTTACAACTGTCATGTTTTTGGCCAGAGGTATTGTTTTTATGTGTGTTGA
GTATTGCCCTATTTTCTTCATAACTGGTAATACACTTCAGTGTATAATGCTAGTTTATT
 
GTTTCTTAGGCTATTTTTGTACTTGTTACTTTGGCCTCTTTTGTTTACTCAACCGCTAC
TTTAGACTGACTCTTGGTGTTTATGATTACTTAGTTTCTACACAGGAGTTTAGATATAT
GAATTCACAGGGACTACTCCCACCCAAGAATAGCATAGATGCCTTCAAACTCAACATT
AAATTGTTGGGTGTTGGTGGCAAACCTTGTATCAAAGTAGCCACTGTACAGTCTAAAA
TGTCAGATGTAAAGTGCACATCAGTAGTCTTACTCTCAGTTTTGCAACAACTCAGAGT
AGAATCATCATCTAAATTGTGGGCTCAATGTGTCCAGTTACACAATGACATTCTCTTA
GCTAAAGATACTACTGAAGCCTTTGAAAAAATGGTTTCACTACTTTCTGTTTTGCTTTC
CATGCAGGGTGCTGTAGACATAAACAAGCTTTGTGAAGAAATGCTGGACAACAGGGC
AACCTTACAAGCTATAGCCTCAGAGTTTAGTTCCCTTCCATCATATGCAGCTTTTGCT
ACTGCTCAAGAAGCTTATGAGCAGGCTGTTGCTAATGGTGATTCTGAAGTTGTTCTTA
AAAAGTTGAAGAAGTCTTTGAATGTGGCTAAATCTGAATTTGACCGTGATGCAGCCAT
GCAACGTAAGTTGGAAAAGATGGCTGATCAAGCTATGACCCAAATGTATAAACAGGC
TAGATCTGAGGACAAGAGGGCAAAAGTTACTAGTGCTATGCAGACAATGCTTTTCAC
TATGCTTAGAAAGTTGGATAATGATGCACTCAACAACATTATCAACAATGCAAGAGAT
GGTTGTGTTCCCTTGAACATAATACCTCTTACAACAGCAGCCAAACTAATGGTTGTCA
TACCAGACTATAACACATATAAAAATACGTGTGATGGTACAACATTTACTTATGCATCA
GCATTGTGGGAAATCCAACAGGTTGTAGATGCAGATAGTAAAATTGTTCAACTTAGTG
AAATTAGTATGGACAATTCACCTAATTTAGCATGGCCTCTTATTGTAACAGCTTTAAGG
GCCAATTCTGCTGTCAAATTACAGAATAATGAGCTTAGTCCTGTTGCACTACGACAGA
TGTCTTGTGCTGCCGGTACTACACAAACTGCTTGCACTGATGACAATGCGTTAGCTTA
CTACAACACAACAAAGGGAGGTAGGTTTGTACTTGCACTGTTATCCGATTTACAGGAT
TTGAAATGGGCTAGATTCCCTAAGAGTGATGGAACTGGTACTATCTATACAGAACTG
GAACCACCTTGTAGGTTTGTTACAGACACACCTAAAGGTCCTAAAGTGAAGTATTTAT
ACTTTATTAAAGGATTAAACAACCTAAATAGAGGTATGGTACTTGGTAGTTTAGCTGC
CACAGTACGTCTACAAGCTGGTAATGCAACAGAAGTGCCTGCCAATTCAACTGTATTA
TCTTTCTGTGCTTTTGCTGTAGATGCTGCTAAAGCTTACAAAGATTATCTAGCTAGTG
GGGGACAACCAATCACTAATTGTGTTAAGATGTTGTGTACACACACTGGTACTGGTC
AGGCAATAACAGTTACACCGGAAGCCAATATGGATCAAGAATCCTTTGGTGGTGCAT
CGTGTTGTCTGTACTGCCGTTGCCACATAGATCATCCAAATCCTAAAGGATTTTGTGA
CTTAAAAGGTAAGTATGTACAAATACCTACAACTTGTGCTAATGACCCTGTGGGTTTT
ACACTTAAAAACACAGTCTGTACCGTCTGCGGTATGTGGAAAGGTTATGGCTGTAGT
TGTGATCAACTCCGCGAACCCATGCTTCAGTCAGCTGATGCACAATCGTTTTTAAACG
GGTTTGCGGTGTAAGTGCAGCCCGTCTTACACCGTGCGGCACAGGCACTAGTACTG
ATGTCGTATACAGGGCTTTTGACATCTACAATGATAAAGTAGCTGGTTTTGCTAAATT
CCTAAAAACTAATTGTTGTCGCTTCCAAGAAAAGGACGAAGATGACAATTTAATTGAT
TCTTACTTTGTAGTTAAGAGACACACTTTCTCTAACTACCAACATGAAGAAACAATTTA
TAATTTACTTAAGGATTGTCCAGCTGTTGCTAAACATGACTTCTTTAAGTTTAGAATAG
 
ACGGTGACATGGTACCACATATATCACGTCAACGTCTTACTAAATACACAATGGCAGA
CCTCGTCTATGCTTTAAGGCATTTTGATGAAGGTAATTGTGACACATTAAAAGAAATA
CTTGTCACATACAATTGTTGTGATGATGATTATTTCAATAAAAAGGACTGGTATGATTT
TGTAGAAAACCCAGATATATTACGCGTATACGCCAACTTAGGTGAACGTGTACGCCA
AGCTTTGTTAAAAACAGTACAATTCTGTGATGCCATGCGAAATGCTGGTATTGTTGGT
GTACTGACATTAGATAATCAAGATCTCAATGGTAACTGGTATGATTTCGGTGATTTCA
TACAAACCACGCCAGGTAGTGGAGTTCCTGTTGTAGATTCTTATTATTCATTGTTAAT
GCCTATATTAACCTTGACCAGGGCTTTAACTGCAGAGTCACATGTTGACACTGACTTA
ACAAAGCCTTACATTAAGTGGGATTTGTTAAAATATGACTTCACGGAAGAGAGGTTAA
AACTCTTTGACCGTTATTTTAAATATTGGGATCAGACATACCACCCAAATTGTGTTAAC
TGTTTGGATGACAGATGCATTCTGCATTGTGCAAACTTTAATGTTTTATTCTCTACAGT
GTTCCCACCTACAAGTTTTGGACCACTAGTGAGAAAAATATTTGTTGATGGTGTTCCA
TTTGTAGTTTCAACTGGATACCACTTCAGAGAGCTAGGTGTTGTACATAATCAGGATG
TAAACTTACATAGCTCTAGACTTAGTTTTAAGGAATTACTTGTGTATGCTGCTGACCCT
GCTATGCACGCTGCTTCTGGTAATCTATTACTAGATAAACGCACTACGTGCTTTTCAG
TAGCTGCACTTACTAACAATGTTGCTTTTCAAACTGTCAAACCCGGTAATTTTAACAAA
GACTTCTATGACTTTGCTGTGTCTAAGGGTTTCTTTAAGGAAGGAAGTTCTGTTGAAT
TAAAACACTTCTTCTTTGCTCAGGATGGTAATGCTGCTATCAGCGATTATGACTACTA
TCGTTATAATCTACCAACAATGTGTGATATCAGACAACTACTATTTGTAGTTGAAGTTG
TTGATAAGTACTTTGATTGTTACGATGGTGGCTGTATTAATGCTAACCAAGTCATCGT
CAACAACCTAGACAAATCAGCTGGTTTTCCATTTAATAAATGGGGTAAGGCTAGACTT
TATTATGATTCAATGAGTTATGAGGATCAAGATGCACTTTTCGCATATACAAAACGTAA
TGTCATCCCTACTATAACTCAAATGAATCTTAAGTATGCCATTAGTGCAAAGAATAGA
GCTCGCACCGTAGCTGGTGTCTCTATCTGTAGTACTATGACCAATAGACAGTTTCATC
AAAAATTATTGAAATCAATAGCCGCCACTAGAGGAGCTACTGTAGTAATTGGAACAAG
CAAATTCTATGGTGGTTGGCACAACATGTTAAAAACTGTTTATAGTGATGTAGAAAAC
CCTCACCTTATGGGTTGGGATTATCCTAAATGTGATAGAGCCATGCCTAACATGCTTA
GAATTATGGCCTCACTTGTTCTTGCTCGCAAACATACAACGTGTTGTAGCTTGTCACA
CCGTTTCTATAGATTAGCTAATGAGTGTGCTCAAGTATTGAGTGAAATGGTCATGTGT
GGCGGTTCACTATATGTTAAACCAGGTGGAACCTCATCAGGAGATGCCACAACTGCT
TATGCTAATAGTGTTTTTAACATTTGTCAAGCTGTCACGGCCAATGTTAATGCACTTTT
ATCTACTGATGGTAACAAAATTGCCGATAAGTATGTCCGCAATTTACAACACAGACTT
TATGAGTGTCTCTATAGAAATAGAGATGTTGACACAGACTTTGTGAATGAGTTTTACG
CATATTTGCGTAAACATTTCTCAATGATGATACTCTCTGACGATGCTGTTGTGTGTTTC
AATAGCACTTATGCATCTCAAGGTCTAGTGGCTAGCATAAAGAACTTTAAGTCAGTTC
TTTATTATCAAAACAATGTTTTTATGTCTGAAGCAAAATGTTGGACTGAGACTGACCTT
ACTAAAGGACCTCATGAATTTTGCTCTCAACATACAATGCTAGTTAAACAGGGTGATG
 
ATTATGTGTACCTTCCTTACCCAGATCCATCAAGAATCCTAGGGGCCGGCTGTTTTGT
AGATGATATCGTAAAAACAGATGGTACACTTATGATTGAACGGTTCGTGTCTTTAGCT
ATAGATGCTTACCCACTTACTAAACATCCTAATCAGGAGTATGCTGATGTCTTTCATTT
GTACTTACAATACATAAGAAAGCTACATGATGAGTTAACAGGACACATGTTAGACATG
TATTCTGTTATGCTTACTAATGATAACACTTCAAGGTATTGGGAACCTGAGTTTTATGA
GGCTATGTACACACCGCATACAGTCTTACAGGCTGTTGGGGCTTGTGTTCTTTGCAA
TTCACAGACTTCATTAAGATGTGGTGCTTGCATACGTAGACCATTCTTATGTTGTAAAT
GCTGTTACGACCATGTCATATCAACATCACATAAATTAGTCTTGTCTGTTAATCCGTAT
GTTTGCAATGCTCCAGGTTGTGATGTCACAGATGTGACTCAACTTTACTTAGGAGGTA
TGAGCTATTATTGTAAATCACATAAACCACCCATTAGTTTTCCATTGTGTGCTAATGGA
CAAGTTTTTGGTTTATATAAAAATACATGTGTTGGTAGCGATAATGTTACTGACTTTAA
TGCAATTGCAACATGTGACTGGACAAATGCTGGTGATTACATTTTAGCTAACACCTGT
ACTGAAAGACTCAAGCTTTTTGCAGCAGAAACGCTCAAAGCTACTGAGGAGACATTT
AAACTGTCTTATGGTATTGCTACTGTACGTGAAGTGCTGTCTGACAGAGAATTACATC
TTTCATGGGAAGTTGGTAAACCTAGACCACCACTTAACCGAAATTATGTCTTTACTGG
TTATCGTGTAACTAAAAACAGTAAAGTACAAATAGGAGAGTACACCTTTGAAAAAGGT
GACTATGGTGATGCTGTTGTTTACCGAGGTACAACAACTTACAAATTAAATGTTGGTG
ATTATTTTGTGCTGACATCACATACAGTAATGCCATTAAGTGCACCTACACTAGTGCC
ACAAGAGCACTATGTTAGAATTACTGGCTTATACCCAACACTCAATATCTCAGATGAG
TTTTCTAGCAATGTTGCAAATTATCAAAAGGTTGGTATGCAAAAGTATTCTACACTCCA
GGGACCACCTGGTACTGGTAAGAGTCATTTTGCTATTGGCCTAGCTCTCTACTACCC
TTCTGCTCGCATAGTGTATACAGCTTGCTCTCATGCCGCTGTTGATGCACTATGTGAG
AAGGCATTAAAATATTTGCCTATAGATAAATGTAGTAGAATTATACCTGCACGTGCTC
GTGTAGAGTGTTTTGATAAATTCAAAGTGAATTCAACATTAGAACAGTATGTCTTTTGT
ACTGTAAATGCATTGCCTGAGACGACAGCAGATATAGTTGTCTTTGATGAAATTTCAA
TGGCCACAAATTATGATTTGAGTGTTGTCAATGCCAGATTACGTGCTAAGCACTATGT
GTACATTGGCGACCCTGCTCAATTACCTGCACCACGCACATTGCTAACTAAGGGCAC
ACTAGAACCAGAATATTTCAATTCAGTGTGTAGACTTATGAAAACTATAGGTCCAGAC
ATGTTCCTCGGAACTTGTCGGCGTTGTCCTGCTGAAATTGTTGACACTGTGAGTGCT
TTGGTTTATGATAATAAGCTTAAAGCACATAAAGACAAATCAGCTCAATGCTTTAAAAT
GTTTTATAAGGGTGTTATCACGCATGATGTTTCATCTGCAATTAACAGGCCACAAATA
GGCGTGGTAAGAGAATTCCTTACACGTAACCCTGCTTGGAGAAAAGCTGTCTTTATTT
CACCTTATAATTCACAGAATGCTGTAGCCTCAAAGATTTTGGGACTACCAACTCAAAC
TGTTGATTCATCACAGGGCTCAGAATATGACTATGTCATATTCACTCAAACCACTGAA
ACAGCTCACTCTTGTAATGTAAACAGATTTAATGTTGCTATTACCAGAGCAAAAGTAG
GCATACTTTGCATAATGTCTGATAGAGACCTTTATGACAAGTTGCAATTTACAAGTCTT
GAAATTCCACGTAGGAATGTGGCAACTTTACAAGCTGAAAATGTAACAGGACTCTTTA
 
AAGATTGTAGTAAGGTAATCACTGGGTTACATCCTACACAGGCACCTACACACCTCA
GTGTTGACACTAAATTCAAAACTGAAGGTTTATGTGTTGACATACCTGGCATACCTAA
GGACATGACCTATAGAAGACTCATCTCTATGATGGGTTTTAAAATGAATTATCAAGTT
AATGGTTACCCTAACATGTTTATCACCCGCGAAGAAGCTATAAGACATGTACGTGCAT
GGATTGGCTTCGATGTCGAGGGGTGTCATGCTACTAGAGAAGCTGTTGGTACCAATT
TACCTTTACAGCTAGGTTTTTCTACAGGTGTTAACCTAGTTGCTGTACCTACAGGTTA
TGTTGATACACCTAATAATACAGATTTTCCAGAGTTAGTGCTAAACCACCGCCTGGA
GATCAATTTAAACACCTCATACCACTTATGTACAAAGGACTTCCTTGGAATGTAGTGC
GTATAAAGATTGTACAAATGTTAAGTGACACACTTAAAAATCTCTCTGACAGAGTCGT
ATTTGTCTTATGGGCACATGGCTTTGAGTTGACATCTATGAAGTATTTTGTGAAAATA
GGACCTGAGCGCACCTGTTGTCTATGTGATAGACGTGCCACATGCTTTTCCACTGCT
TCAGACACTTATGCCTGTTGGCATCATTCTATTGGATTTGATTACGTCTATAATCCGTT
TATGATTGATGTTCAACAATGGGGTTTTACAGGTAACCTACAAAGCAACCATGATCTG
TATTGTCAAGTCCATGGTAATGCACATGTAGCTAGTTGTGATGCAATCATGACTAGGT
GTCTAGCTGTCCACGAGTGCTTTGTTAAGCGTGTTGACTGGACTATTGAATATCCTAT
AATTGGTGATGAACTGAAGATTAATGCGGCTTGTAGAAAGGTTCAACACATGGTTGTT
AAAGCTGCATTATTAGCAGACAAATTCCCAGTTCTTCACGACATTGGTAACCCTAAAG
CTATTAAGTGTGTACCTCAAGCTGATGTAGAATGGAAGTTCTATGATGCACAGCCTTG
TAGTGACAAAGCTTATAAAATAGAAGAATTATTCTATTCTTATGCCACACATTCTGACA
AATTCACAGATGGTGTATGCCTATTTTGGAATTGCAATGTCGATAGATATCCTGCTAA
TTCCATTGTTTGTAGATTTGACACTAGAGTGCTATCTAACCTTAACTTGCCTGGTTGT
GATGGTGGCAGTTTGTATGTAAATAAACATGCATTCCACACACCAGCTTTTGATAAAA
GTGCTTTTGTTAATTTAAAACAATTACCATTTTTCTATTACTCTGACAGTCCATGTGAG
TCTCATGGAAAACAAGTAGTGTCAGATATAGATTATGTACCACTAAAGTCTGCTACGT
GTATAACACGTTGCAATTTAGGTGGTGCTGTCTGTAGACATCATGCTAATGAGTACAG
ATTGTATCTCGATGCTTATAACATGATGATCTCAGCTGGCTTTAGCTTGTGGGTTTAC
AAACAATTTGATACTTATAACCTCTGGAACACTTTTACAAGACTTCAGAGTTTAGAAAA
TGTGGCTTTTAATGTTGTAAATAAGGGACACTTTGATGGACAACAGGGTGAAGTACCA
GTTTCTATCATTAATAACACTGTTTACACAAAAGTTGATGGTGTTGATGTAGAATTGTT
TGAAAATAAAACAACATTACCTGTTAATGTAGCATTTGAGCTTTGGGCTAAGCGCAAC
ATTAAACCAGTACCAGAGGTGAAAATACTCAATAATTTGGGTGTGGACATTGCTGCTA
ATACTGTGATCTGGGACTACAAAAGAGATGCTCCAGCACATATATCTACTATTGGTGT
TTGTTCTATGACTGACATAGCCAAGAAACCAACTGAAACGATTTGTGCACCACTCACT
GTCTTTTTTGATGGTAGAGTTGATGGTCAAGTAGACTTATTTAGAAATGCCCGTAATG
GTGTTCTTATTACAGAAGGTAGTGTTAAAGGTTTACAACCATCTGTAGGTCCCAAACA
AGCTAGTCTTAATGGAGTCACATTAATTGGAGAAGCCGTAAAAACACAGTTCAATTAT
TATAAGAAAGTTGATGGTGTTGTCCAACAATTACCTGAAACTTACTTTACTCAGAGTA
 
GAAATTTACAAGAATTTAAACCCAGGAGTCAAATGGAAATTGATTTCTTAGAATTAGCT
ATGGATGAATTCATTGAACGGTATAAATTAGAAGGCTATGCCTTCGAACATATCGTTT
ATGGAGATTTTAGTCATAGTCAGTTAGGTGGTTTACATCTACTGATTGGACTAGCTAA
ACGTTTTAAGGAATCACCTTTTGAATTAGAAGATTTTATTCCTATGGACAGTACAGTTA
AAAACTATTTCATAACAGATGCGCAAACAGGTTCATCTAAGTGTGTGTGTTCTGTTATT
GATTTATTACTTGATGATTTTGTTGAAATAATAAAATCCCAAGATTTATCTGTAGTTTCT
AAGGTTGTCAAAGTGACTATTGACTATACAGAAATTTCATTTATGCTTTGGTGTAAAGA
TGGCCATGTAGAAACATTTTACCCAAAATTACAATCTAGTCAAGCGTGGCAACCGGG
TGTTGCTATGCCTAATCTTTACAAAATGCAAAGAATGCTATTAGAAAAGTGTGACCTT
CAAAATTATGGTGATAGTGCAACATTACCTAAAGGCATAATGATGAATGTCGCAAAAT
ATACTCAACTGTGTCAATATTTAAACACATTAACATTAGCTGTACCCTATAATATGAGA
GTTATACATTTTGGTGCTGGTTCTGATAAAGGAGTTGCACCAGGTACAGCTGTTTTAA
GACAGTGGTTGCCTACGGGTACGCTGCTTGTCGATTCAGATCTTAATGACTTTGTCT
CTGATGCAGATTCAACTTTGATTGGTGATTGTGCAACTGTACATACAGCTAATAAATG
GGATCTCATTATTAGTGATATGTACGACCCTAAGACTAAAAATGTTACAAAAGAAAAT
GACTCTAAAGAGGGTTTTTTCACTTACATTTGTGGGTTTATACAACAAAAGCTAGCTC
TTGGAGGTTCCGTGGCTATAAAGATAACAGAACATTCTTGGAATGCTGATCTTTATAA
GCTCATGGGACACTTCGCATGGTGGACAGCCTTTGTTACTAATGTGAATGCGTCATC
ATCTGAAGCATTTTTAATTGGATGTAATTATCTTGGCAAACCACGCGAACAAATAGAT
GGTTATGTCATGCATGCAAATTACATATTTTGGAGGAATACAAATCCAATTCAGTTGT
CTTCCTATTCTTTATTTGACATGAGTAAATTTCCCCTTAAATTAAGGGGTACTGCTGTT
ATGTCTTTAAAAGAAGGTCAAATCAATGATATGATTTTATCTCTTCTTAGTAAAGGTAG
ACTTATAATTAGAGAAAACAACAGAGTTGTTATTTCTAGTGATGTTCTTGTTAACAACT
AA
Sequence #36:
   > **RNA P&P SARS-CoV-2 *lacZ*-1** 
GGCGCCGUCUUGUUGACCAACAGUUUGUUGACUAAAUAACGGUUAACAAGCUGUU
UCAGAGAGGACGCAAAUAAAGUCCUCUUUGGAGUAGUUUGUUGAUUUACUUUAAG
GAGGGCAAAAUGGGAAGCUUCGAUCCCGCCGUCGUUUUACAACG
Sequence #37:
   > **RNA P&P SARS-CoV-2 *lacZ*-2** 
GGCGCCGUCUUGUUGACCAACAGUUUGUUGACUAAAUAACGGUUAACAAGCUGUU
UCAGAGAGGACGCAAAUAAAGUCCUCUUUGGAGUAGUUUGUUGAUUUACUUUAAG
GAGGGCAAAAUGGGAAGCUUCGAUCCCGCCGUCGUUUUACAACG
Sequence #38:
   > **Synthetic RNA target of SARS-CoV-2** (*orf1ab* gene, nt position 3215-3244)
   AGUCAACAAACUGUUGGUCAACAAGACGGC
Sequence #39:
   > **5' primer of RNA P&P SARS-CoV-2 *lacZ*-1**
   T7 promoter (in *italics*) 
TCATAAGCTTTAATACGACTCACTATAGGCGCCGTCTTGTTGACCAACAGTTTGTTGA
CTAAATAACGGTTAACAAGCTGTTTCAGAGAGGACGCAAATAAAGTCCTCTTTGGAGT
AGTTTGTTGATTTACTTTAAGGAGGGCAAAATGGGAAGCTTCGATCCCGCCGTCGTT
TTACAACG
Sequence #40:
   > **5' primer of RNA P&P SARS-CoV-2 *lacZ*-2**
   T7 promoter (in *italics*) 
TCATAAGCTTTAATACGACTCACTATAGGCGCCGTCTTGTTGACCAACAGTTTGTTGA
CTAAATAACGGTTAACAAGCTGTTTCAAAGAGGACGCAAATAAAGTCCTCTTTGGAGT
AGTTTGTTGATTTACTTTAAGGAGGGCAAAATGGGAAGCTTCGATCCCGCCGTCGTT
TTACAACG
Sequence #41:
   > **PCR product of RNA P&P *lacZ*-1**
   T7 promoter (in *italics*), YEp358 vector underlined, *lacZ* CDS in grey 
TCATAAGCTTTAATACGACTCACTATAGGCGCCGTCTTGTTGACCAACAGTTTGTTGA
CTAAATAACGGTTAACAAGCTGTTTCAGAGAGGACGCAAATAAAGTCCTCTTTGGAGT
AGTTTGTTGATTTACTTTAAGGAGGGCAAAATGGAAGCTTCGATCCCGCCGTCGTT
TTACAACGTCGTGACTGGGAAAACCCTGGCGTTACCCAACTTAATCGCCTTGCAGCA
CATCCCCCTTTCGCCAGCTGGCGTAATAGCGAAGAGGCCCGCACCGATCGCCCTTC
CCAACAGTTGCGCAGCCTGAATGGCGAATGGCGCTTTGCCTGGTTTCCGGCACCAG
AAGCGGTGCCGGAAAGCTGGCTGGAGTGCGATCTTCCTGAGGCCGATACTGTCGTC
GTCCCCTCAAACTGGCAGATGCACGGTTACGATGCGCCCATCTACACCAACGTGAC
CTATCCCATTACGGTCAATCCGCCGTTTGTTCCCACGGAGAATCCGACGGGTTGTTA
CTCGCTCACATTTAATGTTGATGAAAGCTGGCTACAGGAAGGCCAGACGCGAATTAT
TTTTGATGGCGTTAACTCGGCGTTTCATCTGTGGTGCAACGGGCGCTGGGTCGGTTA
CGGCCAGGACAGTCGTTTGCCGTCTGAATTTGACCTGAGCGCATTTTTACGCGCCG
 
GAGAAAACCGCCTCGCGGTGATGGTGCTGCGCTGGAGTGACGGCAGTTATCTGGAA
GATCAGGATATGTGGCGGATGAGCGGCATTTTCCGTGACGTCTCGTTGCTGCATAAA
CCGACTACACAAATCAGCGATTTCCATGTTGCCACTCGCTTTAATGATGATTTCAGCC
GCGCTGTACTGGAGGCTGAAGTTCAGATGTGCGGCGAGTTGCGTGACTACCTACGG
GTAACAGTTTCTTTATGGCAGGGTGAAACGCAGGTCGCCAGCGGCACCGCGCCTTT
CGGCGGTGAAATTATCGATGAGCGTGGTGGTTATGCCGATCGCGTCACACTACGTCT
GAACGTCGAAAACCCGAAACTGTGGAGCGCCGAAATCCCGAATCTCTATCGTGCGG
TGGTTGAACTGCACACCGCCGACGGCACGCTGATTGAAGCAGAAGCCTGCGATGTC
GGTTTCCGCGAGGTGCGGATTGAAAATGGTCTGCTGCTGCTGAACGGCAAGCCGTT
GCTGATTCGAGGCGTTAACCGTCACGAGCATCATCCTCTGCATGGTCAGGTCATGGA
TGAGCAGACGATGGTGCAGGATATCCTGCTGATGAAGCAGAACAACTTTAACGCCGT
GCGCTGTTCGCATTATCCGAACCATCCGCTGTGGTACACGCTGTGCGACCGCTACG
GCCTGTATGTGGTGGATGAAGCCAATATTGAAACCCACGGCATGGTGCCAATGAATC
GTCTGACCGATGATCCGCGCTGGCTACCGGCGATGAGCGAACGCGTAACGCGAATG
GTGCAGCGCGATCGTAATCACCCGAGTGTGATCATCTGGTCGCTGGGGAATGAATC
AGGCCACGGCGCTAATCACGACGCGCTGTATCGCTGGATCAAATCTGTCGATCCTTC
CCGCCCGGTGCAGTATGAAGGCGGCGGAGCCGACACCACGGCCACCGATATTATTT
GCCCGATGTACGCGCGCGTGGATGAAGACCAGCCCTTCCCGGCTGTGCCGAAATG
GTCCATCAAAAAATGGCTTTCGCTACCTGGAGAGACGCGCCCGCTGATCCTTTGCGA
ATACGCCCACGCGATGGGTAACAGTCTTGGCGGTTTCGCTAAATACTGGCAGGCGTT
TCGTCAGTATCCCCGTTTACAGGGCGGCTTCGTCTGGGACTGGGTGGATCAGTCGC
TGATTAAATATGATGAAAACGGCAACCCGTGGTCGGCTTACGGCGGTGATTTTGGCG
ATACGCCGAACGATCGCCAGTTCTGTATGAACGGTCTGGTCTTTGCCGACCGCACG
CCGCATCCAGCGCTGACGGAAGCAAAACACCAGCAGCAGTTTTTCCAGTTCCGTTTA
TCCGGGCAAACCATCGAAGTGACCAGCGAATACCTGTTCCGTCATAGCGATAACGAG
CTCCTGCACTGGATGGTGGCGCTGGATGGTAAGCCGCTGGCAAGCGGTGAAGTGC
CTCTGGATGTCGCTCCACAAGGTAAACAGTTGATTGAACTGCCTGAACTACCGCAGC
CGGAGAGCGCCGGGCAACTCTGGCTCACAGTACGCGTAGTGCAACCGAACGCGAC
CGCATGGTCAGAAGCCGGGCACATCAGCGCCTGGCAGCAGTGGCGTCTGGCGGAA
AACCTCAGTGTGACGCTCCCCGCCGCGTCCCACGCCATCCCGCATCTGACCACCAG
CGAAATGGATTTTTGCATCGAGCTGGGTAATAAGCGTTGGCAATTTAACCGCCAGTC
AGGCTTTCTTTCACAGATGTGGATTGGCGATAAAAAACAACTGCTGACGCCGCTGCG
CGATCAGTTCACCCGTGCACCGCTGGATAACGACATTGGCGTAAGTGAAGCGACCC
GCATTGACCCTAACGCCTGGGTCGAACGCTGGAAGGCGGCGGGCCATTACCAGGC
CGAAGCAGCGTTGTTGCAGTGCACGGCAGATACACTTGCTGATGCGGTGCTGATTA
CGACCGCTCACGCGTGGCAGCATCAGGGGAAAACCTTATTTATCAGCCGGAAAACC
TACCGGATTGATGGTAGTGGTCAAATGGCGATTACCGTTGATGTTGAAGTGGCGAGC
 
GATACACCGCATCCGGCGCGGATTGGCCTGAACTGCCAGCTGGCGCAGGTAGCAG
AGCGGGTAAACTGGCTCGGATTAGGGCCGCAAGAAAACTATCCCGACCGCCTTACT
GCCGCCTGTTTTGACCGCTGGGATCTGCCATTGTCAGACATGTATACCCCGTACGTC
TTCCCGAGCGAAAACGGTCTGCGCTGCGGGACGCGCGAATTGAATTATGGCCCACA
CCAGTGGCGCGGCGACTTCCAGTTCAACATCAGCCGCTACAGTCAACAGCAACTGA
TGGAAACCAGCCATCGCCATCTGCTGCACGCGGAAGAAGGCACATGGCTGAATATC
GACGGTTTCCATATGGGGATTGGTGGCGACGACTCCTGGAGCCCGTCAGTATCGGC
GGAATTCCAGCTGAGCGCCGGTCGCTACCATTACCAGTTGGTCTGGTGTCAAAAATA
ACTAGCATAACCCCTCTCTAAACGGAGGGGTTT
Sequence #42:
   > **PCR product of RNA P&P *lacZ*-2**
   T7 promoter (in *italics*), YEp358 vector underlined, *lacZ* CDS in grey 
TCATAAGCTTTAATACGACTCACTATAGGCGCCGTCTTGTTGACCAACAGTTTGTTGA
CTAAATAACGGTTAACAAGCTGTTTCAAAGAGGACGCAAATAAAGTCCTCTTTGGAGT
AGTTTGTTGATTTACTTTAAGGAGGGCAAAATGGAAGCTTCGATCCCGCCGTCGTT
TTACAACGTCGTGACTGGGAAAACCCTGGCGTTACCCAACTTAATCGCCTTGCAGCA
CATCCCCCTTTCGCCAGCTGGCGTAATAGCGAAGAGGCCCGCACCGATCGCCCTTC
CCAACAGTTGCGCAGCCTGAATGGCGAATGGCGCTTTGCCTGGTTTCCGGCACCAG
AAGCGGTGCCGGAAAGCTGGCTGGAGTGCGATCTTCCTGAGGCCGATACTGTCGTC
GTCCCCTCAAACTGGCAGATGCACGGTTACGATGCGCCCATCTACACCAACGTGAC
CTATCCCATTACGGTCAATCCGCCGTTTGTTCCCACGGAGAATCCGACGGGTTGTTA
CTCGCTCACATTTAATGTTGATGAAAGCTGGCTACAGGAAGGCCAGACGCGAATTAT
TTTTGATGGCGTTAACTCGGCGTTTCATCTGTGGTGCAACGGGCGCTGGGTCGGTTA
CGGCCAGGACAGTCGTTTGCCGTCTGAATTTGACCTGAGCGCATTTTTACGCGCCG
GAGAAAACCGCCTCGCGGTGATGGTGCTGCGCTGGAGTGACGGCAGTTATCTGGAA
GATCAGGATATGTGGCGGATGAGCGGCATTTTCCGTGACGTCTCGTTGCTGCATAAA
CCGACTACACAAATCAGCGATTTCCATGTTGCCACTCGCTTTAATGATGATTTCAGCC
GCGCTGTACTGGAGGCTGAAGTTCAGATGTGCGGCGAGTTGCGTGACTACCTACGG
GTAACAGTTTCTTTATGGCAGGGTGAAACGCAGGTCGCCAGCGGCACCGCGCCTTT
CGGCGGTGAAATTATCGATGAGCGTGGTGGTTATGCCGATCGCGTCACACTACGTCT
GAACGTCGAAAACCCGAAACTGTGGAGCGCCGAAATCCCGAATCTCTATCGTGCGG
TGGTTGAACTGCACACCGCCGACGGCACGCTGATTGAAGCAGAAGCCTGCGATGTC
GGTTTCCGCGAGGTGCGGATTGAAAATGGTCTGCTGCTGCTGAACGGCAAGCCGTT
GCTGATTCGAGGCGTTAACCGTCACGAGCATCATCCTCTGCATGGTCAGGTCATGGA
TGAGCAGACGATGGTGCAGGATATCCTGCTGATGAAGCAGAACAACTTTAACGCCGT
GCGCTGTTCGCATTATCCGAACCATCCGCTGTGGTACACGCTGTGCGACCGCTACG
 
GCCTGTATGTGGTGGATGAAGCCAATATTGAAACCCACGGCATGGTGCCAATGAATC
GTCTGACCGATGATCCGCGCTGGCTACCGGCGATGAGCGAACGCGTAACGCGAATG
GTGCAGCGCGATCGTAATCACCCGAGTGTGATCATCTGGTCGCTGGGGAATGAATC
AGGCCACGGCGCTAATCACGACGCGCTGTATCGCTGGATCAAATCTGTCGATCCTTC
CCGCCCGGTGCAGTATGAAGGCGGCGGAGCCGACACCACGGCCACCGATATTATTT
GCCCGATGTACGCGCGCGTGGATGAAGACCAGCCCTTCCCGGCTGTGCCGAAATG
GTCCATCAAAAAATGGCTTTCGCTACCTGGAGAGACGCGCCCGCTGATCCTTTGCGA
ATACGCCCACGCGATGGGTAACAGTCTTGGCGGTTTCGCTAAATACTGGCAGGCGTT
TCGTCAGTATCCCCGTTTACAGGGCGGCTTCGTCTGGGACTGGGTGGATCAGTCGC
TGATTAAATATGATGAAAACGGCAACCCGTGGTCGGCTTACGGCGGTGATTTTGGCG
ATACGCCGAACGATCGCCAGTTCTGTATGAACGGTCTGGTCTTTGCCGACCGCACG
CCGCATCCAGCGCTGACGGAAGCAAAACACCAGCAGCAGTTTTTCCAGTTCCGTTTA
TCCGGGCAAACCATCGAAGTGACCAGCGAATACCTGTTCCGTCATAGCGATAACGAG
CTCCTGCACTGGATGGTGGCGCTGGATGGTAAGCCGCTGGCAAGCGGTGAAGTGC
CTCTGGATGTCGCTCCACAAGGTAAACAGTTGATTGAACTGCCTGAACTACCGCAGC
CGGAGAGCGCCGGGCAACTCTGGCTCACAGTACGCGTAGTGCAACCGAACGCGAC
CGCATGGTCAGAAGCCGGGCACATCAGCGCCTGGCAGCAGTGGCGTCTGGCGGA
AAACCTCAGTGTGACGCTCCCCGCCGCGTCCCACGCCATCCCGCATCTGACCACCAG
CGAAATGGATTTTTGCATCGAGCTGGGTAATAAGCGTTGGCAATTTAACCGCCAGTC
AGGCTTTCTTTCACAGATGTGGATTGGCGATAAAAAACAACTGCTGACGCCGCTGCG
CGATCAGTTCACCCGTGCACCGCTGGATAACGACATTGGCGTAAGTGAAGCGACCC
GCATTGACCCTAACGCCTGGGTCGAACGCTGGAAGGCGGCGGGCCATTACCAGGC
CGAAGCAGCGTTGTTGCAGTGCACGGCAGATACACTTGCTGATGCGGTGCTGATTA
CGACCGCTCACGCGTGGCAGCATCAGGGGAAAACCTTATTTATCAGCCGGAAAACC
TACCGGATTGATGGTAGTGGTCAAATGGCGATTACCGTTGATGTTGAAGTGGCGAGC
GATACACCGCATCCGGCGCGGATTGGCCTGAACTGCCAGCTGGCGCAGGTAGCAG
AGCGGGTAAACTGGCTCGGATTAGGGCCGCAAGAAAACTATCCCGACCGCCTTACT
GCCGCCTGTTTTGACCGCTGGGATCTGCCATTGTCAGACATGTATACCCCGTACGTC
TTCCCGAGCGAAAACGGTCTGCGCTGCGGGACGCGCGAATTGAATTATGGCCCACA
CCAGTGGCGCGGCGACTTCCAGTTCAACATCAGCCGCTACAGTCAACAGCAACTGA
TGGAAACCAGCCATCGCCATCTGCTGCACGCGGAAGAAGGCACATGGCTGAATATC
GACGGTTTCCATATGGGGATTGGTGGCGACGACTCCTGGAGCCCGTCAGTATCGGC
GGAATTCCAGCTGAGCGCCGGTCGCTACCATTACCAGTTGGTCTGGTGTCAAAAATA
ACTAGCATAACCCCTCTCTAAACGGAGGGGTTT
Sequence #43:
   *>* ***orf8* gene** (CDS); MN908947.3:27894-28259 Severe acute respiratory syndrome coronavirus 2 isolate Wuhan-Hu-1 (SARS-CoV-2), complete genome 
ATGAAATTTCTTGTTTTCTTAGGAATCATCACAACTGTAGCTGCATTTCACCAAGAATG
TAGTTTACAGTCATGTACTCAACATCAACCATATGTAGTTGATGACCCGTGTCCTATT
CACTTCTATTCTAAATGGTATATTAGAGTAGGAGCTAGAAAATCAGCACCTTTAATTGA
ATTGTGCGTGGATGAGGCTGGTTCTAAATCACCCATTCAGTACATCGATATCGGTAAT
TATACAGTTTCCTGTTTACCTTTTACAATTAATTGCCAGGAACCTAAATTGGGTAGTCT
TGTAGTGCGTTGTTCGTTCTATGAAGACTTTTTAGAGTATCATGACGTTCGTGTTGTTT
TAGATTTCATCTAA
Sequence #44:
   > **RNA P&P SARS-CoV-2 GFP-1** 
GGCGGUUAAUGGGUUGUUGGUUAGCGAGGCGGCAUAAAUCGCCGUCUUGCUGGC
CUAGGGAGGCGGCCAGGUACAUCGCCUCCUUAGGUUGGUAAGACGGUAACUUAA
GGAGGCGAAAAUGCAGAACCUGGCGGCAGCGCAAAAGAUGCGUAAA
Sequence #45:
   > **RNA P&P SARS-CoV-2 GFP-2** 
GGCCCUAUUCACUUUAUUCUAAAUGGUAUAUUUAAGACUGGUAUACCGUUUAGAU
AAGGAGGGCACCGGGGUAUUGUCCUCCUUGUUUGGACGGUAUAUCCGAAUAAGG
AGGACAAGAUGCAAAACCUGGCGGCAGCGCAAAAGAUGCGUAAA
Sequence #46:
   > **Synthetic RNA target of SARS-CoV-2 GFP-1** (*orf1ab* gene, nt position 3215-3244, reverse complement)
   GCCGUCUUGUUGACCAACAGUUUGUUGACU
Sequence #47:
   > **Synthetic RNA target of SARS-CoV-2 GFP-2** (*orf8* gene, nt position 28005-28034, reverse complement)
   AAUAUACCAUUUAGAAUAGAAGUGAAUAGG
Sequence #48:
   > **5' primer of RNA P&P SARS-CoV-2 GFP-1**
   T7 promoter (in *italics*) 
TCATAAGCTTTAATACGACTCACTATAGGCGGTTAATGGGTTGTTGGTTAGCGAGGC
GGCATAAATCGCCGTCTTGCTGGCCTAGGGAGGCGGCCAGGTACATCGCCTCCTTA
 
GGTTGGTAAGACGGTAACTTAAGGAGGCGAAAATGCAGAACCTGGCGGCAGCGCAA
AAGATGCGTAAA
Sequence #49:
   > **5' primer of RNA P&P SARS-CoV-2 GFP-2**
   T7 promoter (in *italics*) 
TCATAAGCTTTAATACGACTCACTATAGGCCCTATTCACTTCTATTCTAAATGGTATAT
TTAAGACTGGTATACCGTTTAGATAAGGAGGCACCGGGGTATTGTCCTCCTTGTTT
GGACGGTATATCCGAATAAGGAGGACAAGATGCAAAACCTGGCGGCAGCGCAAAAG
ATGCGTAAA
Sequence #50:
   > **PCR product of RNA P&P SARS-CoV-2 GFP-1**
   T7 promoter (in *italics), gfpmut3b-ASV* CDS in grey 
TCATAAGCTTTAATACGACTCACTATAGGCGGTTAATGGGTTGTTGGTTAGCGAGGC
GGCATAAATCGCCGTCTTGCTGGCCTAGGGAGGCGGCCAGGTACATCGCCTCCTTA
GGTTGGTAAGACGGTAACTTAAGGAGGCGAAAATGCAGAACCTGGCGGCAGCGCAA
AAGATGCGTAAAGGAGAAGAACTTTTCACTGGAGTTGTCCCAATTCTTGTTGAATTAG
ATGGTGATGTTAATGGGCACAAATTTTCTGTCAGTGGAGAGGGTGAAGGTGATGCAA
CATACGGAAAACTTACCCTTAAATTTATTTGCACTACTGGAAAACTACCTGTTCCGTG
GCCAACACTTGTCACTACTTTCGGTTATGGTGTTCAATGCTTTGCGAGATACCCAGAT
CACATGAAACAGCATGACTTTTTCAAGAGTGCCATGCCCGAAGGTTACGTACAGGAA
AGAACTATATTTTTCAAAGATGACGGGAACTACAAGACACGTGCTGAAGTCAAGTTTG
AAGGTGATACCCTTGTTAATAGAATCGAGTTAAAAGGTATTGATTTTAAAGAAGATGG
AAACATTCTTGGACACAAATTGGAATACAACTATAACTCACACAATGTATACATCATG
GCAGACAAACAAAAGAATGGAATCAAAGTTAACTTCAAAATTAGACACAACATTGAAG
ATGGAAGCGTTCAACTAGCAGACCATTATCAACAAAATACTCCGATTGGCGATGGCC
CTGTCCTTTTACCAGACAACCATTACCTGTCCACACAATCTGCCCTTTCGAAAGATCC
CAACGAAAAGAGAGACCACATGGTCCTTCTTGAGTTTGTAACCGCTGCTGGGATTAC
ACATGGCATGGATGAACTATACAAAGGCCTGCAGCAAACGACGAAACTACGCTGC
ATCAGTTTAATAATAAGATAAACCAGAGCGGCACGGCAAGCAGAGTATACGAGATTC
GGTAGCCACCGCTGAGCAATAACTAGCATAACCCCTTGGGGCCTCTAAACGGGTCTT
GAGGGGTTTTTTGCATATGCAACGG
Sequence #51:
   > **PCR product of RNA P&P SARS-CoV-2 GFP-2**
   T7 promoter (in *italics*), *gfpmut3b-ASV* CDS in grey 
TCATAAGCTTTAATACGACTCACTATAGGCAGTTAAGTTATTTATGGCTGAGAGTGGA
CTGTAAATAAGTCCGCTCTCAGTCGAAGGAGGCGTCTTTTGCAAACGCCTCCTTTGG
TTGAGAGCGGATTGACTAAAGGAGGCGTATATGTCGAACCTGGCGGCAGCGCAAAA
GATGCGTAAAGGAGAAGAACTTTTCACTGGAGTTGTCCCAATTCTTGTTGAATTAGAT
GGTGATGTTAATGGGCACAAATTTTCTGTCAGTGGAGAGGGTGAAGGTGATGCAACA
TACGGAAAACTTACCCTTAAATTTATTTGCACTACTGGAAAACTACCTGTTCCGTGGC
CAACACTTGTCACTACTTTCGGTTATGGTGTTCAATGCTTTGCGAGATACCCAGATCA
CATGAAACAGCATGACTTTTTCAAGAGTGCCATGCCCGAAGGTTACGTACAGGAAAG
AACTATATTTTTCAAAGATGACGGGAACTACAAGACACGTGCTGAAGTCAAGTTTGAA
GGTGATACCCTTGTTAATAGAATCGAGTTAAAAGGTATTGATTTTAAAGAAGATGGAA
ACATTCTTGGACACAAATTGGAATACAACTATAACTCACACAATGTATACATCATGGC
AGACAAACAAAAGAATGGAATCAAAGTTAACTTCAAAATTAGACACAACATTGAAGAT
GGAAGCGTTCAACTAGCAGACCATTATCAACAAAATACTCCGATTGGCGATGGCCCT
GTCCTTTTACCAGACAACCATTACCTGTCCACACAATCTGCCCTTTCGAAAGATCCCA
ACGAAAAGAGAGACCACATGGTCCTTCTTGAGTTTGTAACCGCTGCTGGGATTACAC
ATGGCATGGATGAACTATACAAAGGCCTGCAGCAAACGACGAAAACTACGCTGCAT
CAGTTTAATAATAAGATAAACCAGAGCGGCACGGCAAGCAGAGTATACGAGATTCGG
TAGCCACCGCTGAGCAATAACTAGCATAACCCCTTGGGGCCTCTAAACGGGTCTTGA
GGGGTTTTTTGCATATGCAACGG
Sequence #52:
   > **MK176509.1 Synthetic construct near-infrared fluorescent protein *miRFP670nano* gene,** CDS 
ATGGCAAACCTGGACAAGATGCTGAATACCACAGTAACAGAGGTGCGGCAGTTCCT
GCAGGTGGACAGAGTGTGCGTGTTCCAGTTTGAGGAGGATTATAGCGGAGTGGTGG
TGGTGGAGGCCGTGGACGATAGGTGGATCTCCATCCTGAAGACCCAGGTGCGGGAT
AGATACTTCATGGAGACAAGGGGCGAGGAGTATTCTCACGGCCGCTACCAGGCCAT
CGCCGACATCTACACCGCAAACCTGACAGAGTGCTACAGGGATCTGCTGACACAGTT
TCAGGTGAGAGCAATCCTGGCCGTGCCCATCCTGCAGGGCAAGAAGCTGTGGGGC
CTGTTGGTGGCACACCAGCTGGCGGCCCCTAGACAGTGGCAGACCTGGGAGATCG
ACTTTCTGAAGCAGCAGGCCGTGGTGGTGGGCATCGCCATCCAGCAGAGCTAA
Sequence #53:
   > **RNA P&P SARS-CoV-2 miRFP/GFP-1**
   *miRFP670nano* gene CDS underlined; 
GGCGGUUGAUGGGUUGUUGGUCAACAGGGCGGCGAUAAUCGCCGUUCUGUUGGC
UAGCAAGGAGGAAAAUAUGGCAAACCUGGACAAGAUGCUGAAUACCACAGUAACAG
AGGUGCGGCAGUUCCUGCAGGUGGACAGAGUGUGCGUGUUCCAGUUUGAGGAGG
AUUAUAGCGGAGUGGUGGUGGAGGCCGUGGACGAUAGGUGGAUCUCCAUCC
 
UGAAGACCCAGGUGCGGAUAGAUACUUCAUGGAGACAAGGGCGAGGAGUAUU
CUCACGGCCGCUACCAGGCCAUCGCCGACAUCUACACCGCAAACCUGACAGAGUG
CUACAGGGAUCUGCUGACACAGUUUCAGGUGAGAGCAAUCCUGGCCGUGCCCAUC
CUGCAGGGCAAGAAGCUGUGGGGCCUGUUGGUGGCACACCAGCUGGCGGCCCCU
AGACAGUGGCAGACCUGGGAGAUCGACUUUCUGAAGCAGCAGGCCGUGGUGGUG
GGCAUCGCCAUCCAGCAGAGCUAAUUUCCUCCUUGUUAGCCAAUAGAAUGGUCUA
ACAAGGAGGAAACAAUGUAUACAACAAACCUGGCGGCAGCGCAAAAGAUG
Sequence #54:
   > **RNA P&P SARS-CoV-2 miRFP/GFP-2**
   *miRFP670nano* gene CDS underlined; 
GGCGGUUGAUGGGUUGUUGGUCAACAGGGCGGCGAUAAUUGCCGUUCUGUUGGC
UAGCAAGGAGGAAAAUAUGGCAAACCUGGACAAGAUGCUGAAUACCACAGUAACAG
AGGUGCGGCAGUUCCUGCAGGUGGACAGUGUGCGUGUUCCAGUUUGAGGAGG
AUUAUAGCGGAGUGGUGGUGGUGGAGGCCGUGGACGAUAGGUGGAUCUCCAUCC
UGAAGACCCAGGUGCGGGAUAGAUACUUCAUGGAGACAAGGGGCGAGGAGUAUU
CUCACGGCCGCUACCAGGCCAUCGCCGACAUCUACACCGCAAACCUGACAGAGUG
CUACAGGGAUCUGCUGACACAGUUUCAGGUGAGAGCAAUCCUGGCCGUGCCCAUC
CUGCAGGGCAAGAAGCUGUGGGGCCUGUUGGUGGCACACCAGCUGGCGGCCCCU
AGACAGUGGCAGACCUGGGAGAUCGACUUUCUGAAGCAGCAGGCCGUGGUGGUG
GGCAUCGCCAUCCAGCAGAGCUAAUUUCCUCCUUGUUAGCCAAUAGAAUGGUCUA
ACAAGGAGGAAACAAUGUAUACAACAAACCUGGCGGCAGCGCAAAAGAUG
Sequence #55:
   > **5' primer #1 for RNA P&P SARS-CoV-2 miRFP/GFP-1** (PCR#1)
   T7 promoter (in *italics*) 
TCATAAGCTTTAATACGACTCACTATAGGCGGTTGATGGGTTGTTGGTCAACAGGGC
GGCGATAATCGCCGTTCTGTTGGCTAGCAAGGAGGAAAATATGGCAAACCTGGACAA
G
Sequence #56:
   > **5' primer #1 for RNA P&P SARS-CoV-2 miRFP/GFP-2** (PCR#1)
   T7 promoter (in *italics*) 
TCATAAGCTTTAATACGACTCACTATAGGCGGTTGATGGGTTGTTGGTCAACAGGGC
GGCGATAATTGCCGTTCTGTTGGCTAGCAAGGAGGAAAATATGGCAAACCTGGACAA
G
Sequence #57:
   > **3' primer** (reverse) **for RNA P&P SARS-CoV-2 and *E.coli* miRFP/GFP-1/-2** (PCR#1)
   PCR#1/2 32-nt overlapping region in **bold**
   **CAGCTGGTGTGCCACCAACAGGCCCCACAGCT**TCTTGCCCTGCAGGATGGGC
Sequence #58:
   > **PCR product #1 of RNA P&P SARS-CoV-2 miRFP/GFP-1**
   T7 promoter (in *italics*); part of *miRFP670nano* CDS underlined; 32-nt PCR#1/2 overlapping region in **bold** 
TCATAAGCTTTAATACGACTCACTATAGGCGGTTGATGGGTTGTTGGTCAACAGGGC
GGCGATAATCGCCGTTCTGTTGGCTAGCAAGGAGGAAAATATGGCAAACCTGGACAA
GATGCTGAATACCACAGTAACAGAGGTGCGGCAGTTCCTGCAGGTGGACAGAGTGT
GCGTGTTCCAGTTTGAGGAGGATTATAGCGGAGTGGTGGTGGAGGCCGTGGAC
GATAGGTGGATCTCCATCCTGAAGACCCAGGTGCGGGATAGATACTTCATGGAGACA
AGGGGCGAGGAGTATTCTCACGGCCGCTACCAGGCCATCGCCGACATCTACACCGC
AAACCTGACAGAGTGCTACAGGGATCTGCTGACACAGTTTCAGGTGAGAGCAATCCT
GGCCGTGCCCATCCTGCAGGGCAAGAAGCTGTGGGGCCTGTTGGTGGCACACCAG
CTG
Sequence #59:
   > **PCR product #1 of RNA P&P SARS-CoV-2 miRFP/GFP-2**
   T7 promoter (in *italics*); part of *miRFP670nano* CDS underlined; 32-nt PCR#1/2 overlapping region in **bold** 
TCATAAGCTTTAATACGACTCACTATAGGCGGTTGATGGGTTGTTGGTCAACAGGGC
GGCGATAATTGCCGTTCTGTTGGCTAGCAAGGAGGAAAATATGGCAAACCTGGACAA
GATGCTGAATACCACAGTAACAGAGGTGCGGCAGTTCCTGCAGGTGGACAGAGTGT
GCGTGTTCCAGTTTGAGGAGGATTATAGCGGAGTGGTGGTGGAGGCCGTGGAC
GATAGGTGGATCTCCATCCTGAAGACCCAGGTGCGGGATAGATACTTCATGGAGACA
AGGGGCGAGGAGTATTCTCACGGCCGCTACCAGGCCATCGCCGACATCTACACCGC
AAACCTGACAGAGTGCTACAGGGATCTGCTGACACAGTTTCAGGTGAGAGCAATCCT
GGCCGTGCCCATCCTGCAGGGCAAGAAGCTGTGGGGCCTGTTGGTGGCACACCAG
CTG
Sequence #60:
   > **5' primer #2 for RNA P&P SARS-CoV-2 miRFP/GFP-1/-2** (PCR#2) 
AGCTGTGGGGCCTGTTGGTGGCACACCAGCTGGCGGCCCCTAGACAGTGGCAGAC
CTGGGAGATCGACTTTCTGAAGCAGCAGGCCGTGGTGGTGGGCATCGCCATCCAGC
AGAGCTAATTTCCTCCTTGTTAGCCAATAGAATGGTCTAACAAGGAGGAAACAATGTA
TACAACAAACCTGGCGGCAGCGCAAAAGATG
Sequence #61:
   > **PCR product#2 for RNA P&P SARS-CoV-2 miRFP/GFP-1**
   End of *miRFP670nano* CDS underlined; 32-nt overlapping region in **bold;** *gfpmut3b-ASV* CDS in grey 
AGCTGTGGGGCCTGTTGGTGGCACACCAGCTGGCGGCCCCTAGACAGTGGCAGAC
CTGGGAGATCGACTTTCTGAAGCAGCAGGCCGTGGTGGGCATCGCCATCCAGC
AGAGCTAATTTCCTCCTTGTTAGCCAATAGAATGGTCTAACAAGGAGGAAACAATGTA
TACAACAAACCTGGCGGCAGCGCAAAAGATGCGTAAAGGAGAAGAACTTTTCACTGG
AGTTGTCCCAATTCTTGTTGAATTAGATGGTGATGTTAATGGGCACAAATTTTCTGTC
AGTGGAGAGGGTGAAGGTGATGCAACATACGGAAAACTTACCCTTAAATTTATTTGC
ACTACTGGAAAACTACCTGTTCCGTGGCCAACACTTGTCACTACTTTCGGTTATGGTG
TTCAATGCTTTGCGAGATACCCAGATCACATGAAACAGCATGACTTTTTCAAGAGTGC
CATGCCCGAAGGTTACGTACAGGAAAGAACTATATTTTTCAAAGATGACGGGAACTA
CAAGACACGTGCTGAAGTCAAGTTTGAAGGTGATACCCTTGTTAATAGAATCGAGTTA
AAAGGTATTGATTTTAAAGAAGATGGAAACATTCTTGGACACAAATTGGAATACAACT
ATAACTCACACAATGTATACATCATGGCAGACAAACAAAAGAATGGAATCAAAGTTAA
CTTCAAAATTAGACACAACATTGAAGATGGAAGCGTTCAACTAGCAGACCATTATCAA
CAAAATACTCCGATTGGCGATGGCCCTGTCCTTTTACCAGACAACCATTACCTGTCCA
CACAATCTGCCCTTTCGAAAGATCCCAACGAAAAGAGAGACCACATGGTCCTTCTTG
AGTTTGTAACCGCTGCTGGGATTACACATGGCATGGATGAACTATACAAAGGCCTG
CAGCAAACGACGAAAACTACGCTGCATCAGTTTAATAATAAGATAAACCAGAGCGGC
ACGGCAAGCAGAGTATACGAGATTCGGTAGCCACCGCTGAGCAATAACTAGCATAAC
CCCTTGGGGCCTCTAAACGGGTCTTGAGGGGTTTTTTGCATATGCAACGG
Sequence #62:
   > **PCR product#2 for RNA P&P SARS-CoV-2 miRFP/GFP-2**
   End of *miRFP670nano* CDS underlined; 32-nt overlapping region in **bold;** *gfpmut3b-ASV* CDS in grey 
AGCTGTGGGGCCTGTTGGTGGCACACCAGCTGGCGGCCCCTAGACAGTGGCAGAC
CTGGGAGATCGACTTTCTGAAGCAGCAGGCCGTGGTGGGCATCGCCATCCAGC
AGAGCTAATTTCCTCCTTGTTAGCCAATAGAATGGTCTAACAAGGAGGAAACAATGTA
TACAACAAACCTGGCGGCAGCGCAAAAGATGCGTAAAGGAGAAGAACTTTTCACTGG
AGTTGTCCCAATTCTTGTTGAATTAGATGGTGATGTTAATGGGCACAAATTTTCTGTC
AGTGGAGAGGGTGAAGGTGATGCAACATACGGAAAACTTACCCTTAAATTTATTTGC
ACTACTGGAAAACTACCTGTTCCGTGGCCAACACTTGTCACTACTTTCGGTTATGGTG
TTCAATGCTTTGCGAGATACCCAGATCACATGAAACAGCATGACTTTTTCAAGAGTGC
CATGCCCGAAGGTTACGTACAGGAAAGAACTATATTTTTCAAAGATGACGGGAACTA
CAAGACACGTGCTGAAGTCAAGTTTGAAGGTGATACCCTTGTTAATAGAATCGAGTTA
AAAGGTATTGATTTTAAAGAAGATGGAAACATTCTTGGACACAAATTGGAATACAACT
ATAACTCACACAATGTATACATCATGGCAGACAAACAAAAGAATGGAATCAAAGTTAA
CTTCAAAATTAGACACAACATTGAAGATGGAAGCGTTCAACTAGCAGACCATTATCAA
CAAAATACTCCGATTGGCGATGGCCCTGTCCTTTTACCAGACAACCATTACCTGTCCA
CACAATCTGCCCTTTCGAAAGATCCCAACGAAAAGAGAGACCACATGGTCCTTCTTG
AGTTTGTAACCGCTGCTGGGATTACACATGGCATGGATGAACTATACAAAGGCCTG
CAGCAAACGACGAAAACTACGCTGCATCAGTTTAATAATAAGATAAACCAGAGCGGC
ACGGCAAGCAGAGTATACGAGATTCGGTAGCCACCGCTGAGCAATAACTAGCATAAC
CCCTTGGGGCCTCTAAACGGGTCTTGAGGGGTTTTTTGCATATGCAACGG
Sequence #63:
   > **5' primer T7** (forward)
   TCATAAGCTTTAATACGACTC
Sequence #64:
   > **3' primer GFP term** (reverse)
   CCGTTGCATATGCAAAAAACC
Sequence #65:
   > **PCR final product of RNA P&P SARS-CoV-2 miRFP/GFP-1**
   T7 promoter (in *italics*), *gfpmut3b-ASV* CDS in grey 
TCATAAGCTTTAATACGACTCACTATAGGCGGTTGATGGGTTGTTGGTCAACAGGGC
GGCGATAATCGCCGTTCTGTTGGCTAGCAAGGAGGAAAATATGGCAAACCTGGACAA
GATGCTGAATACCACAGTAACAGAGGTGCGGCAGTTCCTGCAGGTGGACAGAGTGT
GCGTGTTCCAGTTTGAGGAGGATTATAGCGGAGTGGTGGTGGAGGCCGTGGAC
GATAGGTGGATCTCCATCCTGAAGACCCAGGTGCGGGATAGATACTTCATGGAGACA
AGGGGCGAGGAGTATTCTCACGGCCGCTACCAGGCCATCGCCGACATCTACACCGC
AAACCTGACAGAGTGCTACAGGGATCTGCTGACACAGTTTCAGGTGAGAGCAATCCT
GGCCGTGCCCATCCTGCAGGGCAAGAAGCTGTGGGGCCTGTTGGTGGCACACCAG
CTGGCGGCCCCTAGACAGTGGCAGACCTGGGAGATCGACTTTCTGAAGCAGCAGGC
CGTGGTGGTGGGCATCGCCATCCAGCAGAGCTAATTTCCTCCTTGTTAGCCAATAGA
ATGGTCTAACAAGGAGGAAACAATGTATACAACAAACCTGGCGGCAGCGCAAAAGAT
GCGTAAAGGAGAAGAACTTTTCACTGGAGTTGTCCCAATTCTTGTTGAATTAGATGGT
GATGTTAATGGGCACAAATTTTCTGTCAGTGGAGAGGGTGAAGGTGATGCAACATAC
GGAAAACTTACCCTTAAATTTATTTGCACTACTGGAAAACTACCTGTTCCGTGGCCAA
CACTTGTCACTACTTTCGGTTATGGTGTTCAATGCTTTGCGAGATACCCAGATCACAT
GAAACAGCATGACTTTTTCAAGAGTGCCATGCCCGAAGGTTACGTACAGGAAAGAAC
TATATTTTTCAAAGATGACGGGAACTACAAGACACGTGCTGAAGTCAAGTTTGAAGGT
GATACCCTTGTTAATAGAATCGAGTTAAAAGGTATTGATTTTAAAGAAGATGGAAACA
TTCTTGGACACAAATTGGAATACAACTATAACTCACACAATGTATACATCATGGCAGA
CAAACAAAAGAATGGAATCAAAGTTAACTTCAAAATTAGACACAACATTGAAGATGGA
AGCGTTCAACTAGCAGACCATTATCAACAAAATACTCCGATTGGCGATGGCCCTGTC
CTTTTACCAGACAACCATTACCTGTCCACACAATCTGCCCTTTCGAAAGATCCCAACG
AAAAGAGAGACCACATGGTCCTTCTTGAGTTTGTAACCGCTGCTGGGATTACACATG
GCATGGATGAACTATACAAAGGCCTGCAGCAAACGACGAAAACTACGCTGCATCAG
TTTAATAATAAGATAAACCAGAGCGGCACGGCAAGCAGAGTATACGAGATTCGGTAG
CCACCGCTGAGCAATAACTAGCATAACCCCTTGGGGCCTCTAAACGGGTCTTGAGG
GGTTTTTTGCATATGCAACGG
Sequence #66:
   > **PCR product of RNA P&P SARS-CoV-2 miRFP/GFP-1**
   T7 promoter (in *italics); miRFP670nano* underlined; *gfpmut3b-ASV* CDS in grey 
TCATAAGCTTTAATACGACTCACTATAGGCGGTTGATGGGTTGTTGGTCAACAGGGC
GGCGATAATTGCCGTTCTGTTGGCTAGCAAGGAGGAAAATATGGCAAACCTGGACAA
GATGCTGAATACCACAGTAACAGAGGTGCGGCAGTTCCTGCAGGTGGACAGAGTGT
GCGTGTTCCAGTTTGAGGAGGATTATAGCGGAGTGGTGGTGGAGGCCGTGGAC
GATAGGTGGATCTCCATCCTGAAGACCCAGGTGCGGGATAGATACTTCATGGAGACA
AGGGGCGAGGAGTATTCTCACGGCCGCTACCAGGCCATCGCCGACATCTACACCGC
AAACCTGACAGAGTGCTACAGGGATCTGCTGACACAGTTTCAGGTGAGAGCAATCCT
GGCCGTGCCCATCCTGCAGGGCAAGAAGCTGTGGGGCCTGTTGGTGGCACACCAG
CTGGCGGCCCCTAGACAGTGGCAGACCTGGGAGATCGACTTTCTGAAGCAGCAGGC
CGTGGTGGTGGGCATCGCCATCCAGCAGAGCTAATTTCCTCCTTGTTAGCCAATAGA
ATGGTCTAACAAGGAGGAAACAATGTATACAACAAACCTGGCGGCAGCGCAAAAGAT
GCGTAAAGGAGAAGAACTTTTCACTGGAGTTGTCCCAATTCTTGTTGAATTAGATGGT
GATGTTAATGGGCACAAATTTTCTGTCAGTGGAGAGGGTGAAGGTGATGCAACATAC
GGAAAACTTACCCTTAAATTTATTTGCACTACTGGAAAACTACCTGTTCCGTGGCCAA
CACTTGTCACTACTTTCGGTTATGGTGTTCAATGCTTTGCGAGATACCCAGATCACAT
GAAACAGCATGACTTTTTCAAGAGTGCCATGCCCGAAGGTTACGTACAGGAAAGAAC
TATATTTTTCAAAGATGACGGGAACTACAAGACACGTGCTGAAGTCAAGTTTGAAGGT
GATACCCTTGTTAATAGAATCGAGTTAAAAGGTATTGATTTTAAAGAAGATGGAAACA
TTCTTGGACACAAATTGGAATACAACTATAACTCACACAATGTATACATCATGGCAGA
CAAACAAAAGAATGGAATCAAAGTTAACTTCAAAATTAGACACAACATTGAAGATGGA
AGCGTTCAACTAGCAGACCATTATCAACAAAATACTCCGATTGGCGATGGCCCTGTC
CTTTTACCAGACAACCATTACCTGTCCACACAATCTGCCCTTTCGAAAGATCCCAACG
AAAAGAGAGACCACATGGTCCTTCTTGAGTTTGTAACCGCTGCTGGGATTACACATG
GCATGGATGAACTATACAAAGGCCTGCAGCAAACGACGAAAACTACGCTGCATCAG
TTTAATAATAAGATAAACCAGAGCGGCACGGCAAGCAGAGTATACGAGATTCGGTAG
CCACCGCTGAGCAATAACTAGCATAACCCCTTGGGGCCTCTAAACGGGTCTTGAGG
GGTTTTTTGCATATGCAACGG
Sequence #67:
   > **RNA P&P *E.coli* miRFP/GFP-1**
   *miRFP670nano* gene CDS underlined; 
GGCAAGUUUGAUGUGGCUGUGCUGCGUGUAUGGGGAUAACUUGUAUGUGUGGCG
CAGCAAGGAGGAAAAUAUGGCAAACCUGGACAAGAUGCUGAAUACCACAGUAACAG
AGGUGCGGCAGUUCCUGCAGGUGGACAGAGUGUGCGUGUUCCAGUUUGAGGAGG
AUUAUAGCGGAGUGGUGGUGGUGGAGGCCGUGGACGAUAGGUGGAUCUCCAUCC
UGAAGACCCAGGUGCGGGAUAGAUACUUCAUGGAGACAAGGGGCGAGGAGUAUU
CUCACGGCCGCUACCAGGCCAUCGCCGACAUCUACACCGCAAACCUGACAGAGUG
CUACAGGGAUCUGCUGACACAGUUUCAGGUGAGAGCAAUCCUGGCCGUGCCCAUC
CUGCAGGGCAAGAAGCUGUGGGGCCUGUUGGUGGCACACCAGCUGGCGGCCCCU
AGACAGUGGCAGACCUGGGAGAUCGACUUUCUGAAGCAGCAGGCCGUGGUGGUG
GGCAUCGCCAUCCAGCAGAGCUAAUUUCCUCCUUGUUGCGCUGUAUGUACGGGC
AAUAAGGAGGAAAUAAUGUCAACCACCAACCUGGCGGCAGCGCAAAAGAUG
Sequence #68:
   > **RNA P&P *E.coli* miRFP/GFP-2**
   *miRFP670nano* gene CDS underlined; 
GGCUAGUUGUGUUGUGUAUGAAGAAGGCCUUGAAUAACGGGGUCUUCUUUGU
GGUUAAGGAGGAAAAUAUGGCAAACCUGGACAAGAUGCUGAAUACCACAGUAACA
GAGGUGCGGCAGUUCCUGCAGGUGGACAGAGUGUGCGUGUUCCAGUUUGAGGAG
GAUUAUAGCGGAGUGGUGGUGGUGGAGGCCGUGGACGAUAGGUGGAUCUCCAUC
CUGAAGACCCAGGUGCGGGAUAGAUACUUCAUGGAGACAAGGGGCGAGGAGUAU
UCUCACGGCCGCUACCAGGCCAUCGCCGACAUCUACACCGCAAACCUGACAGAGU
GCUACAGGGAUCUGCUGACACAGUUUCAGGUGAGAGCAAUCCUGGCCGUGCCCA
UCCUGCAGGGCAAGAAGCUGUGGGGCCUGUUGGUGGCACACCAGCUGGCGGCCC
CUAGACAGUGGCAGACCUGGGAGAUCGACUUUCUGAAGCAGCAGGCCGUGGUGG
UGGGCAUCGCCAUCCAGCAGAGCUAAUUUCCUCCUUAGUCACGAAGAAGACUUCU
GACUAAGGAGGGAAUAAUGUCGAAGACGAACCUGGCGGCAGCGCAAAAGAUG
Sequence #69:
   > **RNA P&P *E.coli* miRFP/GFP-3**
   *miRFP670nano* gene CDS underlined; 
GGCUAGUUGUGUUGUGUAUGAAGAAGGCCUUGAAUAACGGGGUCUUCUUCGU
GGUUAAGGAGGAAAAUAUGGCAAACCUGGACAAGAUGCUGAAUACCACAGUAACA
GAGGUGCGGCAGUUCCUGCAGGUGGACAGAGUGCGUGUUCCAGUUUGAGGAG
GAUUAUAGCGGAGUGGUGGUGGUGGAGGCCGUGGACGAUAGGUGGAUCUCCAUC
CUGAAGACCCAGGUGCGGGAUAGAUACUUCAUGGAGACAAGGGGCGAGGAGUAU
UCUCACGGCCGCUACCAGGCCAUCGCCGACAUCUACACCGCAAACCUGACAGAGU
GCUACAGGGAUCUGCUGACACAGUUUCAGGUGAGAGCAAUCCUGGCCGUGCCCA
UCCUGCAGGGCAAGAAGCUGUGGGCCUGUUGGUGGCACACCAGCUGGCGGCCC
CUAGACAGUGGCAGACCUGGGAGAUCGACUUUCUGAAGCAGCAGGCCGUGGUG
UGGGCAUCGCCAUCCAGCAGAGCUAAUUUCCUCCUUAGUCACGAAGAAGACUUCU
GACUAAGGAGGGAAUAAUGUCGAAGACGAACCUGGCGGCAGCGCAAAAGAUG
Sequence #70:
   > **RNA P&P *E.coli* miRFP/GFP-4**
   *miRFP670nano* gene CDS underlined; 
GGCUAGUUGUGUUGUGUAUGAAGAGGGCCUUGAAUAACGGGGUCUUCUUCGU
GGUUAAGGAGGAAAAUAUGGCAAACCUGGACAAGAUGCUGAAUACCACAGUAACA
GAGGUGCGGCAGUUCCUGCAGGUGGACAGAGUGCGUGUUCCAGUUUGAGGAG
GAUUAUAGCGGAGUGGUGGUGGUGGAGGCCGUGGACGAUAGGUGGAUCUCCAUC
CUGAAGACCCAGGUGCGGGAUAGAUACUUCAUGGAGACAAGGGGCGAGGAGUAU
UCUCACGGCCGCUACCAGGCCAUCGCCGACAUCUACACCGCAAACCUGACAGAGU
GCUACAGGGAUCUGCUGACACAGUUUCAGGUGAGAGCAAUCCUGGCCGUGCCCA
UCCUGCAGGGCAAGAAGCUGUGGGCCUGUUGGUGGCACACCAGCUGGCGGCCC
CUAGACAGUGGCAGACCUGGGAGAUCGACUUUCUGAAGCAGCAGGCCGUGGUGG
UGGGCAUCGCCAUCCAGCAGAGCUAAUUUCCUCCUUAGUCACGAAGAAGACUUCU
GACUAAGGAGGGAAUAAUGUCGAAGACGAACCUGGCGGCAGCGCAAAAGAUG
Sequence #71:
   > **5' primer #1 for RNA P&P *E.coli* miRFP/GFP-1** (PCR#1)
   T7 promoter (in *italics*) 
TCATAAGCTTTAATACGACTCACTATAGGCAAGTTTGATGTGGCTGTGCTGCGTGTAT
GGGGATAACTTGTATGTGTGGCGCAGCAAGGAGGAAAATATGGCAAACCTGGACAA
G
Sequence #72:
   > **5' primer #1 for RNA P&P *E.coli* miRFP/GFP-2** (PCR#1)
   T7 promoter (in *italics*) 
TCATAAGCTTTAATACGACTCACTATAGGCTAGTTGTGTTGTGTGTATGAAGAAGGCC
TTGAATAACGGGGTCTTCTTTGTGGTTAAGGAGGAAAATATGGCAAACCTGGACAAG
Sequence #73:
   > **5' primer #1 for RNA P&P *E.coli* miRFP/GFP-3** (PCR#1)
   T7 promoter (in *italics*) 
TCATAAGCTTTAATACGACTCACTATAGGCTAGTTGTGTTGTGTGTATGAAGAAGGCC
TTGAATAACGGGGTCTTCTTCGTGGTTAAGGAGGAAAATATGGCAAACCTGGACAAG
Sequence #74:
   > **5' primer #1 for RNA P&P *E.coli* miRFP/GFP-4** (PCR#1)
   T7 promoter (in *italics*) 
TCATAAGCTTTAATACGACTCACTATAGGCTAGTTGTGTTGTGTGTATGAAGAGGGCC
TTGAATAACGGGGTCTTCTTCGTGGTTAAGGAGGAAAATATGGCAAACCTGGACAAG
Sequence #75:
   > **PCR product #1 of RNA P&P *E.coli* miRFP/GFP-1**
   T7 promoter (in *italics*); part of *miRFP670nano* CDS underlined; 32-nt PCR#1/2 overlapping region in **bold** 
TCATAAGCTTTAATACGACTCACTATAGGCAAGTTTGATGTGGCTGTGCTGCGTGTAT
GGGGATAACTTGTATGTGTGGCGCAGCAAGGAGGAAAATATGGCAAACCTGGACAA
GATGCTGAATACCACAGTAACAGAGGTGCGGCAGTTCCTGCAGGTGGACAGAGTGT
GCGTGTTCCAGTTTGAGGAGGATTATAGCGGAGTGGTGGTGGAGGCCGTGGAC
GATAGGTGGATCTCCATCCTGAAGACCCAGGTGCGGGATAGATACTTCATGGAGACA
AGGGGCGAGGAGTATTCTCACGGCCGCTACCAGGCCATCGCCGACATCTACACCGC
AAACCTGACAGAGTGCTACAGGGATCTGCTGACACAGTTTCAGGTGAGAGCAATCCT
GGCCGTGCCCATCCTGCAGGGCAAGAAGCTGTGGGGCCTGTTGGTGGCACACCAG
CTG
Sequence #76:
   > **PCR product #1 of RNA P&P *E.coli* miRFP/GFP-2**
   T7 promoter (in *italics*); part of *miRFP670nano* CDS underlined; 32-nt PCR#1/2 overlapping region in **bold** 
TCATAAGCTTTAATACGACTCACTATAGGCTAGTTGTGTTGTGTATGAAGAAGGCC
TTGAATAACGGGGTCTTCTTTGTGGTTAAGGAGGAAAATATGGCAAACCTGGACAAG
ATGCTGAATACCACAGTAACAGAGGTGCGGCAGTTCCTGCAGGTGGACAGAGTGTG
CGTGTTCCAGTTTGAGGAGGATTATAGCGGAGTGGTGGTGGAGGCCGTGGACG
ATAGGTGGATCTCCATCCTGAAGACCCAGGTGCGGGATAGATACTTCATGGAGACAA
GGGGCGAGGAGTATTCTCACGGCCGCTACCAGGCCATCGCCGACATCTACACCGCA
AACCTGACAGAGTGCTACAGGGATCTGCTGACACAGTTTCAGGTGAGAGCAATCCTG
GCCGTGCCCATCCTGCAGGGCAAGAAGCTGTGGGGCCTGTTGGTGGCACACCAGC
TG
Sequence #77:
   > **PCR product #1 of RNA P&P *E.coli* miRFP/GFP-3**
   T7 promoter (in *italics*); part of *miRFP670nano* CDS underlined; 32-nt PCR#1/2 overlapping region in **bold** 
TCATAAGCTTTAATACGACTCACTATAGGCTAGTTGTGTTGTGTATGAAGAAGGCC
TTGAATAACGGGGTCTTCTTCGTGGTTAAGGAGGAAAATATGGCAAACCTGGACAAG
ATGCTGAATACCACAGTAACAGAGGTGCGGCAGTTCCTGCAGGTGGACAGAGTGTG
CGTGTTCCAGTTTGAGGAGGATTATAGCGGAGTGGTGGTGGAGGCCGTGGACG
ATAGGTGGATCTCCATCCTGAAGACCCAGGTGCGGGATAGATACTTCATGGAGACAA
GGGGCGAGGAGTATTCTCACGGCCGCTACCAGGCCATCGCCGACATCTACACCGCA
AACCTGACAGAGTGCTACAGGGATCTGCTGACACAGTTTCAGGTGAGAGCAATCCTG
GCCGTGCCCATCCTGCAGGGCAAGAAGCTGTGGGGCCTGTTGGTGGCACACCAGC
TG
Sequence #78:
   > **PCR product #1 of RNA P&P *E.coli* miRFP/GFP-4**
   T7 promoter (in *italics*); part of *miRFP670nano* CDS underlined; 32-nt PCR#1/2 overlapping region in **bold** 
TCATAAGCTTTAATACGACTCACTATAGGCTAGTTGTGTTGTGTATGAAGAGGGCC
TTGAATAACGGGGTCTTCTTCGTGGTTAAGGAGGAAAATATGGCAAACCTGGACAAG
ATGCTGAATACCACAGTAACAGAGGTGCGGCAGTTCCTGCAGGTGGACAGAGTGTG
CGTGTTCCAGTTTGAGGAGGATTATAGCGGAGTGGTGGTGGTGGAGGCCGTGGACG
ATAGGTGGATCTCCATCCTGAAGACCCAGGTGCGGGATAGATACTTCATGGAGACAA
GGGGCGAGGAGTATTCTCACGGCCGCTACCAGGCCATCGCCGACATCTACACCGCA
AACCTGACAGAGTGCTACAGGGATCTGCTGACACAGTTTCAGGTGAGAGCAATCCTG
GCCGTGCCCATCCTGCAGGGCAAGAAGCTGTGGGGCCTGTTGGTGGCACACCAGC
TG
Sequence #79:
   > **5' primer #2 for RNA P&P *E.coli* miRFP/GFP-1** (PCR#2) 
AGCTGTGGGGCCTGTTGGTGGCACACCAGCTGGCGGCCCCTAGACAGTGGCAGAC
CTGGGAGATCGACTTTCTGAAGCAGCAGGCCGTGGTGGTGGGCATCGCCATCCAGC
AGAGCTAATTTCCTCCTTGTTGCGCTGTATGTACGGGCAATAAGGAGGAAATAATGT
CAACCACCAACCTGGCGGCAGCGCAAAAGATG
Sequence #80:
   > **5' primer #2 for RNA P&P *E.coli* miRFP/GFP-2/-3/-4** (PCR#2) 
AGCTGTGGGGCCTGTTGGTGGCACACCAGCTGGCGGCCCCTAGACAGTGGCAGAC
CTGGGAGATCGACTTTCTGAAGCAGCAGGCCGTGGTGGTGGGCATCGCCATCCAGC
AGAGCTAATTTCCTCCTTAGTCACGAAGAAGACTTCTGACTAAGGAGGGAATAATGTC
GAAGACGAACCTGGCGGCAGCGCAAAAGATG
Sequence #81:
   > **PCR product#2 for RNA P&P *E.coli* miRFP/GFP-1**
   End of *miRFP670nano* CDS underlined: 32-nt overlapping region in **bold;** *gfpmut3b-ASV* CDS in grey 
AGCTGTGGGGCCTGTTGGTGGCACACCAGCTGGCGGCCCCTAGACAGTGGCAGAC
CTGGGAGATCGACTTTCTGAAGCAGCAGGCCGTGGTGGGCATCGCCATCCAGC
AGAGCTAATTTCCTCCTTGTTGCGCTGTATGTACGGGCAATAAGGAGGAAATAATGT
CAACCACCAACCTGGCGGCAGCGCAAAAGATGCGTAAAGGAGAAGAACTTTTCACT
GGAGTTGTCCCAATTCTTGTTGAATTAGATGGTGATGTTAATGGGCACAAATTTTCTG
TCAGTGGAGAGGGTGAAGGTGATGCAACATACGGAAAACTTACCCTTAAATTTATTTG
CACTACTGGAAAACTACCTGTTCCGTGGCCAACACTTGTCACTACTTTCGGTTATGGT
GTTCAATGCTTTGCGAGATACCCAGATCACATGAAACAGCATGACTTTTTCAAGAGTG
CCATGCCCGAAGGTTACGTACAGGAAAGAACTATATTTTTCAAAGATGACGGGAACT
ACAAGACACGTGCTGAAGTCAAGTTTGAAGGTGATACCCTTGTTAATAGAATCGAGTT
AAAAGGTATTGATTTTAAAGAAGATGGAAACATTCTTGGACACAAATTGGAATACAAC
TATAACTCACACAATGTATACATCATGGCAGACAAACAAAAGAATGGAATCAAAGTTA
ACTTCAAAATTAGACACAACATTGAAGATGGAAGCGTTCAACTAGCAGACCATTATCA
ACAAAATACTCCGATTGGCGATGGCCCTGTCCTTTTACCAGACAACCATTACCTGTCC
ACACAATCTGCCCTTTCGAAAGATCCCAACGAAAAGAGAGACCACATGGTCCTTCTT
GAGTTTGTAACCGCTGCTGGGATTACACATGGCATGGATGAACTATACAAAGGCCT
GCAGCAAACGACGAAAACTACGCTGCATCAGTTTAATAATAAGATAAACCAGAGCGG
CACGGCAAGCAGAGTATACGAGATTCGGTAGCCACCGCTGAGCAATAACTAGCATAA
CCCCTTGGGGCCTCTAAACGGGTCTTGAGGGGTTTTTTGCATATGCAACGG
Sequence #82:
   > **PCR product#2 for RNA P&P *E.coli* miRFP/GFP-2/-3/-4**
   End of *miRFP670nano* CDS underlined; 32-nt overlapping region in **bold;** *gfpmut3b-ASV* CDS in grey 
AGCTGTGGGGCCTGTTGGTGGCACACCAGCTGGCGGCCCCTAGACAGTGGCAGAC
CTGGGAGATCGACTTTCTGAAGCAGCAGGCCGTGGTGGGCATCGCCATCCAGC
AGAGCTAATTTCCTCCTTAGTCACGAAGAAGACTTCTGACTAAGGAGGGAATAATGTC
GAAGACGAACCTGGCGGCAGCGCAAAAGATGCGTAAAGGAGAAGAACTTTTCACTG
GAGTTGTCCCAATTCTTGTTGAATTAGATGGTGATGTTAATGGGCACAAATTTTCTGT
CAGTGGAGAGGGTGAAGGTGATGCAACATACGGAAAACTTACCCTTAAATTTATTTG
CACTACTGGAAAACTACCTGTTCCGTGGCCAACACTTGTCACTACTTTCGGTTATGGT
GTTCAATGCTTTGCGAGATACCCAGATCACATGAAACAGCATGACTTTTTCAAGAGTG
CCATGCCCGAAGGTTACGTACAGGAAAGAACTATATTTTTCAAAGATGACGGGAACT
ACAAGACACGTGCTGAAGTCAAGTTTGAAGGTGATACCCTTGTTAATAGAATCGAGTT
AAAAGGTATTGATTTTAAAGAAGATGGAAACATTCTTGGACACAAATTGGAATACAAC
TATAACTCACACAATGTATACATCATGGCAGACAAACAAAAGAATGGAATCAAAGTTA
ACTTCAAAATTAGACACAACATTGAAGATGGAAGCGTTCAACTAGCAGACCATTATCA
ACAAAATACTCCGATTGGCGATGGCCCTGTCCTTTTACCAGACAACCATTACCTGTCC
ACACAATCTGCCCTTTCGAAAGATCCCAACGAAAAGAGAGACCACATGGTCCTTCTT
GAGTTTGTAACCGCTGCTGGGATTACACATGGCATGGATGAACTATACAAAGGCCT
GCAGCAAACGACGAAAACTACGCTGCATCAGTTTAATAATAAGATAAACCAGAGCGG
CACGGCAAGCAGAGTATACGAGATTCGGTAGCCACCGCTGAGCAATAACTAGCATAA
CCCCTTGGGGCCTCTAAACGGGTCTTGAGGGGTTTTTTGCATATGCAACGG
Sequence #83:
   > **PCR final product of RNA P&P *E.coli* miRFP/GFP-1**
   T7 promoter (in *italics*), *gfpmut3b-ASV* CDS in grey 
TCATAAGCTTTAATACGACTCACTATAGGCAAGTTTGATGTGGCTGTGCTGCGTGTAT
GGGGATAACTTGTATGTGTGGCGCAGCAAGGAGGAAAATATGGCAAACCTGGACAA
GATGCTGAATACCACAGTAACAGAGGTGCGGCAGTTCCTGCAGGTGGACAGAGTGT
GCGTGTTCCAGTTTGAGGAGGATTATAGCGGAGTGGTGGTGGAGGCCGTGGAC
GATAGGTGGATCTCCATCCTGAAGACCCAGGTGCGGGATAGATACTTCATGGAGACA
AGGGGCGAGGAGTATTCTCACGGCCGCTACCAGGCCATCGCCGACATCTACACCGC
AAACCTGACAGAGTGCTACAGGGATCTGCTGACACAGTTTCAGGTGAGAGCAATCCT
GGCCGTGCCCATCCTGCAGGGCAAGAAGCTGTGGGGCCTGTTGGTGGCACACCAG
CTGGCGGCCCCTAGACAGTGGCAGACCTGGGAGATCGACTTTCTGAAGCAGCAGGC
CGTGGTGGTGGGCATCGCCATCCAGCAGAGCTAATTTCCTCCTTGTTGCGCTGTATG
TACGGGCAATAAGGAGGAAATAATGTCAACCACCAACCTGGCGGCAGCGCAAAAGA
TGCGTAAAGGAGAAGAACTTTTCACTGGAGTTGTCCCAATTCTTGTTGAATTAGATGG
TGATGTTAATGGGCACAAATTTTCTGTCAGTGGAGAGGGTGAAGGTGATGCAACATA
CGGAAAACTTACCCTTAAATTTATTTGCACTACTGGAAAACTACCTGTTCCGTGGCCA
ACACTTGTCACTACTTTCGGTTATGGTGTTCAATGCTTTGCGAGATACCCAGATCACA
TGAAACAGCATGACTTTTTCAAGAGTGCCATGCCCGAAGGTTACGTACAGGAAAGAA
CTATATTTTTCAAAGATGACGGGAACTACAAGACACGTGCTGAAGTCAAGTTTGAAGG
TGATACCCTTGTTAATAGAATCGAGTTAAAAGGTATTGATTTTAAAGAAGATGGAAAC
ATTCTTGGACACAAATTGGAATACAACTATAACTCACACAATGTATACATCATGGCAG
ACAAACAAAAGAATGGAATCAAAGTTAACTTCAAAATTAGACACAACATTGAAGATGG
AAGCGTTCAACTAGCAGACCATTATCAACAAAATACTCCGATTGGCGATGGCCCTGT
CCTTTTACCAGACAACCATTACCTGTCCACACAATCTGCCCTTTCGAAAGATCCCAAC
GAAAAGAGAGACCACATGGTCCTTCTTGAGTTTGTAACCGCTGCTGGGATTACACAT
GGCATGGATGAACTATACAAAGGCCTGCAGCAAACGACGAAAACTACGCTGCATCA
GTTTAATAATAAGATAAACCAGAGCGGCACGGCAAGCAGAGTATACGAGATTCGGTA
 
GCCACCGCTGAGCAATAACTAGCATAACCCCTTGGGGCCTCTAAACGGGTCTTGAG
GGGTTTTTTGCATATGCAACGG
Sequence #84:
   > **PCR final product of RNA P&P *E.coli* miRFP/GFP-2**
   T7 promoter (in *italics), gfpmut3b-ASV* CDS in grey 
TCATAAGCTTTAATACGACTCACTATAGGCTAGTTGTGTTGTGTGTATGAAGAAGGCC
TTGAATAACGGGGTCTTCTTTGTGGTTAAGGAGGAAAATATGGCAAACCTGGACAAG
ATGCTGAATACCACAGTAACAGAGGTGCGGCAGTTCCTGCAGGTGGACAGAGTGTG
CGTGTTCCAGTTTGAGGAGGATTATAGCGGAGTGGTGGTGGAGGCCGTGGACG
ATAGGTGGATCTCCATCCTGAAGACCCAGGTGCGGGATAGATACTTCATGGAGACAA
GGGGCGAGGAGTATTCTCACGGCCGCTACCAGGCCATCGCCGACATCTACACCGCA
AACCTGACAGAGTGCTACAGGGATCTGCTGACACAGTTTCAGGTGAGAGCAATCCTG
GCCGTGCCCATCCTGCAGGGCAAGAAGCTGTGGGGCCTGTTGGTGGCACACCAGC
TGGCGGCCCCTAGACAGTGGCAGACCTGGGAGATCGACTTTCTGAAGCAGCAGGCC
GTGGTGGTGGGCATCGCCATCCAGCAGAGCTAATTTCCTCCTTAGTCACGAAGAAGA
CTTCTGACTAAGGAGGGAATAATGTCGAAGACGAACCTGGCGGCAGCGCAAAAGAT
GCGTAAAGGAGAAGAACTTTTCACTGGAGTTGTCCCAATTCTTGTTGAATTAGATGGT
GATGTTAATGGGCACAAATTTTCTGTCAGTGGAGAGGGTGAAGGTGATGCAACATAC
GGAAAACTTACCCTTAAATTTATTTGCACTACTGGAAAACTACCTGTTCCGTGGCCAA
CACTTGTCACTACTTTCGGTTATGGTGTTCAATGCTTTGCGAGATACCCAGATCACAT
GAAACAGCATGACTTTTTCAAGAGTGCCATGCCCGAAGGTTACGTACAGGAAAGAAC
TATATTTTTCAAAGATGACGGGAACTACAAGACACGTGCTGAAGTCAAGTTTGAAGGT
GATACCCTTGTTAATAGAATCGAGTTAAAAGGTATTGATTTTAAAGAAGATGGAAACA
TTCTTGGACACAAATTGGAATACAACTATAACTCACACAATGTATACATCATGGCAGA
CAAACAAAAGAATGGAATCAAAGTTAACTTCAAAATTAGACACAACATTGAAGATGGA
AGCGTTCAACTAGCAGACCATTATCAACAAAATACTCCGATTGGCGATGGCCCTGTC
CTTTTACCAGACAACCATTACCTGTCCACACAATCTGCCCTTTCGAAAGATCCCAACG
AAAAGAGAGACCACATGGTCCTTCTTGAGTTTGTAACCGCTGCTGGGATTACACATG
GCATGGATGAACTATACAAAGGCCTGCAGCAAACGACGAAAACTACGCTGCATCAG
TTTAATAATAAGATAAACCAGAGCGGCACGGCAAGCAGAGTATACGAGATTCGGTAG
CCACCGCTGAGCAATAACTAGCATAACCCCTTGGGGCCTCTAAACGGGTCTTGAGG
GGTTTTTTGCATATGCAACGG
Sequence #85:
   > **PCR final product of RNA P&P *E.coli* miRFP/GFP-3**
   T7 promoter (in *italics), gfpmut3b-ASV* CDS in grey 
TCATAAGCTTTAATACGACTCACTATAGGCTAGTTGTGTTGTGTATGAAGAAGGCC
TTGAATAACGGGGTCTTCTTCGTGGTTAAGGAGGAAAATATGGCAAACCTGGACAAG
ATGCTGAATACCACAGTAACAGAGGTGCGGCAGTTCCTGCAGGTGGACAGAGTGTG
CGTGTTCCAGTTTGAGGAGGATTATAGCGGAGTGGTGGTGGAGGCCGTGGACG
ATAGGTGGATCTCCATCCTGAAGACCCAGGTGCGGGATAGATACTTCATGGAGACAA
GGGGCGAGGAGTATTCTCACGGCCGCTACCAGGCCATCGCCGACATCTACACCGCA
AACCTGACAGAGTGCTACAGGGATCTGCTGACACAGTTTCAGGTGAGAGCAATCCTG
GCCGTGCCCATCCTGCAGGGCAAGAAGCTGTGGGGCCTGTTGGTGGCACACCAGC
 
TGGCGGCCCCTAGACAGTGGCAGACCTGGGAGATCGACTTTCTGAAGCAGGCC
GTGGTGGTGGGCATCGCCATCCAGCAGAGCTAATTTCCTCCTTAGTCACGAAGAAGA
CTTCTGACTAAGGAGGGAATAATGTCGAAGACGAACCTGGCGGCAGCGCAAAAGAT
GCGTAAAGGAGAAGAACTTTTCACTGGAGTTGTCCCAATTCTTGTTGAATTAGATGGT
GATGTTAATGGGCACAAATTTTCTGTCAGTGGAGAGGGTGAAGGTGATGCAACATAC
GGAAAACTTACCCTTAAATTTATTTGCACTACTGGAAAACTACCTGTTCCGTGGCCAA
CACTTGTCACTACTTTCGGTTATGGTGTTCAATGCTTTGCGAGATACCCAGATCACAT
GAAACAGCATGACTTTTTCAAGAGTGCCATGCCCGAAGGTTACGTACAGGAAAGAAC
TATATTTTTCAAAGATGACGGGAACTACAAGACACGTGCTGAAGTCAAGTTTGAAGGT
GATACCCTTGTTAATAGAATCGAGTTAAAAGGTATTGATTTTAAAGAAGATGGAAACA
TTCTTGGACACAAATTGGAATACAACTATAACTCACACAATGTATACATCATGGCAGA
CAAACAAAAGAATGGAATCAAAGTTAACTTCAAAATTAGACACAACATTGAAGATGGA
AGCGTTCAACTAGCAGACCATTATCAACAAAATACTCCGATTGGCGATGGCCCTGTC
CTTTTACCAGACAACCATTACCTGTCCACACAATCTGCCCTTTCGAAAGATCCCAACG
AAAAGAGAGACCACATGGTCCTTCTTGAGTTTGTAACCGCTGCTGGGATTACACATG
GCATGGATGAACTATACAAAGGCCTGCAGCAAACGACGAAAACTACGCTGCATCAG
TTTAATAATAAGATAAACCAGAGCGGCACGGCAAGCAGAGTATACGAGATTCGGTAG
CCACCGCTGAGCAATAACTAGCATAACCCCTTGGGGCCTCTAAACGGGTCTTGAGG
GGTTTTTTGCATATGCAACGG
Sequence #86:
   > **PCR final product of RNA P&P *E.coli* miRFP/GFP-4**
   T7 promoter (in *italics*), *gfpmut3b-ASV* CDS in grey

TCATAAGCTTTAATACGACTCACTATAGGCTAGTTGTGTTGTGTGTATGAAGAGGGCC
TTGAATAACGGGGTCTTCTTCGTGGTTAAGGAGGAAAATATGGCAAACCTGGACAAG
ATGCTGAATACCACAGTAACAGAGGTGCGGCAGTTCCTGCAGGTGGACAGAGTGTG
CGTGTTCCAGTTTGAGGAGGATTATAGCGGAGTGGTGGTGGAGGCCGTGGACG
ATAGGTGGATCTCCATCCTGAAGACCCAGGTGCGGGATAGATACTTCATGGAGACAA
GGGGCGAGGAGTATTCTCACGGCCGCTACCAGGCCATCGCCGACATCTACACCGCA
AACCTGACAGAGTGCTACAGGGATCTGCTGACACAGTTTCAGGTGAGAGCAATCCTG
GCCGTGCCCATCCTGCAGGGCAAGAAGCTGTGGGGCCTGTTGGTGGCACACCAGC
TGGCGGCCCCTAGACAGTGGCAGACCTGGGAGATCGACTTTCTGAAGCAGCAGGCC
GTGGTGGTGGGCATCGCCATCCAGCAGAGCTAATTTCCTCCTTAGTCACGAAGAAGA
CTTCTGACTAAGGAGGGAATAATGTCGAAGACGAACCTGGCGGCAGCGCAAAAGAT
GCGTAAAGGAGAAGAACTTTTCACTGGAGTTGTCCCAATTCTTGTTGAATTAGATGGT
GATGTTAATGGGCACAAATTTTCTGTCAGTGGAGAGGGTGAAGGTGATGCAACATAC
GGAAAACTTACCCTTAAATTTATTTGCACTACTGGAAAACTACCTGTTCCGTGGCCAA
CACTTGTCACTACTTTCGGTTATGGTGTTCAATGCTTTGCGAGATACCCAGATCACAT
GAAACAGCATGACTTTTTCAAGAGTGCCATGCCCGAAGGTTACGTACAGGAAAGAAC
TATATTTTTCAAAGATGACGGGAACTACAAGACACGTGCTGAAGTCAAGTTTGAAGGT
GATACCCTTGTTAATAGAATCGAGTTAAAAGGTATTGATTTTAAAGAAGATGGAAACA
TTCTTGGACACAAATTGGAATACAACTATAACTCACACAATGTATACATCATGGCAGA
CAAACAAAAGAATGGAATCAAAGTTAACTTCAAAATTAGACACAACATTGAAGATGGA
AGCGTTCAACTAGCAGACCATTATCAACAAAATACTCCGATTGGCGATGGCCCTGTC
CTTTTACCAGACAACCATTACCTGTCCACACAATCTGCCCTTTCGAAAGATCCCAACG
AAAAGAGAGACCACATGGTCCTTCTTGAGTTTGTAACCGCTGCTGGGATTACACATG
GCATGGATGAACTATACAAAGGCCTGCAGCAAACGACGAAAACTACGCTGCATCAG
TTTAATAATAAGATAAACCAGAGCGGCACGGCAAGCAGAGTATACGAGATTCGGTAG
CCACCGCTGAGCAATAACTAGCATAACCCCTTGGGGCCTCTAAACGGGTCTTGAGG
GGTTTTTTGCATATGCAACGG

## Claims

1. An RNA molecule comprising a sensor part and a trigger part, wherein the trigger part is an mRNA coding for a protein and the sensor part comprises 2 stem loops divided in the following regions in order from 5' to 3': T (T1,T2), A, B, C (C1,C2), D (D1,D2), E, F (F1,F2) and G, wherein
- region T1 is unpaired;
- T2 forms a stem with B where A is the loop region;
- C can be paired or unpaired;
- D forms a stem with F, where E is the loop region;
- F2 must contain an RBS;
- stem loop D1 basepairs with F2 and D2 with F1;
- G contains an ATG and a linker and is immediately followed by the trigger part;
- further with the following structural limitations:
a) T has a length of >= 30 nucleotides;
b) T has the same number of nucleotides as B;
c) D1 has the same number of nucleotides as F2 and D2 has the same number of nucleotides as F1:
d) RBS in F1 is at least 3 nucleotides away from its start;
e) (B + C1) has the same number of nucleotides as (D + E);
f) D2 + E has the same number of nucleotides as T;
g) C1 has the same number of nucleotides as D1;
h) RBS in F2 must be at least 3 nucleotides away from F2 3' end;
further wherein in the absence of a target RNA, the RBS in region F2 is sequestered in the DEF stem loop and thus, translation of the trigger is not possible;
further wherein in the presence of a target RNA, the sensor part hybridizes the target RNA and a conformational change is produced, in which region T is sequestered by target RNA, B and C1 form a stem loop with D and E, where C2 is the loop region, F and G are partially or totally unpaired and thus the RBS in F2 is not sequestered and translation of the trigger is possible; and wherein RBS do not form stems with regions that also hybridize to the target RNA or form a stem with regions that hybridize with the regions which hybridize the target RNA.

2. An RNA molecule, according to claim 1, wherein in the sensor part:
- C contains an RBS in C1 and a sensor reporter gene different from the trigger part,
- RBS in C1 must be at least 3 nucleotides away from C1 each end;
- ATG of the reporter gene can be either on C1 or C2 as long as the distance from the RBS is from 3 to 10, preferably 5-6 nucleotides;
- D+E must be proportional to the length of C2; and
wherein in the absence of RNA target, RBS in C1 is partially or totally unpaired, and wherein in the presence of RNA target, C1-D1 are part of the stem BC1-DE.

3. The RNA molecule according to claim 1, wherein the trigger part is an mRNA coding for a bioluminescence protein, a fluorescent protein or a colorimetric reporter protein.

4. The RNA molecule according to claim 2, wherein both the sensor and the trigger reporter genes are mRNAs coding for a bioluminescence protein, a fluorescent protein or a colorimetric reporter protein, as long as both reporter genes are different.

5. The RNA molecule, according to claim 3 or 4, wherein said bioluminescence protein is selected from luciferase and aequorin, said fluorescent protein is selected from GFP, YFP and RFP and said colorimetric reporter protein is LacZ.

6. The RNA molecule of claim 1,3,5 where there is no sensor reporter gene and the trigger is a LacZ mRNA and the sensor part corresponds to the following artificial RNA sequences selected from SEQ ID NO:, 4, 5 and 6 , where their respective artificial RNA targets are the following: SEQ ID NO: 1 for SEQ ID NO: 4 and 5; and SEQ ID NO: 2 for SEQ ID NO: 6.

7. The RNA molecule according to any of claims 1 to 6, wherein said artificial RNA target is a biomarker characterizing a disease selected from a viral infection, a bacterial infection, cancer or a genetic disease.

8. The RNA molecule according to claim 7, being dependent of claim 1, 3, 5 and 6, wherein said biomarker is a region of the e.coli rRNA16s mRNA reverse complemented, the trigger is a LacZ mRNA and wherein the sensor part corresponds to the following artificial RNA sequences: SEQ ID NO: 16 and 17 or alternatively, where the trigger is a GFP mRNA and wherein the sensor part corresponds to the following artificial RNA sequences: SEQ ID NO: 24, 25 and 26, or alternatively, being dependent of claim 2, 4, 5, wherein the sensor reporter gene is RFP and the trigger is a GFP mRNA corresponding to sequences SEQ ID NOs: 67, 68, 69 and 70.

9. The RNA molecule according to claim 7, being dependent of claim 1, 3, 5 and 6, wherein said biomarker is a region of the SARS-CoV-2 viral genome (SEQ ID NO: 38), the trigger is a LacZ mRNA and wherein the sensor part corresponds to the following artificial RNA sequences: SEQ ID NO: 36 and SEQ ID NO: 37 or alternatively, wherein said biomarker are regions of the SARS-CoV-2 viral genome reverse complemented (SEQ ID NO: 46, SEQ ID NO: 47), the trigger is a GFP mRNA and wherein the sensor part corresponds to the following artificial RNA sequences: SEQ ID NO: 44 and 45, or alternatively, being dependent of claim 2, 4, 5, wherein said biomarker are regions of the SARS-CoV-2 viral genome reverse complemented , the sensor reporter gene is RFP and the trigger is a GFP mRNA corresponding to sequences SEQ ID NOs:53 and 54.

10. Use of the RNA molecule according to claims 1 to 9 in a colorimetric or fluorometric assay upon RNA target binding of said RNA molecule, wherein said RNA molecule is contained with a cell extract in the recipient to be used in an in-vitro diagnostic of a certain disease.

11. A process for detecting in vitro a disease comprising the steps of:
a) obtaining an isolated sample of blood or saliva or swabs of body fluids from an individual;
c) extracting and purifying the RNA;
d) amplifying the RNA obtained in step c) through isothermal amplification;
e) detecting the RNA from cells and/or microorganisms causing the disease through an RNA molecule according to any claims 1, 3, 5, 6, 7, 8, 9; and
f) identifying a positive result of the disease by confirming the expression of the trigger mRNA.

12. A process for detecting in vitro a disease comprising the steps of:
a) obtaining an isolated sample of blood or saliva or swabs of body fluids from an individual;
c) extracting and purifying the RNA;
d) amplifying the RNA obtained in step c) through isothermal amplification;
e) detecting the RNA from cells and/or microorganisms causing the disease through an RNA molecule according to any claims 2, 4, 5, 7, 8, 9; and
f) identifying a positive result of the disease by confirming the expression of the trigger mRNA.
g) identifying a negative result of the disease by confirming the expression of the sensor mRNA.
h) Null if no expression detected.

13. The process, according to claims 11 or 12, wherein
1) the isothermal amplification is selected from NASBA, TMA and SMART technologies, and/or
2) the RNA molecule according to claims 1 to 9 is lyophilized, along with a cell extract, in a microfluidics device, and/or
3) the identification of a positive result in step f) is confirmed through a colorimetric or fluorometric assay wherein the trigger is a LacZ mRNA or GFP.
4) the identification of a negative result in step f) is confirmed through a fluorimetric assay wherein the sensor reporter is RFP.

14. The process, according to claim any of claims 11 to 13, wherein the process further comprises between steps a) and c):
b) treating said isolated sample or swab with at least a lysate buffer and/or a heating step.

15. A kit for detecting in vitro a disease comprising:
a) at least one receiving means for receiving said sample or swab isolated from the individual;
b) a lysate buffer for treating the sample or swab;
c) heating capacity.
d) at least the RNA molecule according to any of claims 1 to 9 for the detection of a disease in the individual;
e) instructions for using the kit.

16. The process, according to any of claims 11 to 14, the kit, according to claim 15, or the use, according to claim 10, wherein said disease is a bloodstream infection caused by pathogenic bacteria or COVID-19.
